(19) Europäisches Patentamt
European Patent Office
Office européen des brevets

(11) EP 1 943 356 B1

(12) **EUROPEAN PATENT SPECIFICATION**

(45) Date of publication and mention of the grant of the patent:
**14.05.2014 Bulletin 2014/20**

(21) Application number: **06806038.3**

(22) Date of filing: **04.10.2006**

(51) Int Cl.:
***C12Q 1/68*** *(2006.01)*

(86) International application number:
**PCT/EP2006/009605**

(87) International publication number:
**WO 2007/039290 (12.04.2007 Gazette 2007/15)**

# (54) METHODS AND NUCLEIC ACIDS FOR THE ANALYSIS OF GENE EXPRESSION ASSOCIATED WITH THE PROGNOSIS OF CELL PROLIFERATIVE DISORDERS

VERFAHREN UND NUKLEINSÄUREN ZUR ANALYSE DER GENEXPRESSION IN ZUSAMMENHANG MIT DER PROGNOSE VON ZELLPROLIFERATIONSSTÖRUNGEN

METHODES ET ACIDES NUCLEIQUES POUR L'ANALYSE D'EXPRESSION GENIQUE ASSOCIEE AU PRONOSTIC DE TROUBLES DE PROLIFERATION CELLULAIRE

(84) Designated Contracting States:
**AT BE BG CH CY CZ DE DK EE ES FI FR GB GR HU IE IS IT LI LT LU LV MC NL PL PT RO SE SI SK TR**

(30) Priority: **03.10.2005 US 723125 P**
**30.11.2005 US 740923 P**
**15.06.2006 EP 06090106**

(43) Date of publication of application:
**16.07.2008 Bulletin 2008/29**

(73) Proprietor: **Epigenomics AG**
**10178 Berlin (DE)**

(72) Inventor: **BERLIN, Kurt**
**14532 Stahnsdorf (DE)**

(74) Representative: **Zwicker, Jörk et al**
**ZSP Patentanwälte**
**Partnerschaftsgesellschaft**
**Radlkoferstrasse 2**
**81373 München (DE)**

(56) References cited:
**EP-A- 1 379 694**
**WO-A-01/19845**
**WO-A-02/070742**
**WO-A-2004/035803**
**WO-A-2006/071466**

- **DATABASE MEDLINE [Online] US NATIONAL LIBRARY OF MEDICINE (NLM), BETHESDA, MD, US 04 August 1993 GASPARINI G ET AL: 'Evaluating the potential usefulness of new prognostic and predictive indicators in node-negative breast cancer patients.' Database accession no. NLM8331681 & GASPARINI G ET AL: "Evaluating the potential usefulness of new prognostic and predictive indicators in node-negative breast cancer patients.", JOURNAL OF THE NATIONAL CANCER INSTITUTE 4 AUG 1993 LNKD- PUBMED:8331681, vol. 85, no. 15, 4 August 1993 (1993-08-04), pages 1206-1219, ISSN: 0027-8874**
- **DATABASE MEDLINE [Online] US NATIONAL LIBRARY OF MEDICINE (NLM), BETHESDA, MD, US 2004 CIANFROCCA MARY ET AL: 'Prognostic and predictive factors in early-stage breast cancer.' Database accession no. NLM15561805 & CIANFROCCA MARY ET AL: "Prognostic and predictive factors in early-stage breast cancer.", THE ONCOLOGIST 2004 LNKD- PUBMED: 15561805, vol. 9, no. 6, 2004, pages 606-616, ISSN: 1083-7159**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 943 356 B1

Printed by Jouve, 75001 PARIS (FR)

**Description**

FIELD OF THE INVENTION

The present invention relates to human DNA sequences that exhibit heterogeneous expression patterns in cancer patients. Particular embodiments of the invention provide methods for determining the prognosis of said patients.

PRIOR ART

The following invention relates to the use of the gene PITX2 as a prognostic marker in the treatment of cancer. The gene PITX2 (NM_000325) encodes the paired-like homeodomain transcription factor 2 which is known to be expressed during development of anterior structures such as the eye, teeth, and anterior pituitary. In the state of the art it is known that hypermethylation and accordingly underexpression of this gene are associated with the development of cancer. Toyota et al., (2001. Blood. 97:2823-9.) found hypermethylation of the PITX2 gene in a large proportion of acute myeloid leukemias. However, this document does not disclose that the marker is also relevant in determining the prognosis of cancer patients. EP 04 029 486.0 is the closest single document relevant for the assessment of novelty. Said document discloses that PITX2 an indicator of breast cancer prognosis in EP 04 029 486.0. Said document does not disclose that said gene is a prognostic marker applicable across multiple types of cancer accordingly the invention is new.
Furthermore, on the basis of this document the person skilled in the art would not expect that said gene would also be a prognostic indicator in other cancerous disease. Due to the heterogeneity of cancers there is currently no single molecular prognostic indicator applicable across all classes of cancers. Accordingly the use of the gene PITX2 as a prognostic indicator across a plurality of cancer types is a surprising effect.
WO 2004/035803 discloses that PITX2 could be used for predicting the responsiveness of a subject with a cell proliferative disorder of the breast to a therapy.
WO 01/19845 discloses that PITX2 is methylated in leukaemia, breast and prostate cancer.

SUMMARY OF THE INVENTION

The present invention provides novel and efficient methods and nucleic acids for providing a prognosis of cell proliferative disorders, most preferably cancer but not breast cancer. It is particularly preferred that said cancers are selected from the group consisting bladder cancer, colorectal cancer, endometrial cancer, kidney (renal cell) cancer, leukemia, lung (Including bronchus) cancer, melanoma, non-Hodgkin's lymphoma, pancreatic cancer, prostate cancer, skin cancer and thyroid cancer.
The invention solves this longstanding need in the art by providing the gene PITX2 (SEQ ID NO: 1) as a marker of cancer prognosis. In a particularly preferred embodiment of the invention, the methylation status of CpG positions of the gene PITX2 and/or regulatory regions thereof is indicative of the prognosis of cell proliferative disorders, most preferably cancer (but not breast cancer) or features thereof. It is particularly preferred that said cancers are selected from the group consisting bladder cancer, colorectal cancer, endometrial cancer, kidney (renal cell) cancer, leukemia, lung (Including bronchus) cancer, melanoma, non-Hodgkin's lymphoma, pancreatic cancer, prostate cancer, skin cancer and thyroid cancer.
To enable this analysis the invention provides a method for the analysis of biological samples for genomic methylation associated with the development of cell proliferative disorders, most preferably cancer but not breast cancer. It is particularly preferred that said cancers are selected from the group consisting bladder cancer, colorectal cancer, endometrial cancer, kidney (renal cell) cancer, leukemia, lung (Including bronchus) cancer, melanoma, non-Hodgkin's lymphoma, pancreatic cancer, prostate cancer, skin cancer and thyroid cancer. Said method is characterized in that at least one nucleic acid, or a fragment thereof of the gene PITX2 and/or regulatory regions thereof (SEQ ID NO: 1) is/are contacted with a reagent or series of reagents capable of distinguishing between methylated and non methylated CpG dinucleotides within the genomic sequence thereof.
It is particularly preferred that the method and nucleic acids according to the invention are utilized for at least one of: prognosis of; treatment of; monitoring of; and treatment and monitoring of cell proliferative disorders, most preferably cancer but not breast cancer. It is particularly preferred that said cancers are selected from the group consisting bladder cancer, colorectal cancer, endometrial cancer, kidney (renal cell) cancer, leukemia, lung (Including bronchus) cancer, melanoma, non-Hodgkin's lymphoma, pancreatic cancer, prostate cancer, skin cancer and thyroid cancer.
The present invention provides a method for ascertaining genetic and/or epigenetic parameters of genomic DNA. The method has utility for the improved prognostic classification of cell proliferative disorders, most preferably cancer but not breast cancer, more specifically by enabling the improved identification of and differentiation between aggressive and non-aggressive forms of said disorder. It is particularly preferred that said cancers are selected from the group consisting bladder cancer, colorectal cancer, endometrial cancer, kidney (renal cell) cancer, leukemia, lung (Including bronchus) cancer, melanoma, non-Hodgkin's lymphoma, pancreatic cancer, prostate cancer, skin cancer and thyroid cancer.

The invention presents several substantial improvements over the state of the art. Although some methylation assays for the detection of cancer are known, there is currently no molecular classification system for the *prognostic* classification of tumors.

The DNA source may be any suitable source. Preferably, the source of the DNA sample is selected from the group consisting of cells or cell lines, histological slides, biopsies, paraffin-embedded tissue, bodily fluids, ejaculate, urine, blood, sputum, stool, tissues for example but not limited to those from colon, prostate, lung or liver, and combinations thereof. Preferably, the source is biopsies, bodily fluids, ejaculate, urine, or blood.

Specifically, the present invention provides a method for providing a prognosis of cell proliferative disorders, most preferably cancer but not breast cancer, comprising: obtaining a biological sample comprising genomic nucleic acid(s); contacting the nucleic acid(s), or a fragment thereof, with one reagent or a plurality of reagents sufficient for distinguishing between methylated and non methylated CpG dinucleotide sequences within a target sequence of the subject nucleic acid, wherein the target sequence comprises, or hybridizes under stringent conditions to, a sequence comprising at least 16 contiguous nucleotides of SEQ ID NO: 1 said contiguous nucleotides comprising at least one CpG dinucleotide sequence; and determining, based at least in part on said distinguishing, the methylation state of at least one target CpG dinucleotide sequence, or an average, or a value reflecting an average methylation state of a plurality of target CpG dinucleotide sequences. Preferably, distinguishing between methylated and non methylated CpG dinucleotide sequences within the target sequence comprises methylation state-dependent conversion or non-conversion of at least one such CpG dinucleotide sequence to the corresponding converted or non-converted dinucleotide sequence within a sequence selected from the group consisting of SEQ ID NO: 2 to SEQ ID NO: 5, and contiguous regions thereof corresponding to the target sequence. It is particularly preferred that said cancers are selected from the group consisting bladder cancer, colorectal cancer, endometrial cancer, kidney (renal cell) cancer, leukemia, lung (Including bronchus) cancer, melanoma, non-Hodgkin's lymphoma, pancreatic cancer, prostate cancer, skin cancer and thyroid cancer.

Additional embodiments provide a method for providing a prognosis of cell proliferative disorders, most preferably cancer but not breast cancer, comprising: obtaining a biological sample having subject genomic DNA; extracting the genomic DNA; treating the genomic DNA, or a fragment thereof, with one or more reagents to convert 5-position unmethylated cytosine bases to uracil or to another base that is detectably dissimilar to cytosine in terms of hybridization properties; contacting the treated genomic DNA, or the treated fragment thereof, with an amplification enzyme and at least two primers comprising, in each case a contiguous sequence at least 9 nucleotides in length that is complementary to, or hybridizes under moderately stringent or stringent conditions to a sequence selected from the group consisting SEQ ID NO: 2 to SEQ ID NO: 5 and complements thereof, wherein the treated DNA or the fragment thereof is either amplified to produce an amplificate, or is not amplified; and determining, based on a presence or absence of, or on a property of said amplificate, the methylation state of at least one CpG dinucleotide sequence selected from the group consisting of SEQ ID NO:1, or an average, or a value reflecting an average methylation state of a plurality of CpG dinucleotide sequences thereof.

Preferably, at least one such hybridizing nucleic acid molecule or peptide nucleic acid molecule is bound to a solid phase. Preferably, determining comprises use of at least one method selected from the group consisting of: hybridizing at least one nucleic acid molecule comprising a contiguous sequence at least 9 nucleotides in length that is complementary to, or hybridizes under moderately stringent or stringent conditions to a sequence selected from the group consisting of SEQ ID NO:2 to SEQ ID NO:5, and complements thereof; hybridizing at least one nucleic acid molecule, bound to a solid phase, comprising a contiguous sequence at least 9 nucleotides in length that is complementary to, or hybridizes under moderately stringent or stringent conditions to a sequence selected from the group consisting of SEQ ID NO: 2 to SEQ ID NO: 5, , and complements thereof; hybridizing at least one nucleic acid molecule comprising a contiguous sequence at least 9 nucleotides in length that is complementary to, or hybridizes under moderately stringent or stringent conditions to a sequence selected from the group consisting of SEQ ID NO: 2 to SEQ ID NO: 5 , and complements thereof, and extending at least one such hybridized nucleic acid molecule by at least one nucleotide base; and sequencing of the amplificate.

Further embodiments provide a method for providing a prognosis of cell proliferative disorders, most preferably cancer but not breast cancer, comprising: obtaining a biological sample having subject genomic DNA; extracting the genomic DNA; contacting the genomic DNA, or a fragment thereof, comprising one or more sequences selected from the group consisting of SEQ ID NO:1 or a sequence that hybridizes under stringent conditions thereto, with one or more methylation-sensitive restriction enzymes, wherein the genomic DNA is either digested thereby to produce digestion fragments, or is not digested thereby; and determining, based on a presence or absence of, or on property of at least one such fragment, the methylation state of at least one CpG dinucleotide sequence of one or more sequences selected from the group consisting of SEQ ID NO:1, or an average, or a value reflecting an average methylation state of a plurality of CpG dinucleotide sequences thereof. Preferably, the digested or undigested genomic DNA is amplified prior to said determining.

Additional embodiments provide novel genomic and chemically modified nucleic acid sequences, as well as oligonucleotides and/or PNA-oligomers for analysis of cytosine methylation patterns within sequences from the group consisting of SEQ ID NO: 1.

BRIEF DESCRIPTION OF THE DRAWINGS

Figure 1 shows the distribution of follow up times of patients as analyzed in Example 1. The white bars represent the distribution of all censored (no PSA relapse) patients. The grey bars show the distribution of the PSA-free survival time for all of the relapse patients. Frequency is shown on the Y-axis and time (months) is shown on the X-axis.

Figure 2 shows Kaplan-Meier survival analysis of the PITX2 marker (A & B) and ROC curve analysis (C) of the marker PITX2 in differentiating between prostate cancer patients according to Example 1. Proportion of recurrence-free patients is shown on the Y-axis, time in years is shown on the x-axis.

Figure 3 shows Kaplan-Meier survival analysis of PITX2 performance on sub-populations based on stage according to Example 1. Proportion of recurrence-free patients is shown on the Y-axis, time in years is shown on the x-axis. Figure A shows all T2 and T3 patients, wherein the dark grey plot shows clinical stage T3 patients, and light grey plot shows clinical stage T2 patients. Figure B shows all T3 patients, wherein the dark grey plot shows hypomethylated samples, and light grey plot shows hypomethylated samples. Figure C shows all T2 patients, wherein the dark grey plot shows hypomethylated samples, and light grey plot shows hypomethylated samples. Proportion of recurrence-free patients is shown on the Y-axis, time in years is shown on the x-axis.

Figure 4 shows Kaplan-Meier survival analysis of PITX2 performance on sub-populations based on Gleason score according to Example 1. Figure A shows the performance of Gleason score as a prognostic marker. Gleason 5 and 6 patients are in light grey, Gleason 7 patients are in dark-grey, and Gleason 8, 9, and 10 patients are in black. Figure C shows the performance of PITX2 on Gleason 5 and 6 patients. Figure B shows the performance of PITX2 on Gleason 7 patients. Figure D shows the performance of PITX2 on Gleason 8, 9, and 10 patients. In figures B to D light grey shows hypomethylated samples, black indicates hypermethylated samples. Proportion of recurrence-free patients is shown on the Y-axis, time in years is shown on the x-axis.

Figure 5 shows Kaplan-Meier survival analysis of PITX2 performance on sub-populations based on nomogram score according to Example 1. Figure A shows the performance of Nomogram score as a prognostic marker. High risk are in light grey, low risk patients are in black. Figure C shows the performance of PITX2 on high risk patients. Figure B shows the performance of PITX2 on low risk patients.

DETAILED DESCRIPTION OF THE INVENTION

<u>Definitions:</u>

As used herein the term expression shall be taken to mean the transcription and translation of a gene. The level of expression of a gene may be determined by the analysis of any factors associated with or indicative of the level of transcription and translation of a gene including but not limited to methylation analysis, loss of heterozygosity (hereinafter also referred to as LOH), RNA expression levels and protein expression levels.

Furthermore the activity of the transcribed gene may be affected by genetic variations such as but not limited genetic mutations (including but not limited to SNPs, point mutations, deletions, insertions, repeat length, rearrangements and other polymorphisms).

The scope of the present invention is directed to cell proliferative disorders, more preferably cancers but not breast cancers. Accordingly the term "cancer but not breast cancer" and all equivalents thereof should be taken to mean all disorders of the group consisting of: Acute Lymphoblastic Leukemia; Acute Myeloid Leukemia; Adrenocortical Carcinoma; AIDS-Related Cancers; AIDS-Related Lymphoma; Anal Cancer; Astrocytoma (Cerebellar and Cerebral); Basal Cell Carcinoma; Bile Duct Cancer; Bladder Cancer; Bone Cancer, Osteosarcoma/Malignant Fibrous Histiocytoma; Brain Stem Glioma; Brain Tumor, - Brain Stem Glioma, - Cerebellar Astrocytoma, - Cerebral Astrocytoma/Malignant Glioma; -Ependymoma, - Medulloblastoma, - Supratentorial Primitive Neuroectodermal Tumors, - Visual Pathway and Hypothalamic Glioma; Bronchial Adenomas/Carcinoids; Burkitt's Lymphoma; Carcinoid Tumor; Carcinoid Tumor, Gastrointestinal;Central Nervous System Lymphoma, Primary; Cerebellar Astrocytoma; Cerebral Astrocytoma/Malignant Glioma; Cervical Cancer; Chronic Lymphocytic Leukemia; Chronic Myelogenous Leukemia; Chronic Myeloproliferative Disorders;

Colon Cancer; Colorectal Cancer; Cutaneous T-Cell Lymphoma, Mycosis Fungoides and Sézary Syndrome; Endometrial Cancer; Ependymoma; Esophageal Cancer; Ewing's Family of Tumors; Extracranial Germ Cell Tumor; Extragonadal Germ Cell Tumor; Extrahepatic Bile Duct Cancer; Eye Cancer, Intraocular Melanoma; Eye Cancer, Retinoblastoma; Gallbladder Cancer; Gastric (Stomach) Cancer; Gastrointestinal Carcinoid Tumor; Germ Cell Tumor, Extracranial; Germ Cell Tumor, Extragonadal; Germ Cell Tumor, Ovarian; Gestational Trophoblastic Tumor; Glioma; GliomaBrain Stem; Glioma, Cerebral Astrocytoma; Glioma, Visual Pathway and Hypothalamic; Hairy Cell Leukemia; Head and Neck Cancer; Hepatocellular (Liver) Cancer (Primary); Hodgkin's Lymphoma; Hypopharyngeal Cancer; Hypothalamic and Visual Pathway Glioma; Intraocular Melanoma; Islet Cell Carcinoma (Endocrine Pancreas); Kaposi's Sarcoma; Kidney (Renal Cell) Cancer; Kidney Cancer; Laryngeal Cancer; Leukemia, Acute Lymphoblastic; Leukemia, Acute Myeloid; Leukemia, Chronic Lymphocytic; Lip and Oral Cavity Cancer; Liver Cancer(Primary); Lung Cancer, Non-Small Cell; Lung Cancer, Small Cell; Lymphoma, AIDS-Related; Lymphoma, Burkitt's; Lymphoma, Cutaneous T-Cell; Lymphoma, Hodgkin's; Lymphoma, Non-Hodgkin's; Lymphoma, Primary Central Nervous System; Macroglobulinemia, Waldenström's; Malignant Fibrous Histiocytoma of Bone/Osteosarcoma; Medulloblastoma; Melanoma; Melanoma, Intraocular (Eye); Merkel Cell Carcinoma; Mesothelioma Malignant; Mesothelioma; Metastatic Squamous Neck Cancer with Occult Primary; Multiple Endocrine Neoplasia Syndrome; Multiple Myeloma/Plasma Cell Neoplasm; Mycosis Fungoides; Myelodysplastic Syndromes; Myelodysplastic/Myeloproliferative Diseases; Myelogenous Leukemia, Chronic; Myeloid Leukemia Acute; Myeloma, Multiple; Myeloproliferative Disorders, Chronic; Nasal Cavity and Paranasal Sinus Cancer; Nasopharyngeal Cancer; Neuroblastoma; Non-Hodgkin's Lymphoma; Non-Small Cell Lung Cancer; Oral Cancer; Oral Cavity Cancer, Lip and; Oropharyngeal Cancer; Osteosarcoma/Malignant Fibrous Histiocytoma of Bone; Ovarian Cancer; Ovarian Epithelial Cancer;Ovarian Germ Cell Tumor; Ovarian Low Malignant Potential Tumor; Pancreatic Cancer; Pancreatic Cancer, Islet Cell; Paranasal Sinus and Nasal Cavity Cancer; Parathyroid Cancer; Penile Cancer; Pheochromocytoma; Pineoblastoma and Supratentorial Primitive Neuroectodermal Tumors; Pituitary Tumor; Plasma Cell Neoplasm/Multiple Myeloma; Pleuropulmonary Blastoma; Primary Central Nervous System Lymphoma; Prostate Cancer; Rectal Cancer; Renal Cell (Kidney) Cancer; Renal Pelvis and Ureter, Transitional Cell Cancer; Retinoblastoma; Rhabdomyosarcoma; Salivary Gland Cancer; Sarcoma, Ewing's Family of Tumors; Sarcoma, Kaposi's; Sarcoma, Soft Tissue; Sarcoma, Uterine; Sezary Syndrome; Skin Cancer (non-Melanoma); Skin Cancer; Skin Cancer (Melanoma); Skin Carcinoma, Merkel Cell; Small Cell Lung Cancer; Small Intestine Cancer; Soft Tissue Sarcoma; Squamous Cell Carcinoma; Squamous Neck Cancer with Occult Primary; Stomach (Gastric) Cancer; Supratentorial Primitive Neuroectodermal Tumors; T-Cell Lymphoma, CutaneousTesticular Cancer; Thymoma; Thymoma and Thymic Carcinoma; Thyroid Cancer; Transitional Cell Cancer of the Renal Pelvis and Ureter; Trophoblastic Tumor, Gestational; Ureter and Renal Pelvis, Transitional Cell Cancer; Urethral Cancer; Uterine Cancer, Endometrial; Uterine Sarcoma; Vaginal Cancer; Visual Pathway and Hypothalamic Glioma; Vulvar Cancer; Waldenström's Macroglobulinemia; and Wilms' Tumor.

As used herein the term "prognosis" shall be taken to mean a prediction of the progression of the disease (for example but not limited to regression, stasis and metastasis), in particular aggressiveness and metastatic potential of a tumor.

As used herein the term "prognostic marker" shall be taken to mean an indicator of a prediction of the progression of the disease, in particular aggressiveness and metastatic potential of a tumor.

As used herein the term "prognostic classification" or "prognosis" shall be taken to mean the classification of a cell proliferative disorder, preferably cancer but not breast cancer according to a prediction of the progression of the disease, in particular aggressiveness and metastatic potential of a tumor.

It is preferably used to define patients with high, low and intermediate risks of death or recurrence after treatment that result from the inherent heterogeneity of the disease process. As used herein the term "aggressive" as used with respect to a tumor shall be taken to mean a cell proliferative disorder that has the biological capability to rapidly spread outside of its primary location or organ. Indicators of tumor aggressiveness standard in the art include but are not limited to tumor stage, tumor grade, Gleason grade, nodal status and survival. As used herein the term "survival" shall not be limited to mean survival until mortality (wherein said mortality may be either irrespective of cause or cell proliferative disorder related) but may be used in combination with other terms to define clinical terms, for example but not limited to "recurrence-free survival" (wherein the term recurrence shall include both localized and distant recurrence);metastasis free survival; disease free survival (wherein the term disease shall include cancer and diseases associated therewith). The length of said survival may be calculated by reference to a defined start point (e.g. time of diagnosis or start of treatment) and a defined end point (e.g. death, recurrence or metastasis).

The term "Observed/Expected Ratio" ("O/E Ratio") refers to the frequency of CpG dinucleotides within a particular DNA sequence, and corresponds to the [number of CpG sites / (number of C bases x number of G bases)].

The term "CpG island" refers to a contiguous region of genomic DNA that satisfies the criteria of (1) having a frequency of CpG dinucleotides corresponding to an "Observed/Expected Ratio" >0.6, and (2) having a "GC Content" >0.5. CpG islands are typically, but not always, between about 0.2 to about 1 kb, or to about 2kb in length.

The term "methylation state" or "methylation status" refers to the presence or absence of 5-methylcytosine ("5-mCyt") at one or a plurality of CpG dinucleotides within a DNA sequence. Methylation states at one or more particular CpG methylation sites (each having two CpG dinucleotide sequences) within a DNA sequence include "unmethylated," "fully-methylated" and "hemi-methylated."

The term "hemi-methylation" or "hemimethylation" refers to the methylation state of a palindromic CpG methylation site, where only a single cytosine in one of the two CpG dinucleotide sequences of the palindromic CpG methylation site is methylated (e.g., 5'-CC$^M$GG-3' (top strand): 3'-GGCC-5' (bottom strand)).

The term 'AUC' as used herein is an abbreviation for the area under a curve. In particular it refers to the area under a Receiver Operating Characteristic (ROC) curve. The ROC curve is a plot of the true positive rate against the false positive rate for the different possible cutpoints of a diagnostic test. It shows the tradeoff between sensitivity and specificity depending on the selected cutpoint (any increase in sensitivity will be accompanied by a decrease in specificity). The area under an ROC curve (AUC) is a measure for the accuracy of a diagnostic test (the larger the area the better, optimum is 1, a random test would have a ROC curve lying on the diagonal with an area of 0.5; for reference: J.P. Egan. Signal Detection Theory and ROC Analysis, Academic Press, New York, 1975).

The term "hypermethylation" refers to the average methylation state corresponding to an *increased* presence of 5-mCyt at one or a plurality of CpG dinucleotides within a DNA sequence of a test DNA sample, relative to the amount of 5-mCyt found at corresponding CpG dinucleotides within a normal control DNA sample.

The term "hypomethylation" refers to the average methylation state corresponding to a *decreased* presence of 5-mCyt at one or a plurality of CpG dinucleotides within a DNA sequence of a test DNA sample, relative to the amount of 5-mCyt found at corresponding CpG dinucleotides within a normal control DNA sample.

The term "microarray" refers broadly to both "DNA microarrays," and 'DNA chip(s),' as recognized in the art, encompasses all art-recognized solid supports, and encompasses all methods for affixing nucleic acid molecules thereto or synthesis of nucleic acids thereon.

"Genetic parameters" are mutations and polymorphisms of genes and sequences further required for their regulation. To be designated as mutations are, in particular, insertions, deletions, point mutations, inversions and polymorphisms and, particularly preferred, SNPs (single nucleotide polymorphisms).

"Epigenetic parameters" are, in particular, cytosine methylations. Further epigenetic parameters include, for example, the acetylation of histones which, however, cannot be directly analyzed using the described method but which, in turn, correlate with the DNA methylation.

The term "bisulfite reagent" refers to a reagent comprising bisulfite, disulfite, hydrogen sulfite or combinations thereof, useful as disclosed herein to distinguish between methylated and unmethylated CpG dinucleotide sequences.

The term "Methylation assay" refers to any assay for determining the methylation state of one or more CpG dinucleotide sequences within a sequence of DNA.

The term "MS.AP-PCR" (Methylation-Sensitive Arbitrarily-Primed Polymerase Chain Reaction) refers to the art-recognized technology that allows for a global scan of the genome using CG-rich primers to focus on the regions most likely to contain CpG dinucleotides, and described by Gonzalgo et al., Cancer Research 57: 594-599, 1997.

The term "MethyLight™" refers to the art-recognized fluorescence-based real-time PCR technique described by Eads et al., Cancer Res. 59:2302-2306, 1999.

The term "HeavyMethyl™" assay, in the embodiment thereof implemented herein, refers to an assay, wherein methylation specific *blocking* probes (also referred to herein as *blockers)* covering CpG positions between, or covered by the amplification primers enable methylation-specific selective amplification of a nucleic acid sample.

The term "HeavyMethyl™ MethyLight™" assay, in the embodiment thereof implemented herein, refers to a HeavyMethyl™ MethyLight™ assay, which is a variation of the MethyLight™ assay, wherein the MethyLight™ assay is combined with methylation specific *blocking* probes covering CpG positions between the amplification primers.

The term "Ms-SNuPE" (Methylation-sensitive Single Nucleotide Primer Extension) refers to the art-recognized assay described by Gonzalgo & Jones, Nucleic Acids Res. 25:2529-2531, 1997.

The term "MSP" (Methylation-specific PCR) refers to the art-recognized methylation assay described by Herman et al. Proc. Natl. Acad. Sci. USA 93:9821-9826, 1996, and by US Patent No. 5,786,146.

The term "COBRA" (Combined Bisulfite Restriction Analysis) refers to the art-recognized methylation assay described by Xiong & Laird, Nucleic Acids Res. 25:2532-2534, 1997.

The term "MCA" (Methylated CpG Island Amplification) refers to the methylation assay described by Toyota et al., Cancer Res. 59:2307-12, 1999, and in WO 00/26401A1.

The term "hybridization" is to be understood as a bond of an oligonucleotide to a complementary sequence along the lines of the Watson-Crick base pairings in the sample DNA, forming a duplex structure.

"Stringent hybridization conditions," as defined herein, involve hybridizing at 68°C in 5x SSC/5x Denhardt's solution/1.0% SDS, and washing in 0.2x SSC/0.1% SDS at room temperature, or involve the art-recognized equivalent thereof (e.g., conditions in which a hybridization is carried out at 60°C in 2.5 x SSC buffer, followed by several washing steps at 37°C in a low buffer concentration, and remains stable). Moderately stringent conditions, as defined herein, involve including washing in 3x SSC at 42°C, or the art-recognized equivalent thereof. The parameters of salt concentration and temperature can be varied to achieve the optimal level of identity between the probe and the target nucleic acid. Guidance regarding such conditions is available in the art, for example, by Sambrook et al., 1989, Molecular Cloning, A Laboratory Manual, Cold Spring Harbor Press, N.Y.; and Ausubel et al. (eds.), 1995, Current Protocols in Molecular Biology, (John Wiley & Sons, N.Y.) at Unit 2.10.

The terms "array SEQ ID NO," "composite array SEQ ID NO," or "composite array sequence" refer to a sequence, hypothetical or otherwise, consisting of a head-to-tail (5' to 3') linear composite of all individual contiguous sequences of a subject array (e.g., a head-to-tail composite of SEQ ID NO:1-71, in that order).

The terms "array SEQ ID NO node," "composite array SEQ ID NO node," or "composite array sequence node" refer to a *junction* between any two individual contiguous sequences of the "array SEQ ID NO," the "composite array SEQ ID NO," or the "composite array sequence."

In reference to composite array sequences, the phrase "contiguous nucleotides" refers to a contiguous sequence region of any individual contiguous sequence of the composite array, but does not include a region of the composite array sequence that includes a "node," as defined herein above.

<u>Overview:</u>

The present invention provides for a molecular genetic marker that has utility for providing a prognosis of cell proliferative disorders, most preferably cancer but not breast cancer. It is particularly preferred that said cancers are selected from the group consisting bladder cancer, colorectal cancer, endometrial cancer, kidney (renal cell) cancer, leukemia, lung (Including bronchus) cancer, melanoma, non-Hodgkin's lymphoma, pancreatic cancer, prostate cancer, skin cancer and thyroid cancer. In particular embodiments said marker may be used for classifying the cancer according to aggressiveness and/or invasiveness. It is particularly preferred that the method and nucleic acids according to the invention are utilized for at least one of: prognosis of; treatment of; monitoring of; and treatment and monitoring of cell proliferative disorders, most preferably cancer but not breast cancer.

The term 'prognosis' is taken to mean a prediction of outcome of disease progression (wherein the term progression shall be taken to also include recurrence after treatment). Prognosis may be expressed in terms of overall patient survival, disease- or relapse-free survival, increased tumor-related complications and rate of progression of tumor or metastases, wherein a decrease in any of said factors (with the exception of increased tumor-related complications rate of progression) as relative to a pre-determined level, is a 'negative' outcome and increase thereof is a 'positive' outcome. A decrease in tumor-related complications and/or rate of progression of tumor or metastases as relative to a pre-determined level, is considered a 'positive' outcome and increase thereof is a 'negative' outcome.

Hereinafter prognosis may also be referred to in terms of 'aggressiveness' wherein an aggressive cancer is determined to have a high risk of negative outcome and wherein a non-aggressive cancer has a low risk of negative outcome.

In one aspect the prognostic marker according to the present invention is used to provide an estimate of the risk of negative outcome. For example, characterization of a cancer in terms of predicted outcome enables the physician to determine the risk of recurrence and/or death. This aids in treatment selection as the absolute reduction of risk of recurrence and death after treatments such as adjuvant hormonal, chemo-, and radiation therapy can be determined based on the predicted negative outcome. The absolute reduction in risk attributable to treatment may then be compared to the drawbacks of said treatment (e.g. side effects, cost) in order to determine the suitability of said treatment for the patient.

Conversely, wherein a cancer is characterized as non-aggressive (i.e. positive outcome with low risk of death and/or recurrence) the patient will derive low absolute benefit from adjuvant or other treatment and may be appropriately treated by watchful waiting (commonly prescribed in prostate cancer). Therein lies a great advantage of the present invention. By providing a means for determining which patients will not significantly benefit from treatment the present prevents the over-prescription of therapies.

According to the predicted outcome (i.e. prognosis) of the disease an appropriate treatment or treatments may be selected. Wherein a cancer is characterized as aggressive it is particularly preferred that adjuvant treatment such as, but not limited to, hormonal, chemo- or radiation therapy is provided in addition to or instead of further treatments.

The herein described marker has further utility in predicting outcome of a patient after surgical treatment. This will hereinafter also be referred to as a 'predictive' marker. Over expression of the gene PITX2 is associated with negative outcome of cancer patients. Patients with predicted positive outcome (i.e. hypomethylation or over-expression) after said treatment will accordingly have a decreased absolute reduction of risk of recurrence and death after treatment with post surgical adjuvant therapies. Patients with predicted negative outcome (i.e. hypermethylation or under-expression) after said treatment will accordingly have a relatively larger absolute reduction of risk of recurrence and death after post surgical adjuvant treatment. Accordingly patients with a negative outcome after said treatment will be considered more suitable candidates for adjuvant treatment than patients with a positive outcome. Patients with a positive outcome may accordingly be prevented from over-prescription of adjuvant treatment.

*Bisulfite modification of DNA is an art-recognized tool used to assess CpG methylation status. 5*-methylcytosine is the most frequent covalent base modification in the DNA of eukaryotic cells. It plays a role, for example, in the regulation of the transcription, in genetic imprinting, and in tumorigenesis. Therefore, the identification of 5-methylcytosine as a component of genetic information is of considerable interest. However, 5-methylcytosine positions cannot be identified by sequencing, because 5-methylcytosine has the same base pairing behavior as cytosine. Moreover, the epigenetic information carried by 5-methylcytosine is completely lost during, *e.g.,* PCR amplification.

The most frequently used method for analyzing DNA for the presence of 5-methylcytosine is based upon the specific reaction of bisulfite with cytosine whereby, upon subsequent alkaline hydrolysis, cytosine is converted to uracil, which corresponds to thymine in its base pairing behavior. Significantly, however, 5-methylcytosine remains unmodified under these conditions. Consequently, the original DNA is *converted* in such a manner that methylcytosine, which originally could not be distinguished from cytosine by its hybridization behavior, can now be detected as the only remaining cytosine using standard, art-recognized molecular biological techniques, for example, by amplification and hybridization, or by sequencing. All of these techniques are based on differential base pairing properties, which can now be fully exploited.

The prior art, in terms of sensitivity, is defined by a method comprising enclosing the DNA to be analyzed in an agarose matrix, thereby preventing the diffusion and renaturation of the DNA (bisulfite only reacts with single-stranded DNA), and replacing all precipitation and purification steps with fast dialysis (Olek A, et al., A modified and improved method for bisulfite based cytosine methylation analysis, Nucleic Acids Res. 24:5064-6, 1996). It is thus possible to analyze individual cells for methylation status, illustrating the utility and sensitivity of the method. An overview of art-recognized methods for detecting 5-methylcytosine is provided by Rein, T., et al., Nucleic Acids Res., 26:2255, 1998.

The bisulfite technique, barring few exceptions (e.g., Zeschnigk M, et al., Eur J Hum Genet. 5:94-98, 1997), is currently only used in research. In all instances, short, specific fragments of a known gene are amplified subsequent to a bisulfite treatment, and either completely sequenced (Olek & Walter, Nat Genet. 1997 17:275-6, 1997), subjected to one or more primer extension reactions (Gonzalgo & Jones, Nucleic Acids Res., 25:2529-31, 1997; WO 95/00669; U.S. Patent No. 6,251,594) to analyze individual cytosine positions, or treated by enzymatic digestion (Xiong & Laird, Nucleic Acids Res., 25:2532-4, 1997). Detection by hybridization has also been described in the art (Olek et al., WO 99/28498). Additionally, use of the bisulfite technique for methylation detection with respect to individual genes has been described (Grigg & Clark, Bioessays, 16:431-6, 1994; Zeschnigk M, et al., Hum Mol Genet., 6:387-95, 1997; Feil R, et al., Nucleic Acids Res., 22:695-, 1994; Martin V, et al., Gene, 157:261-4, 1995; WO 9746705 and WO 9515373).

The present invention provides for the use of the bisulfite technique, in combination with one or more methylation assays, for determination of the methylation status of CpG dinucleotide sequences within sequences from the group consisting of SEQ ID NO:1. Preferably said group consists of SEQ ID Nos:35. 63, 19 and most preferably said sequence is SEQ ID NO: 961 According to the present invention, determination of the methylation status of CpG dinucleotide sequences within sequences from the group consisting of SEQ ID NO:1 and SEQ ID NO: 961has prognostic utility.

*Methylation Assay Procedures.* Various methylation assay procedures are known in the art, and can be used in conjunction with the present invention. These assays allow for determination of the methylation state of one or a plurality of CpG dinucleotides (e.g., CpG islands) within a DNA sequence. Such assays involve, among other techniques, DNA sequencing of bisulfite-treated DNA, PCR (for sequence-specific amplification), Southern blot analysis, and use of methylation-sensitive restriction enzymes.

For example, genomic sequencing has been simplified for analysis of DNA methylation patterns and 5-methylcytosine distribution by using bisulfite treatment (Frommer et al., Proc. Natl. Acad. Sci. USA 89:1827-1831, 1992). Additionally, restriction enzyme digestion of PCR products amplified from bisulfite-converted DNA is used, e.g., the method described by Sadri & Hornsby (Nucl. Acids Res. 24:5058-5059, 1996), or COBRA (Combined Bisulfite Restriction Analysis) (Xiong & Laird, Nucleic Acids Res. 25:2532-2534, 1997).

COBRA. COBRA analysis is a quantitative methylation assay useful for determining DNA methylation levels at specific gene loci in small amounts of genomic DNA (Xiong & Laird, Nucleic Acids Res. 25:2532-2534, 1997). Briefly, restriction enzyme digestion is used to reveal methylation-dependent sequence differences in PCR products of sodium bisulfite-treated DNA. Methylation-dependent sequence differences are first introduced into the genomic DNA by standard bisulfite treatment according to the procedure described by Frommer et al. (Proc. Natl. Acad. Sci. USA 89:1827-1831, 1992). PCR amplification of the bisulfite converted DNA is then performed using primers specific for the CpG islands of interest, followed by restriction endonuclease digestion, gel electrophoresis, and detection using specific, labeled hybridization probes. Methylation levels in the original DNA sample are represented by the relative amounts of digested and undigested PCR product in a linearly quantitative fashion across a wide spectrum of DNA methylation levels. In addition, this technique can be reliably applied to DNA obtained from microdissected paraffin-embedded tissue samples. Typical reagents (e.g., as might be found in a typical COBRA-based kit) for COBRA analysis may include, but are not limited to: PCR primers for specific gene (or bisulfite treated DNA sequence or CpG island); restriction enzyme and appropriate buffer; gene-hybridization oligo; control hybridization oligo; kinase labeling kit for oligo probe; and labeled nucleotides. Additionally, bisulfite conversion reagents may include: DNA denaturation buffer; sulfonation buffer; DNA recovery reagents or kits (e.g., precipitation, ultrafiltration, affinity column); desulfonation buffer; and DNA recovery components.

Preferably, assays such as "MethyLight™" (a fluorescence-based real-time PCR technique) (Eads et al., Cancer Res. 59:2302-2306, 1999), Ms-SNuPE (Methylation-sensitive Single Nucleotide Primer Extension) reactions (Gonzalgo & Jones, Nucleic Acids Res. 25:2529-2531, 1997), methylation-specific PCR ("MSP"; Herman et al., Proc. Natl. Acad. Sci. USA 93:9821-9826, 1996; US Patent No. 5,786,146), and methylated CpG island amplification ("MCA"; Toyota et al., Cancer Res. 59:2307-12, 1999) are used alone or in combination with other of these methods.

*MethyLight™.* The MethyLight™ assay is a high-throughput quantitative methylation assay that utilizes fluorescence-based real-time PCR (TaqMan™) technology that requires no further manipulations after the PCR step (Eads et al., Cancer Res. 59:2302-2306, 1999). Briefly, the MethyLight™ process begins with a mixed sample of genomic DNA that is converted, in a sodium bisulfite reaction, to a mixed pool of methylation-dependent sequence differences according to standard procedures (the bisulfite process converts unmethylated cytosine residues to uracil). Fluorescence-based PCR is then performed either in an "unbiased" (with primers that do not overlap known CpG methylation sites) PCR reaction, or in a "biased" (with PCR primers that overlap known CpG dinucleotides) reaction. Sequence discrimination can occur either at the level of the amplification process or at the level of the fluorescence detection process, or both.

The MethyLight™ assay may be used as a quantitative test for methylation patterns in the genomic DNA sample, wherein sequence discrimination occurs at the level of probe hybridization. In this quantitative version, the PCR reaction provides for unbiased amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not "cover" known methylation sites (a fluorescence-based version of the "MSP" technique), or with oligonucleotides covering potential methylation sites.

The MethyLight™ process can by used with a "TaqMan®" probe in the amplification process. For example, double-stranded genomic DNA is treated with sodium bisulfite and subjected to one of two sets of PCR reactions using TaqMan® probes; e.g., with either biased primers and TaqMan® probe, or unbiased primers and TaqMan® probe. The TaqMan® probe is dual-labeled with fluorescent "reporter" and "quencher" molecules, and is designed to be specific for a relatively high GC content region so that it melts out at about 10°C higher temperature in the PCR cycle than the forward or reverse primers. This allows the TaqMan® probe to remain fully hybridized during the PCR annealing/extension step. As the Taq polymerase enzymatically synthesizes a new strand during PCR, it will eventually reach the annealed TaqMan® probe. The Taq polymerase 5' to 3' endonuclease activity will then displace the TaqMan® probe by digesting it to release the fluorescent reporter molecule for quantitative detection of its now unquenched signal using a real-time fluorescent detection system.

Typical reagents (e.g., as might be found in a typical MethyLightTM-based kit) for MethyLightTM analysis may include, but are not limited to: PCR primers for specific gene (or bisulfite treated DNA sequence or CpG island); TaqMan® probes; optimized PCR buffers and deoxynucleotides; and Taq polymerase.

*Ms-SNuPE.* The Ms-SNuPE technique is a quantitative method for assessing methylation differences at specific CpG sites based on bisulfite treatment of DNA, followed by single-nucleotide primer extension (Gonzalgo & Jones, Nucleic Acids Res. 25:2529-2531, 1997). Briefly, genomic DNA is reacted with sodium bisulfite to convert unmethylated cytosine to uracil while leaving 5-methylcytosine unchanged. Amplification of the desired target sequence is then performed using PCR primers specific for bisulfite-converted DNA, and the resulting product is isolated and used as a template for methylation analysis at the CpG site(s) of interest. Small amounts of DNA can be analyzed (*e.g.*, microdissected pathology sections), and it avoids utilization of restriction enzymes for determining the methylation status at CpG sites.

Typical reagents (*e.g.*, as might be found in a typical Ms-SNuPE-based kit) for Ms-SNuPE analysis may include, but are not limited to: PCR primers for specific gene (or bisulfite treated DNA sequence or CpG island); optimized PCR buffers and deoxynucleotides; gel extraction kit; positive control primers; Ms-SNuPE primers for specific gene; reaction buffer (for the Ms-SNuPE reaction); and labeled nucleotides. Additionally, bisulfite conversion reagents may include: DNA denaturation buffer; sulfonation buffer; DNA recovery regents or kit (*e.g.*, precipitation, ultrafiltration, affinity column); desulfonation buffer; and DNA recovery components.

MSP. MSP (methylation-specific PCR) allows for assessing the methylation status of virtually any group of CpG sites within a CpG island, independent of the use of methylation-sensitive restriction enzymes (Herman et al. Proc. Natl. Acad. Sci. USA 93:9821-9826, 1996; US Patent No. 5,786,146). Briefly, DNA is modified by sodium bisulfite converting all unmethylated, but not methylated cytosines to uracil, and subsequently amplified with primers specific for methylated versus unmethylated DNA. MSP requires only small quantities of DNA, is sensitive to 0.1% methylated alleles of a given CpG island locus, and can be performed on DNA extracted from paraffin-embedded samples. Typical reagents (*e.g.*, as might be found in a typical MSP-based kit) for MSP analysis may include, but are not limited to: methylated and unmethylated PCR primers for specific gene (or bisulfite treated DNA sequence or CpG island), optimized PCR buffers and deoxynucleotides, and specific probes.

*MCA.* The MCA technique is a method that can be used to screen for altered methylation patterns in genomic DNA, and to isolate specific sequences associated with these changes (Toyota et al., Cancer Res. 59:2307-12, 1999). Briefly, restriction enzymes with different sensitivities to cytosine methylation in their recognition sites are used to digest genomic DNAs from primary tumors, cell lines, and normal tissues prior to arbitrarily primed PCR amplification. Fragments that show differential methylation are cloned and sequenced after resolving the PCR products on high-resolution polyacrylamide gels. The cloned fragments are then used as probes for Southern analysis to confirm differential methylation of these regions. Typical reagents (*e.g.,* as might be found in a typical MCA-based kit) for MCA analysis may include, but are not limited to: PCR primers for arbitrary priming Genomic DNA; PCR buffers and nucleotides, restriction enzymes and appropriate buffers; gene-hybridization oligos or probes; control hybridization oligos or probes.

<u>The genomic sequences according to SEQ ID NO: 1 and non-naturally occurring treated Variants thereof according to SEQ ID NOS: 2 to 5, were determined to have utility for providing a prognosis and/or treatment of cell proliferative disorders, most preferably cancer but not breast cancer.</u>

In one embodiment the invention provides a method for providing a prognosis of cell proliferative disorders, most preferably cancer but not breast cancer in a subject. It is particularly preferred that said cancers are selected from the group consisting bladder cancer, colorectal cancer, endometrial cancer, kidney (renal cell) cancer, leukemia, lung (Including bronchus) cancer, melanoma, non-Hodgkin's lymphoma, pancreatic cancer, prostate cancer, skin cancer and thyroid cancer. In a particularly preferred embodiment the invention provides a method for the classification based on aggressiveness of a cancer.

Said method comprises the following steps:

i) determining the expression levels of the gene PITX2 and/or regulatory regions thereof; and
ii) determining the prognosis of said cell proliferative disorders.

Said expression level may be determined by any means standard in the art including but not limited to methylation analysis, loss of heterozygosity (hereinafter also referred to as LOH), RNA expression levels and protein expression levels.

Accordingly the present invention also provides prognostic assays and methods, both quantitative and qualitative for detecting the expression of the gene PITX2 in a subject with a cell proliferative disorder, preferably cancer but not breast cancer and determining therefrom upon the prognosis in said subject.

Aberrant expression of mRNA transcribed from the gene PITX2 are associated with prognosis of cancer. Over expression is associated with poor prognosis , under expression is associated with good prognosis.

To detect the presence of mRNA encoding a gene or genomic sequence, a sample is obtained from a patient. The sample may be any suitable sample comprising cellular matter of the tumor, most preferably the primary tumor. Suitable sample types include The DNA source may be any suitable source. Preferably, the source of the DNA sample is selected from the group consisting of cells or cell lines, histological slides, biopsies, paraffin-embedded tissue, bodily fluids, ejaculate, urine, blood, sputum, stool, tissues for example but not limited to those from colon, prostate, lung or liver and combinations thereof. Preferably, the source is biopsies, bodily fluids, ejaculate, urine, or blood.

In a particularly preferred embodiment of the method said source is primary tumor tissue. The sample may be treated to extract the RNA contained therein. The resulting nucleic acid from the sample is then analyzed. Many techniques are known in the state of the art for determining absolute and relative levels of gene expression, commonly used techniques suitable for use in the present invention include in situ hybridization (e.g. FISH), Northern analysis, RNase protection assays (RPA), microarrays and PCR-based techniques, such as quantitative PCR and differential display PCR or any other nucleic acid detection method.

Particularly preferred is the use of the reverse transcription/polymerization chain reaction technique (RT-PCR). The method of RT-PCR is well known in the art (for example, see Watson and Fleming, supra).

The RT-PCR method can be performed as follows. Total cellular RNA is isolated by, for example, the standard guanidium isothiocyanate method and the total RNA is reverse transcribed. The reverse transcription method involves synthesis of DNA on a template of RNA using a reverse transcriptase enzyme and a 3' end oligo dT primer and/or random hexamer primers. The cDNA thus produced is then amplified by means of PCR. (Belyavsky et al. Nucl Acid Res 17:2919-2932, 1989; Krug and Berger, Methods in Enzymology, Academic Press, N.Y., Vol.152, pp. 316-325, 1987. Further preferred is the "Real-time" variant of RT- PCR, wherein the PCR product is detected by means of hybridization probes (e.g. TaqMan, Lightcycler, Molecular Beacons & Scorpion) or SYBR green. The detected signal from the probes or SYBR green is then quantitated either by reference to a standard curve or by comparing the Ct values to that of a calibration standard. Analysis of housekeeping genes is often used to normalize the results.

In Northern blot analysis total or poly(A)+ mRNA is run on a denaturing agarose gel and detected by hybridization to a labeled probe in the dried gel itself or on a membrane. The resulting signal is proportional to the amount of target RNA in the RNA population.

Comparing the signals from two or more cell populations or tissues reveals relative differences in gene expression levels. Absolute quantitation can be performed by comparing the signal to a standard curve generated using known amounts of an in vitro transcript corresponding to the target RNA. Analysis of housekeeping genes, genes whose expression levels are expected to remain relatively constant regardless of conditions, is often used to normalize the results, eliminating any apparent differences caused by unequal transfer of RNA to the membrane or unequal loading of RNA on the gel.

The first step in Northern analysis is isolating pure, intact RNA from the cells or tissue of interest. Because Northern blots distinguish RNAs by size, sample integrity influences the degree to which a signal is localized in a single band. Partially degraded RNA samples will result in the signal being smeared or distributed over several bands with an overall loss in sensitivity and possibly an erroneous interpretation of the data. In Northern blot analysis, DNA, RNA and oligonucleotide probes can be used and these probes are preferably labeled (e.g. radioactive labels, mass labels or fluorescent labels). The size of the target RNA, not the probe, will determine the size of the detected band, so methods such as random-primed labeling, which generates probes of variable lengths, are suitable for probe synthesis. The specific activity of the probe will determine the level of sensitivity, so it is preferred that probes with high specific activities, are used.

In an RNase protection assay, the RNA target and an RNA probe of a defined length are hybridized in solution. Following hybridization, the RNA is digested with RNases specific for single-stranded nucleic acids to remove any unhybridized, single-stranded target RNA and probe. The RNases are inactivated, and the RNA is separated e.g. by denaturing polyacrylamide gel electrophoresis. The amount of intact RNA probe is proportional to the amount of target RNA in the RNA population. RPA can be used for relative and absolute quantitation of gene expression and also for mapping RNA structure, such as intron/exon boundaries and transcription start sites. The RNase protection assay is preferable to Northern blot analysis as it generally has a lower limit of detection.

The antisense RNA probes used in RPA are generated by in vitro transcription of a DNA template with a defined endpoint and are typically in the range of 50-600 nucleotides. The use of RNA probes that include additional sequences not homologous to the target RNA allows the protected fragment to be distinguished from the full-length probe. RNA probes are typically used instead of DNA probes due to the ease of generating single-stranded RNA probes and the reproducibility and reliability of RNA:RNA duplex digestion with RNases (Ausubel *et al.* 2003), particularly preferred are probes with high specific activities.

Particularly preferred is the use of microarrays. The microarray analysis process can be divided into two main parts. First is the immobilization of known gene sequences onto glass slides or other solid support followed by hybridization of the fluorescently labeled cDNA (comprising the sequences to be interrogated) to the known genes immobilized on the glass slide. After hybridization, arrays are scanned using a fluorescent microarray scanner. Analyzing the relative fluorescent intensity of different genes provides a measure of the differences in gene expression.

DNA arrays can be generated by immobilizing presynthesized oligonucleotides onto prepared glass slides. In this case, representative gene sequences are manufactured and prepared using standard oligonucleotide synthesis and purification methods. These synthesized gene sequences are complementary to the genes of interest (in this case PITX2) and tend to be shorter sequences in the range of 25-70 nucleotides. Alternatively, immobilized oligos can be chemically synthesized in situ on the surface of the slide. In situ oligonucleotide synthesis involves the consecutive addition of the appropriate nucleotides to the spots on the microarray; spots not receiving a nucleotide are protected during each stage of the process using physical or virtual masks.

In expression profiling microarray experiments, the RNA templates used are representative of the transcription profile of the cells or tissues under study. RNA is first isolated from the cell populations or tissues to be compared. Each RNA sample is then used as a template to generate fluorescently labeled cDNA via a reverse transcription reaction. Fluorescent labeling of the cDNA can be accomplished by either direct labeling or indirect labeling methods. During direct labeling, fluorescently modified nucleotides, (e.g., Cy®3- or Cy®5-dCTP) are incorporated directly into the cDNA during the reverse transcription. Alternatively, indirect labeling can be achieved by incorporating aminoallyl-modified nucleotides during cDNA synthesis and then conjugating an N-hydroxysuccinimide (NHS)-ester dye to the aminoallyl-modified cDNA after the reverse transcription reaction is complete. Alternatively, the probe may be unlabelled, but may be detectable by specific binding with a ligand which is labeled, either directly or indirectly. Suitable labels and methods for labeling ligands (and probes) are known in the art, and include, for example, radioactive labels which may be incorporated by known methods (e.g., nick translation or kinasing). Other suitable labels include but are not limited to biotin, fluorescent groups, chemiluminescent groups (e.g., dioxetanes, particularly triggered dioxetanes), enzymes, antibodies, and the like.

To perform differential gene expression analysis, cDNA generated from different RNA samples are labeled with Cy®3. The resulting labeled cDNA is purified to remove unincorporated nucleotides, free dye and residual RNA. Following purification, the labeled cDNA samples are hybridized to the microarray. The stringency of hybridization is determined by a number of factors during hybridization and during the washing procedure, including temperature, ionic strength, length of time and concentration of formamide. These factors are outlined in, for example, Sambrook et al. (Molecular Cloning: A Laboratory Manual, 2nd ed., 1989). The microarray is scanned post-hybridization using a fluorescent microarray scanner. The fluorescent intensity of each spot indicates the level of expression for that gene; bright spots correspond to strongly expressed genes, while dim spots indicate weak expression.

Once the images are obtained, the raw data must be analyzed. First, the background fluorescence must be subtracted from the fluorescence of each spot. The data is then normalized to a control sequence, such as an exogenously added RNA, or a housekeeping gene panel to account for any nonspecific hybridization, array imperfections or variability in the array setup, cDNA labeling, hybridization or washing. Data normalization allows the results of multiple arrays to be compared.

The present invention further provides for methods for the detection of the presence of the polypeptide encoded by said gene sequences in a sample obtained from a patient.

Aberrant levels of polypeptide expression of the polypeptides encoded by the gene PITX2 are associated with prognosis of cell proliferative disorder, preferably cancer but not breast cancer. It is particularly preferred that said cancers are selected from the group consisting bladder cancer, colorectal cancer, endometrial cancer, kidney (renal cell) cancer, leukemia, lung (Including bronchus) cancer, melanoma, non-Hodgkin's lymphoma, pancreatic cancer, prostate cancer, skin cancer and thyroid cancer. Accordingly over or under expression of said polypeptides are associable with the prognosis of cancers. Over expression is associated with poor prognosis and under expression is associated with good prognosis.

Any method known in the art for detecting polypeptides can be used. Such methods include, but are not limited to mass-spectrometry, immunodiffusion, immunoelectrophoresis, immunochemical methods, binder-ligand assays, immunohistochemical techniques, agglutination and complement assays (e.g., see Basic and Clinical Immunology, Sites and Terr, eds., Appleton & Lange, Norwalk, Conn. pp 217-262, 1991). Preferred are binder-ligand immunoassay methods including reacting antibodies with an epitope or epitopes and competitively displacing a labeled polypeptide or derivative thereof.

Certain embodiments of the present invention comprise the use of antibodies specific to the polypeptide encoded by the PITX2 gene.

Such antibodies are useful for cancer prognostic and/or predictive applications. In certain embodiments production of monoclonal or polyclonal antibodies can be induced by the use of the coded polypeptide as an antigen. Such antibodies may in tum be used to detect expressed polypeptides as markers for cell proliferative disorder, preferably cancer but not breast cancer prognosis. The levels of such polypeptides present may be quantified by conventional methods. Antibody-polypeptide binding may be detected and quantified by a variety of means known in the art, such as labeling with fluorescent or radioactive ligands. The invention further comprises kits for performing the above-mentioned procedures, wherein such kits contain antibodies specific for the investigated polypeptides.

Numerous competitive and non-competitive polypeptide binding immunoassays are well known in the art. Antibodies employed in such assays may be unlabelled, for example as used in agglutination tests, or labeled for use a wide variety of assay methods. Labels that can be used include radionuclides, enzymes, fluorescers, chemiluminescers, enzyme substrates or co-factors, enzyme inhibitors, particles, dyes and the like. Preferred assays include but are not limited to radioimmunoassay (RIA), enzyme immunoassays, e.g., enzyme-linked immunosorbent assay (ELISA), fluorescent immunoassays and the like. Polyclonal or monoclonal antibodies or epitopes thereof can be made for use in immunoassays by any of a number of methods known in the art.

In an alternative embodiment of the method the proteins may be detected by means of western blot analysis. Said analysis is standard in the art, briefly proteins are separated by means of electrophoresis e.g. SDS-PAGE. The separated proteins are then transferred to a suitable membrane (or paper) e.g. nitrocellulose, retaining the spatial separation achieved by electrophoresis. The membrane is then incubated with a generic protein (e.g. milk protein) to bind remaining sticky places on the membrane. An antibody specific to the protein of interest is then added, said antibody being detectably labeled for example by dyes or enzymatic means (e.g. alkaline phosphatase or horseradish peroxidase) . The location of the antibody on the membrane is then detected.

In an alternative embodiment of the method the proteins may be detected by means of immunohistochemistry (the use of antibodies to probe specific antigens in a sample). Said analysis is standard in the art, wherein detection of antigens in tissues is known as immunohistochemistry, while detection in cultured cells is generally termed immunocytochemistry. Briefly the primary antibody to be detected by binding to its specific antigen. The antibody-antigen complex is then bound by a secondary enzyme conjugated antibody. In the presence of the necessary substrate and chromogen the bound enzyme is detected according to colored deposits at the antibody-antigen binding sites. There is a wide range of suitable sample types, antigen-antibody affinity, antibody types, and detection enhancement methods. Thus optimal conditions for immunohistochemical or immunocytochemical detection must be determined by the person skilled in the art for each individual case.

One approach for preparing antibodies to a polypeptide is the selection and preparation of an amino acid sequence of all or part of the polypeptide, chemically synthesizing the amino acid sequence and injecting it into an appropriate animal, usually a rabbit or a mouse (Milstein and Kohler Nature 256:495-497, 1975; Gulfre and Milstein, Methods in Enzymology: Immunochemical Techniques 73:1-46, Langone and Banatis eds., Academic Press, 1981). Methods for preparation of the polypeptides or epitopes thereof include, but are not limited to chemical synthesis, recombinant DNA techniques or isolation from biological samples.

In the final step of the method the prognosis of the patient is determined, whereby overexpression is indicative of negative prognosis. The term overexpression shall be taken to mean expression at a detected level greater than a pre-determined cut off which may be selected from the group consisting of the mean, median or an optimized threshold value.

Another aspect of the invention provides a kit for use in providing a prognosis of a subject with a cell proliferative disorder, preferably cancer but not breast cancer, comprising: a means for detecting PITX2 polypeptides. The means for detecting the polypeptides comprise preferably antibodies, antibody derivatives, or antibody fragments. The polypeptides are most preferably detected by means of Western blotting utilizing a labeled antibody. In another embodiment of the invention the kit further comprising means for obtaining a biological sample of the patient. Preferred is a kit, which further comprises a container suitable for containing the means for detecting the polypeptides in the biological sample of the patient, and most preferably further comprises instructions for use and interpretation of the kit results. In a preferred embodiment the kit for use in determining treatment strategy for a patient with a cell proliferative disorder, preferably cancer but not breast cancer, comprises: (a) a means for detecting PITX2 polypeptides; (b) a container suitable for containing the said means and the biological sample of the patient comprising the polypeptides wherein the means can form complexes with the polypeptides; (c) a means to detect the complexes of (b); and optionally (d) instructions for use and interpretation of the kit results. It is particularly preferred that said cancers are selected from the group consisting bladder cancer, colorectal cancer, endometrial cancer, kidney (renal cell) cancer, leukemia, lung (Including bronchus) cancer, melanoma, non-Hodgkin's lymphoma, pancreatic cancer, prostate cancer, skin cancer and thyroid cancer.

The kit may also contain other components such as buffers or solutions suitable for blocking, washing or coating , packaged in a separate container.

Another aspect of the invention relates to a kit for use in providing a prognosis of a subject with a cell proliferative disorder, preferably cancer but not breast cancer, said kit comprising: a means for measuring the level of transcription of the gene PITX2. It is particularly preferred that said cancers are selected from the group consisting bladder cancer, colorectal cancer, endometrial cancer, kidney (renal cell) cancer, leukemia, lung (Including bronchus) cancer, melanoma, non-Hodgkin's lymphoma, pancreatic cancer, prostate cancer, skin cancer and thyroid cancer. In a preferred embodiment the means for measuring the level of transcription comprise oligonucleotides or polynucleotides able to hybridize under stringent or moderately stringent conditions to the transcription products of PITX2. In a most preferred embodiment the level of transcription is determined by techniques selected from the group of Northern blot analysis, reverse transcriptase PCR, real-time PCR, RNAse protection, and microarray. In another embodiment of the invention the kit further comprises means for obtaining a biological sample of the patient. Preferred is a kit, which further comprises a container suitable for containing the means for measuring the level of transcription and the biological sample of the patient, and most preferably further comprises instructions for use and interpretation of the kit results.

In a preferred embodiment the kit for use in determining treatment strategy for a patient with a cell proliferative disorder, preferably cancer but not breast cancer comprises (a) a plurality of oligonucleotides or polynucleotides able to hybridize under stringent or moderately stringent conditions to the transcription products of the gene PITX2; (b) a container suitable for containing the oligonucleotides or polynucleotides and a biological sample of the patient comprising the transcription products wherein the oligonucleotides or polynucleotide can hybridize under stringent or moderately stringent conditions to the transcription products, (c) means to detect the hybridization of (b); and optionally, (d) instructions for use and interpretation of the kit results.

The kit may also contain other components such as hybridization buffer (where the oligonucleotides are to be used as a probe) packaged in a separate container. Alternatively, where the oligonucleotides are to be used to amplify a target region, the kit may contain, packaged in separate containers, a polymerase and a reaction buffer optimized for primer extension mediated by the polymerase, such as PCR.

Most preferably a kit according to the embodiments of the present invention is used for the determination of expression step of the methods according to other aspects of the invention. In a further aspect, the invention provides a further method for providing a prognosis of a subject with a cell proliferative disorder, preferably cancer but not breast cancer comprising the following steps. It is particularly preferred that said cancers are selected from the group consisting bladder cancer, colorectal cancer, endometrial cancer, kidney (renal cell) cancer, leukemia, lung (Including bronchus) cancer, melanoma, non-Hodgkin's lymphoma, pancreatic cancer, prostate cancer, skin cancer and thyroid cancer. In the first step of the method a sample is obtained from the subject. Commonly used techniques suitable for use in the present invention include in situ hybridization (e.g. FISH), Northern analysis, RNase protection assays (RPA), microarrays and PCR-based techniques, such as quantitative PCR and differential display PCR or any other nucleic acid detection method.

Particularly preferred is the use of the reverse transcription/polymerization chain reaction technique (RT-PCR). The method of RT-PCR is well known in the art (for example, see Watson and Fleming, supra).

The RT-PCR method can be performed as follows. Total cellular RNA is isolated by, for example, the standard guanidium isothiocyanate method and the total RNA is reverse transcribed. The reverse transcription method involves synthesis of DNA on a template of RNA using a reverse transcriptase enzyme and a 3' end oligo dT primer and/or random hexamer primers. The cDNA thus produced is then amplified by means of PCR. (Belyavsky et al, Nucl Acid Res 17:2919-2932, 1989; Krug and Berger, Methods in Enzymology, Academic Press, N.Y., Vol.152, pp. 316-325, 1987). Further preferred is the "Real-time" variant of RT- PCR, wherein the PCR product is detected by means of hybridization probes (e.g. TaqMan, Lightcycler, Molecular Beacons & Scorpion) or SYBR green. The detected signal from the probes or SYBR green is then quantitated either by reference to a standard curve or by comparing the Ct values to that of a calibration standard. Analysis of housekeeping genes is often used to normalize the results.

In Northern blot analysis total or poly(A)+ mRNA is run on a denaturing agarose gel and detected by hybridization to a labeled probe in the dried gel itself or on a membrane. The resulting signal is proportional to the amount of target RNA in the RNA population.

Comparing the signals from two or more cell populations or tissues reveals relative differences in gene expression levels. Absolute quantitation can be performed by comparing the signal to a standard curve generated using known amounts of an in vitro transcript corresponding to the target RNA. Analysis of housekeeping genes, genes whose expression levels are expected to remain relatively constant regardless of conditions, is often used to normalize the results, eliminating any apparent differences caused by unequal transfer of RNA to the membrane or unequal loading of RNA on the gel.

The first step in Northern analysis is isolating pure, intact RNA from the cells or tissue of interest. Because Northern blots distinguish RNAs by size, sample integrity influences the degree to which a signal is localized in a single band. Partially degraded RNA samples will result in the signal being smeared or distributed over several bands with an overall loss in sensitivity and possibly an erroneous interpretation of the data. In Northern blot analysis, DNA, RNA and oligonucleotide probes can be used and these probes are preferably labeled (e.g. radioactive labels, mass labels or fluorescent labels). The size of the target RNA, not the probe, will determine the size of the detected band, so methods such as random-primed labeling, which generates probes of variable lengths, are suitable for probe synthesis. The specific activity of the probe will determine the level of sensitivity, so it is preferred that probes with high specific activities, are used.

In an RNase protection assay, the RNA target and an RNA probe of a defined length are hybridized in solution. Following hybridization, the RNA is digested with RNases specific for single-stranded nucleic acids to remove any unhybridized, single-stranded target RNA and probe. The RNases are inactivated, and the RNA is separated e.g. by denaturing polyacrylamide gel electrophoresis. The amount of intact RNA probe is proportional to the amount of target RNA in the RNA population. RPA can be used for relative and absolute quantitation of gene expression and also for mapping RNA structure, such as intron/exon boundaries and transcription start sites. The RNase protection assay is preferable to Northern blot analysis as it generally has a lower limit of detection.

The antisense RNA probes used in RPA are generated by in vitro transcription of a DNA template with a defined endpoint and are typically in the range of 50-600 nucleotides. The use of RNA probes that include additional sequences not homologous to the target RNA allows the protected fragment to be distinguished from the full-length probe. RNA probes are typically used instead of DNA probes due to the ease of generating single-stranded RNA probes and the reproducibility and reliability of RNA:RNA duplex digestion with RNases (Ausubel *et al.* 2003), particularly preferred are probes with high specific activities.

Particularly preferred is the use of microarrays. The microarray analysis process can be divided into two main parts. First is the immobilization of known gene sequences onto glass slides or other solid support followed by hybridization of the fluorescently labelled cDNA (comprising the sequences to be interrogated) to the known genes immobilized on the glass slide. After hybridization, arrays are scanned using a fluorescent microarray scanner. Analyzing the relative fluorescent intensity of different genes provides a measure of the differences in gene expression.

DNA arrays can be generated by immobilizing presynthesized oligonucleotides onto prepared glass slides. In this case, representative gene sequences are manufactured and prepared using standard oligonucleotide synthesis and purification methods. These synthesized gene sequences are complementary to the genes of interest (in this case PITX2) and tend to be shorter sequences in the range of 25-70 nucleotides. Alternatively, immobilized oligos can be chemically synthesized in situ on the surface of the slide. In situ oligonucleotide synthesis involves the consecutive addition of the appropriate nucleotides to the spots on the microarray; spots not receiving a nucleotide are protected during each stage of the process using physical or virtual masks.

In expression profiling microarray experiments, the RNA templates used are representative of the transcription profile of the cells or tissues under study. RNA is first isolated from the cell populations or tissues to be compared. Each RNA sample is then used as a template to generate fluorescently labelled cDNA via a reverse transcription reaction. Fluorescent labeling of the cDNA can be accomplished by either direct labeling or indirect labeling methods. During direct labeling, fluorescently modified nucleotides (e.g., Cy®3- or Cy®5-dCTP) are incorporated directly into the cDNA during the reverse transcription. Alternatively, indirect labeling can be achieved by incorporating aminoallyl-modified nucleotides during cDNA synthesis and then conjugating an N-hydroxysuccinimide (NHS)-ester dye to the aminoallyl-modified cDNA after the reverse transcription reaction is complete. Alternatively, the probe may be unlabelled, but may be detectable by specific binding with a ligand which is labelled, either directly or indirectly. Suitable labels and methods for labeling ligands (and probes) are known in the art, and include, for example, radioactive labels which may be incorporated by known methods (e.g., nick translation or kinasing). Other suitable labels include but are not limited to biotin, fluorescent groups, chemiluminescent groups (e.g., dioxetanes, particularly triggered dioxetanes), enzymes, antibodies, and the like.

To perform differential gene expression analysis, cDNA generated from different RNA samples are labelled with Cy®3. The resulting labelled cDNA is purified to remove unincorporated nucleotides, free dye and residual RNA. Following purification, the labeled cDNA samples are hybridized to the microarray. The stringency of hybridization is determined by a number of factors during hybridization and during the washing procedure, including temperature, ionic strength, length of time and concentration of formamide. These factors are outlined in, for example, Sambrook et al. (Molecular Cloning: A Laboratory Manual, 2nd ed., 1989). The microarray is scanned post-hybridization using a fluorescent microarray scanner. The fluorescent intensity of each spot indicates the level of expression for that gene; bright spots correspond to strongly expressed genes, while dim spots indicate weak expression.

Once the images are obtained, the raw data must be analyzed. First, the background fluorescence must be subtracted from the fluorescence of each spot. The data is then normalized to a control sequence, such as an exogenously added RNA, or a housekeeping gene panel to account for any nonspecific hybridization, array imperfections or variability in the array setup, cDNA labeling, hybridization or washing. Data normalization allows the results of multiple arrays to be compared.

The present invention further provides for methods for the detection of the presence of the polypeptide encoded by said gene sequences in a sample obtained from a patient.

Aberrant levels of polypeptide expression of the polypeptides encoded by the gene PITX2 are associated with cell proliferative disorder, preferably cancer but not breast cancer prognosis and/or treatment outcome. It is particularly preferred that said cancers are selected from the group consisting bladder cancer, colorectal cancer, endometrial cancer, kidney (renal cell) cancer, leukemia, lung (Including bronchus) cancer, melanoma, non-Hodgkin's lymphoma, pancreatic cancer, prostate cancer, skin cancer and thyroid cancer.

Accordingly over or under expression of said polypeptides are associable with the prognosis and to treatment outcome of cancers. Over expression is associated with poor prognosis and under expression is associated with good prognosis.

Any method known in the art for detecting polypeptides can be used. Such methods include, but are not limited to mass-spectrometry, immunodiffusion, immunoelectrophoresis, immunochemical methods, binder-ligand assays, immunohistochemical techniques, agglutination and complement assays (e.g., see Basic and Clinical Immunology, Sites and Terr, eds., Appleton & Lange, Norwalk, Conn. pp 217-262, 1991). Preferred are binder-ligand immunoassay methods including reacting antibodies with an epitope or epitopes and competitively displacing a labelled polypeptide or derivative thereof.

Certain embodiments of the present invention comprise the use of antibodies specific to the polypeptide encoded by the PITX2 gene.

Such antibodies are useful for cancer prognostic and/or predictive applications. In certain embodiments production of monoclonal or polyclonal antibodies can be induced by the use of the coded polypeptide as an antigen. Such antibodies may in turn be used to detect expressed polypeptides as markers for cell proliferative disorder, preferably cancer but not breast cancer prognosis. The levels of such polypeptides present may be quantified by conventional methods. Antibody-polypeptide binding may be detected and quantified by a variety of means known in the art, such as labeling with fluorescent or radioactive ligands. The invention further comprises kits for performing the above-mentioned procedures, wherein such kits contain antibodies specific for the investigated polypeptides.

Numerous competitive and non-competitive polypeptide binding immunoassays are well known in the art. Antibodies employed in such assays may be unlabelled, for example as used in agglutination tests, or labelled for use a wide variety of assay methods. Labels that can be used include radionuclides, enzymes, fluorescers, chemiluminescers, enzyme substrates or co-factors, enzyme inhibitors, particles, dyes and the like. Preferred assays include but are not limited to radioimmunoassay (RIA), enzyme immunoassays, e.g., enzyme-linked immunosorbent assay (ELISA), fluorescent immunoassays and the like. Polyclonal or monoclonal antibodies or epitopes thereof can be made for use in immunoassays by any of a number of methods known in the art.

In an alternative embodiment of the method the proteins may be detected by means of western blot analysis. Said analysis is standard in the art, briefly proteins are separated by means of electrophoresis e.g. SDS-PAGE. The separated proteins are then transferred to a suitable membrane (or paper) e.g. nitrocellulose, retaining the spatial separation achieved by electrophoresis. The membrane is then incubated with a generic protein (e.g. milk protein) to bind remaining sticky places on the membrane. An antibody specific to the protein of interest is then added, said antibody being detectably labelled for example by dyes or enzymatic means (e.g. alkaline phosphatase or horseradish peroxidase) . The location of the antibody on the membrane is then detected.

In an alternative embodiment of the method the proteins may be detected by means of immunohistochemistry (the use of antibodies to probe specific antigens in a sample). Said analysis is standard in the art, wherein detection of antigens in tissues is known as immunohistochemistry, while detection in cultured cells is generally termed immunocytochemistry. Briefly the primary antibody to be detected by binding to its specific antigen. The antibody-antigen complex is then bound by a secondary enzyme conjugated antibody. In the presence of the necessary substrate and chromogen the bound enzyme is detected according to colored deposits at the antibody-antigen binding sites. There is a wide range of suitable sample types, antigen-antibody affinity, antibody types, and detection enhancement methods. Thus optimal conditions for immunohistochemical or immunocytochemical detection must be determined by the person skilled in the art for each individual case.

One approach for preparing antibodies to a polypeptide is the selection and preparation of an amino acid sequence of all or part of the polypeptide, chemically synthesizing the amino acid sequence and injecting it into an appropriate animal, usually a rabbit or a mouse (Milstein and Kohler Nature 256:495-497, 1975; Gulfre and Milstein, Methods in Enzymology: Immunochemical Techniques 73:1-46. Langone and Banatis eds., Academic Press, 1981). Methods for preparation of the polypeptides or epitopes thereof include, but are not limited to chemical synthesis, recombinant DNA techniques or isolation from biological samples.

In a particularly preferred embodiment the expression level of the gene PITX2 is determined by analysis of the level of methylation of said gene and/or regulatory regions thereof. It is preferred that the level of methylation of said gene and/or regulatory regions thereof is determined by determining the methylation status or level of at least one CpG dinucleotide thereof. It is further preferred that the level of methylation of said gene and/or regulatory regions thereof is determined by determining the methylation status or level of a plurality of CpG dinucleotides thereof. Said analysis comprises the following steps:

i) contacting genomic DNA obtained from the subject with at least one reagent, or series of reagents that distinguishes between methylated and non-methylated CpG dinucleotides within at least one target region of the genomic DNA, wherein said contiguous nucleotides comprise at least one CpG dinucleotide sequence; and
ii) classifying the cell proliferative disorder, (most preferably cancer but not breast cancer) according to its prognosis as determined from the methylation status of said target regions analyzed in i).

Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants e.g. by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA. Preferably, the source of the DNA sample is selected from the group consisting of cells or cell lines, histological slides, biopsies, paraffin-embedded tissue, bodily fluids, ejaculate, urine, blood, and combinations thereof. Preferably, the source is biopsies, bodily fluids, ejaculate, urine, or blood. The genomic DNA sample is then treated in such a manner that cytosine bases which are unmethylated at the 5'-position are converted to uracil, thymine, or another base which is dissimilar to cytosine in terms of hybridization behavior. This will be understood as 'treatment' herein.

The above described treatment of genomic DNA is preferably carried out with bisulfite (hydrogen sulfite, disulfite) and subsequent alkaline hydrolysis which results in a conversion of non-methylated cytosine nucleobases to uracil or to another base which is dissimilar to cytosine in terms of base pairing behavior.

In a preferred embodiment said method is achieved by contacting the nucleic acid of the gene PITX2 and/or its regulatory regions, or sequences thereof according to SEQ ID NO: 1 in a biological sample obtained from a subject with at least one reagent or a series of reagents, wherein said reagent or series of reagents, distinguishes between methylated and non methylated CpG dinucleotides within the target nucleic acid.

In a preferred embodiment, the method comprises the following steps: Preferably, said method comprises the following steps: In the *first step,* a sample of the tissue to be analyzed is obtained. The source may be any suitable source, such as The DNA source may be any suitable source. Preferably, the source of the DNA sample is selected from the group consisting of cells or cell lines, histological slides, biopsies, paraffin-embedded tissue, bodily fluids, ejaculate, urine, blood, sputum, stool, tissues for example but not limited to those from colon, prostate, lung or liver, and combinations thereof. Preferably, the source is biopsies, bodily fluids, ejaculate, urine, or blood. The DNA is then isolated from the sample. Extraction may be by means that are standard to one skilled in the art, including the use of commercially available kits, detergent lysates, sonification and vortexing with glass beads. Briefly, wherein the DNA of interest is encapsulated by a cellular membrane the biological sample must be disrupted and lysed by enzymatic, chemical or mechanical means. The DNA solution may then be cleared of proteins and other contaminants e.g. by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA. Once the nucleic acids have been extracted, the genomic double stranded DNA is used in the analysis.

In the second *step* of the method, the genomic DNA sample is treated in such a manner that cytosine bases which are unmethylated at the 5'-position are converted to uracil, thymine, or another base which is dissimilar to cytosine in terms of hybridization behavior. This will be understood as 'pretreatment' herein.

This is preferably achieved by means of treatment with a bisulfite reagent. The term "bisulfite reagent" refers to a reagent comprising bisulfite, disulfte, hydrogen sulfite or combinations thereof, useful as disclosed herein to distinguish between methylated and unmethylated CpG dinucleotide sequences. Methods of said treatment are known in the art (e.g. PCT/EP2004/011715). It is preferred that the bisulfite treatment is conducted in the presence of denaturing solvents such as but not limited to n-alkylenglycol, particularly diethylene glycol dimethyl ether (DME), or in the presence of dioxane or dioxane derivatives. In a preferred embodiment the denaturing solvents are used in concentrations between 1% and 35% (v/v). It is also preferred that the bisulfite reaction is carried out in the presence of scavengers such as but not limited to chromane derivatives, e.g., 6-hydroxy-2,5,7,8,-tetramethylchromane 2-carboxylic acid (see: PCT/EP2004/011715). The bisulfite conversion is preferably carried out at a reaction temperature between 30°C and 70°C, whereby the temperature is increased to over 85°C for short periods of times during the reaction (see: PCT/EP2004/011715). The bisulfite treated DNA is preferably purified prior to the quantification. This may be conducted by any means known in the art, such as but not limited to ultrafiltration, preferably carried out by means of Microcon^(TM) columns (manufactured by Millipore^(TM)). The purification is carried out according to a modified manufacturer's protocol (see: PCT/EP2004/011715).

In the *third step* of the method, fragments of the pretreated DNA are amplified, using sets of primer oligonucleotides according to the present invention, and an amplification enzyme. The amplification of several DNA segments can be carried out simultaneously in one and the same reaction vessel. Typically, the amplification is carried out using a polymerase chain reaction (PCR). The set of primer oligonucleotides includes at least two oligonucleotides whose sequences are each reverse complementary to, identical to, or hybridize under stringent or highly stringent conditions to an at least 16-base-pair long segment of the base sequences of one of SEQ ID NO: 2 to SEQ ID NO: 5 and sequences complementary thereto.

In an alternate embodiment of the method, the methylation status of preselected CpG positions within SEQ ID NO: 1, may be detected by use of methylation-specific primer oligonucleotides. This technique (MSP) has been described in United States Patent No. 6,265,171 to Herman. The use of methylation status specific primers for the amplification of bisulfite treated DNA allows the differentiation between methylated and unmethylated nucleic acids. MSP primers pairs contain at least one primer that hybridizes to a bisulfite treated CpG dinucleotide. Therefore, the sequence of said primers comprises at least one CpG or TpG dinucleotide. MSP primers specific for non-methylated DNA contain a 'T' at the 3' position of the C position in the CpG. Preferably, therefore, the base sequence of said primers is required to comprise a sequence having a length of at least 9 nucleotides which hybridizes to a pretreated nucleic acid sequence according to one of SEQ ID NO: 2 to SEQ ID NO: 5 and sequences complementary thereto, wherein the base sequence of said oligomers comprises at least one CpG dinucleotide.

A further preferred embodiment of the method comprises the use of *blocker* oligonucleotides. The use of such blocker oligonucleotides has been described by Yu et al., BioTechniques 23:714-720, 1997. Blocking probe oligonucleotides are hybridized to the bisulfite treated nucleic acid concurrently with the PCR primers. PCR amplification of the nucleic acid is terminated at the 5' position of the blocking probe, such that amplification of a nucleic acid is suppressed where the complementary sequence to the blocking probe is present. The probes may be designed to hybridize to the bisulfite treated nucleic acid in a methylation status specific manner. For example, for detection of methylated nucleic acids within a population of unmethylated nucleic acids, suppression of the amplification of nucleic acids which are unmethylated at the position in question would be carried out by the use of blocking probes comprising a 'CpA' or 'TpG' at the position in question, as opposed to a 'CpG' if the suppression of amplification of methylated nucleic acids is desired.

For PCR methods using blocker oligonucleotides, efficient disruption of polymerase-mediated amplification requires that blocker oligonucleotides not be elongated by the polymerase. Preferably, this is achieved through the use of blockers that are 3'-deoxyoligonucleotides, or oligonucleotides derivatized at the 3' position with other than a "free" hydroxyl group. For example, 3'-O-acetyl oligonucleotides are representative of a preferred class of blocker molecule.

Additionally, polymerase-mediated decomposition of the blocker oligonucleotides should be precluded. Preferably, such preclusion comprises either use of a polymerase lacking 5'-3' exonuclease activity, or use of modified blocker oligonucleotides having, for example, thioate bridges at the 5'-termini thereof that render the blocker molecule nuclease-resistant. Particular applications may not require such 5' modifications of the blocker. For example, if the blocker- and primer-binding sites overlap, thereby precluding binding of the primer (e.g., with excess blocker), degradation of the blocker oligonucleotide will be substantially precluded. This is because the polymerase will not extend the primer toward, and through (in the 5'-3' direction) the blocker - a process that normally results in degradation of the hybridized blocker oligonucleotide.

A particularly preferred blocker/PCR embodiment, for purposes of the present invention and as implemented herein, comprises the use of peptide nucleic acid (PNA) oligomers as blocking oligonucleotides. Such PNA blocker oligomers are ideally suited, because they are neither decomposed nor extended by the polymerase. Preferably, therefore, the base sequence of said *blocking oligonucleotides* is required to comprise a sequence having a length of at least 9 nucleotides which hybridizes to a pretreated nucleic acid sequence according to one of SEQ ID NO: 2 to SEQ ID NO: 5, and sequences complementary thereto, wherein the base sequence of said oligonucleotides comprises at least one CpG, TpG or CpA dinucleotide.

The fragments obtained by means of the amplification can carry a directly or indirectly detectable label. Preferred are labels in the form of fluorescence labels, radionuclides, or detachable molecule fragments having a typical mass that can be detected in a mass spectrometer. Where said labels are mass labels, it is preferred that the labeled amplificates have a single positive or negative net charge, allowing for better detectability in the mass spectrometer. The detection may be carried out and visualized by means of, e.g., matrix assisted laser desorption/ionization mass spectrometry (MALDI) or using electron spray mass spectrometry (ESI).

Matrix Assisted Laser Desorption/Ionization Mass Spectrometry (MALDI-TOF) is a very efficient development for the analysis of biomolecules (Karas and Hillenkamp, Anal Chem., 60:2299-301, 1988). An analyte is embedded in a light-absorbing matrix. The matrix is evaporated by a short laser pulse thus transporting the analyte molecule into the vapor phase in an unfragmented manner. The analyte is ionized by collisions with matrix molecules. An applied voltage accelerates the ions into a field-free flight tube. Due to their different masses, the ions are accelerated at different rates. Smaller ions reach the detector sooner than bigger ones. MALDI-TOF spectrometry is well suited to the analysis of peptides and proteins. The analysis of nucleic acids is somewhat more difficult (Gut and Beck, Current Innovations and Future Trends, 1:147-57, 1995). The sensitivity with respect to nucleic acid analysis is approximately 100-times less than for peptides, and decreases disproportionally with increasing fragment size. Moreover, for nucleic acids having a multiply negatively charged backbone, the ionization process via the matrix is considerably less efficient. In MALDI-TOF spectrometry, the selection of the matrix plays an eminently important role. For desorption of peptides, several very efficient matrixes have been found which produce a very fine crystallization. There are now several responsive matrixes for DNA, however, the difference in sensitivity between peptides and nucleic acids has not been reduced. This difference in sensitivity can be reduced, however, by chemically modifying the DNA in such a manner that it becomes more similar to a peptide. For example, phosphorothioate nucleic acids, in which the usual phosphates of the backbone are substituted with thiophosphates, can be converted into a charge-neutral DNA using simple alkylation chemistry (Gut and Beck, Nucleic Acids Res. 23: 1367-73, 1995). The coupling of a charge tag to this modified DNA results in an increase in MALDI-TOF sensitivity to the same level as that found for peptides. A further advantage of charge tagging is the increased stability of the analysis against impurities, which makes the detection of unmodified substrates considerably more difficult.

In the *fourth step* of the method, the amplificates obtained during the third step of the method are analyzed in order to ascertain the methylation status of the CpG dinucleotides prior to the treatment.

In embodiments where the amplificates were obtained by means of MSP amplification, the presence or absence of an amplificate is in itself indicative of the methylation state of the CpG positions covered by the primer, according to the base sequences of said primer.

Amplificates obtained by means of both standard and methylation specific PCR may be further analyzed by means of hybridization-based methods such as, but not limited to, array technology and probe based technologies as well as by means of techniques such as sequencing and template directed extension.

In one embodiment of the method, the amplificates synthesized in *step* three are subsequently hybridized to an array or a set of oligonucleotides and/or PNA probes. In this context, the hybridization takes place in the following manner: the set of probes used during the hybridization is preferably composed of at least 2 oligonucleotides or PNA-oligomers; in the process, the amplificates serve as probes which hybridize to oligonucleotides previously bonded to a solid phase; the non-hybridized fragments are subsequently removed; said oligonucleotides contain at least one base sequence having a length of at least 9 nucleotides which is reverse complementary or identical to a segment of the base sequences specified in the present Sequence Listing; and the segment comprises at least one CpG , TpG or CpA dinucleotide.

In a preferred embodiment, said dinucleotide is present in the central third of the oligomer. For example, wherein the oligomer comprises one CpG dinucleotide, said dinucleotide is preferably the fifth to ninth nucleotide from the 5'-end of a 13-mer. One oligonucleotide exists for the analysis of each CpG dinucleotide within the sequence according to SEQ ID NO: 1, and the equivalent positions within SEQ ID NO: 2 TO SEQ ID NO: 5. Said oligonucleotides may also be present in the form of peptide nucleic acids. The non-hybridized amplificates are then removed. The hybridized amplificates are then detected. In this context, it is preferred that labels attached to the amplificates are identifiable at each position of the solid phase at which an oligonucleotide sequence is located.

In yet a further embodiment of the method, the genomic methylation status of the CpG positions may be ascertained by means of oligonucleotide probes that are hybridized to the bisulfite treated DNA concurrently with the PCR amplification primers (wherein said primers may either be methylation specific or standard).

A particularly preferred embodiment of this method is the use of fluorescence-based Real Time Quantitative PCR (Heid et al., Genome Res. 6:986-994, 1996; *also* see United States Patent No. 6,331,393) employing a dual-labeled fluorescent oligonucleotide probe (TaqMan™ PCR, using an ABI Prism 7700 Sequence Detection System, Perkin Elmer Applied Biosystems, Foster City, California). The TaqMan™ PCR reaction employs the use of a nonextendible interrogating oligonucleotide, called a TaqMan™ probe, which, in preferred embodiments, is designed to hybridize to a GpC-rich sequence located between the forward and reverse amplification primers. The TaqMan™ probe further comprises a fluorescent reporter moiety and a quencher moiety covalently bound to linker moieties (*e.g.*, phosphoramidites) attached to the nucleotides of the TaqMan™ oligonucleotide. For analysis of methylation within nucleic acids subsequent to bisulfite treatment, it is required that the probe be methylation specific, as described in United States Patent No. 6,331,393, also known as the MethylLight assay. Variations on the TaqMan™ detection methodology that are also suitable for use with the described invention include the use of dual-probe technology (Lightcycler) or fluorescent amplification primers (Sunrise technology). Both these techniques may be adapted in a manner suitable for use with bisulfite treated DNA, and moreover for methylation analysis within CpG dinucleotides.

A further suitable method for the use of probe oligonucleotides for the assessment of methylation by analysis of bisulfite treated nucleic acids In a further preferred embodiment of the method, the *fifth step* of the method comprises the use of template-directed oligonucleotide extension, such as MS-SNuPE as described by Gonzalgo and Jones, Nucleic Acids Res. 25:2529-2531, 1997.

In yet a further embodiment of the method, the *fourth step* of the method comprises sequencing and subsequent sequence analysis of the amplificate generated in the *third step* of the method (Sanger F., et al., Proc Natl Acad Sci USA 74:5463-5467, 1977).

In one preferred embodiment of the method the nucleic acid according to SEQ ID NO: 1, are isolated and treated according to the first three steps of the method outlined above, namely:

a) obtaining, from a subject, a biological sample having subject genomic DNA;
b) extracting or otherwise isolating the genomic DNA; and
c) treating the genomic DNA of b), or a fragment thereof, with one or more reagents to convert cytosine bases that are unmethylated in the 5-position thereof to uracil or to another base that is detectably dissimilar to cytosine in terms of hybridization properties;

and wherein the subsequent amplification of d) is carried out in a methylation specific manner, namely by use of methylation specific primers or *blocking oligonucleotides,* and further wherein the detection of the amplificates is carried out by means of a real-time detection probes, as described above.

Wherein the subsequent amplification of d) is carried out by means of methylation specific primers, as described above, said methylation specific primers comprise a sequence having a length of at least 9 nucleotides which hybridizes to a pretreated nucleic acid sequence according to one of SEQ ID NO: 2 to SEQ ID NO: 5, and sequences complementary thereto, wherein the base sequence of said oligomers comprises at least one CpG dinucleotide.

Step e) of the method, namely the detection of the specific amplificates indicative of the methylation status of one or more CpG positions according to SEQ ID NO: 1 is carried out by means of real-time detection methods as described above.

In an alternative most preferred embodiment of the method the subsequent amplification of d) is carried out in the presence of *blocking oligonucleotides,* as described above. Said *blocking oligonucleotides* comprising a sequence having a length of at least 9 nucleotides which hybridizes to a pretreated nucleic acid sequence according to one of SEQ ID NO: 2 to SEQ ID NO: 5 and sequences complementary thereto, wherein the base sequence of said oligomers comprises at least one CpG, TpG or CpA dinucleotide. Step e) of the method, namely the detection of the specific amplificates indicative of the methylation status of one or more CpG positions according to SEQ ID NO: 1 is carried out by means of real-time detection methods as described above.

In a further preferred embodiment of the method the nucleic acids according to SEQ ID NO: 1 are isolated and treated according to the first three steps of the method outlined above, namely:

a) obtaining, from a subject, a biological sample having subject genomic DNA;
b) extracting or otherwise isolating the genomic DNA;
c) treating the genomic DNA of b), or a fragment thereof, with one or more reagents to convert cytosine bases that are unmethylated in the 5-position thereof to uracil or to another base that is detectably dissimilar to cytosine in terms of hybridization properties; and wherein
d) amplifying subsequent to treatment in c) is carried out in a methylation specific manner, namely by use of methylation specific primers or *blocking oligonucleotides,* and further wherein
e) detecting of the amplificates is carried out by means of a real-time detection probes, as described above.

Wherein the subsequent amplification of c) is carried out by means of methylation specific primers, as described above, said methylation specific primers comprise a sequence having a length of at least 9 nucleotides which hybridizes to a pretreated nucleic acid sequence according to one of SEQ ID NO: 2 to SEQ ID NO: 5 and sequences complementary thereto, wherein the base sequence of said oligomers comprises at least one CpG dinucleotide.

Additional embodiments of the invention provide a method for the analysis of the methylation status of genomic DNA according to the invention (SEQ ID NO: 1, and the complement thereof) without the need for pretreatment.

In the *first step* of such additional embodiments, the genomic DNA sample is isolated from tissue or cellular sources. Preferably, such sources include cell lines, histological slides, paraffin embedded tissues, body fluids, or tissue embedded in paraffin. In the *second step,* the genomic DNA is extracted. Extraction may be by means that are standard to one skilled in the art, including but not limited to the use of detergent lysates, sonification and vortexing with glass beads. Once the nucleic acids have been extracted, the genomic double-stranded DNA is used in the analysis.

In a preferred embodiment, the DNA may be cleaved prior to the treatment, and this may be by any means standard in the state of the art, in particular with methylation-sensitive restriction endonucleases.

In the *third step,* the DNA is then digested with one or more methylation sensitive restriction enzymes. The digestion is carried out such that hydrolysis of the DNA at the restriction site is informative of the methylation status of a specific CpG dinucleotide.

In the *fourth step,* which is optional but a preferred embodiment, the restriction fragments are amplified. This is preferably carried out using a polymerase chain reaction, and said amplificates may carry suitable detectable labels as discussed above, namely fluorophore labels, radionucleotides and mass labels.

In the *fifth step* the amplificates are detected. The detection may be by any means standard in the art, for example, but not limited to, gel electrophoresis analysis, hybridization analysis, incorporation of detectable tags within the PCR products, DNA array analysis, MALDI or ESI analysis.

In the final step of the method the prognosis of the patient is determined. Hypermethylation and over expression of the gene PITX2 and/or genomic sequences thereof according to SEQ ID NO: 1 are associated with negative prognosis. Patients with predicted positive outcome (i.e. hypomethylation or under expression) after said treatment will accordingly have a decreased *absolute* reduction of risk of recurrence and death after treatment with primary or adjuvant treatment. Patients with predicted negative outcome (i.e. hypermethylation or over expression) after said treatment will accordingly have a relatively larger *absolute* reduction of risk of recurrence and death after said treatment. Accordingly patients with a negative outcome will be considered more suitable candidates for aggressive treatment such as chemotherapy or other adjuvant therapies than patients with a positive outcome. Patients with a positive outcome may accordingly be prevented from over prescription of e.g. chemotherapeutic treatment.

The present invention provides novel uses for the genomic sequence of SEQ ID NO:1. Additional embodiments provide modified variants of SEQ ID NO:1, as well as oligonucleotides and/or PNA-oligomers for analysis of cytosine methylation patterns of SEQ ID NO:1.

An objective of the invention comprises analysis of the methylation state of one or more CpG dinucleotides of the gene PITX2, preferably of SEQ ID NO: 1.

The disclosed invention provides treated nucleic acids, derived from genomic SEQ ID NO:1, wherein the treatment is suitable to convert at least one unmethylated cytosine base of the genomic DNA sequence to uracil or another base that is detectably dissimilar to cytosine in terms of hybridization. The genomic sequences in question may comprise one, or more, consecutive or random methylated CpG positions. Said treatment preferably comprises use of a reagent selected from the group consisting of bisulfite, hydrogen sulfite, disulfite, and combinations thereof. In a preferred embodiment of the invention, the objective comprises analysis of a non-naturally occurring modified nucleic acid comprising a sequence of at least 16 contiguous nucleotide bases in length of a sequence selected from the group consisting of SEQ ID NO: 2 TO SEQ ID NO: 5. Particularly preferred is a non-naturally occurring modified nucleic acid comprising a sequence of at least 16 contiguous nucleotide bases in length of a sequence selected from the group consisting of SEQ ID NO: 65 to SEQ ID NO: 320 and SEQ ID NO: 962 to SEQ ID NO: 965 that is not identical to or complementary to SEQ ID NO: 1 to SEQ ID NO: 64 and SEQ ID NO: 961 or other human genomic DNA. Further preferred is a non-naturally occurring modified nucleic acid comprising a sequence of at least 16 contiguous nucleotide bases in length of a sequence selected from the group consisting of SEQ ID Nos: 133,134,261,262, 189,190,317,318, 101,102,229,230,962 - 965 that is not identical to or complementary to SEQ ID Nos: 961, 35, 63 and 19 or other human genomic DNA.

It is further preferred that said sequence comprises at least one CpG, TpA or CpA dinucleotide and sequences complementary thereto. The sequences of SEQ ID NO: 2 TO SEQ ID NO: 5 provide non-naturally occurring modified versions of the nucleic acid according to SEQ ID NO:1. wherein the modification of each genomic sequence results in the synthesis of a nucleic acid having a sequence that is unique and distinct from said genomic sequence as follows. For each sense strand genomic DNA, e.g., SEQ ID NO:1, four converted versions are disclosed. A first version wherein "C" is converted to "T," but "CpG" remains "CpG" (*i.e.*, corresponds to case where, for the genomic sequence, all "C" residues of CpG dinucleotide sequences are methylated and are thus not converted); a second version discloses the complement of the disclosed genomic DNA sequence (*i.e. anti*sense strand), wherein "C" is converted to "T," but "CpG" remains "CpG" (*i.e.*, corresponds to case where, for all "C" residues of CpG dinucleotide sequences are methylated and are thus not converted). The 'upmethylated' converted sequence of SEQ ID NO:1 corresponds to SEQ ID NO:2 to SEQ ID NO:3. A third chemically converted version of each genomic sequences is provided, wherein "C" is converted to "T" for all "C" residues, including those of "CpG" dinucleotide sequences (*i.e.*, corresponds to case where, for the genomic sequences, all "C" residues of CpG dinucleotide sequences are *un*methylated); a final chemically converted version of each sequence, discloses the complement of the disclosed genomic DNA sequence (*i.e.* antisense strand), wherein "C" is converted to "T" for all "C" residues, including those of "CpG" dinucleotide sequences (*i.e.*, corresponds to case where, for the complement (antisense strand) of each genomic sequence, all "C" residues of CpG dinucleotide sequences are *un*methylated). The 'down methylated' converted sequences of SEQ ID NO:1 correspond to SEQ ID NO: 4 to SEQ ID NO: 5.

In an alternative preferred embodiment, such analysis comprises the use of an oligonucleotide or oligomer for detecting the cytosine methylation state within genomic or treated (chemically modified) DNA, according to SEQ ID NO:1 to SEQ ID NO: 5. Said oligonucleotide or oligomer comprising a nucleic acid sequence having a length of at least nine (9) nucleotides which hybridizes, under moderately stringent or stringent conditions (as defined herein above), to a treated nucleic acid sequence according to SEQ ID NO:2 to SEQ ID NO: 5 and/or sequences complementary thereto, or to a genomic sequence according to SEQ ID NO:1 and/or sequences complementary thereto.

Thus, the present invention includes nucleic acid molecules (e.g., oligonucleotides and peptide nucleic acid (PNA) molecules (PNA-oligomers)) that hybridize under moderately stringent and/or stringent hybridization conditions to all or a portion of the sequences SEQ ID NO: 1 to SEQ ID NO: 5 , or to the complements thereof. Particularly preferred is a nucleic acid molecule that hybridizes under moderately stringent and/or stringent hybridization conditions to all or a portion of the sequences SEQ ID NO: 2 to SEQ ID NO: 5 but is not identical to or complementary to SEQ ID NO: 1 or other human genomic DNA. Further preferred is a nucleic acid molecule that hybridizes under moderately stringent and/or stringent hybridization conditions to all or a portion of the sequences SEQ ID NO: 2 to SEQ ID NO: 5 but is not identical to or complementary to SEQ ID NO: 1 or other human genomic DNA.

The hybridizing portion of the hybridizing nucleic acids is typically at least 9, 15, 20, 25, 30 or 35 nucleotides in length. However, longer molecules have inventive utility, and are thus within the scope of the present invention.

Preferably, the hybridizing portion of the inventive hybridizing nucleic acids is at least 95%, or at least 98%, or 100% identical to the sequence, or to a portion thereof of SEQ ID NO: 1 to SEQ ID NO: 5, or to the complements thereof.

Hybridizing nucleic acids of the type described herein can be used, for example, as a primer (e.g., a PCR primer), or a diagnostic and/or prognostic probe or primer. Preferably, hybridization of the oligonucleotide probe to a nucleic acid sample is performed under stringent conditions and the probe is 100% identical to the target sequence. Nucleic acid duplex or hybrid stability is expressed as the melting temperature or Tm, which is the temperature at which a probe dissociates from a target DNA. This melting temperature is used to define the required stringency conditions.

Hybridizing nucleic acids of the type described herein can be used, for example, as a primer (e.g., a PCR primer), or a prognostic probe or primer. Preferably, hybridization of the oligonucleotide probe to a nucleic acid sample is performed under stringent conditions and the probe is 100% identical to the target sequence. Nucleic acid duplex or hybrid stability is expressed as the melting temperature or Tm, which is the temperature at which a probe dissociates from a target DNA. This melting temperature is used to define the required stringency conditions.

For target sequences that are related and substantially identical to the corresponding sequence of SEQ ID NO:1 (such as allelic variants and SNPs), rather than identical, it is useful to first establish the lowest temperature at which only homologous hybridization occurs with a particular concentration of salt (*e.g.*, SSC or SSPE). Then, assuming that 1% mismatching results in a 1°C decrease in the Tm, the temperature of the final wash in the hybridization reaction is reduced accordingly (for example, if sequences having > 95% identity with the probe are sought, the final wash temperature is decreased by 5°C). In practice, the change in Tm can be between 0.5°C and 1.5°C per 1% mismatch.

Examples of inventive oligonucleotides of length X (in nucleotides), as indicated by polynucleotide positions with reference to, e.g., SEQ ID NO:1, include those corresponding to sets (sense and antisense sets) of consecutively overlapping oligonucleotides of length X, where the oligonucleotides within each consecutively overlapping set (corresponding to a given X value) are defined as the finite set of Z oligonucleotides from nucleotide positions:

$$n \text{ to } (n + (X-1));$$

where n=1, 2, 3, ... (Y-(X-1));
where Y equals the length (nucleotides or base pairs) of SEQ ID NO: 1 (28536);
where X equals the common length (in nucleotides) of each oligonucleotide in the set (e.g., X=20 for a set of consecutively overlapping 20-mers); and
where the number (Z) of consecutively overlapping oligomers of length X for a given SEQ ID NO of length Y is equal to Y-(X-1). For example Z= 28536-19= 28517 for either sense or antisense sets of SEQ ID NO:1, where X=20.

Preferably, the set is limited to those oligomers that comprise at least one CpG, TpG or CpA dinucleotide.

Examples of inventive 20-mer oligonucleotides include the following set of oligomers (and the antisense set complementary thereto), indicated by polynucleotide positions with reference to SEQ ID NO:1: 1-20, 2-21, 3-22, 4-23, 5-24, ...... 28517- 28536.

Preferably, the set is limited to those oligomers that comprise at least one CpG, TpG or CpA dinucleotide.

Likewise, examples of inventive 25-mer oligonucleotides include the following set of 2,256 oligomers (and the antisense set complementary thereto), indicated by polynucleotide positions with reference to SEQ ID NO:1:

1-25, 2-26, 3-27, 4-28, 5-29, ...... 28512- 28536.

Preferably, the set is limited to those oligomers that comprise at least one CpG, TpG or CpA dinucleotide.

The present invention encompasses, for SEQ ID NO:1 to SEQ ID NO: 5 multiple consecutively overlapping sets of oligonucleotides or modified oligonucleotides of length X, where, e.g., X= 9, 10, 17, 20, 22, 23, 25, 27, 30 or 35 nucleotides.

The oligonucleotides or oligomers according to the present invention constitute effective tools useful to ascertain genetic and epigenetic parameters of the genomic sequence corresponding to SEQ ID NO:1. Preferred sets of such oligonucleotides or modified oligonucleotides of length X are those consecutively overlapping sets of oligomers corresponding to SEQ ID NO:1 to SEQ ID NO:5 (and to the complements thereof). Preferably, said oligomers comprise at least one CpG, TpG or CpA dinucleotide.

Particularly preferred oligonucleotides or oligomers according to the present invention are those in which the cytosine of the CpG dinucleotide (or of the corresponding converted TpG or CpA dinucleotide) sequences is within the middle third of the oligonucleotide; that is, where the oligonucleotide is, for example, 13 bases in length, the CpG, TpG or CpA dinucleotide is positioned within the fifth to ninth nucleotide from the 5'-end.

The oligonucleotides of the invention can also be modified by chemically linking the oligonucleotide to one or more moieties or conjugates to enhance the activity, stability or detection of the oligonucleotide. Such moieties or conjugates include chromophores, fluorophores, lipids such as cholesterol, cholic acid, thioether, aliphatic chains, phospholipids, polyamines, polyethylene glycol (PEG), palmityl moieties, and others as disclosed in, for example, United States Patent Numbers 5,514,758, 5,565,552, 5,567,810, 5,574,142, 5,585,481, 5,587,371, 5,597,696 and 5,958,773. The probes may also exist in the form of a PNA (peptide nucleic acid) which has particularly preferred pairing properties. Thus, the oligonucleotide may include other appended groups such as peptides, and may include hybridization-triggered cleavage agents (Krol et al., BioTechniques 6:958-976, 1988) or intercalating agents (Zon, Pharm. Res. 5:539-549, 1988). To this end, the oligonucleotide may be conjugated to another molecule, e.g., a chromophore, fluorophor, peptide, hybridization-triggered cross-linking agent, transport agent, hybridization-triggered cleavage agent, etc.

The oligonucleotide may also comprise at least one art-recognized modified sugar and/or base moiety, or may comprise a modified backbone or non-natural internucleoside linkage.

The oligonucleotides or oligomers according to particular embodiments of the present invention are typically used in 'sets,' which contain at least one oligomer for analysis of at least one of the CpG dinucleotides of genomic sequences SEQ ID NO:1 and sequences complementary thereto, or to the corresponding CpG, TpG or CpA dinucleotide within a sequence of the treated nucleic acids according to SEQ ID NO: 2 to SEQ ID NO: 5 and sequences complementary thereto. However, it is anticipated that for economic or other factors it may be preferable to analyze a limited selection of the CpG dinucleotides within said sequences, and the content of the set of oligonucleotides is altered accordingly.

Therefore, in particular embodiments, the present invention provides a set of at least two (2) (oligonucleotides and/or PNA-oligomers) useful for detecting the cytosine methylation state in treated genomic DNA (SEQ ID NO: 2 to SEQ ID NO:5), or in genomic DNA (SEQ ID NO:1 and sequences complementary thereto). These probes enable diagnosis and/or classification of genetic and epigenetic parameters of cell proliferative disorders, most preferably cancer but not breast cancer. The set of oligomers may also be used for detecting single nucleotide polymorphisms (SNPs) in treated genomic DNA (SEQ ID NO: 2 to SEQ ID NO: 5), or in genomic DNA (SEQ ID NO:1 and sequences complementary thereto).

In preferred embodiments, at least one, and more preferably all members of a set of oligonucleotides is bound to a solid phase.

In further embodiments, the present invention provides a set of at least two (2) oligonucleotides that are used as 'primer' oligonucleotides for amplifying DNA sequences of one of SEQ ID NO:1 to SEQ ID NO:5 and sequences complementary thereto, or segments thereof.

It is anticipated that the oligonucleotides may constitute all or part of an "array" or "DNA chip" (*i.e.*, an arrangement of different oligonucleotides and/or PNA-oligomers bound to a solid phase). Such an array of different oligonucleotide- and/or PNA-oligomer sequences can be characterized, for example, in that it is arranged on the solid phase in the form of a rectangular or hexagonal lattice. The solid-phase surface may be composed of silicon, glass, polystyrene, aluminum, steel, iron, copper, nickel, silver, or gold. Nitrocellulose as well as plastics such as nylon, which can exist in the form of pellets or also as resin matrices, may also be used. An overview of the Prior Art in oligomer array manufacturing can be gathered from a special edition of Nature Genetics (Nature Genetics Supplement, Volume 21, January 1999, and from the literature cited therein). Fluorescently labeled probes are often used for the scanning of immobilized DNA arrays. The simple attachment of Cy3 and Cy5 dyes to the 5'-OH of the specific probe are particularly suitable for fluorescence labels. The detection of the fluorescence of the hybridized probes may be carried out, for example, via a confocal microscope. Cy3 and Cy5 dyes, besides many others, are commercially available.

It is also anticipated that the *oligonucleotides,* or particular sequences thereof, may constitute all or part of an "virtual array" wherein the oligonucleotides, or particular sequences thereof, are used, for example, as 'specifiers' as part of, or in combination with a diverse population of unique labeled probes to analyze a complex mixture of analytes. Such a method, for example is described in US 2003/0013091 (United States serial number 09/898,743, published 16 January 2003). In such methods, enough labels are generated so that each nucleic acid in the complex mixture (*i.e.*, each analyte) can be uniquely bound by a unique label and thus detected (each label is directly counted, resulting in a digital read-out of each molecular species in the mixture).

It is particularly preferred that the oligomers according to the invention are utilized for at least one of: prognosis of; treatment of; monitoring of; and treatment and monitoring of cell proliferative disorders, most preferably cancer but not breast cancer. This is enabled by use of said sets for providing a prognosis of a biological sample isolated from a patient. Particularly preferred are those sets of oligomer that comprise at least two oligonucleotides selected from one of the following sets of oligonucleotides.

In one embodiment of the method, this is achieved by analysis of the methylation status of at least one target sequence comprising, or hybridizing under stringent conditions to at least 16 contiguous nucleotides of the gene PITX2 and/or regulatory regions thereof.

The present invention further provides a method for ascertaining genetic and/or epigenetic parameters of the genomic sequences according to SEQ ID NO:1 within a subject by analyzing cytosine methylation and single nucleotide polymorphisms. In a preferred embodiment the present invention further provides a method for ascertaining genetic and/or epigenetic parameters of the genomic sequences according to SEQ ID NO:1 within a subject by analyzing cytosine methylation and single nucleotide polymorphisms. Said method comprising contacting a nucleic acid comprising SEQ ID NO:1 in a biological sample obtained from said subject with at least one reagent or a series of reagents, wherein said reagent or series of reagents, distinguishes between methylated and non-methylated CpG dinucleotides within the target nucleic acid.

Preferably, said method comprises the following steps: In the *first step,* a sample of the tissue to be analyzed is obtained. The source may be any suitable source. Preferably, the source of the DNA sample is selected from the group consisting of cells or cell lines, histological slides, biopsies, paraffin-embedded tissue, bodily fluids, ejaculate, urine, blood, and combinations thereof. Preferably, the source is biopsies, bodily fluids, ejaculate, urine, or blood.

The genomic DNA is then isolated from the sample. Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants e.g. by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA.

Once the nucleic acids have been extracted, the genomic double stranded DNA is used in the analysis.

In the *second step* of the method, the genomic DNA sample is treated in such a manner that cytosine bases which are unmethylated at the 5'-position are converted to uracil, thymine, or another base which is dissimilar to cytosine in terms of hybridization behavior. This will be understood as 'pretreatment' or 'treatment' herein.

The above-described treatment of genomic DNA is preferably carried out with bisulfite (hydrogen sulfite, disulfite) and subsequent alkaline hydrolysis which results in a conversion of non-methylated cytosine nucleobases to uracil or to another base which is dissimilar to cytosine in terms of base pairing behavior.

In the *third step* of the method, fragments of the treated DNA are amplified, using sets of primer oligonucleotides according to the present invention, and an amplification enzyme. The amplification of several DNA segments can be carried out simultaneously in one and the same reaction vessel. Typically, the amplification is carried out using a polymerase chain reaction (PCR). The set of primer oligonucleotides includes at least two oligonucleotides whose sequences are each reverse complementary, identical, or hybridize under stringent or highly stringent conditions to an at least 16-base-pair long segment of the base sequences of one of SEQ ID NO: 2 to SEQ ID NO: 5 (preferably one of SEQ ID Nos: 133,134,261,262, 189,190,317,318, 101,102,229,230 and most preferably one of SEQ ID Nos: 962 - 965) and sequences complementary thereto.

In an alternate embodiment of the method, the methylation status of preselected CpG positions within the nucleic acid sequences comprising one or more of SEQ ID NO:1 may be detected by use of methylation-specific primer oligonucleotides. This technique (MSP) has been described in United States Patent No. 6,265,171 to Herman. The use of methylation status specific primers for the amplification of bisulfite treated DNA allows the differentiation between methylated and unmethylated nucleic acids. MSP primers pairs contain at least one primer which hybridizes to a bisulfite treated CpG dinucleotide. Therefore, the sequence of said primers comprises at least one CpG dinucleotide. MSP primers specific for non-methylated DNA contain a "T' at the position of the C position in the CpG. Preferably, therefore, the base sequence of said primers is required to comprise a sequence having a length of at least 9 nucleotides which hybridizes to a treated nucleic acid sequence according to one of SEQ ID NO:2 to SEQ ID NO:5 and sequences complementary thereto, wherein the base sequence of said oligomers comprises at least one CpG dinucleotide.

A further preferred embodiment of the method comprises the use of *blocker* oligonucleotides. The use of such blocker oligonucleotides has been described by Yu et al., BioTechniques 23:714-720. 1997. Blocking probe oligonucleotides are hybridized to the bisulfite treated nucleic acid concurrently with the PCR primers. PCR amplification of the nucleic acid is terminated at the 5' position of the blocking probe, such that amplification of a nucleic acid is suppressed where the complementary sequence to the blocking probe is present. The probes may be designed to hybridize to the bisulfite treated nucleic acid in a methylation status specific manner. For example, for detection of methylated nucleic acids within a population of unmethylated nucleic acids, suppression of the amplification of nucleic acids which are unmethylated at the position in question would be carried out by the use of blocking probes comprising a 'CpA' or 'TpA' at the position in question, as opposed to a 'CpG' if the suppression of amplification of methylated nucleic acids is desired.

For PCR methods using blocker oligonucleotides, efficient disruption of polymerase-mediated amplification requires that blocker oligonucleotides not be elongated by the polymerase. Preferably, this is achieved through the use of blockers that are 3'-deoxyoligonucleotides, or oligonucleotides derivatized at the 3' position with other than a "free" hydroxyl group. For example, 3'-O-acetyl oligonucleotides are representative of a preferred class of blocker molecule.

Additionally, polymerase-mediated decomposition of the blocker oligonucleotides should be precluded. Preferably, such preclusion comprises either use of a polymerase lacking 5'-3' exonuclease activity, or use of modified blocker oligonucleotides having, for example, thioate bridges at the 5'-terminii thereof that render the blocker molecule nuclease-resistant. Particular applications may not require such 5' modifications of the blocker. For example, if the blocker- and primer-binding sites overlap, thereby precluding binding of the primer (*e.g.*, with excess blocker), degradation of the blocker oligonucleotide will be substantially precluded. This is because the polymerase will not extend the primer toward, and through (in the 5'-3' direction) the blocker-a process that normally results in degradation of the hybridized blocker oligonucleotide.

A particularly preferred blocker/PCR embodiment, for purposes of the present invention and as implemented herein, comprises the use of peptide nucleic acid (PNA) oligomers as blocking. oligonucleotides. Such PNA blocker oligomers are ideally suited, because they are neither decomposed nor extended by the polymerase.

Preferably, therefore, the base sequence of said *blocking oligonucleotides* is required to comprise a sequence having a length of at least 9 nucleotides which hybridizes to a treated nucleic acid sequence according to one of SEQ ID NO: 2 to SEQ ID NO: 5 and sequences complementary thereto, wherein the base sequence of said oligonucleotides comprises at least one CpG, TpG or CpA dinucleotide. More preferably the base sequence of said *blocking oligonucleotides* is required to comprise a sequence having a length of at least 9 nucleotides which hybridizes to a treated nucleic acid sequence according to one of preferably SEQ ID NO:2 to SEQ ID NO:5 and sequences complementary thereto, wherein the base sequence of said oligonucleotides comprises at least one CpG, TpG or CpA dinucleotide.

The fragments obtained by means of the amplification can carry a directly or indirectly detectable label. Preferred are labels in the form of fluorescence labels, radionuclides, or detachable molecule fragments having a typical mass which can be detected in a mass spectrometer. Where said labels are mass labels, it is preferred that the labeled amplificates have a single positive or negative net charge, allowing for better detectability in the mass spectrometer. The detection may be carried out and visualized by means of, e.g., matrix assisted laser desorption/ionization mass spectrometry (MALDI) or using electron spray mass spectrometry (ESI).

Matrix Assisted Laser Desorption/Ionization Mass Spectrometry (MALDI-TOF) is a very efficient development for the analysis of biomolecules (Karas & Hillenkamp, Anal Chem., 60:2299-301, 1988). An analyte is embedded in a light-absorbing matrix. The matrix is evaporated by a short laser pulse thus transporting the analyte molecule into the vapor phase in an unfragmented manner. The analyte is ionized by collisions with matrix molecules. An applied voltage accelerates the ions into a field-free flight tube. Due to their different masses, the ions are accelerated at different rates. Smaller ions reach the detector sooner than bigger ones. MALDI-TOF spectrometry is well suited to the analysis of peptides and proteins. The analysis of nucleic acids is somewhat more difficult (Gut & Beck, Current Innovations and Future Trends, 1:147-57, 1995). The sensitivity with respect to nucleic acid analysis is approximately 100-times less than for peptides, and decreases disproportionately with increasing fragment size. Moreover, for nucleic acids having a multiply negatively charged backbone, the ionization process via the matrix is considerably less efficient. In MALDI-TOF spectrometry, the selection of the matrix plays an eminently important role. For desorption of peptides, several very efficient matrixes have been found which produce a very fine crystallization. There are now several responsive matrixes for DNA, however, the difference in sensitivity between peptides and nucleic acids has not been reduced. This difference in sensitivity can be reduced, however, by chemically modifying the DNA in such a manner that it becomes more similar to a peptide. For example, phosphorothioate nucleic acids, in which the usual phosphates of the backbone are substituted with thiophosphates, can be converted into a charge-neutral DNA using simple alkylation chemistry (Gut & Beck, Nucleic Acids Res. 23: 1367-73, 1995). The coupling of a charge tag to this modified DNA results in an increase in MALDI-TOF sensitivity to the same level as that found for peptides. A further advantage of charge tagging is the increased stability of the analysis against impurities, which makes the detection of unmodified substrates considerably more difficult.

In the *fourth step* of the method, the amplificates obtained during the third step of the method are analyzed in order to ascertain the methylation status of the CpG dinucleotides prior to the treatment.

In embodiments where the amplificates were obtained by means of MSP amplification, the presence or absence of an amplificate is in itself indicative of the methylation state of the CpG positions covered by the primer, according to the base sequences of said primer.

Amplificates obtained by means of both standard and methylation specific PCR may be further analyzed by means of hybridization-based methods such as, but not limited to, array technology and probe based technologies as well as by means of techniques such as sequencing and template directed extension.

In one embodiment of the method, the amplificates synthesized in *step three* are subsequently hybridized to an array or a set of oligonucleotides and/or PNA probes. In this context, the hybridization takes place in the following manner: the set of probes used during the hybridization is preferably composed of at least 2 oligonucleotides or PNA-oligomers; in the process, the amplificates serve as probes which hybridize to oligonucleotides previously bonded to a solid phase; the non-hybridized fragments are subsequently removed; said oligonucleotides contain at least one base sequence having a length of at least 9 nucleotides which is reverse complementary or identical to a segment of the base sequences specified in the present Sequence Listing; and the segment comprises at least one CpG , TpG or CpA dinucleotide

In a preferred embodiment, said dinucleotide is present in the central third of the oligomer. For example, wherein the oligomer comprises one CpG dinucleotide, said dinucleotide is preferably the fifth to ninth nucleotide from the 5'-end of a 13-mer. One oligonucleotide exists for the analysis of each CpG dinucleotide within the sequence according to SEQ ID NO:1, and the equivalent positions within SEQ ID NO: 2 to SEQ ID NO: 5. Said oligonucleotides may also be present in the form of peptide nucleic acids. The non-hybridized amplificates are then removed. The hybridized amplificates are then detected. In this context, it is preferred that labels attached to the amplificates are identifiable at each position of the solid phase at which an oligonucleotide sequence is located.

In yet a further embodiment of the method, the genomic methylation status of the CpG positions may be ascertained by means of oligonucleotide probes that are hybridized to the bisulfite treated DNA concurrently with the PCR amplification primers (wherein said primers may either be methylation specific or standard).

A particularly preferred embodiment of this method is the use of fluorescence-based Real Time Quantitative PCR (Heid et al., Genome Res. 6:986-994, 1996; *also* see United States Patent No. 6,331,393) employing a dual-labeled fluorescent oligonucleotide probe (TaqMan™ PCR, using an ABI Prism 7700 Sequence Detection System, Perkin Elmer Applied Biosystems, Foster City, California). The TaqMan™ PCR reaction employs the use of a nonextendible interrogating oligonucleotide, called a TaqMan™ probe, which, in preferred embodiments, is designed to hybridize to a GpC-rich sequence located between the forward and reverse amplification primers. The TaqMan™ probe further comprises a fluorescent "reporter moiety" and a "quencher moiety" covalently bound to linker moieties (e.g., phosphoramidites) attached to the nucleotides of the TaqMan™ oligonucleotide. For analysis of methylation within nucleic acids subsequent to bisulfite treatment, it is required that the probe be methylation specific, as described in United States Patent No. 6,331,393, also known as the MethylLight™ assay. Variations on the TaqMan™ detection methodology that are also suitable for use with the described invention include the use of dual-probe technology (Lightcycler™) or fluorescent amplification primers (Sunrise™ technology). Both these techniques may be adapted in a manner suitable for use with bisulfite treated DNA, and moreover for methylation analysis within CpG dinucleotides.

A further suitable method for the use of probe oligonucleotides for the assessment of methylation by analysis of bisulfite treated nucleic acids In a further preferred embodiment of the method, the *fifth step* of the method comprises the use of template-directed oligonucleotide extension, such as MS-SNuPE as described by Gonzalgo & Jones, Nucleic Acids Res. 25:2529-2531, 1997.

In yet a further embodiment of the method, the *fourth step* of the method comprises sequencing and subsequent sequence analysis of the amplificate generated in the *third step* of the method (Sanger F., et al., Proc Natl Acad Sci USA 74:5463-5467, 1977).

In the most preferred embodiment of the method the genomic nucleic acids are isolated and treated according to the first three steps of the method outlined above, namely:

a) obtaining, from a subject, a biological sample having subject genomic DNA;
b) extracting or otherwise isolating the genomic DNA;
c) treating the genomic DNA of b), or a fragment thereof, with one or more reagents to convert cytosine bases that are unmethylated in the 5-position thereof to uracil or to another base that is detectably dissimilar to cytosine in terms of hybridization properties; and wherein
d) amplifying subsequent to treatment in c) is carried out in a methylation specific manner, namely by use of methylation specific primers or *blocking oligonucleotides,* and further wherein

e) detecting of the amplificates is carried out by means of a real-time detection probe, as described above.

Preferably, where the subsequent amplification of d) is carried out by means of methylation specific primers, as described above, said methylation specific primers comprise a sequence having a length of at least 9 nucleotides which hybridizes to a treated nucleic acid sequence according to one of SEQ ID NO: 2 to SEQ ID NO: 5 and sequences complementary thereto, wherein the base sequence of said oligomers comprises at least one CpG dinucleotide. More preferably, where the subsequent amplification of d) is carried out by means of methylation specific primers, as described above, said methylation specific primers comprise a sequence having a length of at least 9 nucleotides which hybridizes to a treated nucleic acid sequence according to one of SEQ ID NO:2 to SEQ ID NO:5 and sequences complementary thereto, wherein the base sequence of said oligomers comprises at least one CpG dinucleotide.

In an alternative most preferred embodiment of the method, the subsequent amplification of d) is carried out in the presence of *blocking oligonucleotides,* as described above. Said *blocking oligonucleotides* comprising a sequence having a length of at least 9 nucleotides which hybridizes to a treated nucleic acid sequence according to one of SEQ ID NO:2 to SEQ ID NO:5 and sequences complementary thereto, wherein the base sequence of said oligomers comprises at least one CpG. TpG or CpA dinucleotide. Preferably said *blocking oligonucleotides* comprising a sequence having a length of at least 9 nucleotides which hybridizes to a treated nucleic acid sequence according to one of SEQ ID NO:2 to SEQ ID NO:5 and sequences complementary thereto, wherein the base sequence of said oligomers comprises at least one CpG, TpG or CpA dinucleotide.

Step e) of the method, namely the detection of the specific amplificates indicative of the methylation status of one or more CpG positions according to SEQ ID NO:1 is carried out by means of real-time detection methods as described above.

Additional embodiments of the invention provide a method for the analysis of the methylation status of genomic DNA according to the invention SEQ ID NO: 1 and complements thereof without the need for pretreatment.

In the *first step* of such additional embodiments, the genomic DNA sample is isolated from tissue or cellular sources. Preferably, such sources include cell lines, histological slides, body fluids, or tissue embedded in paraffin. In the *second step,* the genomic DNA is extracted. Extraction may be by means that are standard to one skilled in the art, including but not limited to the use of detergent lysates, sonification and vortexing with glass beads. Once the nucleic acids have been extracted, the genomic doublestranded DNA is used in the analysis.

In a preferred embodiment, the DNA may be cleaved prior to the treatment, and this may be by any means standard in the state of the art, in particular with methylation-sensitive restriction endonucleases.

In the *third step,* the DNA is then digested with one or more methylation sensitive restriction enzymes. The digestion is carried out such that hydrolysis of the DNA at the restriction site is informative of the methylation status of a specific CpG dinucleotide.

In the *fourth step,* which is optional but a preferred embodiment, the restriction fragments are amplified. This is preferably carried out using a polymerase chain reaction, and said amplificates may carry suitable detectable labels as discussed above, namely fluorophore labels, radionuclides and mass labels.

In the *fifth step* the amplificates are detected. The detection may be by any means standard in the art, for example, but not limited to, gel electrophoresis analysis, hybridization analysis, incorporation of detectable tags within the PCR products, DNA array analysis, MALDI or ESI analysis.

Subsequent to the determination of the methylation state of the genomic nucleic acids the prognosis of the cancer is deduced based upon the methylation state of at least one CpG dinucleotide sequence of SEQ ID NO:1, or an average, or a value reflecting an average methylation state of a plurality of CpG dinucleotide sequences of SEQ ID NO:1. Preferably said prognosis is based upon the methylation state of at least one CpG dinucleotide sequence of the gene PITX2, or an average, or a value reflecting an average methylation state of a plurality of CpG dinucleotide sequences of SEQ ID NO:1. Hypermethylation of said CpG positions are associated with good prognosis, and hypomethylation is associated with poor prognosis. The cut-off point for determining said hypo and hyper methylation is may be the median methylation level for a given population, or is preferably an optimized cut-off level. For the analysis of PITX2 it is preferred that the cut-off is between 20% and 10% methylation, and most preferably 14.27%. Wherein the methods according to the present invention of expression analysis (most preferably by means of methylation analysis), of the herein described marker are used to determine the prognosis of a cancer patient said methods are preferably used in combination with other clinical prognostic variables used to determine prognosis (i.e. that said variables are factored in or taken into account).

## Kits

Moreover, an additional aspect of the present invention is a kit comprising, for example: a bisulfite-containing reagent; a set of primer oligonucleotides containing at least two oligonucleotides whose sequences in each case correspond, are complementary, or hybridize under stringent or highly stringent conditions to a 16-base long segment of the sequences SEQ ID NO:1 to SEQ ID NO: 5; oligonucleotides and/or PNA-oligomers; as well as instructions for carrying out and evaluating the described method. In a further preferred embodiment, said kit may further comprise standard reagents for performing a CpG position-specific methylation analysis, wherein said analysis comprises one or more of the following techniques: MS-SNuPE, MSP, MethyLight™, HeavyMethyl™, COBRA, and nucleic acid sequencing. However, a kit along the lines of the present invention can also contain only part of the aforementioned components.

Preferably said kit comprises a bisulfite-containing reagent; a set of primer oligonucleotides containing at least two oligonucleotides whose sequences in each case correspond, are complementary, or hybridize under stringent or highly stringent conditions to a 16-base long segment of the sequences SEQ ID NO:2 to SEQ ID NO:5; oligonucleotides and/or PNA-oligomers; as well as instructions for carrying out and evaluating the described method In a further preferred embodiment, said kit may further comprise standard reagents for performing a CpG position-specific methylation analysis, wherein said analysis comprises one or more of the following techniques: MS-SNuPE, MSP, MethyLight™, HeavyMethyl™, COBRA, and nucleic acid sequencing. However, a kit along the lines of the present invention can also contain only part of the aforementioned components.

The described invention further provides a composition of matter useful for providing a prognosis of cancer patients. Said composition comprising at least one nucleic acid 18 base pairs in length of a segment of a nucleic acid sequence selected from the group consisting SEQ ID NO:2 to SEQ ID NO:5, and one or more substances taken from the group comprising : magnesium chloride, dNTP, Taq polymerase, bovine serum albumen, an oligomer in particular an oligonucleotide or peptide nucleic acid (PNA)-oligomer, said oligomer comprising in each case at least one base sequence having a length of at least 9 nucleotides which is complementary to, or hybridizes under moderately stringent or stringent conditions to a pretreated genomic DNA according to one of the SEQ ID NO:2 to SEQ ID NO:5 and sequences complementary thereto. It is preferred that said composition of matter comprises a buffer solution appropriate for the stabilization of said nucleic acid in an aqueous solution and enabling polymerase based reactions within said solution. Suitable buffers are known in the art and commercially available.

While the present invention has been described with specificity in accordance with certain of its preferred embodiments, the following EXAMPLES and TABLES serve only to illustrate the invention and are not intended to limit the invention within the principles and scope of the broadest interpretations and equivalent configurations thereof.

**TABLES 1-5**

Table 1: Results of the Cox regression analysis for PITX2 according to Example 1. Using stepwise regression the marker remains in the model. P-values refer to the null-hypothesis "hazard ratio equals zero".

| Variable | P value | Hazard Ratio | Lower Confidence Interval | Upper Confidence Interval |
|---|---|---|---|---|
| PITX2 | 0.0043 | 2.222 | 1.284 | 3.845 |
| Disease stage | 0.0692 | 1.713 | 0.965 | 3.061 |
| Gleason category | 0.0107 | 1.798 | 1.146 | 2.821 |
| PSA | 0.075 | 1.254 | 0.977 | 1.609 |
| Nomogram category | 0.0866 | 2.187 | 0.894 | 5.353 |

Table 2: Components for all QM assays according to Example 1

| **Component** | **Company** | **Stock conc.** |
|---|---|---|
| Reaction buffer ROX | Eurogentec | 10x |
| MgCl2 | Eurogentec | 50mM |
| DNTPs | MBI | 25mM each |
| Forward primer | TIB Molbiol | 6,25μM |
| Reverse primer | TIB Molbiol | 6,25μM |
| cg Probe | Eurogentec | 4μM |

(continued)

| Component | Company | Stock conc. |
|---|---|---|
| tg Probe | Eurogentec | 4μM |
| HotGoldStar-Taq | Eurogentec | 5U/μl |
| Water | Fluka | |

Table 3A: Optimized Reaction conditions for all QM assays according to Example 1

| Gene | dNTPs | Buffer | $MgCl_2$ | Primers | Probes | Taq | Baseline | Threshold | Annealing |
|---|---|---|---|---|---|---|---|---|---|
| PITX2 | 250μM | 1x | 3mM | 625 nM | 200 nM | 1U | 3/23 | 0,05 | 62°C |
| | | | | | | | | | |
| | | | | | | | | | |
| | | | | | | | | | |
| | | | | | | | | | |
| | | | | | | | | | |

Table 3B: Cycle program for QM assays according to Example 1. For annealing temperatures, see Table 3A.

| | T [°C] | t | Cycles |
|---|---|---|---|
| Initial denat. | 95.0 | 10min | |
| Denaturation | 95.0 | 15sec | 45x (PITX2 50x) |
| Annealing | Variable | 60sec | |

Table 4: Clinical characteristics of the patient population according to Example 1. Age is given as the mean, and all other variables are given as the number of patients. Not all information was available for all patients.

| Clinical Variable | | Baylor | Stanford | VMMC | Total |
|---|---|---|---|---|---|
| Age (mean) | | 61.1 | 61.7 | 61.1 | 61.3 |
| PSA | 0-4 | 25 | 33 | 18 | 76 |
| | 4-10 | 120 | 139 | 99 | 358 |
| | >10 | 60 | 72 | 30 | 162 |
| Gleason Score | 5-6 | 137 | 164 | 118 | 419 |
| | 7 | 37 | 44 | 19 | 100 |
| | 8-10 | 26 | 31 | 25 | 82 |
| Stage | Organ-confined | 110 | 211 | 113 | 434 |
| | Not organ-confined | 94 | 33 | 35 | 162 |
| PSA-based recurrence | | 22 | 10 | 13 | 45 |
| Decision to treat based recurrence | | 3 | 14 | 4 | 21 |
| Total Samples | | 206 | 244 | 162 | 612 |

**Example 1**

The aim of the investigation was to confirm the significance of the gene PITX as a prognostic marker and to optimize methylation cut-offs. It was decided to investigate prostate cancer. The marker should be suitable to split patients who undergo prostatectomy into two groups: one with a high chance of PSA recurrence and one with a low chance of PSA recurrence. In addition, the markers should provide additional information to Gleason grade analysis. A marker meeting these criteria will have an important clinical role in selection of prostatectomy patients for adjuvant therapy. It was decided to undertake the analysis by means of methylation analysis on a real-time platform (QM Assay). The QM assay (= Quantitative Methylation Assay) is a Real-time PCR based method for quantitative DNA methylation detection. The assay principle is based on non-methylation specific amplification of the target region and a methylation specific detection by competitive hybridization of two different probes specific for the CG or the TG status, respectively. For the present study, TaqMan probes were used that were labeled with two different fluorescence dyes ("FAM" for CG specific probes, "VIC" for TG specific probes) and were further modified by a quencher molecule ("TAMRA" or "Minor Groove Binder/non-fluorescent quencher"). Evaluation of the QM assay raw data is possible by measuring absolute fluorescence intensities (FI) in the logarithmic phase of amplification.

The assay was used to analyze the methylation levels of 612 paraffin embedded prostatectomy samples from a cohort of node-negative patients from three institutions.

The primary aim of the invention was to provide a marker that can differentiate between patients with low chance for PSA recurrence after surgery and those with a high chance for PSA recurrence. The performance of these markers as compared to traditional prognostic indicators such as Gleason grading and stage information is also provided.

It is a further aim of the present invention to determine where the marker is most informative in relation to current clinical prognostic assessment and accordingly provide particularly preferred use embodiments of the present invention. It is particularly preferred that a molecular test according to the present invention is combined, either formally or informally, with information from other prognostic sources, and in the case of prostate cancer particular Gleason grading.

Methods: QM Assay Description

The QM-assay was developed to enhance performance without drastically altering standard conditions in order to allow future multiplexing. Primer and probe concentrations, $MgCl_2$ concentration and annealing temperature were optimized under fixed buffer and polymerase conditions. The assay was designed and optimized to ensure quantitative methylation analysis of between 10 and 100 percent methylation. The assay products were checked on an agarose gel and no undesired products were detected. The results of the optimization procedure are shown in Tables 2 and 3.

Oligonucleotide sequences:

| | | | |
|---|---|---|---|
| Forward primer | gtaggggagggaagtagatgtt | | |
| Reverse primer | ttctaatcctcctttccacaataa | | |
| CG-probe | agtcggagtcgggagagcga | Label 5- FAM | Label 3'-TAMRA |
| TG-probe | agttggagttgggagagtgaaaggaga | Label 5- VIC | Label 3'-TAMRA |

Sample Set

Paraffin-embedded prostatectomy tissue samples from 612 patients were analyzed, see Table 4. The samples were provided by the Baylor College of Medicine SPORE, Stanford University Department of Urology, and Virginia Mason Hospital in Seattle. The samples from Stanford and Virginia Mason were prepared by first finding the surgical block with the highest percent tumor, then sectioning the block. Three tubes were prepared, each with three 10 micron thick sections. The procedure was slightly different at Baylor. A core of tissue was removed from the tumor within the prostatectomy block, and then this core was cut into 10 micron sections. Ten sections were included into each of three tubes.

An adjacent section was mounted on a slide and H&E stained for histological analysis. A pathologist reviewed these slides for an independent determination of Gleason grading and percent tumor. The Gleason results were used for all analyses in this report. The original provider Gleason values are available, but they were not used for analysis due to known and hypothetical biases among the providers. Stanford, for instance, uses a percentage Gleason 4/5 for reporting grade, while the other two providers use the traditional system. The measured Gleason values provided an independent and uniform measurement.

A few samples were found to have no tumor cells on the H&E slide, and these patients were omitted from the analysis. In addition, we found a few patients that did not have a PSA nadir after surgery. These patients were also excluded from the study. In total, 612 patients were included in the data analysis.

Due to their coring technique, the percent tumor of the samples provided by Baylor were higher than the other providers.

All patients, aged 40-80, undergoing surgery at the three institutions during certain years were included in the study, with the exception of patients who received neo-adjuvant or adjuvant therapy (before PSA rise) and patients with positive nodes at the time of surgery. For Baylor, the time period was 1993-1998, for Virginia Mason it was 1996-2000, and for Stanford it was 1996-1999.

The overall cohort is similar to other prostatectomy cohorts described in the literature, such as the cohort collected by William Catalona and described in 2004 (Roehl et al.). The patient cohorts from each provider are similar for nearly all clinical parameters. One exception is the type of recurrence. While other institutions typically wait until the patient's PSA rises to 0.2ng/ml or higher after surgery, the Stanford Department of Urology treats many patients when their PSA rises to 0.05. Therefore, Stanford has a higher rate of recurrence based on the decision to treat criteria and a lower rate of recurrence based on the PSA level (0.2ng/ml) criteria. See section 6.1 for a summary of the event definition criteria.

Figure 1 provides a histogram of follow-up times for the patient cohort (all three providers included). The white bars consist of the patients who did not have a recurrence before they were censored, and the shaded bars consist of the patients who experienced recurrence. By selecting patients who received surgery from 1993-2000, we have ensured that the median follow-up time of the cohort (66 months) is long enough to have a significant number of patients who have relapsed.

For deparaffination, the 627 provided PET samples were processed directly in the tube in which they were delivered by the providers. One ml (Virginia Mason and Baylor) or 1.8 ml (Stanford) of limonene was added to each tube and incubated at room temperature for 10 minutes in a thermomixer with occasional vortexing. The samples were centrifuged at 16,000 x g for 5 minutes. The limonene supernatant was removed, and if no pellet was detected, centrifugation was repeated at higher speed and the remaining limonene was removed. For samples from Stanford, the deparaffination process was repeated once with 1.6 ml of limonene to get rid of residual paraffin.

For lysis of the tissue, 190 μl lysis buffer and 20 μl proteinase K was added to each deparaffinated sample. For Stanford samples, 570 μl lysis buffer and 60 μl proteinase K was used. After vortexing, samples were centrifuged briefly and incubated on a thermoshaker at 60°C for 40 hours. After the incubation, samples were checked to ensure that lysis was complete, and the proteinase was then inactivated at 95°C for 10 minutes. If the lysed samples were not directly used for DNA extraction, they were stored at -20°C.

The lysates were randomized based on the sample provider and PSA recurrence. The DNA was isolated using a QIAGEN DNeasy Tissue kit with a few modifications. 400 μl buffer AUE was distributed to collection tubes and 200 μl of lysate were added. The samples were mixed by shaking for 15 seconds. The lysate/buffer mixtures were applied to the 96-well DNeasy plate columns. The plate was sealed and centrifuged at 5790xg for 10 minutes. The columns were washed once with 500 μl of AW1 and then 500 μl AW2. The DNA was eluted with 120 μl buffer AE. Therefore, the final volume of extracted DNA was approximately 120μl. The DNA was stored at -20°C.

Bisulfite Treatment

The CFF real-time PCR assay was used to quantify the DNA concentration of the samples after extraction. CFF sequence:

**TAAGAGTAATAATGGATGGATGATGGATAGATGAATGGATGAAGAAAGAAAGGATGAGTGAGAGAAAGGAAGGGAGATGGGAGG (84bp)**

| | |
|---|---|
| CFF-Forward primer | TAAGAGTAATAATGGATGGATGATG |
| CFF-Reverse primer | CCTCCCATCTCCCTTCC |
| CFF TaqMan probe | ATGGATGAAGAAAGAAAGGATGAGT |

The inventors adjusted the concentration of each genomic DNA sample so that 1ug of CFF1 measured DNA was present in 44 μl. The bisulfite treatment of genomic DNA derived from paraffin embedded tissue was performed using a 96 well protocol. Forty-four μl genomic DNA (with approximately 1μg of amplifiable DNA), 83 μl 4.9M bisulfite solution (pH 5.45-5.5), and 13 μL DME solution were pipetted into the wells of the plate. The samples were thoroughly mixed then placed in a thermocycler with the following program:

- 5:00 min denaturation of DNA at 99°C
- 22:00 min incubation at 60°C
- 3:00 min denaturation of DNA at 99°C
- 1:27:00 hours incubation at 60°C
- 3:00 min denaturation of DNA at 99°C
- 2:57:00 hours incubation at 60°C
- Cooling at 20°C

After the incubations, each sample was divided into two 70 μL aliquots. Each aliquot was combined with 280 μL of prepared Buffer AVL/Carrier RNA and 280 μL ethanol. The wells were sealed and the samples were mixed vigorously for 15 seconds. The plate was incubated for 10 minutes at room temperature. The first aliquot was applied to the QIAamp 96 plate and the plate was centrifuged for four minutes at 5790 x g. The process was repeated with the second aliquot so that both aliquots were applied to the same binding column. The columns were washed with 500 μL buffer AW1, then 500 μL 0.2 M NaOH, and then twice with 500 μL buffer AW2. The DNA was eluted with 100 μL elution buffer (Qiagen) pre-heated to 70 deg C. The bisDNAs were stored at -20°C.

The bisulfite treated DNA samples were stored in 8 x 96 well plates (plate 01-08). The samples and controls were combined onto two 384-well PCR reaction plates for each QM assay. Each QM assay plate contained the samples of 4 x 96 well plates (85 wells actually used per plate) and 1 x96 well plate with standard DNA (7 mixtures of the calibration DNA and water for the no template control PCR reaction). The QM assay plates were run three times.

The 384-well PCR plates were pipetted with the TECAN workstation. The pipetting program transferred first 10 μl of the mastermix and then 10 μl of the respective DNA into the designated well. The master mix was pipetted in a falcon tube and distributed to 8 x 500 μl screw cap vials for automatic pipetting with TECAN workstation.

All QM assays were run on an ABI TAQMAN 7900HT real-time device (SDS 2.2. software) with a reaction volume of 20 μl. PITX2 and CCND2 assays were run with 9600 emulation, and the other assays were not. An automatic sample setup was used to transfer the correct sample names and detector/reporter dyes to the TAQMAN software. The cycling conditions were manually adjusted and ROX was used as passive reference dye. All 384 well PCR plates we analyzed with the SDS2.2 software using the manual analysis settings (baseline setting with start and stop values and manual threshold) to produce results files for each run individually.

Methods: Evaluation of Marker Performance

<u>Definition of Events</u>

After a successful prostatectomy on a patient with non-metastatic disease, there should be no prostate cells left in his body and therefore his PSA levels should drop to zero. A patient's PSA levels are typically measured every 6-12 months after surgery to ensure that the patient remains free of prostate cancer. If PSA becomes detectable and rises to a certain level, the doctor and patient may decide on additional therapy. Therefore, the return and rise of PSA levels are the primary indication of disease recurrence.

A post-surgical PSA relapse is typically indicated by either a gradual or rapid rise in levels over a series of sequential tests. Depending on the clinical characteristics of the patient or the approach of the institution, patients may be treated as soon as PSA is detected, when it reaches a certain threshold, or when clinical symptoms accompany the PSA rise. Most institutions consider a PSA level of 0.2 ng/ml to be significant, and if a patient's PSA reaches this level and is confirmed to be rising in subsequent tests, he will be offered additional therapy. Stanford Department of Urology, one of the sample providers, considers 0.05 ng/ml to be a PSA recurrence, and considers treatment for patients when their PSA reaches this level.

An event in this study includes all PSA-based recurrences. A PSA level of 0.2ng/ml, confirmed in subsequent tests, has been demonstrated to provide the best sensitivity and specificity for detection of recurrence (Freedland et al. 2003). Rise of PSA to this level normally precedes any development of clinical recurrence; therefore, nearly all of the patients in this study are free of clinical recurrence at the time of PSA recurrence. Because Stanford often treats patients with PSA recurrence before they reach this cut-off of 0.2ng/ml, many of their recurrence patients would be censored in the present study if the PSA level of 0.2ng/ml was the only considered event. Therefore, patients from any of the three institutions who receive therapy due to PSA levels are also considered an event in this study.

To summarize, an event is defined in the present study as any rise in PSA to 0.2ng/ml (confirmed in subsequent test) OR a decision to treat the patient based on PSA criteria.

Raw QM Data Processing

All analyses in this report are based on the CT evaluation. Assuming optimal real-time PCR conditions in the exponential amplification phase, the concentration of methylated DNA ($C_{meth}$) can be determined by

$$C_{meth} = \frac{100}{1 + 2^{(CT_{CG} - CT_{TG})}} [\%],$$

where

$CT_{CG}$ denotes the threshold cycle of the CG reporter (FAM channel) and
$CT_{TG}$ denotes the threshold cycle of the TG reporter (VIC channel).

The thresholds for the cycles were determined by visual inspection of the amplification plots (ABI PRISM 7900 HT Sequence Detection System User Guide). The values for the cycles *($CT_{CG}$* and *$CT_{TG}$)* were calculated with these thresholds by the ABI 7900 software. Whenever the amplification curve did not exceed the threshold, the value of the cycle was set to the maximum cycle e.g. 50.

The R software package, version 2.2. (Gentleman and Ihaka 1997), was used for the statistical analysis. In addition, we used the "survival" package, version 2.11-5 (http://cran.at.r-project.org/src/contrib/Descriptions/survival.html), for survival analysis.

Proprietary code was used for k-fold-cross validation, ROC analysis and plot functions.

Each dataset is represented in a proprietary data object, called. "Annotated Data Matrix" (ADM). This data object contains the measurements after quality control and averaging, as well as all necessary annotations for the samples and assays.

QM Assay calibration curves

A series of mixtures of methylated MDA-DNA and unmethylated MDA-DNA, ranging from 0 to 100 percent methylated, were included in triplicate on each QM PCR plate. These DNAs were used to ensure uniform QM assay performance on all PCR plates. All assays showed strong quantitative abilities between 10 and 100%, and some assays were able to consistently distinguish 5% methylated DNA from unmethylated DNA.

Statistical Methods

After quality control, each assay was statistically analyzed.

Cox Regression

The relation between recurrence-free survival times (RFS) and covariates were analyzed using Cox Proportional Hazard models (Cox and Oates 1984; Harrel 2001).

The hazard, i.e. the instantaneous risk of a relapse, is modeled as

$$h(t \mid x) = h_0(t) \cdot \exp(\beta x) \qquad (3)$$

and

$$h(t \mid x_1,\ldots,x_k) = h_0(t)\cdot\exp(\beta_1 x_1 + \ldots + \beta_k x_k) \qquad (4)$$

for univariate and multiple regression analyses, respectively, where t is the time measured in months after surgery, $h_0(t)$ is the (unspecified) baseline hazard, $x_i$ are the covariates (e.g. measurements of the assays) and $\beta_i$ are the regression coefficients (parameters of the model). $\beta_i$ will be estimated by maximizing the partial likelihood of the Cox Proportional Hazard model

Likelihood ratio tests are performed to test whether methylation is related to the hazard. The difference between 2Log(Likelihood) of the full model and the null-model is approximately □$^2$-distributed with k degrees of freedom under the null hypotheses $□_1$, = ... = $□_k$=0.

The assumption of proportional hazards were evaluated by scaled Schoenfeld residuals (Thernau and Grambsch 2000). For the calculation, analysis and diagnostics of the Cox Proportional Hazard Model the R functions "coxph" and "coxph.zph" of the "survival" package are used.

Stepwise Regression Analysis

For multiple Cox regression models a stepwise procedure (Venables and Ripley 1999; Harrel 2001) was used in order to find sub-models including only relevant variables. Two effects are usually achieved by these procedures:

- Variables (methylation rates) that are basically unrelated to the dependent variable (DFS/MFS) are excluded as they do not add relevant information to the model.
- Out of a set of highly correlated variables, only the one with the best relation to the dependent variable is retained.

Inclusion of both types of variables can lead to numerical instabilities and a loss of power. Moreover, the predictor's performance can be low due to over-fitting.

The applied algorithm aims at minimizing the Akaike information criterion (AIC), which is defined as

$$AIC = -2□\text{maximized log-likelihood} + 2□\#\text{parameters}.$$

The AIC is related to the performance of a model, smaller values promise better performance. Whereas the inclusion of additional variables always improves the model fit and thus increases the likelihood, the second term penalizes the estimation of additional parameters. The best model will present a compromise model with good fit and usually a small or moderate number of variables. Stepwise regression calculation with AIC are done with the R function "step".

Kaplan-Meier Survival Curves and Log-Rank Tests

Survival curves were estimated from RFS data using Kaplan-Meier estimator for survival (Kaplan and Meier, 1958). Log-rank tests (Cox and Oates 1984) are used to test for differences of two survival curves, e.g. survival in hyper- vs. hypomethylated groups. In addition, a variant of the Log-rank test usually referred to as the Generalized Wilcoxon test was applied (for description see Hosmer and Lemeshow 1999). For the Kaplan-Meier analysis the functions "survfit" and "survdiff" of the "survival" package are used.

Independence of single markers and marker panels from other covariates

To check whether the present markers give additional and independent information, other relevant clinical factors were included in the Cox Proportional Hazard model and the p-values for the weights for every factor were calculated (Wald-Test) (Themau et al. 2000). For the analysis of additional factors in the Cox Proportional Hazard model, the R function "coxph" is used.

Density Estimation

For numerical variables, kernel density estimation was performed with a Gaussian kernel and variable bandwidth. The bandwidth is determined using Silverman's "rule-of-thumb" (Silverman 1986). For the calculation of the densities the R function "density" is used.

Analysis of Sensitivity and Specificity

The method of calculating sensitivity and specificity using the Bayes-formula was based on the Kaplan-Meier estimates (Heagerty et al. 2000) for the survival probabilities in the marker positive and marker negative groups for a given time $T_{Threshold}$. The ROCs were calculated for different reference times $T_{Threshold}$ (3 year, 4 years, 5 years , 6 years).

k-fold Crossvalidation

For the analysis of model selection and model robustness k-fold crossvalidation (Hastie et al. 2001) was used. The set of observations is randomly split into k chunks. In turn, every chunk was used as a test set, whereas the remaining k-1 chunks constitute the training set. This procedure is repeated m times.

Results

The 605 samples were processed as described above. All samples were analyzed by the QM assays with three replicates. The data was filtered for quality control, and analyzed as described in the methods section. The clinical performance of each marker is summarized below and the Kaplan-Meier survival curves and ROC curves according to figure 2. P-values for comparison of survival curves reported in the graphs are based on the ordinary Log-rank test. The results of using the Generalized Wilcoxon test are essentially the same (data not shown).

The performance of the markers was first examined using the median methylation level as a cut-off. Since this cut-off was fixed before looking at the data, the p values can be used to judge the performance of the markers. Any marker with a significant p value using the median methylation as a cut-off is considered to be validated. The median methylation level might not be the best cut-off for all markers, and for these markers the prognostic separation can be further optimized by choosing the methylation cut-off that results in the lowest p value. Since the cut-off is optimized specifically for p value, the p value no longer can be used to indicate statistical significance.

For judging the significance of the marker performance using the median methylation as a cut-off, we used a p value of 0.005 (assuming correction for 10 markers and panels). Based on p-value and event separation, PITX2 is a strong candidate.

Figure 2 A shows the Kaplan-Meier survival analysis of the PITX2 marker of the 585 patient samples that passed the quality control filter using the optimized methylation cut-off value (13.5%). Figure 2B shows the Kaplan-Meier survival analysis of the PITX2 marker using the predefined median methylation value as a cut-off, the p-value was 0.000017. Figure 2C shows the ROC curve analysis of the PITX2 marker after 5 years of follow-up. The median methylation cut-off is marked as a triangle, and the optimized methylation cut-off is shown as a diamond. The AUC was 0.64.

Several clinical prognostic factors are commonly used for assessing prostate cancer. Histological analysis of the tumor with quantification of the tumor differentiation state using the Gleason grading system is a particularly important prognostic indicator in current clinical practice. The analysis was continued by determining whether the markers could improve Gleason analysis by subdividing patients within a Gleason category. We also investigated whether the markers could add information to other prognostic indicators, such as nomogram risk estimation (Han et al. 2003) and disease stage.

For these analyses, we used Kaplan-Meier analysis to determine whether the PITX2 is still informative on population sub-groups, and Cox regression analysis to determine whether the markers provide information independent of the prognostic clinical variables. Gleason score was divided into three groups (6 or lower, 7, and 8 through 10), stage was divided into two groups (T2/organ-confined and T3/non-organ confined), PSA was divided into four groups (0 to 4 ng/ml, 4 to 10 ng/ml, 10 to 20 ng/ml, and greater than 20 ng/ml), and nomogram estimation of 5 year PSA-free survival was divided into two groups (90 to 100% and 0 to 89%).

With Cox regression modeling, PITX2 is a valuable prognostic marker independent of other clinical prognostic information (Table 1). In other words, PITX2 methylation adds more information to Gleason than either PSA or disease stage. The hazard ratio for PITX2 is 2.2. In the survival analysis of sub-groups, PITX2 has the potential to be a significant marker for all prostate cancer patients.

It is particularly interesting to see strong separation within the patient sub-group with organ-confined disease (Figure 96). Patients with organ-confined disease (T2) should be cured by surgery. Those that are not cured by surgery must have had some cells leave the prostate before surgery, and therefore had tumor cells with aggressive characteristics early in the development. PITX2 can separate the T2 group into a hypomethylated group with a very small chance for recurrence (~5%) and a hyper-methylated group with a prognosis more like T3 patients.

Figure 3 shows the survival analysis of PITX2 performance on sub-populations based on stage. The upper left plot shows the performance of disease stage as a prognostic marker. The upper right plot shows the performance of PITX2 on pT2 patients. The lower left plot shows the performance of PITX2 on pT3 patients.

PITX2 is also capable of stratifying patients within Gleason sub-categories. Figure 4 shows that survival analysis on low Gleason patients (Score 5 or 6) and high Gleason patients (Score 8, 9, or 10) results in low p values. Patients with high Gleason scores are currently candidates for clinical trials on post-surgical adjuvant therapies. But the PITX2 values suggest that this is not a uniform group. PITX2 hypomethylated, high Gleason patients have 85% probability of disease free survival at ten years, while hypermethylated high Gleason patients have a very low chance (~35%). These patients with high likelihood for disease recurrence are the patients who should be selected for adjuvant therapy or clinical trials.

Figure 4 shows the survival analysis of PITX2 performance on sub-populations based on Gleason score categories. The upper left plot shows the performance of Gleason score as a prognostic marker. Gleason 5 and 6 patients are in light grey, Gleason 7 patients are in dark-grey, and Gleason 8, 9, and 10 patients are in black. The upper right plot shows the performance of PITX2 on Gleason 5 and 6 patients. The lower left plot shows the performance of PITX2 on Gleason 7 patients. The lower right plot shows the performance of PITX2 on Gleason 8, 9, and 10 patients.

Prostate cancer nomograms are created based on large cohorts of patients. They mathematically combine information from stage, Gleason, and pre-operative PSA levels into one prognostic indicator. As Figure 5 shows, the nomogram by itself is very strong. But PITX2 is capable of further sub-dividing the patients.

Figure 5 shows the survival analysis of PITX2 performance on sub-populations based on nomogram risk estimation. The upper left plot shows the performance of the nomogram as a prognostic marker. The upper right plot shows the performance of PITX2 on patients with a 90% chance of 5-year PSA-free survival according to the nomogram. The lower left plot shows the performance of PITX2 on patients with less than 90% chance of 5-year PSA-free survival according to the nomogram.

PITX2 shows significant prognostic information when the median methylation level is used as a cut-off. Setting the methylation cut-off even higher than the median improves the performance. This has the effect of decreasing the marker positive group and increasing the specificity of the test.

The patients whose samples were analyzed in this study are representative of the population who would be targeted for a prostatectomy test. Therefore, it is possible to speculate on the information these markers could provide for future patients. PITX2, for example, has a sensitivity of around 60% and a specificity of 70%. In the Kaplan-Meier analysis in Figure 2, the marker positive group has approximately three times the risk of recurrence after ten years that the marker negative group has. In Figure 4, Gleason 8-10 patients that are positive for PITX2 have a 65% chance for PSA recurrence in 10 years. In contrast, the Gleason 8-10 patients who were marker negative had only a 15% chance of PSA relapse. The addition of the methylation marker information to the Gleason stratification will allow clinicians to identify a poor prognosis sub-group who can most benefit from adjuvant therapy. If the marker is incorporated into the patient selection procedure for adjuvant therapy clinical trials, clinicians may begin to see a clear benefit to the addition of early adjuvant treatments for poor prognosis patients.

In addition to adding information to Gleason, PITX2 can also stratify patients with organ-confined disease. Patients with disease that is truly confined to the organ will be cured by complete removal of the organ. Patients with disease that appears to be confined to the organ, but have undetected micrometastases, will not be cured by surgery. These two groups of patients, both with small operable lesions, have tumors with very different capacities for metastases. PITX2 seems to be detecting these underlying differences in basic tumor aggressiveness. The ability of PITX2 to add information to currently, used markers is essential. Gleason and staging already provide significant prognostic information, a new test that would not replace but complement these traditional sources of information is both more valuable and more likely to be readily adopted in clinical practice.

In the analysis on sub-groups of patients, the marker often seemed strongest on patients with poor prognosis based on traditional clinical variables. Gleason 8-10 patients and patients with low nomogram probability for PSA free survival are well stratified by the present marker into good and poor prognosis groups. For a prostatectomy test, these are the ideal patients to target, since the test would be used to select a group of poor prognosis patients who can most benefit from adjuvant therapy. Overall, this analysis demonstrates that the PITX2 marker is especially well suited for identifying poor prognosis patients.

## Claims

**1.** A method for providing a prognosis of a subject with a prostate cancer comprising the follow-ing steps of:

a) in a biological sample obtained from said subject,
b) determining the methylation status of the gene PITX2 in said sample, and
c) determining therefrom the prognosis of said subject, wherein optionally at least one further prognostic variable is factored in when said prognosis is determined, and wherein hypermethylation is indicative of a negative prognosis, and
d) optionally, determining a suitable treatment for said subject.

**2.** The method according to claim 1, wherein said prognosis is determined in terms of at least one of the group consisting of overall patient survival, disease- or relapse-free survival, tumor-related complications and rate of progression of tumor.

**3.** The method according to any of claims 1 to 2, wherein said sample is selected from the group consisting of cells or cell lines, histological slides, biopsies, paraffin-embedded tissue, bodily fluids, ejaculate, urine, blood, sputum, stool, tissues for example but not limited to those from colon, prostate, lung or liver, and combinations thereof.

**4.** The method according to claims 1 to 3, comprising contacting genomic DNA isolated from a biological sample obtained from the subject with at least one reagent, or series of reagents that distinguishes between methylated and non-methylated CpG dinucleotides within at least one target region of the genomic DNA, wherein the target region comprises, or hybridizes under stringent conditions to a sequence of at least 16 contiguous nucleotides of the gene PITX2 and/or regulatory regions thereof, wherein said contiguous nucleotides comprise at least one CpG dinucleotide sequence, and whereby providing a prognosis of cell proliferative disorders, most preferably cancer but not breast cancer is, at least in part, afforded.

**5.** The method according to claim 4, comprising the following steps of:

a) isolating genomic DNA from a biological sample taken from said subject;
b) treating said genomic DNA, or a fragment thereof, with one or more reagents to convert 5-position unmethylated cytosine bases to uracil or to another base that is detectably dissimilar to cytosine in terms of hybridization properties;
c) contacting said treated genomic DNA, or the treated fragment thereof, with an amplification enzyme and at least two primers comprising, in each case a contiguous sequence at least 18 nucleotides in length that is complementary to, or hybridizes under moderately stringent or stringent conditions to a sequence selected from the group consisting of SEQ ID NO: 2 to SEQ ID NO: 5 and complements thereof, wherein the treated DNA or a fragment thereof is either amplified to produce one or more amplificates, or is not amplified;
d) determining, based on the presence or absence of, or on the quantity or on a property of said amplificate, the methylation state of at least one CpG dinucleotide sequence of the gene PITX2 or an average, or a value reflecting an average methylation state of a plurality of CpG dinucleotide sequences of the gene PITX2, and
e) determining from said methylation state as determined the prognosis of said subject.

**6.** Use of the method according to any of claims 1 to 5, for providing the prognosis of a subject with prostate cancer.

## Patentansprüche

**1.** Verfahren zum Bereitstellen einer Prognose eines Probanden mit einem Prostatakrebs, welches die folgenden Schritte umfasst:

a) in einer biologischen Probe, welche von einem Probanden erhalten wurde,
b) Ermitteln des Methylierungszustands des PITX2-Gens in der Probe, und
c) Ermitteln der Prognose des Probanden daraus, wobei optional, wenn die Prognose ermittelt wird, mindestens eine weitere prognostische Variable mit einbezogen wird, und wobei Hypermethylierung indikativ für eine negative Prognose ist, und
d) optional, Ermitteln einer geeigneten Behandlung für den Probanden.

**2.** Verfahren gemäß Patentanspruch 1, wobei die Prognose bezogen auf mindestens eines aus der Gruppe, bestehend aus Gesamtpatientenüberlebensdauer, krankheitsfreies oder rückfallfreies Überleben, Tumor-bezogene Komplikationen und Geschwindigkeit des Fortschreitens des Tumors, ermittelt wird.

**3.** Verfahren gemäß einem der Patentansprüche 1 bis 2, wobei die Probe aus der Gruppe ausgewählt ist, welche aus Zellen oder Zelllinien, histologischen Schnitten, Biopsien, in Paraffin eingebettetem Gewebe, Körperflüssigkeiten, Ejakulat, Urin, Blut, Sputum, Stuhl, Gewebe, zum Beispiel aber nicht beschränkt auf solches vom Darm, von der Prostata, der Lunge oder der Leber und Kombinationen davon, besteht.

**4.** Verfahren gemäß den Patentansprüchen 1 bis 3, umfassend In-Kontakt-Bringen von genomischer DNA, die aus einer biologischen Probe isoliert wurde, welche von einem Probanden erhalten wurde, mit mindestens einem Reagenz oder einer Reihe von Reagenzien, das/die zwischen methylierten und nicht-methylierten CpG-Dinukleotiden innerhalb mindestens einer Zielregion der genomischen DNA unterscheidet, wobei die Zielregion eine Sequenz von mindestens 16 zusammenhängenden Nukleotiden des PITX2-Gens und/oder regulatorischen Regionen davon umfasst oder unter stringenten Bedingungen daran hybridisiert, wobei die zusammenhängenden Nukleotide mindestens eine CpG-Dinukleotidsequenz umfassen, wodurch das Bereitstellen einer Prognose von Zellproliferationserkrankungen, am meisten bevorzugt Krebs aber kein Brustkrebs, zumindest teilsweise, ermöglicht wird.

**5.** Verfahren gemäß Patentanspruch 4 umfassend die folgenden Schritte:

a) Isolieren genomischer DNA aus einer biologischen Probe, die von einem Probanden genommen wurde;
b) Behandeln der genomischen DNA oder eines Fragments davon mit einem oder mehreren Reagenzien, um Cytosinbasen, die in ihrer 5-Position unmethyliert sind, zu Uracil oder zu einer anderen Base umzuwandeln, die im Hinblick auf ihre Hybridisierungseigenschaften nachweisbar unterschiedlich zu Cytosin ist;
c) In-Kontakt-Bringen der behandelten genomischen DNA oder des behandelten Fragments derselben mit einem Amplifikationsenzym und mindestens zwei Primem, die in jedem Fall eine zusammenhängende Sequenz von mindestens 18 Nukleotiden Länge umfassen, die zu einer Sequenz, welche aus der Gruppe ausgewählt ist, die aus SEQ ID NO: 2 bis SEQ ID NO: 5 und Komplementen davon besteht, komplementär ist oder unter moderat stringenten oder stringenten Bedingungen daran hybridisiert, wobei die behandelte DNA oder ein Fragment derselben entweder amplifiziert wird, um ein oder mehrere Amplifikate zu erzeugen oder nicht amplifiziert wird;
d) Bestimmen des Methylierungszustands von wenigstens einer CpG-Dinukleotidsequenz des PITX2-Gens oder von einem Durchschnittswert oder von einem Wert, der einen durchschnittlichen Methylierungszustand einer Vielzahl von CpG-Dinukleotidsequenzen des PITX2-Gens reflektiert, auf der Grundlage der Anwesenheit oder Abwesenheit oder Menge oder einer Eigenschaft des Amplifikats, und
e) Bestimmen der Prognose des Probanden auf der Basis des ermittelten Methylierungszustands.

**6.** Verwendung des Verfahrens gemäß einem der Patentansprüche 1 bis 5 zum Bereitstellen der Prognose eines Probanden mit Prostatakrebs.

## Revendications

**1.** Procédé pour procurer un pronostic d'un sujet vis-à-vis d'un cancer de la prostate, comprenant les étapes suivantes de:

a) dans un échantillon biologique obtenu à partir dudit sujet,
b) détermination de l'état de méthylation du gène PITX2 dans ledit échantillon, et
c) détermination à partir de cela du pronostic dudit sujet, tandis qu'en option au moins un autre pronostic variable est pris en compte lorsque ledit pronostic est déterminé, et tandis que l'hyperméthylation est indicative d'un pronostic négatif, et
d) en option, détermination d'un traitement approprié pour ledit sujet.

**2.** Procédé selon la revendication 1, dans lequel ledit pronostic est déterminé en termes d'au moins l'un de ceux du groupe consistant en survie complète du patient, survie sans maladie ou rechute, complications liées à une tumeur et taux de progression de tumeur.

**3.** Procédé selon l'une quelconque des revendications 1 à 2, dans lequel ledit échantillon est choisi dans le groupe consistant en cellules ou lignées cellulaires, lames histologiques, biopsies, tissu enrobé de paraffine, fluides corporels, éjaculat, urine, sang, crachat, selles, tissus tels que, mais sans que cela soit limitatif, ceux provenant du côlon, de la prostate, des poumons ou du foie, et leurs combinaisons.

**4.** Procédé selon les revendications 1 à 3, comprenant la mise en contact d'ADN génomique isolé à partir d'un échantillon biologique obtenu à partir du sujet, avec au moins un réactif, ou une série de réactifs qui distingue entre des dinucléotides CpG méthylés et non-méthylés dans au moins une région cible de l'ADN génomique, tandis que la région cible comprend, ou hybride dans des conditions stringentes à une séquence d'au moins 16 nucléotides contigus du gène PITX2 et/ou des régions régulatrices de celui-ci, tandis que lesdits nucléotides contigus comprennent au moins une séquence dinucléotidique CpG, et tandis que le fait de procurer ainsi un pronostic de troubles de prolifération des cellules, établit, au moins en partie, le plus préférablement un cancer, mais pas un cancer du sein.

**5.** Procédé selon la revendication 4, comprenant les étapes suivantes de:

a) isolement d'ADN génomique à partir d'un échantillon biologique prélevé à partir dudit sujet;
b) traitement dudit ADN génomique, ou d'un fragment de celui-ci, avec un ou plusieurs réactifs pour convertir les bases cytosine non-méthylées en position 5 en uracile ou en une autre base qui n'est pas similaire à la cytosine de manière détectable en termes de propriétés d'hybridation;
c) mise en contact dudit ADN génomique traité, ou du fragment traité de celui-ci, avec une enzyme d'amplification et au moins deux amorces comprenant, dans chaque cas une séquence contiguë d'au moins 18 nucléotides de long qui est complémentaire de, ou hybride dans des conditions modérément stringentes ou stringentes à une séquence choisie dans le groupe consistant en SEQ ID NO: 2 à SEQ ID NO: 5 et leurs compléments, tandis que l'ADN traité ou un fragment de celui-ci est soit amplifié pour produire un ou plusieurs produits d'amplification, ou n'est pas amplifié;
d) détermination, sur la base de la présence ou de l'absence dudit produit d'amplification, ou sur la base de la quantité ou sur la base d'une propriété dudit produit d'amplification, de l'état de méthylation d'au moins une séquence dinucléotidique CpG du gène PITX2 ou d'une moyenne ou d'une valeur reflétant un état de méthylation moyen d'une pluralité de séquences dinucléotidiques CpG du gène PITX2, et
e) détermination, à partir dudit état de méthylation tel que déterminé, du pronostic dudit sujet.

**6.** Utilisation du procédé selon l'une quelconque des revendications 1 à 5, pour procurer le pronostic d'un sujet ayant un cancer de la prostate.

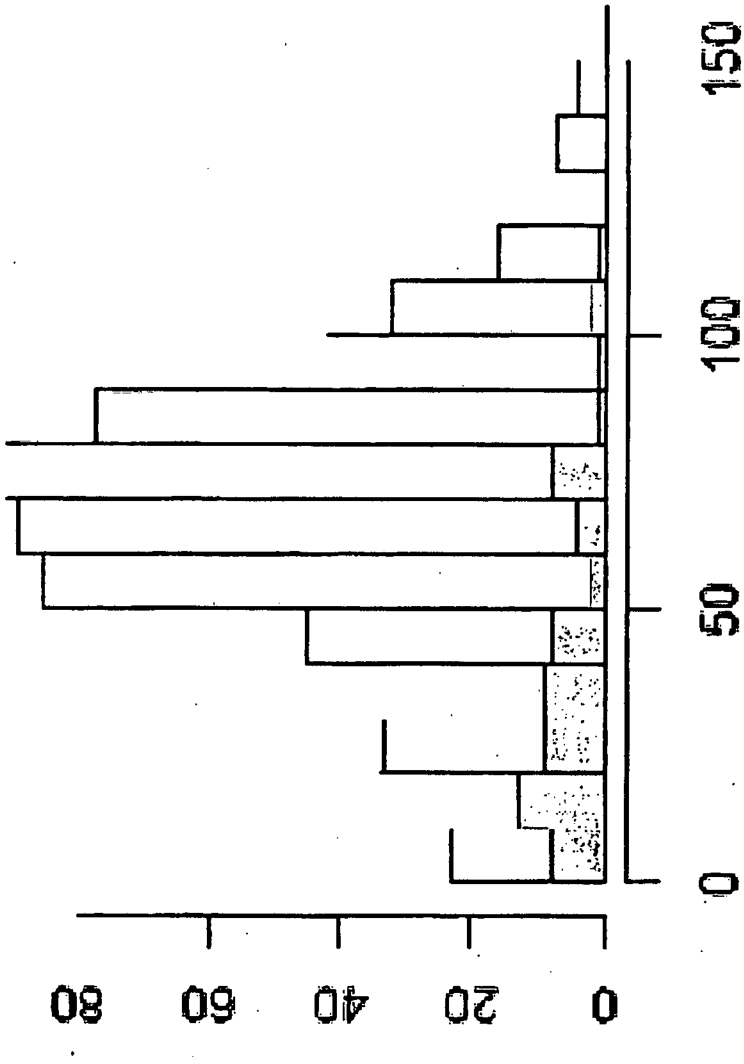


Figure 1

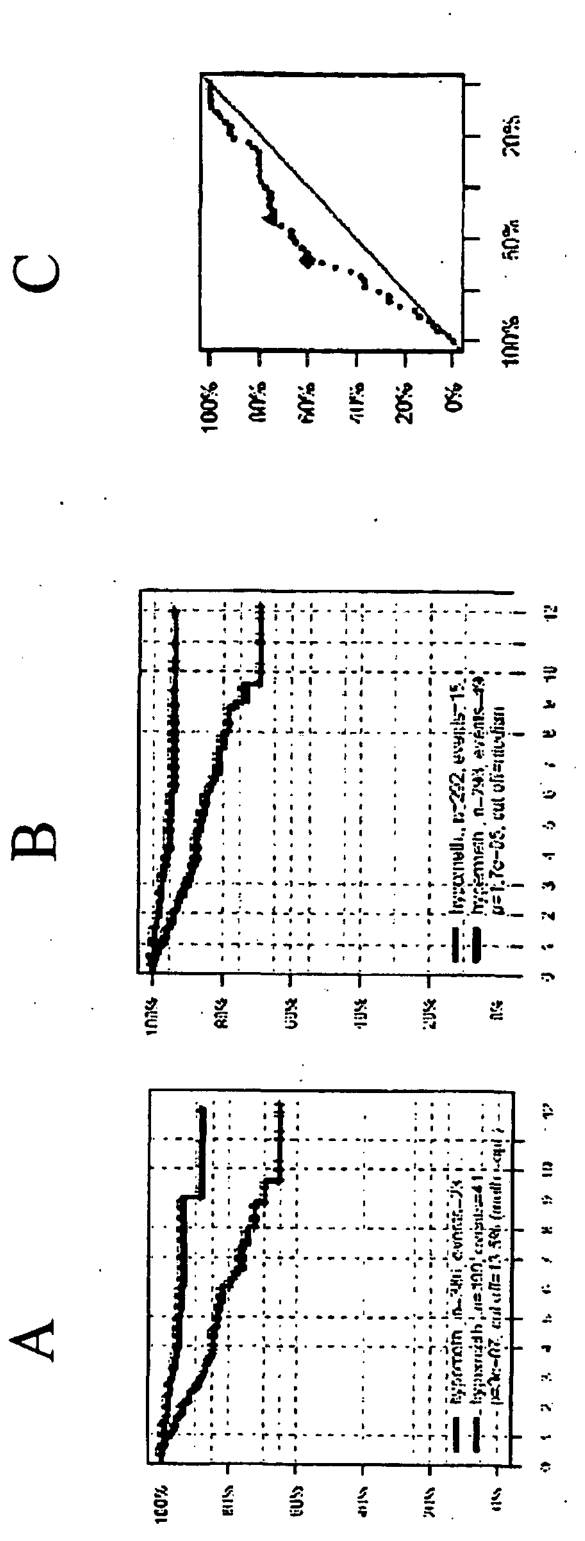


Figure 2

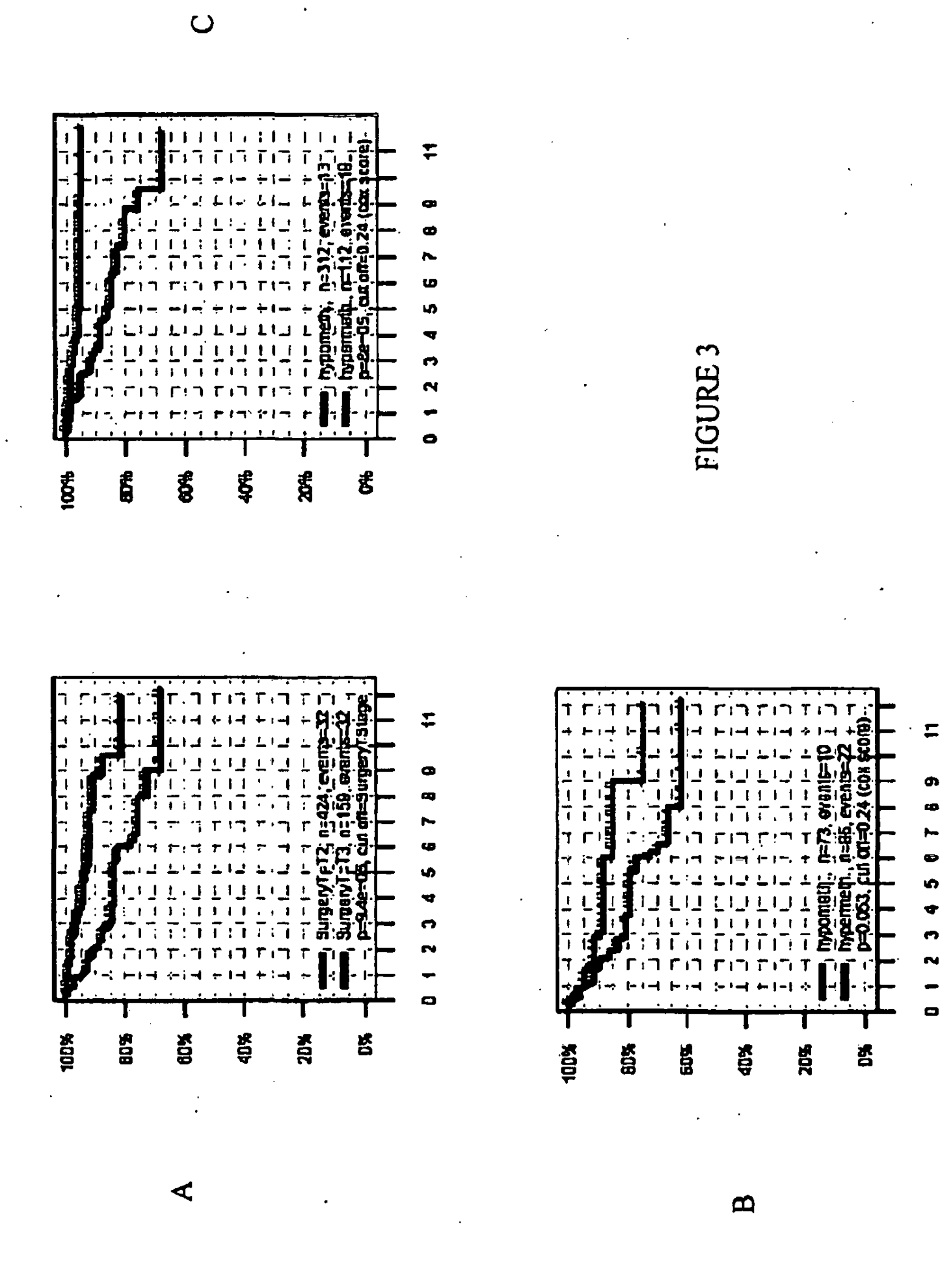
A
SurgeryT=T2, n=424, events=32
SurgeryT=T3, n=159, events=32
p=9.4e-08, cut off=Surgery/TStage
B
hypometh., n=73, events=10
hypermeth., n=85, events=22
p=0.053, cut off=0.24 (cox score)
C
hypometh., n=312, events=13
hypermeth., n=112, events=18
p=2e-05, cut off=0.24 (cox score)
100%
80%
60%
40%
20%
0%
0 1 2 3 4 5 6 7 8 9 11

FIGURE 3

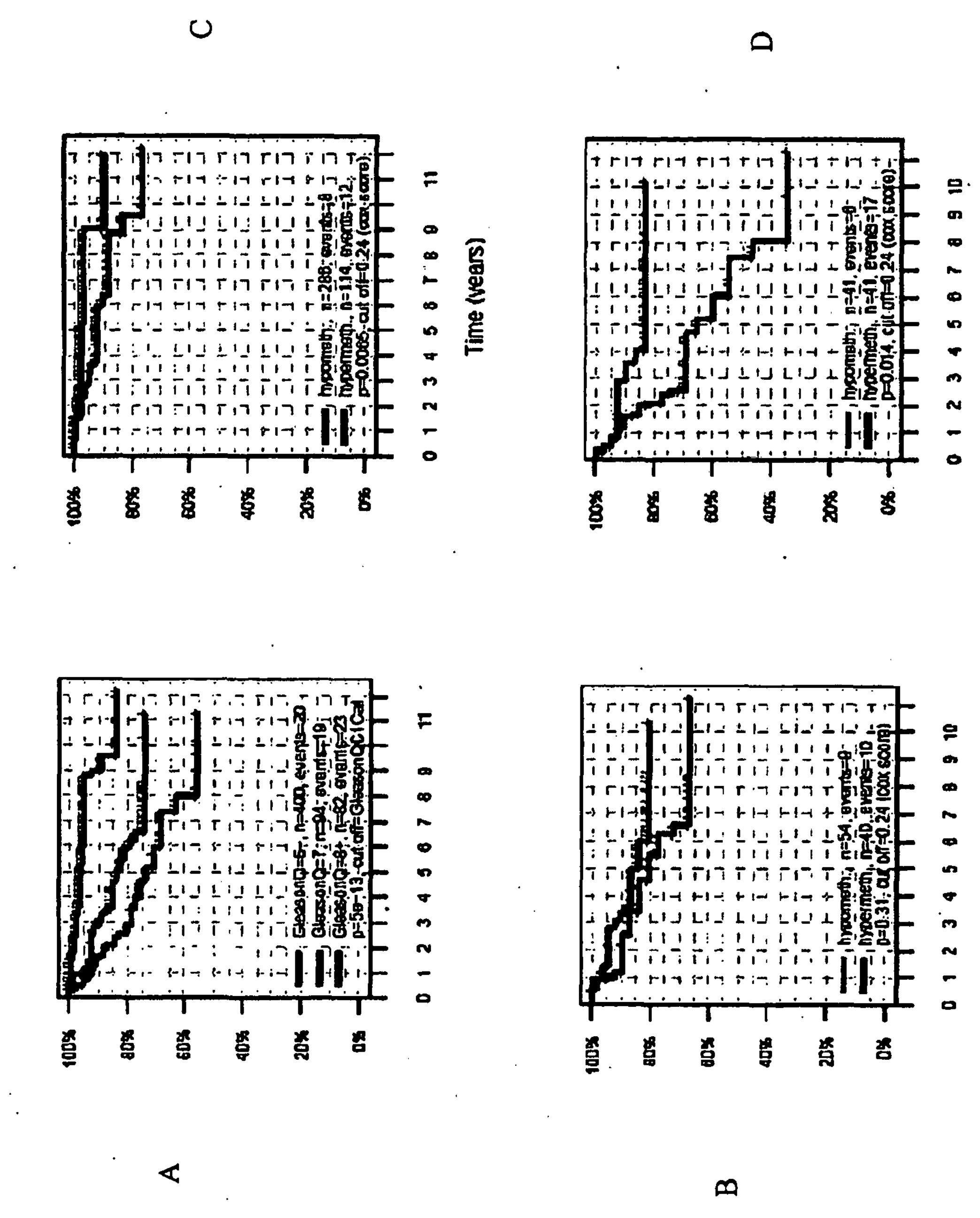


FIGURE 4

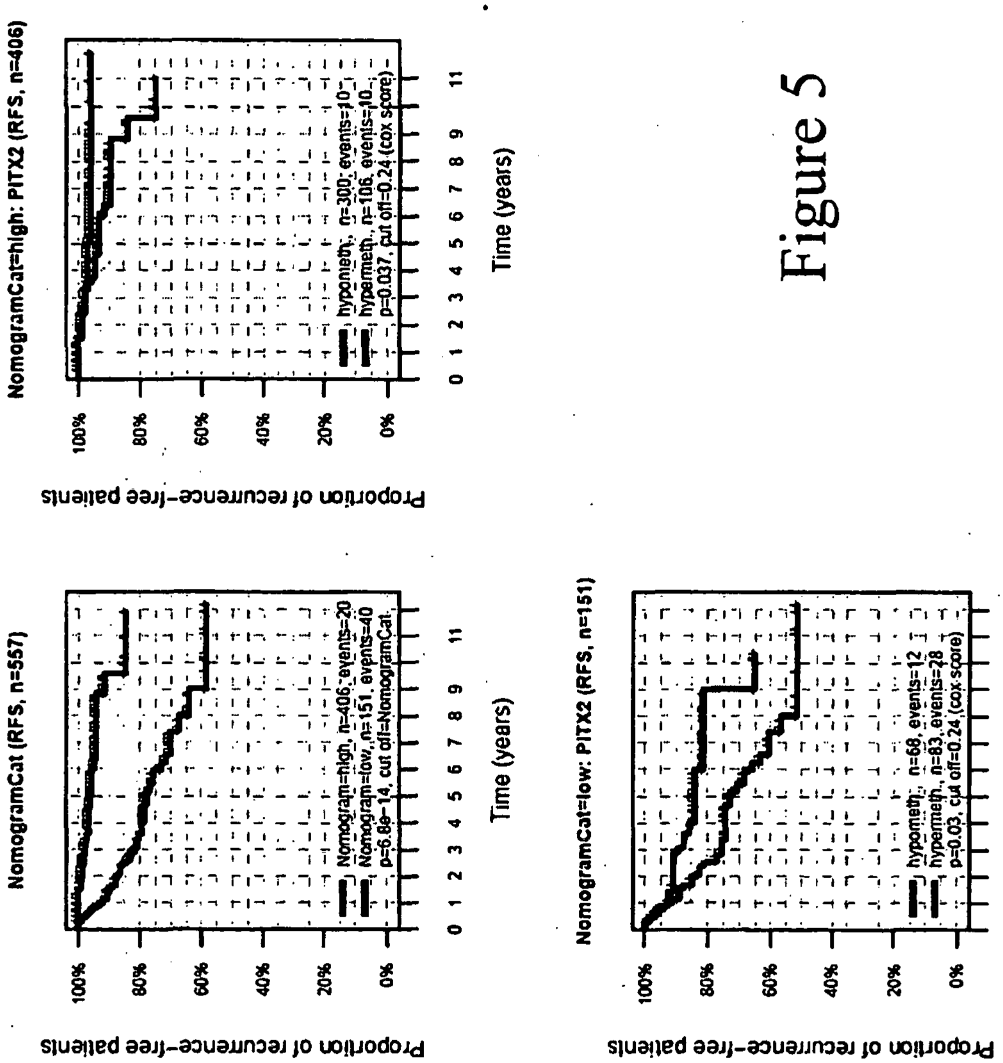
NomogramCat (RFS, n=557)
Nomogram=high, n=406, events=20
Nomogram=low, n=151, events=40
p=6.8e-14, cut off=NomogramCat
NomogramCat=high: PITX2 (RFS, n=406)
hypometh., n=300, events=10
hypermeth., n=106, events=10
p=0.037, cut off=0.24 (cox score)
NomogramCat=low: PITX2 (RFS, n=151)
hypometh., n=68, events=12
hypermeth., n=83, events=28
p=0.03, cut off=0.24 (cox score)
Proportion of recurrence-free patients
100%
80%
60%
40%
20%
0%
Time (years)
0 1 2 3 4 5 6 7 8 9 11

Figure 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 04029486 A
- WO 2004035803 A
- WO 0119845 A
- US 5786146 A
- WO 0026401 A1
- WO 9500669 A
- US 6251594 B
- WO 9928498 A, Olek
- WO 9746705 A
- WO 9515373 A
- EP 2004011715 W
- US 6265171 B, Herman
- US 6331393 B
- US 5514758 A
- US 5565552 A
- US 5567810 A
- US 5574142 A
- US 5585481 A
- US 5587371 A
- US 5597696 A
- US 5958773 A
- US 20030013091 A
- US 09898743 B


### Non-patent literature cited in the description


- **TOYOTA et al.** *Blood,* 2001, vol. 97, 2823-9
- **J.P. EGAN.** Signal Detection Theory and ROC Analysis. Academic Press, 1975
- **GONZALGO et al.** *Cancer Research,* 1997, vol. 57, 594-599
- **EADS et al.** *Cancer Res,* 1999, vol. 59, 2302-2306
- **GONZALGO ; JONES.** *Nucleic Acids Res.,* 1997, vol. 25, 2529-2531
- **HERMAN et al.** *Proc. Natl. Acad. Sci. USA,* 1996, vol. 93, 9821-9826
- **XIONG ; LAIRD.** *Nucleic Acids Res.,* 1997, vol. 25, 2532-2534
- **TOYOTA et al.** *Cancer Res.,* 1999, vol. 59, 2307-12
- **SAMBROOK et al.** Molecular Cloning, A Laboratory Manual. Cold Spring Harbor Press, 1989
- Current Protocols in Molecular Biology. John Wiley & Sons, 1995
- **OLEK A et al.** A modified and improved method for bisulfite based cytosine methylation analysis. *Nucleic Acids Res,* 1996, vol. 24, 5064-6
- **REIN, T. et al.** *Nucleic Acids Res.,* 1998, vol. 26, 2255
- **ZESCHNIGK M et al.** *Eur J Hum Genet.,* 1997, vol. 5, 94-98
- **OLEK ; WALTER.** *Nat Genet.,* 1997, vol. 17, 275-6
- **GONZALGO ; JONES.** *Nucleic Acids Res.,* 1997, vol. 25, 2529-31
- **XIONG ; LAIRD.** *Nucleic Acids Res.,* 1997, vol. 25, 2532-4
- **GRIGG ; CLARK.** *Bioessays,* 1994, vol. 16, 431-6
- **ZESCHNIGK M et al.** *Hum Mol Genet.,* 1997, vol. 6, 387-95
- **FEIL R et al.** *Nucleic Acids Res.,* 1994, vol. 22, 695
- **MARTIN V et al.** *Gene,* 1995, vol. 157, 261-4
- **FROMMER et al.** *Proc. Natl. Acad. Sci. USA,* 1992, vol. 89, 1827-1831
- **SADRI ; HORNSBY.** *Nucl. Acids Res.,* 1996, vol. 24, 5058-5059
- **EADS et al.** *Cancer Res.,* 1999, vol. 59, 2302-2306
- **TOYOTA et al.** *Cancer Res,* 1999, vol. 59, 2307-12
- **BELYAVSKY et al.** *Nucl Acid Res,* 1989, vol. 17, 2919-2932
- **KRUG ; BERGER.** Methods in Enzymology. Academic Press, 1987, vol. 152, 316-325
- **SAMBROOK et al.** Molecular Cloning: A Laboratory Manual. 1989
- Basic and Clinical Immunology. Appleton & Lange, 1991, 217-262
- **MILSTEIN ; KOHLER.** *Nature,* 1975, vol. 256, 495-497
- **GULFRE ; MILSTEIN.** Methods in Enzymology: Immunochemical Techniques. Academic Press, 1981, vol. 73, 1-46
- **YU et al.** *BioTechniques,* 1997, vol. 23, 714-720
- **KARAS ; HILLENKAMP.** *Anal Chem.,* 1988, vol. 60, 2299-301

- **GUT ; BECK.** *Current Innovations and Future Trends,* 1995, vol. 1, 147-57
- **GUT ; BECK.** *Nucleic Acids Res,* 1995, vol. 23, 1367-73
- **HEID et al.** *Genome Res.,* 1996, vol. 6, 986-994
- **SANGER F. et al.** *Proc Natl Acad Sci USA,* 1977, vol. 74, 5463-5467
- **KROL et al.** *BioTechniques,* 1988, vol. 6, 958-976
- **ZON.** *Pharm. Res.,* 1988, vol. 5, 539-549
- Nature Genetics. 1999, vol. 21
- **GUT ; BECK.** *Nucleic Acids Res.,* 1995, vol. 23, 1367-73

Sequence Listing

<110> Epigenomics AG

<120> METHODS AND NUCLEIC ACIDS FOR THE ANALYSIS OF GENE EXPRESSION ASSOCIATED WITH THE PROGNOSIS OF PROSTATE CELL PROLIFERATIVE DISORDERS

<130> E30842PCT

<160> 5

<210> 1
<211> 28536
<212> DNA
<213> Homo Sapiens

<400> 1

```
gctctgggag ctaggtttct ctcgctggat gggaaaagga ggtgcggaaa ggggcaggcg      60
gcctgcgggc tagcggggat gagagttgtg gggagaacgg aggagagagt aaaattattc     120
cttttggaag tccaagggaa gggagctgag gtgagagaaa tggcctctct taggtcccaa     180
gtctccgccc ccagcctgca ccactcaagt cggggaagtg acaactcttc ctaaacctcg     240
acttccattt tttgtttcct gcagattgat tgatgtcctc gcgttgttct agttcagcaa     300
ccaaactagg ccgaggtaca cctcttttac ccagagctcc caaatcactc accaacctgc     360
aggagaccta aatcctccgg gcaggtctcg ttagcgcttc cagcccccat ccccaaatcc     420
cctcttctag ctccttcctt cccctccgcc ctttggccct ccccgccccc taccaggctg     480
agaggcggag aggccgggac cgctgggccc tgcggctcca gaagccaaag tccgcccgtg     540
gggaccgttt tcctctccac tgctctcaag tgaacaacat ggtgggaggt gaccctcgtc     600
caaagggctt ttatctgccc cccactgctg cgtgatccct ccgcaagtcg gaggcagagc     660
tttaaaaaga aagaaagtgt gcgttgagca ctgatcttaa atcggggaca ttatctgcga     720
tctctttaag tggcagcagc agtttcttgc agaacaaaga ctcagtgttc gagctccaaa     780
agcttctagg taaagtttca ttcagatgaa agcaactaag gcgaaaaaac aatgatattg     840
tcggctccag aaaatcggcg gttacttttt gctctaagtt ccccagcgtg gtgtttgttc     900
tcgcccactt tggttgtgcg gggggttgac aagcataaca aaagatctca gcacatccct     960
ccccccacct ccacaacgac cctcctagcg ccatgaggat gaattctctg ggactaagtg    1020
gtatttgttt ctatttgtta tgaaatcaaa tttcacattt ttttaaagct gaaaatcgcg    1080
gataaagaag gatcgggtgc ttaaagttca tttaaacact cacaccgaac tcattccctg    1140
tttagatgtc agaggatggc agggagggcc agggctgtaa ttcatcttga tttgcttcat    1200
tgtcctgcag tttgcaaact ataatcctgt ataatttcag gaacaagcag gtttagaatt    1260
aatgggtcaa tattatttaa aacaaaacac cgggagcatg acctttgcct caactacata    1320
ttgctcgaca agactcagga aactccactc taacctctca acccctgagt tctttgagaa    1380
actgaagggc tgtagttatt agaaatcatc tttgtttctt ttaatgtctc caaaggattt    1440
ggaaatagta atgcaatata acatgaagga tctctctact taagcctgaa gataaacgtg    1500
gaagcctaaa tattttgaac tggagatgtt tccctgtaaa tttattatag atgtattcct    1560
cctgagagaa atgcatataa actatacatg tatacatgca caaatatttc tccagaacaa    1620
tttgctagag gtgtaggttt ccccgtaaag gagtaaactt gagagtcatt ttaagctgat    1680
ggacttgcta aatttctttc ttcttttttc tttttcatat tatttgctag ccataatgga    1740
atcctctagg tttaagccaa agaaaaattg gagagacaaa attagatttt gtagcccttt    1800
tcccccccgg gaatgccttt ttttttcttt ttagtttctg atgaatggct atcatttatt    1860
tctaccaaat ttaaataagg actgctgcct tgtatgttta actaggcagg cagagggaac    1920
tggtttgttt aggaagcagt gactgagatg tcctggccaa gttagtgaca gaggagggga    1980
gaaagaatcc agaccaattt gtatgcagta tattttactc ccatgaaata aaacacattt    2040
gtttcatatt tgctgaaaag taaaacaata atattgtacg aaatgttata cacagggtag    2100
gttgtacata gcagtttcag aaacatcatt gcatccacca gagaaactat tctaaaactg    2160
atattcacac attttttata ataataataa tatgttagaa acatacagtg tggcatttag    2220
tatatacact cccttgctcg caagcgaaaa atcctaatcg cttctgtata acatgcttta    2280
ttttaaagcc taacctttaa aaacactgtt gtgatattac taacaactgc ttttataaaa    2340
ttaatttgac atttcgatat atatacatcc tttcagtcat ttaaatgtta acaatgctaa    2400
acttaaaaaa taacaagctt atagtaatgt taaaatgtca tatccagtca aacatttgtt    2460
tgtgtatgtg tccttgcaac tgttagaaat acttgtagtg aaagatgtca gacactgagg    2520
acatcccttt gaaatcaaag gagctctctc tttgattcag tggtttcctt ttctctatat    2580
agcttctctt tctctccctt tctttagtgc ccacgacctt ctagcataat tcccagtctt    2640
tcaagggcgg agttgcccca tccggcaagg tcctaggatc ccggcgctgt gggtgcggct    2700
```

cacacgggcc ggtccactgc atactggcaa gcactcaggt tggaggccgg gttctgcacg 2760
ctggcgtagc cgaagctgga gtgctgcttt gctttcagtc tcaggctggc caggctcgag 2820
ttacacgtgt ccctataaac ctacggagga gtcggcggcg cgtaaggaca ggcaggcgtc 2880
ggcaccgcgg aattcagcga cgggctactc aggttgttca agttattcag gctgttgaga 2940
ctggagcccg ggacgcctgt cactgctgag ggcaccatgc tggacgacat gctcatggac 3000
gagatagagt tgggtgggga aaacatgctc tgtgatgaca gggggttgac gttcatagag 3060
ttgaagaagg ggaagctctt ggtggatagg gaggcggatg taaggccctt ggcggcccag 3120
ttgttgtagg aatagcctgg gtacatgtcg tcgtagggct gcatgagccc attgaactgc 3180
ggcccgaagc cattcttgca tagctcggcc tgctggttgc gctccctctt tctccatttg 3240
gcccgacgat tcttgaacca aacctggggg cggttggggc aagggagcaa acagatgcca 3300
cagtgcagat tactaaaact tccatcggag gccaaccccc gccttccccc gacacacacg 3360
ctagcgcact cacacaccct ggcctcgctt cactgcaccg ccctgcacac caagatacca 3420
gggccagctt tcagttactg gcccgggtct ccaccaagcg caggagacct ggtctgctct 3480
ggcctgcgag ctgggactcg gagctacgcc acaaacctca gccgaacgca tggagacctg 3540
cggacggttt gatcactcag ccaggcgttt ctccaggtcc aaaaacactt aatgtaaaac 3600
aaacgcgggg cagcaggctt ttccaaccct tcccggggca ccttgcaaac ttgcttccat 3660
tccaaagcca cagacccacg gatgaggaga aggggctgga agggcactag aggatcgctc 3720
tttctcccac gcaattcctc ccttccttcc ctgacctcca ctgtcgtccc ccaccccctg 3780
gtacgtgctc ccttaacagg gactaggccg ccaacactct ttctcgccta gcaaaacaac 3840
caaataaaga gcaaaagacc acctcttcgt cagctcgtta actccaggag cttggcatat 3900
taaactccgg gaacccggaa agggtagttt tggagattcc ccttctttc gctctgcctc 3960
ttctttaccc taagcccacc acaggcctgt ccgcgcgcca ggcccagccg ggtcgtttgg 4020
ctttgcaggc ggccacccag gccggccggc ttccacccgt gtccggtggc ccagccgcaa 4080
ccccgatccc aatccacatc gggcctccct gtcgccccag acggcggctt ttgtgtattg 4140
gagagaggcc tggcctgaga tatccgagct gacaccagtg atgtttcaca ttacacatct 4200
ccgccgggcc cagccgtgta atccgctttt tctcttttc ctttcattct tgatttcctt 4260
tttatccccc ttcctctttg cacccgactg ctataaaaag cacgcctcac tcccacttgg 4320
ctcgacaagc agccgccctg gaaggagagg cagctgcaag gagagcccag cgccgcggct 4380
acaaagcact agggtggagc tgcggaatag cgggcggggt gggagggcgt tttcgaagga 4440
tcccagaaaa cccatagact ctgtctttaa ttacttgcca tttctaccct aggccatcta 4500
aactttgctc aggcgagaag agtacgtgag aggcccgttc ccttgatgtg caagagagct 4560
aatgaaagac tgaccttgct caaaaccacg ccgcccagga cccagctctg gctctggaca 4620
gttaaactaa aaccatttc aacttcttcc cggcctttta tccaccagca tagcctcatg 4680
ccttgcacaa atgccaccca gagagtgtct tcattccctc tgatttggga gagcattttg 4740
gtctttattc tttttatcgt tgttttcttc tttttgtttg ctctgctcta accgggggct 4800
ttatttttc tacccagagc acttaatttt tttttttaa cagcaaagcc tctggatgcc 4860
gcttgatttg cttgattctg ttttctgctt ccagaatcct aacaaatttg gaatcttcca 4920
ccgaccagca taaaccagga cgttgctatt gggttattta tttgagctca tttttgccaa 4980
tccataaagt acagatttgc tacaaagtta aggtaagccc tttttacaaa actatgatta 5040
taatttagaa gagggggtgt gagtttcaat ttccagagtt caactcctga gagaagataa 5100
ataaaccaag cagaaaagtc tttcttcttt ttttctttct ccttctaaga ggactagtag 5160
ttgtgtatta aaactttgct cccggagatc acaaaactag gaaatagggt gtgtgggaga 5220
gacctgaatg gccgaaacaa ccgtaaagaa ggtgtaagaa gcgcgagccc aggagggaaa 5280
aagctgggcc agggccggga caaaggtttc ccagggaggg ccaactcttc cgtgtctctg 5340
gcgggtttc cttgttaaag gctcacaggt tggagcctgt tcgcggctct tggcctggta 5400
gggattttat tagctctgct ctggcaactg caagccagga acacaatgtc ctgtgcaggg 5460
gattgcccat gcagcccagc tcgtgagatc gcgggatggc ggggcagtga gccggtgccg 5520
ctctgggagc ctgagccagg gcggcagtcc tgtcggcctc ggagagggaa ctgtaatctc 5580
gcaaccaggc cgccgcgagg ccttctgcct ttgcaaagct gcgccccacc ggcgccctcc 5640
caggcggcgc tgccttccac attctctcct ggtctacttg gcctgtacct ccacaacatc 5700
ctccccccat ccctcccaga ctccgtgctg gctcctaccc ggactcgggc ttccgtaagg 5760
ttggtccaca cagcgatttc ttcgcgtgtg gacatgtccg ggtagcggtt cctctggaaa 5820
gtggcctcca gctcctggag ctgctggctg gtaaagtgag tccgctgccg cctttgccgc 5880
ttcttcttag acgggtcctc ggcgcccacg tcctcattct tcccctgctg gcttttatct 5940
ttctctgaaa acgaaacaca cacactttcc cgtcagcatg cccacctgca acgcggacgc 6000
caactggacc ggcggcagaa gccgtggaag agctgggctg cctggcgccg gaggagggtg 6060
cgcgcggcgg ctccgggccg cgaggagcgc tgcgcctgtg gggtgtgcag gcgcaagtgt 6120
gggtgtccgc gccccatttc ctcccctccc ccagcgccgc acgttttatt tacatgttta 6180
tctcactgca gcggcacatt cacttttata gcctgtgctt tcaagtatat ttatacacct 6240
ctgcgcagac acaccaaatc tcctgggacg cgcacacgcg cgtggtttac agaccccct 6300
ccccctcgca gaaagctcag atttccatgc ggtttgggaa ggctaggaaa agatgtgggg 6360
attcggttgg gcaccgaagt tcgccggccc tttcccaaaa aaaaaaaaaa aatgcctctt 6420
cgcgaagggc atttctgagt ggtttcaggc aatttcctaa cgagtggagc tcctcgggag 6480

ctgaaagccg agaggaaaac agggacagag gtcggcggcc tctgaaggtc ctcgaatcaa 6540
gatgctggga tttttgtgac ccaggaaaca gaagggaggc cagggtacga atagagaggg 6600
cggcagaatt gctcgcgccc ttagcgcccc aggagccggg ccggtcgagg gagaactaaa 6660
gggatgcggg gtagtcaaaa ttccggctcc cggaagttct gcggggagcc aggcgaacga 6720
ccactcccac cacgcctccc cccggagggg ctgacttcct tggggcgaga gggagcgggt 6780
ggcgcagagc agctgagcgg gaatgtctgc agggcggcgc ggcgccttac ctgcggcctc 6840
cgggctggag gtgtcggaga tggtgtgcac ctccagcctg tgcttggagg agtccagcga 6900
ccggggctga ccgggagcca gaaccgaagc catggctaac ggctggggat ggtgacagga 6960
agatgaggag acggccgaca gcttggtccc cgctgctcgg tgctccaagt gaagcggccc 7020
tttcatgcag ttcatggacg agggagcgcg acgctctact agtccttggc tactgccccg 7080
ccgagccccc gtagccgccg ctgcccgctc cgggtcgcgc tctaggcgcg gagtttcccc 7140
gctgcgggga gagccagggg acgcaacccc cgccgagttc tcaagccaag ctgcccccgt 7200
ctcctccgga aggctcaagc gaaaaagtcc ggagacggaa agtcagcggg caaacgaaga 7260
catgggatgt gggcagaagg gcaccactca gagcgtcttt agggagcagg cttccaagct 7320
ccaaagcgaa acaagagtgg gcaaagaccc ccttcttctc tccctccctc ccccaagaac 7380
ccctccaata aggaaagcta acgccgaccg cgctctgccc gccccccccc cacgcggcag 7440
ccctgacaga gaagtgtcaa gagtgacagg gacaggtagg tgatattaga tcccctgcgg 7500
cggcagcagc cgctgcagcc acgacgcggc cctctgagcg caccctccgc aacgcgcaca 7560
cgcacacccc tcgggcggtc gaacaggagc cgggccttgc cgcagctcag ctccaggcac 7620
ccaggcgagc gacggaccag atctgcggct ccgcgcttcc ctgttggcct aacatcttaa 7680
aaccagaggc gggcttcctg gtgccgagac gtcactccgc cgcggccctc cccagccctc 7740
tccgcctccg cctcctccca gacccttctc cgggtgcgac tgacgtggct ccgcaccaat 7800
caggacgccc cgagccgcgg tggagggact gtcctgcctg cacctatcag cagtgcgggg 7860
ccgggctact gcctcgccgt gcgcactggg tctacacagg caagctcccg ggaattcagc 7920
tcctgcccag cccaaggcga tccggctttt agtacgaacc caaaggtgaa gagatgaggc 7980
taggagtcga aggcttggga gaagagagtg gaatggtcaa gaagagaaag gtacaaggat 8040
caacaagaca cccactcttt gtgtctcact acatccattt ccaatccccc accccatata 8100
aaaaggagac acgttactta aaactagaaa atttgaaaaa cagcaacaaa tcacctctcc 8160
gatcttaaat tttccaaaca gcctgtcaag tgaatgctgc gctaatctga agaagcttta 8220
attgcaaaga agacagagcc ctgaaaaggc aggctaataa attagaaatc gagaagcaaa 8280
tggacccgtc aaaagaaaat taccttgact ttaaacgaac aactgtttgg tggttcactc 8340
tggatttata caagaataaa aagtcgcctc agatcacgtt ctctgtgatg cttattagtc 8400
cccagacaga aaacacacaa tagaagagaa accctaaccc agcgttttca aaatgctgaa 8460
agcttatcca ttctacttaa cgttgattaa gacacatatc ctagatcttt caaattcctt 8520
gtacactgta ttaagctcgt cctaacccga gagagccacg ctttaaattc gactctcttg 8580
tttactttat tatcaatcag atttaaatcc ataaagcctg tagaatcaac aaccttgagc 8640
taattatata tgaaatatgc cttaatgaat ttccatacaa ttaagaatgt tgccaaataa 8700
ccaatttcaa ggataatttt taacagtcat tttcttttcc cagtgagctc aaggctgtct 8760
tgagccatta aagtccaagc aggcagaagg ggtgtgtgtg agctaagggc gaaaagccta 8820
gaactgcgct caactagcaa aagcaaaacc ttatttatat aaaacaaaaa aaatcacctt 8880
tggagacatc aactctttat agcactgttt ccaagcaaat ttaatttcca aagaaattaa 8940
agaaagaaat ccaaacatat tcaaaataat ttttgaaagt cctttttgtcc cccagcatag 9000
gtcagctgga gaggacaaac taatctcctc tgggtttctg catgggcgat tgttttacta 9060
tggagttagt gttatcatct ctgaatgtgt atttgtttga cattacagtc aatgatttgc 9120
aatgccagca tgaagtatct ttaaaacact ccctccttgt ccttgttcac aagattggga 9180
aacttattcg atgtggaaca aagtggatga agcagactac aaatatattt gcaacttatg 9240
tgtctctcct ttgccctgac cacccccaaa ccctatctgc aactcctccc catttaaac 9300
ttgcagtcca aagacgcaca tgagaattgt ttttcagtct ttcttcacca gtatcatccc 9360
actttaagaa taatttagct gcaagggagg aatttcttca tagtaagctt taaatcagca 9420
tttctgcttt taacctttta ttccacttta ccccattcca cacatacaga cacctgctca 9480
gagtaaaaca catcctcatg tgacaggtct gcattagctg aggctcatac atccagctat 9540
attaggtcct gcaatcttat cactaaatta tacacattac actagcagcc tgttggtaaa 9600
gaaggttaaa ttaatttaca ttctgctcat tatctggtgc ttaaatgacg cattttatcc 9660
cggagatttg gcggagaatc tccttctcag accccacagc gtttcactga agacaatgct 9720
cttacatttg tagtggtttt taatctgata agactctaat ttgcttaagt cttttaaata 9780
agggttttaa atgtttctag ccgttttctt attgaatttc ctctaattcc cccaagatca 9840
taaagtatat gtgtaaagta aatatttcct cccattgcac tgccagccga tgacctataa 9900
ctaagtcaat aagaatccag ctcttttctg ctgaatgtgt ttactaatca tattccagtt 9960
tcttctttta aacctcagaa tagctgtggt ccccacaata ccatgcccct taaagcttca 10020
tttcatgaag ggactccatc acattaaaga atgaaaaaaa tctccactgt agttagtata 10080
cacagcccct cactccttgt ttttcaagat tcaaacccca gagctgcaaa tatttttgga 10140
agcttgggtg ttaatgcctt attttagaaa gccgagaagc cccacagagc catatagatt 10200
tctaaaccca tctctcataa acccacagaa ttttgataaa agctctggtg gctccactct 10260

```
accgatggaa ctttcatcac gacaaatata catgtatgaa ggacctcaat cagcctccaa   10320
agtggttgaa aaacccaagg gcacgtgact gctcctcata gtgccaacgt gtgcgagatg   10380
ttggaagcac tggggatcag cagcagccta gatgcctaaa aagataaggt gtcctaattt   10440
gtgtggaccc attgaagtca agtggtgaat aaagacaatt atctagataa ttcagattaa   10500
agtaaaagca aaaccatatc tatttgtata tatatattca catccatttt atattacaga   10560
cacacacacg cacacacaca ctggctctgt aaacaactga ctcaaagtga ggattttctt   10620
tgcatttttc cagcaggagt ttcaacattc tcctaatctc ctaatcactt tacacactca   10680
cagcagcggc gattgggtga cattcttctc aggccccctg tgtggcagga caccaacatg   10740
ataagtctgc atggggaaaa ggaggtatgt ggtgggaact aagaaacact gtccagtgaa   10800
aactctgtgg catggtggtg gttgattttg gagatttaat gtacataaga cttgtgggtg   10860
cacaggcata ggcagcatgg atgagaaagg ggccagaaga aaataaatct tatgcatttt   10920
gtgattccag cattactgtg acctctggct aagttcttct taattggttt tagaaattac   10980
tatgagttca gcctctaaca cagaaatttc caatacggag aacattggtg ggatcctggt   11040
agggaaacta gaggtgctgc atggctcacg tggggcaaag aaggaaagcc cagtgccggc   11100
gtgaggtttt gagtctggga gacatcaggg gttgtctcga ttggggtttc ttgcttattc   11160
tttcaaagaa agaccctaga ggagggaaat gtgtgacatg ggtcagccg tgttttgtgc    11220
tggtatttgc caccgattac cagtcttaaa gtcttattta atttcacact cttcagtgtt   11280
agttgtgcaa agtccctctg gccatggcag tgagcggttg ggctgtgccg ccaaactctc   11340
cgtatcaatc tggcctggga ctcaaccaag tgatctctga cttttggaaa gagtctgtct   11400
tcagagttca cccagaagat ggcttaatta gacatctccc tgagctgtta ggccttagac   11460
gggtgggagt cctgccctgc ccaagctagc tcaaggacga ggcccgcctg gactcagctt   11520
ggagccacgt gatgggcgtg agtgtgtgag ctcctggtaa ggcgcagagg tcagatggag   11580
accttgcatc ctgcccgaga agtgccccac cccctccaat atctggcttt tctctgcata   11640
caaaccaagc tgaaaacagt ccactaccca ccacccctca tagctatgga accaaataac   11700
ccagaaatta aaagcttcac tgtagctgtc cttttcccca tttcctaaat ggaatttaaa   11760
aagctctggc ttgtcaaaag gggaagatta ttttctgaat tggaagtctg tagatatatt   11820
gagcaacagc caccctctct gggtccctgc aaatggtacc catttttcca acccacagct   11880
ctagctgctc aaccatttga gatttggggt aactacctgg gggaacagtg ttcagatggc   11940
agtgggagtt accacctcac agtggcctgg ggaagagaag agaaagagat tagaggaggg   12000
ggcatttgct aaaatcactc aacgaacatg ctgttaatgc ttcctcacat ttgcatgtta   12060
ctgccacagt tttcctaggt gtcactgagt ctccagaaag caactacttg ccgaactaag   12120
taaaataagg agaatggtat agcacatgtg tttggagaag gggaaggaag ggtggaatat   12180
gaaattgagc atagatatcc aggtcaggaa agaaggaagt ggcaaggggc taaatgaagt   12240
cccacccccc cgccactttt ttaaacaaca gttggattaa acactcatct gtcttcctct   12300
ttttctcttt tcttccctct ccagcttatg tccatctccc ttttctattt cttttgctct   12360
cccttctttc tgtttctctc ctcccagaca tgctggtagc taacattcag cattagttgt   12420
catggtgacc ataaatcacc taaatccaaa aatacccacc tataatctga gatgaagctt   12480
ttatttccct agcgaaataa tattttaaaa gctgtcagct gacaaaaaaa aaggaaccca   12540
cctcattgta gtaacccaag taatatatta cttctaaagg tttaaattaa aatgtcagcc   12600
tgttaaaaac atgctggtag agtcttggac actcttcccg tcagatcctc acaaagaagt   12660
tgactctgtc attcctggtc gctctttaac atacacacac aattctgtat gctctctccc   12720
tttctattaa tttcttcctc ccctaccaac tactttagtt ctcaaagact ctacgcattg   12780
ggttctaaaa gaaaagaatc tcctcctgga tcagaaaaca gcctcattgg ttgctgaagt   12840
gaaagatgtg gggtttaggg ggaaaggtta ttaggaccat aatggcggtg gtggtaggag   12900
gtcatcctag aggagttaag aagaaaaaaa aatgcaggga gaaggactgg aggtggaaag   12960
acagagtaac agaaaactga gctgggtggt caggtgctgc ggtgaagtct agccccgaaa   13020
tgataggtat atattctccc actcctgcct ttttctcctc tgagagaaaa ctttcctagt   13080
cagagaccct gggggtagg aggcgggcaa acgccgctgc agttgggcct tttgtcttct    13140
accttgggct tgttgtcttt tgcctatctt ggattacagg gtattcgcct agatgaagag   13200
ccattaatta cccattcagt caatattagg aagacgacaa agctttttac ataggattaa   13260
gaagacatca gattgttcca ttagtatatt cctgctcacg aataagcttt cgttatacat   13320
ttccttttcc cccgagccgc tctaacaact gctgcatata ttagcagcgg gcggtgagga   13380
ataacagccg aaccagcctt aagaaacttt gtgtaccgag tcagcagcga ggaacgcgac   13440
ctgtgaagac cacttttgcg ggcagggatc cgcagggact gactactttc ggacaactgg   13500
tacaatttcc cctgggggtg aaaaaccaca acgcggcggg gcacctttca agtgagctgc   13560
agatttgatt ggcgcggggg gtgggggagg ggaggggaga atgggatggc ggaggtcggg   13620
cggaggaaag aaaatggaaa actctcctta cccctatttc gttgcttttt cctcaagcct   13680
catgcccccct tcggcaagca cctgtccctt ccgcgctagc cacagctaga gttctccacc  13740
tttctctaca catccccttc tttctgacaa ggctcaggac cttggtcacc accccacgca   13800
ccaccatttt gcgttttgct agagacggtc tgggctgatc tcgctggtgt ccatgttagg   13860
attaaaatcc cttcatgacg gcgaggaaaa ctgtattatt tgctttcagg gggtacatta   13920
ggagtccacg tagtatatgc tccgcaaaca ttcgctgatt gaatgagagg cgcgggggcg   13980
gggcggcgga gaggggtctg cggccgccaa ggccgccagg gttaacccag gtccttcgaa   14040
```

```
gaaggttggg accgagctgc tgccgcgtgt gaaggtgtgt gtcgcggctg ggggtgccac 14100
aacgggccat ggagcccacc tcccagaggg aggaagtctg tgcacaccag cgacttgggt 14160
cgaacacttt cagcccatcc actgggccaa agctatttaa caattaatgc gttcctgggg 14220
gaggccgggg gaagtacccg ccccttgtcg ggacgcaaag ccaggcgtca ggtccaaagg 14280
ggctgcagtg cagtccgatt tcagcacgga acccagagcc gccgccctga aactttttaa 14340
gccagtgaca gaggagggtc agtccgtcct cctcctgagg gtgaagacca ttttatgagt 14400
tcctttcagg accccaaag caagaaaagc caaagaaagg tctctttgct ctaggggtc 14460
gttctcagct ggcttctaca ccatcttctg ctagctgcgt ccaccctctc tcaaacctct 14520
ggcttttagg ggtctccagt cgctctcgtg ctacccccgt ttcggggttc tatccaggct 14580
gggcatccgc ccaatggata ccaaggagaa tgggactcac tagggaagga aggcagagcc 14640
tggactgctt agaggtggac tctgcctata cagaacgttt agtctttaac gaggacatgg 14700
tatttttggg cggcgttggg ggcagaggcg gagagggtag cgtaatagac tatcacggcc 14760
ttttgaagaa gtcatatgtc attgtgaact ttttttctct ttaaaagcaa agaaaaactt 14820
taaaaaaaca caagaaataa atccttttgt tttacaagca ggctgtggcc aggactttgg 14880
atattccata agttaactta aaatcaggga aggataggtg ctctatcctt cagcagtgct 14940
atagctttgc ccactcgtgt gatttttttt tgtcgtctac tagttcctta aaagccaatt 15000
aaatcaagat tttcagcatt tcttcccatt acatgccttt tcttaattaa tggcattaaa 15060
cgtgtttagg cagcaatttt tcttttcggt caaaaagtag aaaaagacat attgagtgta 15120
ggggaagagc ctttcacgtg cactaaaaca atgcgggcct gaaagtaatg gttaagaaag 15180
caatcattca ttatttctag ttctttatgt gctagttatc aaaagcgaac gaccaggggc 15240
gcctgcgcgg cttgtgactg gcgcaagcag aactcctctg ctcttagccg tttcctgctc 15300
acctacgagg accccatcct accgtaggct tctccacctg tcctcagaat gcattcccca 15360
tgtctaggaa acctggggta gggactgggg gaaggagaca ttttgcgtct ctctggctcc 15420
cagcacaata agaaatgtca gcctcggctt ggcgactgcg agcccgcccc gcgcgaagcg 15480
agattgggga gctcctctag tctggccgga gccagggctg agcccgcgca aagcattctc 15540
cccagaagtc atcgctgctc ttgattttta accatatccc aaacatacta cggtctaata 15600
atttattttt actaccgatt atcaaacaga agaagaattt aacacaatct aaatgacaga 15660
aatcacgcga cgttatctct gcattgcccc catttaaccc catggggtta atcccggaca 15720
agtgagcgtt taactggccc agcagggcga ctggcggtgt agtgcagtgt ccgggcgtga 15780
agcactggat cgctttagac gcttcatttc aacaaatgat cattttctct cagattcacg 15840
gggaaactcc aatgcaagac ctttgttttc ttcttagtaa tacggtttgt ttttttgacc 15900
ggggtcccaa atcgcccccc tccatcccat attacatttg tattcttaca ctttaatccg 15960
gaaagagggg gttaggggcc gagggctgtg gggggggggg ggctttatct gctcacatta 16020
tcaaaggtca atagcctcct aaggctaggc acttatttac tacggagcca ggaaaataga 16080
ggaacagtaa atttgagggg tttttttcca tgtatttgaa aagaaaggca tcttccctcc 16140
tccatccctt acaccccccc ctccgcccca tagaacaagt ttcaattcag gaaaggcctg 16200
tggcgtaggt tggagacttt ttaacttttt acataagcct gtagacctcc tctggcaatg 16260
tccctcgatt cctccagagt gaaaccagcc aatcaagcaa cgacatcgcc aaaacccaag 16320
gcctggcaac cagtacttca ggcaggttcg cgtcgacagg ggcaaacctc cattctaccc 16380
tgggctgcta agcacagtgg ctgccctcca gctcttcagg gatgttgtcg gcccccgcc 16440
cctcccccaa ccagccaaag caaaccttcg ccaactcaag tttcccttgc ttgcctcccg 16500
cgatgaaccg cgcacccaca agtctgggtg gggcgtggct gagagcctga gtgactgagt 16560
gggccctggt ggtgctgcgc gcagcgggat ataacgagcg acagaggtcg ctgttggacc 16620
acttttccac gccagcctag acgccgaggt ttgctggagc gtgcgcaggg gatcagacca 16680
cagggagcga gcgagaggga gagagaggtg ttgggtctca ggagtgcagt ataatttggg 16740
gaaaggaatt aacgtccctg ggacggctgc ttcccgtccc acccagaggc ggagtgtcta 16800
agttcaagca gcaggcgcgt caggtctggc ggcctcgcct tcttgcgctt cgcccgaggc 16860
ccagagtccc ggaggcgggt gcccagcgcg cggcctgcgc ttccctcccg gccttaccat 16920
tggcgccagg atgctgccgc gggaagaact tgctgctggc tgcctctttt cggctctcag 16980
gagagtccgt gaactcgacc ttttgattt cggagtcttt ggagaagaga cattcaacgg 17040
ccgccggctg cacgcctggg ccacgcgcgc gcccgcccca cgtgcggaga gaggcgtccc 17100
ggaccgcggc cgaaaggagc cggggacggg aggaggggga ggggcgaggc aggccggagg 17160
agaaagaggg acaaagagca aagacccagt tagaggaaag agctgacggc atccccgtcc 17220
cccggaccct ctggcaaccc ggggcaggat ggcgtactct ttctgcgcct ccccggttgc 17280
cggcggttcc agccgggagg agtaggctgg ggggcttcgg tacacagcgc gccgctgctt 17340
cctagtccac cgccccgcca cagggagagg ccactggcga tttggttctg atttccttcc 17400
tgcccaggct ggcccctcgg ggaagcgccc ctcgctgggg cctcgccgca gggccagtgc 17460
cctcttgccg ccctcacgtg gcgcggcctc ccgtccgatg acccgggcag gagaaggggg 17520
ttcttaccta attgcacaca cgccgacacc agtttgcggc agttggtctc cattccccgt 17580
tatctgcaaa acagaagaga aggaaggctg taagaagcgg cggccgtcga gtgagcaggg 17640
ctcagatgag actacgttac actagctgtt aggcgcctac tgtgtgctag gcttcaggcg 17700
tgtttttccg acctgacagc ctctggctgt gcagcagcat ctccagccta gcctcggccc 17760
caggacattt atttatcaag aggggatctc cctccagagt tgccgcaaaa gtgcccagag 17820
```

```
gttagaggac tataaagcca cagtgtgctg gggaggctgt ggacctactt tcaagaatct 17880
cggtgccggg gttaagaact catctgaacg caatggcagc gggagtgggt gggtggagag 17940
gacttttctc tttgggaagc tgtatgcaaa gaccacccctt cagtgcttgt ccattagttg 18000
gagctcggta aacatttgta gaacatcagc gccaacgtgc ccctgtccta gacagcagtt 18060
tccctcggtt ttctgtaatt ctgaaacgaa cgggctcttg gcctagggtg tttcaggagc 18120
gagctgagtt cgggcctctc atccatcagg agccactttt ccatatttag ccacactttc 18180
tcctagagat actaacccgg ccatttattt acttactaca aataattacc ctaaagtatg 18240
atttaagatc gcagaggaga gacactgggt ggactgagcg agactgagga gagcagggca 18300
aacgttcttg gagggtttac tgcccgccaa ggacggagaa atagctctgg tataactgct 18360
acccagttct ccccctttct ctcccgggcg agccaaatct ttttcacgtt ttcaaccaca 18420
acgcagcgag ccaagcattt aacgcgctcc cccttctctg ccacaggcaa gccgggagag 18480
gtgggtctcg aggggctcca ccgggtgggt agaagagccg cggttgcttt aaagacaaga 18540
aaagaaggtc cagggctccc taggcccctt cgatctcagc gcctgcctct ctccacgcta 18600
accagggtac gccgacgacc ggagggccca cttcgcgcgg gtgcggggat cggggtggga 18660
gcaagcgttg tcgggttggc ggaggcacag aggcggggca gggagctgcg ggcctgcctc 18720
tggcctgagt accgtttccc tgcgtctcgg cctctcctga agggagctgg gccctgggga 18780
gcctctggcc aaggtcgctg cctacaggag gggctgcccg gcgctgtggc gtggggatcc 18840
agggtgggga cggccaggcg gttcccccac ccgctagcga gaacgcgggc ggggactctg 18900
ccgattcgat ccttgtgggc ccgtgggccc agaagtagca gtttggcggc tccagattta 18960
gtgattctgc agtaaaatca caggattagt ccctgattga gatgtctgtt cgtgagatat 19020

tacaaaattc attatcacag ctttctacta actcgatatg aagtaacaca gatgggattt 19080
tattagtcca gacttcaaat gtttacttat gataatttcg gaggaaattt gcatgtcatc 19140
atcatttcga taatcttttt tttttatatg tctgaactgg ctgcattatt agctggtagc 19200
cggagcactg cagatggtaa ctgcaaatag tttttattta tttatttttt ttaaagaatg 19260
aaatatacaa aagaaaaaga ttgcgttgct tggtgtaaag tcagtcaatt attacacatt 19320
cttccccccca cttccccgtg cttcagtgct gaagaccaaa caaagcaata caaaacaaat 19380
cttcaagaac tcatagagct ccactctaag gactgaaaag aaggtcaagg cgtgttcctt 19440
agctcactcc tacatgtcct tgtgacttgg agatttattt tgcagtcaaa atgagccttg 19500
agacttgcat ttttatgctt catttaatga ccaggcctac tagaagaact gagtctaaat 19560
aactggggaa gataattttt taaaaagaga cctccaattc ccgcctgctg atccttaaac 19620
ttgccctatc aagacaagtc ttctgtgaga aatttggctg ccagacttcg gaactggctt 19680
caatggctaa tctcacaaat tgagatggga gacttttcct gatgggaggt agttctcacc 19740
cccaaagttc atgttctagt tggaatgtat atgccaagga ctcttgtttt ggccaacttg 19800
ggttttatat tgtgagcaca caaaaagcac tacacggcta acggaggacg aggaaccatg 19860
gcaaagcagg caggcaagcc ctaagaaata aaacaatttg ctaaaaaata atttctgatg 19920
actaccgcaa gactgaaagt gcaggaaaaa tacagttcga ataatcccag atcctttcac 19980
atttcccccc ttttcataca ctttgttacc ccataacaaa atctttaatg gaaagtttaa 20040
aaataaacag cacaggaaca tgtgttttaa atgaactaaa ttgtgaaatt agccagtaaa 20100
ttaatttgta gtaagtaatt atttaaggaa attaaaatac tgctcagttc agttctgtat 20160
tttaccatgt gtatgcgttc tttacaacca attaatataa gtgctttagg aacatttgaa 20220
gacaaacacg cttaacttaa ggaacaaagc acctaaataa tttaagtgta attttgctga 20280
gttaaagtaa aacattccac aaatgaagtg gctatttaat tttttaggga aagtttggtt 20340
attgaaatgt tgtatgctca tgttacatca acaaaaatct tcaatttatt ttgcttatgt 20400
gctttgtttt cttgatatta ttggtatttg aattttagat ggatttctgc caaaatgata 20460
ttttgtgtga taaaagcatc tttagttttg attgatagac taaaacaaat gcaaggaaat 20520
ttctttaaat cagattaatt tttcataaaa atattttaga atgtatgaat tctgatattt 20580
acatttataa tggtaaaagt tttttccgtt tagtttagta agacaatact cacacaaaag 20640
agtaaaaaaa aatcacacca ccttatgata gtttgatttc taaattgctt aagaaagtaa 20700
agtggttaaa ctggaaaaga ggaacatatt tcggaggttt agaatcgaaa attttttct 20760
taatctccag ctggaaaata attctctgca tccatttaaa gtgtatctcc tgaagtgcca 20820
gattggagtt gactggtgat caatttaaag gagttacaat ccaaagaaat ggtgagagct 20880
tggcatccag gcctggctcc caggtaattc gcttgggcct gagaggtcac taactgccag 20940
ttaagatgga atctttttct tttctttttt ttcccaatgg ataacaatgg gaaggggggct 21000
aatcttccag tagctgaaac tttgtaccca gccctttatc ttgagaatgc taatccttgg 21060
cccgaggatt tgttcctgca gtgttggcac cgagatttaa gggaagatac ctcgttttaa 21120
atgccagcca cggtctggct tccctctcga cttcagcacc ctgtagattg ttagtgtctg 21180
tggcgggggga cgaaaggaac agggctttgc aaggtctgtt tgccgactgc gttaccttgg 21240
gcgaaactta gccccaaaag ccacaaatca cctacggtga agattctccg aagtggaaca 21300
aatttccaga ctcgcattat ctcacatccc tgcgggatag atggcctcca cttaccggct 21360
accgggagag agctgctgtc tccgcgtccc actgcttccc ggggcgattt ccagcgagcc 21420
gagcctccgg ctgcacggca agcgcccgaa agccgggcct gagaggactg cagggctcct 21480
gagggtgcca agttccgaag gagtccacgg gtgcactggg gcctccgaaa tctagccgcc 21540
```

actggcagtt tctttctgct cctctccagc tttctcgctc ggtctcgcac tctctctcct 21600
ctccctccct ctcatccctc tctcttccct ctgctcctac tccgtgtggg gagtgacgtg 21660
acgtcagcag agattccacc aaactccact gcacagtggc gcgcgggcgg ccggccgagc 21720
ccggctgcgc ggctggcgat ccaggagcga gcacagcgcc cgggcgagcg ccggggggag 21780
cgagcagggg cgacgagaaa cgaggcaggg gagggaagca gatgccagcg ggccgaagag 21840
tcgggagccg gagccgggag agcgaaagga gaggggacct ggcggggcac ttaggagcca 21900
accgaggagc aggagcacgg actcccactg tggaaaggag gaccagaagg gaggatggga 21960
tggaagagaa gaaaaagcaa tctgcgccaa cccggcagcc ctaataaatc aaagggggag 22020
cgccagggca gcggggagac agaaacgtac ttttggggag caaatcagga cgggctggga 22080
ggaagcgaca gggaaagtgg cccaagagac ggaacaaagg acaatgttca tggggttgtt 22140
tgggacgagg cgtgtggagt gtgggtgtga gcgtgcgtgt gtgaccttct ttcaggcctg 22200
cagagttgag gaaagaggtc acagcaaaga gggactgcgg agggaggaaa gtgagagacc 22260
ggtagagggc gggagtggag gtgggcgcgg tggggatggg agaggatgag tgaagagaaa 22320
tctagaagaa tggagtgagc tagtgggaga gggtgggagg gccacagccg ggagcgaacg 22380
agctaggctt gtcagctggg gaaggccggg acgctgggcc cagcttagct gggacaccgc 22440
gcccgaggtc aaggcgggtg gaccaggcat gctgagagtg tcggcgcaca ggtgggcacg 22500
gccacgcact gacccagtgt tcacgaaggg tttgcactgg acaaggctca gacgctcata 22560
gagtctagaa tttcctctgc tgtacctaca ttcaacaagt tcaccctggg tcacggatat 22620
ctcatttttt aaaatgacga ggttaaggtt cctggcgagg atggtattaa attgcacggg 22680
atagaagtgg gggtggggga gagagtttcc ctcaagtcca catttgctcc tgcaaagcaa 22740
agagtatgtg aaattacagg gcatattctc actcgaaaag tgtgccttac ttctgaaccc 22800
tgatttttctg atttcttgac ttgagcaaag atgtgtattt tggtagtgag cagaatattt 22860
tggctctgtc ctgcctctga gtggaaggac tataaatata attcgcctgg aggaccaggt 22920
gtgaaggctt ctgccaggca tatgggacaa tgttttttca atctcaaggg catcctgtta 22980
atgtatgttt ttggaaagtg ccggaacaca gccattgctc ctggattcgg attttcccac 23040
caatattaat tcctgcttga gagcaaaact caggcccgct attaaaaaga catctctttg 23100
gtccctaatt gagaataaag ttccctctaa aagttgtatt gctcttccta aatcaatata 23160
ccaatactcg caattttaga aatatatagt gactcgggag aatgtgcata aaatagatac 23220
gtttaaaaaa gcttggcgct taaaactaac cctagtcact atataggtgc tgggctttcc 23280
ctacttttgg gggctgtctg gaacatgtta tgtgtttctt tgaattactc cgtgttttga 23340
attcatttga gttagcagta aaaacaggca aacaaacttg ctcaatttgt tttgagtgct 23400
aaatcccttc actttgaaat agctaacagt cgacagatgg actcatttta tggaaagggt 23460
tagcctcttc agccacgaag aaaactgatt agagatctac attttaagcc atttctaacc 23520
tccacgtaac atccgtgaaa actcaaactt tctctcttta cccagtggaa actcaaagca 23580
gtgttattta aggggagaga aatgaggggg aaaatgccca cgtgctgttt aattgtattt 23640
cctctctgac tctgagaatt tctatttctg gtttttgaaa tctcgccgag gcaagaaaat 23700
caaatttctt caacaagtcc cacaactgaa ctctagttac aggacaccgg aaagtgcagt 23760
ccgagaaaga catcttcacc tctgcccatc gacgattttt gcagcctccc cattcctctg 23820
agtaatgggc taataatcct cccttttttt ctttttcattt tgtagagatt aagaggcgct 23880
cgtagcagaa cggccttgcc ttcagctggt ggcgaggata ggcaatctca tggaaaagtt 23940
ggaagagaat gagaaaacca aagacagaaa gattcagaga tccgcggaga gacacaggga 24000
gagggaaggg agttgcgctg aaaagacgca aagatacgcg cgtgcaactc cctccccttt 24060
caggtttcag aggtttgcaa accagggctg agaggaaggg gctcgggaag ctcacgttcc 24120
tctcgccccc cttctgtctg gagtctcgcc cgccagaggc tggttaaccc cagtcccggc 24180
cgccgcagac actgcgctga gcttttgggt cctcgccttg cccagcgcca gtgcagctga 24240
agtgagcagc tggtgggaaa tgcaaatggc tcctggagaa atagaagata cagaatgatt 24300
ctcattccct cctcgagtgt gtggaaggag ctggacatac gtttcacgct cctaatcctt 24360
cttttacatt tttagtcata ctcctattaa acaactaatt aatgcccaga atcaccaggg 24420
aatacattag gcatgcaatc gtagaagcag ggtgctgggg ggctacaaac caccgagctg 24480
atttaagacg tggatttcag gtttttccct tgtcaaagca gtaaaggaag agcgggcctt 24540
ggcgactgta tttagattcc gattacttca aattagaagg gggtggaggg agcgtccaag 24600
caaagcaagc aattctctgc cctgcagatg taaacaagat tgtagcatca aaggtactag 24660
ctcctttagg gctagatcgc ctggactggg agcctgggga aggggagaca ctaaccttac 24720
gtatctgtga atttcaagga tgttacattt ttacataaac aactccagtg cggattctct 24780
ggaatggggg gagtaatacc cccattctag aatattaaaa cattttcccc ccaaagcgta 24840
tatccttttt atttcctcaa aattttgaac tatgtctaaa gataatagtc ttccagtaaa 24900
ctggagcatt ggaccatttc cttcaccctt tcctaccgac attttgatga tctgatttta 24960
atgtgtgggg ggcacaggga attaaataca gcccataaaa ctaagcttag atgaaacagt 25020
gctggctaag tgggttcaga taattttaa tgagaatctc aattacactc ctcccccaa 25080
tatgttgaga caagtgacag aaccgttaga atggtaatca aattggaaag ctcagggaga 25140
acaacaattt cgtgatcaaa ttggggcaaa accgtggaca aatgtggggt gacctccgcc 25200
aactccctgt cacccaagag tcaggatttg ggaaaggtac agtattattt cagagcccgc 25260
tgtgacgggc tgtgtgctac catttacttt cttcactctg gattatgatc tcaaccctgg 25320

```
caagcaattt ccccagctcc ttatctgaca acaagcgagt atgtaaatac caatggctag   25380
cgatgtttaa ctgcctcaaa cattattgat ttgttggctg ttctaaattg tcttcctagt   25440
ccaggtcttg tttccgaatt gtctattcta gaggtttgat ccatgcctcc gatgctacaa   25500
tacaataatt gtttttttaa aaaaggcatt taagatgaac caattgattt gcatataaat   25560
taaaattact atgcgttgcc gattccggtg ttttataatc atttcgaaat tagtacttaa   25620
ccacttgagc taaaagaata tataaatgcc tgtattgact cactaatgaa ttacccaatt   25680
aaaacgtccg ggcaatgctg ggcgctggaa agattgttaa atcaagacat attacaggag   25740
ggatatgaag attagaaagg taacagacca atatctcgca ctcaaaacgg agtttccggt   25800
gattcccagc tttaattttg gagcaggggt ctttctcctc tgctgttaaa aagattttgt   25860
gcttgtttgt gagtgagtgc attcaagtgg aaggaacgct cccacggcta cggtggctca   25920
ggccctttgc tcggaccggg accttacagt tctaacccag gagcgttaaa ctctggaaga   25980
ctccgggcca gccctggagg tgcgtggccc cgcaagtcgc caggccaagc ttgctttttc   26040
tgtctgcccc tccggcaggc tgggcgcgct atggcagtga gctttccgcg caaacggaga   26100
gctggaacca aagctgacat ttaatagata tgctaactga gcacttacct tcgtcctgag   26160
aataggaata aaaggtagct cttcttaaga gaggcggtgc aaaggcacgc tataggagtt   26220
cagaaaaggc tggcggcggg aaatctgtag cctgggggct agtcaacatc ccctttcatt   26280
tcaagcactt attgatttgc tgttgtcatc tttggcgacg cagaaggaca cttgaaagaa   26340
tttctgatgg ggctctgatc tgagaaagga ggtgacctgc ccaggctccc accaaattct   26400
taattaccac atcaactgct ttttttatc ccccacccga cctccttcct tttgcttatt   26460
cttaactttt taattattca gaaactccct tacctctcag tggcttccct tctgcagcag   26520
ttttctattc gaaccttttc cccgcctttc cgtggtaggg cctgtatatt gatccctctg   26580
acctttggca catctgggcc ctctgaaatc tctcaatctt ttcagatttg aggatggcag   26640
gctccaccct ctccactgtg tgcacacact cagagatatg aaaacttaca cagactgcct   26700
tcaaacccag ggtatctaac agatgttccc tttccagttc gtctcttgat ctgaaatgcc   26760
tgcctgattc caacttggat accactcttt cttgcccttc ctttttcaaa gcagtttgga   26820
catgtgtgca agtgagccca gaacagctcc acccatattc tttaccaaac tgtaaataaa   26880
agaagaacta atgaagtaga ttggcatata gattgcatca agagcccgaa tccccagttt   26940
ctggattccc cattcaactc tggctgtcat ctacattgac agagtcattc taagcagagg   27000
cccagagaaa cctgcattgt gggacaacag gtaaagccac agtaaaaagt ggaataattt   27060
taaagtcatt ttattagaat gtaaattgta tttctgggtt ttgttcgtaa ccacctagtt   27120
ttaatatata cagagttaga caggaaaaaa taggtcaaca cagttattgg tactagagaa   27180
gacaaattcc atgggctcct cagtgaaaag aagatcccca aagtctataa tttttgatca   27240
tttaatttca tttataattg tgggaatgaa taagacacca actgctttat gtatttcatt   27300
tacatcaacc aatttgtgtt tccatcaaaa gcagttatac agaatttctt ttaacttctg   27360
gtagcaagtt cagaaaatga agcttacagc caccctgaac tggatacatc tcttgagctg   27420
accatttctg taagtgcagg aatataatat tgttcttcta tggtcttttt gcactctttt   27480
agggtttgca agtccttatc aggtctgaca tcactgtttg ggtctacatt cattacaagc   27540
aaatttgatt actatgctga ttttaaaaca gcctatttgg ccagcataat cttagttttc   27600
aaattataaa aacctttttaa tatacgaagt tcccagtttt tacctcctct agctccttgc   27660
tcattcaaaa cttctatttt aattggtgta agtaataata atttgtatta ctatttgtac   27720
tccttttact tttttggaga ttgggctgga ttccagagag aacaccagca tcaccaccac   27780
cacaaacaac aaaatctaaa agtaaagccc ttatttgcat gataattggt acttggaatg   27840
cttctgactt actcaatgcc accttataaa ggtaccttgt aaactttctt ggaatttcta   27900
gcaagagctt gtagtaactg gaacaactct ctgggaagat atcctctttg atgggctttc   27960
agtttctgga ggaatagatt gagagcaatt agggagggag gggacattgg aaattggcag   28020
ctacgtcagc tgaaacaagc ctgggttcag taaggtgact gatgttgtgg ttgattccct   28080
accccgagtt tctctttaat tggggcactg actcttccca ctttgggatc ccaaggcact   28140
cggtgtgtat gcagattcct cccttgtggt cttcaccatg tggtttcgta gcaggtctct   28200
ggttcaatga tattttatag tcatagccct catattcatt atcatgatct caatgtttag   28260
gcttttagtg tatttatatt aaacctgctt tattagtaag ctggagcaca caggagagat   28320
gggggcaagt aaggactcag cagagctcaa attcagacat gtttaaatgg ctttgactgt   28380
gtaaagtgtg gcaatgcttc ctgctgccct agctttccac tctaagcttc acatgtcctc   28440
tggctaatga agtgtgatat aggccacatg ctaggaataa tagtatttgt tgagaacaaa   28500
gtgaactcag gaaacctggt atacacaaaa tgcatc                             28536
```

<210> 2
<211> 28536
<212> DNA
<213> Artificial Sequence

<220>
<223> chemically treated genomic DNA (Homo sapiens)

<400> 2

gttttgggag ttaggttttt ttcgttggat gggaaaagga ggtgcggaaa ggggtaggcg 60
gtttgcgggt tagcggggat gagagttgtg gggagaacgg aggagagagt aaaattattt 120
tttttggaag tttaagggaa gggagttgag gtgagagaaa tggtttttt taggttttaa 180
gttttcgttt ttagtttgta ttatttaagt cggggaagtg ataatttttt ttaaatttcg 240
attttttattt tttgtttttt gtagattgat tgatgttttc gcgttgtttt agtttagtaa 300
ttaaattagg tcgaggtata tttttttat ttagagtttt taaattattt attaatttgt 360
aggagattta aatttttcgg gtaggtttcg ttagcgtttt tagtttttat ttttaaattt 420
tttttttag tttttttttt tttttcgtt ttttggtttt tttcgttttt tattaggttg 480
agaggcggag aggtcgggat cgttgggttt tgcggtttta gaagttaaag ttcgttcgtg 540
gggatcgttt tttttttat tgtttttaag tgaataatat ggtgggaggt gattttcgtt 600
taaagggttt ttatttgttt tttattgttg cgtgattttt tcgtaagtcg gaggtagagt 660
tttaaaaaga aagaaagtgt gcgttgagta ttgattttaa atcggggata ttatttgcga 720
tttttttaag tggtagtagt agtttttttgt agaataaaga tttagtgttc gagttttaaa 780
agttttagg taaagtttta tttagatgaa agtaattaag gcgaaaaaat aatgatattg 840
tcggttttag aaaatcggcg gttatttttt gttttaagtt ttttagcgtg gtgtttgttt 900
tcgtttattt tggttgtgcg gggggttgat aagtataata aaagatttta gtatattttt 960
ttttttattt ttataacgat ttttttagcg ttatgaggat gaattttttg ggattaagtg 1020
gtatttgttt ttatttgtta tgaaattaaa ttttatattt ttttaaagtt gaaaatcgcg 1080
gataaagaag gatcgggtgt ttaaagttta tttaaatatt tatatcgaat ttattttttg 1140
tttagatgtt agaggatggt agggagggtt agggttgtaa tttattttga tttgttttat 1200
tgttttgtag tttgtaaatt ataattttgt ataattttag gaataagtag gtttagaatt 1260
aatgggttaa tattatttaa aataaaatat cgggagtatg atttttgttt taattatata 1320
ttgttcgata agatttagga aattttattt taattttta atttttgagt tttttgagaa 1380
attgaagggt tgtagttatt agaaattatt tttgtttttt ttaatgtttt taaaggattt 1440
ggaaatagta atgtaatata atatgaagga ttttttttatt taagtttgaa gataaacgtg 1500
gaagtttaaa tattttgaat tggagatgtt tttttgtaaa tttattatag atgtattttt 1560
tttgagagaa atgtatataa attatatatg tatatatgta taaatatttt tttagaataa 1620
tttgttagag gtgtaggttt tttcgtaaag gagtaaattt gagagttatt ttaagttgat 1680
ggatttgtta aatttttttt tttttttttt tttttatat tatttgttag ttataatgga 1740
atttttttagg tttaagttaa agaaaaattg gagagataaa attagatttt gtagtttttt 1800
tttttttcgg gaatgttttt tttttttttt ttagttttg atgaatggtt attatttatt 1860
tttattaaat ttaaataagg attgttgttt tgtatgttta attaggtagg tagagggaat 1920
tggtttgttt aggaagtagt gattgagatg ttttggttaa gttagtgata gaggagggga 1980
gaaagaattt agattaattt gtatgtagta tattttattt ttatgaaata aaatatattt 2040
gtttatatt tgttgaaaag taaaataata atattgtacg aaatgttata tatagggtag 2100
gttgtatata gtagttttag aaatattatt gtatttatta gagaaattat tttaaaattg 2160
atatttatat atttttttata ataataataa tatgttagaa atatatagtg tggtatttag 2220
tatatatatt tttttgttcg taagcgaaaa attttaatcg tttttgtata atatgtttta 2280
ttttaaagtt taattttaa aaatattgtt gtgatattat taataattgt ttttataaaa 2340
ttaatttgat atttcgatat atatatattt ttttagttat ttaaatgtta ataatgttaa 2400
atttaaaaaa taataagttt atagtaatgt taaaatgtta tatttagtta aatatttgtt 2460
tgtgtatgtg tttttgtaat tgttagaaat atttgtagtg aaagatgtta gatattgagg 2520
atattttttt gaaattaaag gagttttttt tttgatttag tggttttttt tttttttatat 2580
agtttttttt tttttttttt tttttagtgt ttacgatttt ttagtataat ttttagtttt 2640
ttaagggcgg agttgtttta ttcggtaagg ttttaggatt tcggcgttgt gggtgcggtt 2700
tatacgggtc ggtttattgt atattggtaa gtatttaggt tggaggtcgg gtttttgtacg 2760
ttggcgtagt cgaagttgga gtgttgtttt gtttttagtt ttaggttggt taggttcgag 2820
ttatacgtgt ttttataaat atacggagga gtcggcggcg cgtaaggata ggtaggcgtc 2880
ggtatcgcgg aatttagcga cgggttattt aggttgttta agttatttag gttgttgaga 2940
ttggagttcg ggacgtttgt tattgttgag ggtattatgt tggacgatat gtttatggac 3000
gagatagagt tgggtgggga aaatatgttt tgtgatgata ggggggttgac gtttatagag 3060
ttgaagaagg ggaagttttt ggtggatagg gaggcggatg taaggttttt ggcggtttag 3120
ttgttgtagg aatagtttgg gtatatgtcg tcgtagggtt gtatgagttt attgaattgc 3180
ggttcgaagt tatttttgta tagttcggtt tgttggttgc gttttttttt tttttatttg 3240
gttcgacgat ttttgaatta aatttgggggg cggttggggt aagggagtaa atagatgtta 3300
tagtgtagat tattaaaatt tttatcggag gttaattttc gttttttttc gatatatacg 3360
ttagcgtatt tatatatttt ggtttcgttt tattgtatcg ttttgtatat taagatatta 3420
gggttagttt ttagttattg gttcgggttt ttattaagcg taggagattt ggtttgtttt 3480
ggtttgcgag ttgggattcg gagttacgtt ataaatttta gtcgaacgta tggagatttg 3540
cggacggttt gattatttag ttaggcgttt ttttaggttt aaaaatattt aatgtaaaat 3600
aaacgcgggg tagtaggttt ttttaatttt tttcggggta ttttgtaaat ttgtttttat 3660

```
tttaaagtta tagatttacg gatgaggaga aggggttgga agggtattag aggatcgttt 3720
ttttttttac gtaatttttt tttttttttt ttgattttta ttgtcgtttt ttattttttg 3780
gtacgtgttt ttttaatagg gattaggtcg ttaatatttt ttttcgttta gtaaaataat 3840
taaataaaga gtaaaagatt attttttcgt tagttcgtta attttaggag tttggtatat 3900
taaatttcgg gaattcggaa agggtagttt tggagatttt ttttttttc gttttgtttt 3960
tttttatttt taagtttatt ataggtttgt tcgcgcgtta ggtttagtcg ggtcgtttgg 4020
ttttgtaggc ggttatttag gtcggtcggt ttttattcgt gttcggtggt ttagtcgtaa 4080
tttcgatttt aatttatatc gggttttttt gtcgttttag acggcggttt ttgtgtattg 4140
gagagaggtt tggtttgaga tattcgagtt gatattagtg atgttttata ttatatattt 4200
tcgtcgggtt tagtcgtgta attcgttttt tttttttttt tttttatttt tgattttttt 4260
tttatttttt tttttttttg tattcgattg ttataaaaag tacgttttat ttttatttgg 4320
ttcgataagt agtcgttttg gaaggagagg tagttgtaag gagagtttag cgtcgcggtt 4380
ataaagtatt agggtggagt tgcggaatag cgggcggggt gggagggcgt tttcgaagga 4440
ttttagaaaa tttatagatt ttgtttttaa ttatttgtta tttttatttt aggttattta 4500
aattttgttt aggcgagaag agtacgtgag aggttcgttt ttttgatgtg taagagagtt 4560
aatgaaagat tgattttgtt taaaattacg tcgtttagga tttagttttg gttttggata 4620
gttaaattaa aattattttt aatttttttt cggtttttta tttattagta tagttttatg 4680
ttttgtataa atgttattta gagagtgttt ttattttttt tgatttggga gagtattttg 4740
gtttttattt tttttatcgt tgtttttttt tttttgtttg ttttgtttta atcggggggtt 4800
ttattttttt tatttagagt atttaatttt tttttttttaa tagtaaagtt tttggatgtc 4860
gtttgatttg tttgatttttg tttttttgttt ttagaatttt aataaatttg gaatttttta 4920
tcgattagta taaattagga cgttgttatt gggttattta tttgagttta tttttgttaa 4980
tttataaagt atagatttgt tataaagtta aggtaagttt tttttataaa attatgatta 5040
taatttagaa gaggggggtgt gagttttaat ttttagagtt taatttttga gagaagataa 5100
ataaattaag tagaaaagtt tttttttttt tttttttttt tttttaaga ggattagtag 5160
ttgtgtatta aaatttttgtt ttcggagatt ataaaattag gaaatagggt gtgtgggaga 5220
gatttgaatg gtcgaaataa tcgtaaagaa ggtgtaagaa gcgcgagttt aggagggaaa 5280
aagttgggtt agggtcggga taaaggtttt ttagggaggg ttaatttttt cgtgtttttg 5340
gcgggttttt tttgttaaag gtttataggt tggagtttgt tcgcggtttt tggtttggta 5400
gggattttat tagttttgtt ttggtaattg taagttagga atataatgtt ttgtgtaggg 5460
gattgtttat gtagtttagt tcgtgagatc gcgggatggc ggggtagtga gtcggtgtcg 5520
ttttgggagt ttgagttagg gcggtagttt tgtcggtttc ggagagggaa ttgtaatttc 5580
gtaattaggt cgtcgcgagg tttttgtttt ttgtaaagtt gcgttttatc ggcgtttttt 5640
taggcggcgt tgtttttat atttttttt ggtttatttg gtttgtattt ttataatatt 5700
tttttttat tttttttaga tttcgtgttg gtttttattc ggattcgggt tttcgtaagg 5760
ttggtttata tagcgatttt ttcgcgtgtg gatatgttcg ggtagcggtt tttttggaaa 5820
gtggttttta gtttttggag ttgttggttg gtaaagtgag ttcgttgtcg tttttgtcgt 5880
ttttttttag acgggtttc ggcgtttacg tttttatttt tttttgttg gtttttattt 5940
tttttgaaa acgaaatata tatatttttt cgttagtatg tttatttgta acgcggacgt 6000
taattggatc ggcggtagaa gtcgtggaag agttgggttg tttggcgtcg gaggagggtg 6060
cgcgcggcgg tttcgggtcg cgaggagcgt tgcgtttgtg gggtgtgtag gcgtaagtgt 6120
gggtgttcgc gttttatttt tttttttttt ttagcgtcgt acgttttatt tatatgttta 6180
ttttattgta gcggtatatt tatttttata gtttgtgttt ttaagtatat ttatatattt 6240
ttgcgtagat atattaaatt ttttgggacg cgtatacgcg cgtggtttat agattttttt 6300
tttttcgta gaaagtttag atttttatgc ggtttgggaa ggttaggaaa agatgtgggg 6360
attcggttgg gtatcgaagt tcgtcggttt ttttttaaaa aaaaaaaaaa aatgtttttt 6420
cgcgaagggt attttgagt ggttttaggt aatttttaa cgagtggagt ttttcgggag 6480
ttgaaagtcg agaggaaaat agggatagag gtcggcggtt tttgaaggtt ttcgaattaa 6540
gatgttggga tttttgtgat ttaggaaata gaagggaggt tagggtacga atagagaggg 6600
cggtagaatt gttcgcgttt ttagcgtttt aggagtcggg tcggtcgagg gagaattaaa 6660
gggatgcggg gtagttaaaa tttcggtttt cggaagtttt gcggggagtt aggcgaacga 6720
ttatttttat tacgtttttt ttcggagggg ttgatttttt tggggcgaga gggagcgggt 6780
ggcgtagagt agttgagcgg gaatgtttgt agggcggcgc ggcgttttat ttgcggtttt 6840
cgggttggag gtgtcggaga tggtgtgtat ttttagtttg tgtttggagg agtttagcga 6900
tcggggttga tcgggagtta gaatcgaagt tatggttaac ggttggggat ggtgatagga 6960
agatgaggag acggtcgata gtttggtttt cgttgttcgg tgttttaagt gaagcgggtt 7020
ttttatgtag tttatggacg agggagcgcg acgttttatt agtttttggt tattgtttcg 7080
tcgagttttc gtagtcgtcg ttgttcgttt cgggtcgcgt tttaggcgcg gagtttttc 7140

gttgcgggga gagttagggg acgtaatttt cgtcgagttt ttaagttaag ttgttttcgt 7200
ttttttcgga aggtttaagc gaaaaagttc ggagacggaa agttagcggg taaacgaaga 7260
tatgggatgt gggtagaagg gtattattta gagcgttttt agggagtagg tttttaagtt 7320
ttaaagcgaa ataagagtgg gtaaagattt tttttttttt tttttttttt ttttaagaat 7380
```

```
tttttaata aggaaagtta acgtcgatcg cgttttgttc gttttttttt tacgcggtag 7440
ttttgataga gaagtgttaa gagtgatagg gataggtagg tgatattaga tttttgcgg 7500
cggtagtagt cgttgtagtt acgacgcggt tttttgagcg tatttttcgt aacgcgtata 7560
cgtatatttt tcgggcggtc gaataggagt cgggttttgt cgtagtttag ttttaggtat 7620
ttaggcgagc gacggattag atttgcggtt tcgcgttttt ttgttggttt aatattttaa 7680
aattagaggc gggtttttg gtgtcgagac gttatttcgt cgcggttttt tttagttttt 7740
ttcgttttcg ttttttttta gatttttttt cgggtgcgat tgacgtggtt tcgtattaat 7800
taggacgttt cgagtcgcgg tggagggatt gttttgtttg tatttattag tagtgcgggg 7860
tcgggttatt gtttcgtcgt gcgtattggg tttatatagg taagttttcg ggaatttagt 7920
ttttgtttag tttaaggcga ttcggttttt agtacgaatt taaaggtgaa gagatgaggt 7980
taggagtcga aggtttggga gaagagagtg gaatggttaa gaagagaaag gtataaggat 8040
taataagata tttatttttt gtgttttatt atatttattt ttaatttttt atttatata 8100
aaaaggagat acgttattta aaattagaaa atttgaaaaa tagtaataaa ttatttttc 8160
gatttaaat tttttaaata gtttgttaag tgaatgttgc gttaatttga agaagtttta 8220
attgtaaaga agatagagtt ttgaaaaggt aggttaataa attagaaatc gagaagtaaa 8280
tggattcgtt aaaagaaaat tattttgatt ttaaacgaat aattgtttgg tggtttattt 8340
tggatttata taagaataaa aagtcgtttt agattacgtt ttttgtgatg tttattagtt 8400
tttagataga aaatatataa tagaagagaa attttaattt agcgttttta aaatgttgaa 8460
agtttattta ttttatttaa cgttgattaa gatatatatt ttagattttt taaatttttt 8520
gtatattgta ttaagttcgt tttaattcga gagagttacg ttttaaattc gatttttttg 8580
tttattttat tattaattag atttaaattt ataaagtttg tagaattaat aattttgagt 8640
taattatata tgaaatatgt tttaatgaat ttttatataa ttaagaatgt tgttaaataa 8700
ttaattttaa ggataatttt taatagttat tttttttttt tagtgagttt aaggttgttt 8760
tgagttatta aagtttaagt aggtagaagg ggtgtgtgtg agttaagggc gaaaagttta 8820
gaattgcgtt taattagtaa aagtaaaatt ttatttatat aaaataaaaa aaattatttt 8880
tggagatatt aatttttat agtattgttt ttaagtaaat ttaattttta aagaaattaa 8940
agaaagaaat ttaaatatat ttaaaataat ttttgaaagt tttttttgttt tttagtatag 9000
gttagttgga gaggataaat taattttttt tgggtttttg tatgggcgat tgttttatta 9060
tggagttagt gttattattt ttgaatgtgt atttgtttga tattatagtt aatgatttgt 9120
aatgttagta tgaagtattt ttaaaatatt tttttttgt ttttgtttat aagattggga 9180
aatttattcg atgtggaata aagtggatga agtagattat aaatatattt gtaatttatg 9240
tgtttttttt ttgttttgat tattttaaa ttttatttgt aatttttttt tattttaaat 9300
ttgtagttta aagacgtata tgagaattgt tttttagttt tttttatta gtattatttt 9360
attttaagaa taatttagtt gtaagggagg aatttttta tagtaagttt taaattagta 9420
tttttgtttt taattttta ttttatttta ttttatttta tatatataga tatttgttta 9480
gagtaaaata tatttttatg tgataggttt gtattagttg aggtttatat atttagttat 9540
attaggtttt gtaattttat tattaaatta tatatattat attagtagtt tgttggtaaa 9600
gaaggttaaa ttaatttata ttttgtttat tatttggtgt ttaaatgacg tattttattt 9660
cggagatttg gcggagaatt tttttttag atttatagc gttttattga agataatgtt 9720
tttatatttg tagtggtttt taatttgata agattttaat ttgtttaagt tttttaaata 9780
agggttttaa atgtttttag tcgtttttt attgaatttt ttttaatttt tttaagatta 9840
taaagtatat gtgtaaagta aatatttttt tttattgtat tgttagtcga tgatttataa 9900
ttaagttaat aagaatttag ttttttttg ttgaatgtgt ttattaatta tattttagtt 9960
ttttttttta aatttagaa tagttgtggt ttttataata ttatgttttt taaagtttta 10020
ttttatgaag ggattttatt atattaaaga atgaaaaaaa tttttattgt agttagtata 10080
tatagttttt tattttttgt tttttaagat ttaaatttta gagttgtaaa tatttttgga 10140
agtttgggtg ttaatgtttt attttagaaa gtcgagaagt tttatagagt tatatagatt 10200
tttaaattta ttttttataa atttatagaa ttttgataaa agttttggtg gttttatttt 10260
atcgatggaa tttttattac gataaatata tatgtatgaa ggattttaat tagtttttaa 10320
agtggttgaa aaatttaagg gtacgtgatt gtttttata gtgttaacgt gtgcgagatg 10380
ttggaagtat tggggattag tagtagttta gatgtttaaa aagataaggt gttttaattt 10440
gtgtggattt attgaagtta agtggtgaat aaagataatt atttagataa tttagattaa 10500
agtaaaagta aaattatatt tatttgtata tatatattta tatttatttt atattataga 10560
tatatatacg tatatatata ttggttttgt aaataattga tttaaagtga ggattttttt 10620
tgtatttttt tagtaggagt tttaatattt ttttaatttt ttaattattt tatatattta 10680
tagtagcggc gattgggtga tattttttt aggtttttg tgtggtagga tattaatatg 10740
ataagtttgt atggggaaaa ggaggtatgt ggtgggaatt aagaaatatt gtttagtgaa 10800
aattttgtgg tatggtggtg gttgattttg gagatttaat gtatataaga tttgtgggtg 10860
tataggtata ggtagtatgg atgagaaagg ggttagaaga aaataaattt tatgtatttt 10920
gtgattttag tattattgtg atttttggtt aagttttttt taattggttt tagaaattat 10980
tatgagttta gtttttaata tagaaatttt taatacggag aatattggtg ggattttggt 11040
agggaaatta gaggtgttgt atggtttacg tggggtaaag aaggaaagtt tagtgtcggc 11100
gtgaggtttt gagtttggga gatattaggg gttgtttcga ttggggtttt ttgtttattt 11160
```

```
ttttaaagaa agattttaga ggagggaaat gtgtgatatg gggttagtcg tgttttgtgt 11220
tggtatttgt tatcgattat tagttttaaa gttttattta attttatatt ttttagtgtt 11280
agttgtgtaa agtttttttg gttatggtag tgagcggttg ggttgtgtcg ttaaattttt 11340
cgtattaatt tggtttggga tttaattaag tgatttttga tttttggaaa gagtttgttt 11400
ttagagttta tttagaagat ggtttaatta gatatttttt tgagttgtta ggttttagac 11460
gggtgggagt tttgttttgt ttaagttagt ttaaggacga ggttcgtttg gatttagttt 11520
ggagttacgt gatgggcgtg agtgtgtgag ttttggtaa ggcgtagagg ttagatggag 11580
attttgtatt ttgttcgaga agtgttttat ttttttaat atttggtttt tttttgtata 11640
taaattaagt tgaaaatagt ttattattta ttattttta tagttatgga attaaataat 11700
ttagaaatta aaagttttat tgtagttgtt ttttttttta tttttaaat ggaatttaaa 11760
aagttttggt ttgttaaaag gggaagatta tttttgaat tggaagtttg tagatatatt 11820
gagtaatagt tattttttt gggtttttgt aaatggtatt tattttttta atttatagtt 11880
ttagttgttt aattatttga gatttggggt aattatttgg gggaatagtg tttagatggt 11940
agtgggagtt attattttat agtggtttgg ggaagagaag agaaagagat tagaggaggg 12000
ggtatttgtt aaaattattt aacgaatatg ttgttaatgt ttttttatat ttgtatgtta 12060
ttgttatagt ttttttaggt gttattgagt ttttagaaag taattatttg tcgaattaag 12120
taaaataagg agaatggtat agtatatgtg tttggagaag gggaaggaag ggtggaatat 12180
gaaattgagt atagatattt aggttaggaa agaaggaagt ggtaaggggt taaatgaagt 12240
tttatttttt cgttattttt ttaaataata gttggattaa atatttattt gttttttttt 12300
ttttttttt ttttttttt ttagtttatg tttattttt ttttttattt tttttgtttt 12360
ttttttttt tgttttttt tttttagata tgttggtagt taatatttag tattagttgt 12420
tatggtgatt ataaattatt taaatttaaa aatatttatt tataatttga gatgaagttt 12480
ttatttttt agcgaaataa tattttaaaa gttgttagtt gataaaaaaa aaggaattta 12540
ttttattgta gtaatttaag taatatatta tttttaaagg tttaaattaa aatgttagtt 12600
tgttaaaaat atgttggtag agttttggat attttttcg ttagattttt ataaagaagt 12660
tgattttgtt atttttggtc gttttttaat atatatatat aattttgtat gttttttttt 12720
tttttattaa ttttttttt ttttattaat tattttagtt ttttaaagatt ttacgtattg 12780
ggttttaaaa gaaaagaatt tttttttgga ttagaaaata gttttattgg ttgttgaagt 12840
gaaagatgtg gggtttaggg ggaaaggtta ttaggattat aatggcggtg gtggtaggag 12900
gttattttag aggagttaag aagaaaaaaa aatgtaggga gaaggattgg aggtggaaag 12960
atagagtaat agaaaattga gttgggtggt taggtgttgc ggtgaagttt agtttcgaaa 13020
tgataggtat atattttttt atttttgttt ttttttttt tgagagaaaa ttttttagt 13080
tagagatttt ggggggtagg aggcgggtaa acgtcgttgt agttgggttt tttgtttttt 13140
attttgggtt tgttgttttt tgtttatttt ggattatagg gtattcgttt agatgaagag 13200
ttattaatta tttatttagt taatattagg aagacgataa agtttttat ataggattaa 13260
gaagatatta gattgtttta ttagtatatt tttgtttacg aataagtttt cgttatatat 13320
ttttttttt ttcgagtcgt tttaataatt gttgtatata ttagtagcgg gcggtgagga 13380
ataatagtcg aattagtttt aagaaatttt gtgtatcgag ttagtagcga ggaacgcgat 13440
ttgtgaagat tatttttgcg ggtagggatt cgtagggatt gattattttc ggataattgg 13500
tataattttt tttgggggtg aaaaattata acgcggcggg gtattttta agtgagttgt 13560
agatttgatt ggcgcggggg gtgggggagg ggaggggaga atgggatggc ggaggtcggg 13620
cggaggaaag aaaatggaaa attttttta ttttatttc gttgtttttt ttttaagttt 13680
tatgtttttt tcggtaagta tttgtttttt tcgcgttagt tatagttaga gttttttatt 13740
tttttttata tattttttt tttttgataa ggtttaggat tttggttatt attttacgta 13800
ttattatttt gcgttttgtt agagacggtt tgggttgatt tcgttggtgt ttatgttagg 13860
attaaaattt ttttatgacg gcgaggaaaa ttgtattatt tgtttttagg gggtatatta 13920
ggagtttacg tagtatatgt ttcgtaaata ttcgttgatt gaatgagagg cgcgggggcg 13980
gggcggcgga gaggggtttg cggtcgttaa ggtcgttagg gttaatttag gtttttcgaa 14040
gaaggttggg atcgagttgt tgtcgcgtgt gaaggtgtgt gtcgcggttg ggggtgttat 14100
aacgggttat ggagtttatt ttttagaggg aggaagtttg tgtatattag cgatttgggt 14160
cgaatatttt tagtttattt attgggttaa agttatttaa taattaatgc gtttttgggg 14220
gaggtcgggg gaagtattcg tttttttgtcg ggacgtaaag ttaggcgtta ggtttaaagg 14280
ggttgtagtg tagttcgatt ttagtacgga atttagagtc gtcgttttga aattttttaa 14340
gttagtgata gaggagggtt agttcgtttt ttttttgagg gtgaagatta ttttatgagt 14400
ttttttttagg atttttaaag taagaaaagt taaagaaagg tttttttgtt ttagggggtc 14460
gtttttagtt ggtttttata ttattttttg ttagttgcgt ttattttttt ttaaattttt 14520
ggtttttagg ggtttttagt cgttttcgtg ttattttcgt ttcggggttt tatttaggtt 14580
gggtattcgt ttaatggata ttaaggagaa tgggatttat tagggaagga aggtagagtt 14640
tggattgttt agaggtggat tttgtttata tagaacgttt agtttttaac gaggatatgg 14700
tatttttggg cggcgttggg ggtagaggcg gagagggtag cgtaatagat tattacggtt 14760
ttttgaagaa gttatatgtt attgtgaatt ttttttttt ttaaaagtaa agaaaaattt 14820
taaaaaaata taagaaataa attttttgt tttataagta ggttgtggtt aggattttgg 14880
atattttata agttaattta aaattaggga aggataggtg ttttattttt tagtagtgtt 14940
```

```
atagttttgt ttattcgtgt gatttttttt tgtcgtttat tagtttttta aaagttaatt 15000
aaattaagat ttttagtatt tttttttatt atatgttttt ttttaattaa tggtattaaa 15060
cgtgtttagg tagtaatttt ttttttcggt taaaaagtag aaaaagatat attgagtgta 15120
ggggaagagt tttttacgtg tattaaaata atgcggttt gaaagtaatg gttaagaaag 15180
taattattta ttattttag tttttatgt gttagttatt aaaagcgaac gattaggggc 15240
gtttgcgcgg tttgtgattg gcgtaagtag aattttttg tttttagtcg tttttgttt 15300
atttacgagg attttatttt atcgtaggtt ttttttatttg tttttagaat gtattttta 15360
tgtttaggaa atttggggta gggattgggg gaaggagata ttttgcgttt ttttggtttt 15420
tagtataata agaaatgtta gtttcggttt ggcgattgcg agttcgtttc gcgcgaagcg 15480
agattgggga gttttttag tttggtcgga gttagggttg agttcgcgta aagtattttt 15540
tttagaagtt atcgttgttt ttgattttta attatatttt aaatatatta cggtttaata 15600
atttattttt attatcgatt attaaataga agaagaattt aatataattt aaatgataga 15660
aattacgcga cgttattttt gtattgtttt tatttaattt tatggggtta atttcggata 15720
agtgagcgtt taattggttt agtagggcga ttggcggtgt agtgtagtgt tcgggcgtga 15780
agtattggat cgttttagac gttttatttt aataaatgat tattttttt tagatttacg 15840
gggaaatttt aatgtaagat ttttgttttt tttttagtaa tacggtttgt tttttgatc 15900
ggggttttaa atcgtttttt tttattttat attatatttg tatttttata ttttaattcg 15960
gaaagagggg gttaggggtc gagggttgtg gggggggggg ggttttattt gtttatatta 16020
ttaaaggtta atagtttttt aaggttaggt atttatttat tacggagtta ggaaaataga 16080
ggaatagtaa atttgagggg tttttttta tgtatttgaa aagaaaggta ttttttttt 16140
tttatttttt atattttttt tttcgtttta tagaataagt tttaatttag gaaaggtttg 16200
tggcgtaggt tggagatttt ttaatttttt atataagttt gtagattttt tttggtaatg 16260
tttttcgatt tttttagagt gaaattagtt aattaagtaa cgatatcgtt aaaatttaag 16320
gtttggtaat tagtatttta ggtaggttcg cgtcgatagg ggtaaatttt tattttattt 16380
tgggttgtta agtatagtgg ttgtttttta gtttttttagg gatgttgtcg gtttttcgtt 16440
ttttttttaa ttagttaaag taaattttcg ttaatttaag ttttttttgt ttgtttttcg 16500
cgatgaatcg cgtatttata agtttgggtg gggcgtggtt gagagtttga gtgattgagt 16560
gggttttggt ggtgttgcgc gtagcgggat ataacgagcg atagaggtcg ttgttggatt 16620
atttttttac gttagtttag acgtcgaggt ttgttggagc gtgcgtaggg gattagatta 16680
tagggagcga gcgagaggga gagagaggtg ttgggtttta ggagtgtagt ataatttggg 16740
gaaaggaatt aacgtttttg ggacggttgt ttttcgtttt atttagaggc ggagtgttta 16800
agtttaagta gtaggcgcgt taggtttggc ggtttcgttt ttttgcgttt cgttcgaggt 16860
ttagagtttc ggaggcgggt gtttagcgcg cggtttgcgt tttttttttcg gttttattat 16920
tggcgttagg atgttgtcgc gggaagaatt tgttgttggt tgtttttttt cggtttttag 16980
gagagttcgt gaattcgatt tttttgattt cggagttttt ggagaagaga tatttaacgg 17040
tcgtcggttg tacgtttggg ttacgcgcgc gttcgtttta cgtgcggaga gaggcgtttc 17100
ggatcgcggt cgaaaggagt cggggacggg aggaggggga ggggcgaggt aggtcggagg 17160
agaaagaggg ataaagagta aagatttagt tagaggaaag agttgacggt attttcgttt 17220
ttcggatttt ttggtaattc ggggtaggat ggcgtatttt ttttgcgttt tttcggttgt 17280
cggcggtttt agtcgggagg agtaggttgg ggggtttcgg tatatagcgc gtcgttgttt 17340
tttagtttat cgtttcgtta tagggagagg ttattggcga tttggttttg atttttttttt 17400
tgtttaggtt ggtttttcgg ggaagcgttt ttcgttgggg tttcgtcgta gggttagtgt 17460
tttttttgtcg tttttacgtg gcgcggtttt tcgttcgatg attcgggtag gagaaggggg 17520
ttttttattta attgtatata cgtcgatatt agtttgcggt agttggtttt tatttttcgt 17580
tatttgtaaa atagaagaga aggaaggttg taagaagcgg cggtcgtcga gtgagtaggg 17640
tttagatgag attacgttat attagttgtt aggcgtttat tgtgtgttag gttttaggcg 17700
tgtttttttcg atttgatagt ttttggttgt gtagtagtat ttttagttta gtttcgggtt 17760
taggatattt atttattaag aggggatttt tttttagagt tgtcgtaaaa gtgtttagag 17820
gttagaggat tataaagtta tagtgtgttg gggaggttgt ggatttattt ttaagaattt 17880
cggtgtcggg gttaagaatt tatttgaacg taatggtagc gggagtgggt gggtggagag 17940
gattttttttt tttgggaagt tgtatgtaaa gattattttt tagtgtttgt ttattagttg 18000
gagttcggta aatatttgta gaatattagc gttaacgtgt ttttgtttta gatagtagtt 18060
tttttcggtt ttttgtaatt ttgaaacgaa cgggtttttg gtttagggtg ttttaggagc 18120
gagttgagtt cgggtttttt atttattagg agttattttt ttatatttag ttatattttt 18180
ttttagagat attaattcgg ttatttattt atttattata aataattatt ttaaagtatg 18240
atttaagatc gtagaggaga gatattgggt ggattgagcg agattgagga gagtagggta 18300
aacgtttttg gagggtttat tgttcgttaa ggacggagaa atagttttgg tataattgtt 18360
atttagtttt ttttttttt ttttcgggcg agttaaattt tttttacgtt tttaattata 18420
acgtagcgag ttaagtattt aacgcgtttt tttttttttg ttataggtaa gtcgggagag 18480
gtgggtttcg aggggtttta tcgggtgggt agaagagtcg cggttgtttt aaagataaga 18540
aaagaaggtt tagggttttt taggtttttt cgattttagc gtttgttttt ttttacgtta 18600
attagggtac gtcgacgatc ggagggttta tttcgcgcgg gtgcggggat cggggtggga 18660
gtaagcgttg tcgggttggc ggaggtatag aggcggggta gggagttgcg ggtttgtttt 18720
```

tggtttgagt atcgtttttt tgcgtttcgg tttttttga agggagttgg gttttgggga 18780
gtttttggtt aaggtcgttg tttataggag gggttgttcg gcgttgtggc gtggggattt 18840
agggtgggga cggttaggcg gttttttat tcgttagcga gaacgcgggc ggggattttg 18900
tcgattcgat ttttgtgggt tcgtgggttt agaagtagta gtttggcggt tttagattta 18960
gtgattttgt agtaaaatta taggattagt ttttgattga gatgtttgtt cgtgagatat 19020
tataaaattt attattatag tttttatta attcgatatg aagtaatata gatgggattt 19080
tattagttta gatttaaat gtttatttat gataatttcg gaggaaattt gtatgttatt 19140
attatttcga taatttttt tttttatatg tttgaattgg ttgtattatt agttggtagt 19200
cggagtattg tagatggtaa ttgtaaatag ttttattta tttatttttt ttaaagaatg 19260
aaatatataa aagaaaaaga ttgcgttgtt tggtgtaaag ttagttaatt attatatatt 19320
ttttttttta ttttttcgtg ttttagtgtt gaagattaaa taaagtaata taaaataaat 19380
ttttaagaat ttatagagtt ttattttaag gattgaaaag aaggttaagg cgtgtttttt 19440
agtttatttt tatatgtttt tgtgatttgg agatttattt tgtagttaaa atgagttttg 19500
agatttgtat ttttatgttt tatttaatga ttaggtttat tagaagaatt gagtttaaat 19560
aattggggaa gataattttt taaaaagaga tttttaattt tcgtttgttg atttttaaat 19620
ttgttttatt aagataagtt ttttgtgaga aatttggttg ttagatttcg gaattggttt 19680
taatggttaa ttttataaat tgagatggga gattttttt gatgggaggt agtttttatt 19740
tttaaagttt atgtttagt tggaatgtat atgttaagga tttttgtttt ggttaatttg 19800
ggttttatat tgtgagtata taaaaagtat tatacggtta acggaggacg aggaattatg 19860
gtaaagtagg taggtaagtt ttaagaaata aaataatttg ttaaaaaata atttttgatg 19920
attatcgtaa gattgaaagt gtaggaaaaa tatagttcga ataatttag atttttttat 19980
atttttttt ttttatata ttttgttatt ttataataaa atttttaatg gaaagtttaa 20040
aaataaatag tataggaata tgtgttttaa atgaattaaa ttgtgaaatt agttagtaaa 20100
ttaatttgta gtaagtaatt atttaaggaa attaaaatat tgtttagttt agttttgtat 20160
tttattatgt gtatgcgttt tttataatta attaatataa gtgttttagg aatatttgaa 20220
gataaatacg tttaatttaa ggaataaagt atttaaataa tttaagtgta attttgttga 20280
gttaaagtaa aatattttat aaatgaagtg gttatttaat tttttaggga aagtttggtt 20340
attgaaatgt tgtatgttta tgttatatta ataaaaattt ttaatttatt ttgtttatgt 20400
gttttgtttt tttgatatta ttggtatttg aattttagat ggattttgt taaaatgata 20460
ttttgtgtga taaaagtatt tttagttttg attgatagat taaaataaat gtaaggaaat 20520
ttttttaaat tagattaatt ttttataaaa atattttaga atgtatgaat tttgatattt 20580
atatttataa tggtaaaagt ttttttcgtt tagtttagta agataatatt tatataaaag 20640
agtaaaaaaa aattatatta ttttatgata gtttgatttt taaattgttt aagaaagtaa 20700
agtggttaaa ttggaaaaga ggaatatatt tcggaggttt agaatcgaaa attttttttt 20760
taatttttag ttggaaaata atttttgta tttatttaaa gtgtattttt tgaagtgtta 20820
gattggagtt gattggtgat taatttaaag gagttataat ttaaagaaat ggtgagagtt 20880
tggtatttag gtttggtttt taggtaattc gtttgggttt gagaggttat taattgttag 20940
ttaagatgga attttttttt tttttttttt tttttaatgg ataataatgg gaagggggtt 21000
aatttttag tagttgaaat tttgtattta gtttttatt ttgagaatgt taatttttgg 21060
ttcgaggatt tgtttttgta gtgttggtat cgagatttaa gggaagatat ttcgttttaa 21120
atgttagtta cggtttggtt tttttttcga ttttagtatt ttgtagattg ttagtgtttg 21180
tggcggggga cgaaaggaat agggtttgt aaggtttgtt tgtcgattgc gttattttgg 21240
gcgaaattta gttttaaaag ttataaatta tttacggtga agatttttcg aagtggaata 21300
aattttaga ttcgtattat tttatatttt tgcgggatag atggttttta tttatcggtt 21360
atcgggagag agttgttgtt ttcgcgtttt attgtttttc ggggcgattt ttagcgagtc 21420
gagttttcgg ttgtacggta agcgttcgaa agtcgggttt gagaggattg tagggttttt 21480
gagggtgtta agtttcgaag gagtttacgg gtgtattggg gttttcgaaa tttagtcgtt 21540
attggtagtt tttttttgtt tttttttagt tttttcgttc ggtttcgtat ttttttttt 21600
tttttttttt tttatttttt ttttttttt ttgtttttat ttcgtgtggg gagtgacgtg 21660
acgttagtag agattttatt aaattttatt gtatagtggc gcgcgggcgg tcggtcgagt 21720
tcggttgcgc ggttggcgat ttaggagcga gtatagcgtt cgggcgagcg tcgggggag 21780
cgagtagggg cgacgagaaa cgaggtaggg gagggaagta gatgttagcg ggtcgaagag 21840
tcgggagtcg gagtcgggag agcgaaagga gaggggattt ggcggggtat ttaggagtta 21900
atcgaggagt aggagtacgg atttttattg tggaaaggag gattagaagg gaggatggga 21960
tggaagagaa gaaaaagtaa tttgcgttaa ttcggtagtt ttaataaatt aaagggggag 22020
cgttagggta gcggggagat agaaacgtat ttttggggag taaattagga cgggttggga 22080
ggaagcgata gggaaagtgg tttaagagac ggaataaagg ataatgttta tggggttgtt 22140
tgggacgagg cgtgtggagt gtgggtgtga gcgtgcgtgt gtgatttttt tttaggtttg 22200
tagagttgag gaaagaggtt atagtaaaga gggattgcgg agggaggaaa gtgagagatc 22260
ggtagagggc gggagtggag gtgggcgcgg tggggatggg agaggatgag tgaagagaaa 22320
tttagaagaa tggagtgagt tagtgggaga gggtgggagg gttatagtcg ggagcgaacg 22380
agttaggttt gttagttggg gaaggtcggg acgttgggtt tagtttagtt gggatatcgc 22440
gttcgaggtt aaggcgggtg gattaggtat gttgagagtg tcggcgtata ggtgggtacg 22500

gttacgtatt gatttagtgt ttacgaaggg tttgtattgg ataaggttta gacgtttata 22560
gagtttagaa ttttttttgt tgtatttata tttaataagt ttattttggg ttacggatat 22620
tttatttttt aaaatgacga ggttaaggtt tttggcgagg atggtattaa attgtacggg 22680
atagaagtgg gggtggggga gagagttttt tttaagttta tatttgtttt tgtaaagtaa 22740
agagtatgtg aaattatagg gtatattttt attcgaaaag tgtgttttat ttttgaattt 22800
tgattttttg atttttttgat ttgagtaaag atgtgtattt tggtagtgag tagaatattt 22860
tggttttgtt ttgtttttga gtggaaggat tataaatata attcgtttgg aggattaggt 22920
gtgaaggttt ttgttaggta tatgggataa tgttttttta attttaaggg tattttgtta 22980
atgtatgttt ttggaaagtg tcggaatata gttattgttt ttggattcgg atttttttat 23040
taatattaat ttttgtttga gagtaaaatt taggttcgtt attaaaaaga tattttttttg 23100
gtttttaatt gagaataaag ttttttttaa aagttgtatt gtttttttta aattaatata 23160
ttaatattcg taattttaga aatatatagt gattcgggag aatgtgtata aaatagatac 23220
gtttaaaaaa gtttggcgtt taaaattaat tttagttatt atataggtgt tgggtttttt 23280
ttatttttgg gggttgtttg gaatatgtta tgtgtttttt tgaattattt cgtgttttga 23340
atttatttga gttagtagta aaaataggta aataaatttg tttaatttgt tttgagtgtt 23400
aaattttttt attttgaaat agttaatagt cgatagatgg atttatttta tggaaagggt 23460
tagtttttttt agttacgaag aaaattgatt agagatttat attttaagtt atttttaatt 23520
tttacgtaat attcgtgaaa atttaaattt ttttttttta tttagtggaa atttaaagta 23580
gtgttattta aggggagaga aatgaggggg aaaatgttta cgtgttgttt aattgtattt 23640
ttttttgat tttgagaatt tttattttg gtttttgaaa tttcgtcgag gtaagaaaat 23700
taaatttttt taataagttt tataattgaa ttttagttat aggatatcgg aaagtgtagt 23760
tcgagaaaga tatttttatt tttgtttatc gacgattttt gtagtttttt tattttttttg 23820
agtaatgggt taataatttt tttttttttt tttttttattt tgtagagatt aagaggcgtt 23880
cgtagtagaa cggttttgtt tttagttggt ggcgaggata ggtaatttta tggaaaagtt 23940
ggaagagaat gagaaaatta aagatagaaa gatttagaga ttcgcggaga gatataggga 24000
gagggaaggg agttgcgttg aaaagacgta aagatacgcg cgtgtaattt tttttttttt 24060
taggttttag aggtttgtaa attagggttg agaggaaggg gttcgggaag tttacgtttt 24120
tttcgttttt tttttgtttg gagtttcgtt cgttagaggt tggttaattt tagtttcggt 24180
cgtcgtagat attgcgttga gtttttgggt tttcgttttg tttagcgtta gtgtagttga 24240
agtgagtagt tggtgggaaa tgtaaatggt ttttggagaa atagaagata tagaatgatt 24300
tttatttttt tttcgagtgt gtggaaggag ttggatatac gttttacgtt tttaattttt 24360
tttttatatt tttagttata tttttattaa ataattaatt aatgtttaga attattaggg 24420
aatatattag gtatgtaatc gtagaagtag ggtgttgggg ggttataaat tatcgagttg 24480
atttaagacg tggattttag gttttttttt tgttaaagta gtaaaggaag agcgggtttt 24540
ggcgattgta tttagatttc gattatttta aattagaagg gggtggaggg agcgtttaag 24600
taaagtaagt aatttttttgt tttgtagatg taaataagat tgtagtatta aaggtattag 24660
tttttttagg gttagatcgt ttggattggg agtttgggga aggggagata ttaattttac 24720
gtatttgtga attttaagga tgttatattt ttatataaat aattttagtg cggattttttt 24780
ggaatggggg gagtaatatt tttattttag aatattaaaa tatttttttt ttaaagcgta 24840
tatttttttt attttttttaa aattttgaat tatgtttaaa gataatagtt ttttagtaaa 24900
ttggagtatt ggattatttt ttttattttt ttttatcgat attttgatga tttgatttta 24960
atgtgtgggg ggtataggga attaaatata gtttataaaa ttaagtttag atgaaatagt 25020
gttggttaag tgggtttaga taatttttaa tgagaatttt aattatattt tttttttttaa 25080
tatgttgaga taagtgatag aatcgttaga atggtaatta aattggaaag tttagggaga 25140
ataataattt cgtgattaaa ttggggtaaa atcgtggata aatgtggggt gattttcgtt 25200
aatttttttgt tatttaagag ttaggatttg ggaaaggtat agtattattt tagagttcgt 25260
tgtgacgggt tgtgtgttat tatttatttt ttttattttg gattatgatt ttaatttttgg 25320
taagtaattt ttttagtttt ttatttgata ataagcgagt atgtaaatat taatggttag 25380
cgatgtttaa ttgttttaaa tattattgat ttgttggttg ttttaaattg ttttttttagt 25440
ttaggttttg ttttcgaatt gtttatttta gaggtttgat ttatgttttc gatgttataa 25500
tataataatt gttttttttaa aaaaggtatt taagatgaat taattgattt gtatataaat 25560
taaaattatt atgcgttgtc gatttcggtg ttttataatt atttcgaaat tagtatttaa 25620
ttatttgagt taaaagaata tataaatgtt tgtattgatt tattaatgaa ttatttaatt 25680
aaaacgttcg ggtaatgttg ggcgttggaa agattgttaa attaagatat attataggag 25740
ggatatgaag attagaaagg taatagatta atatttcgta tttaaaacgg agttttcggt 25800
gatttttagt tttaattttg gagtaggggt ttttttttttt tgttgttaaa aagattttgt 25860
gtttgtttgt gagtgagtgt atttaagtgg aaggaacgtt tttacggtta cggtggttta 25920
ggtttttttgt tcggatcggg attttatagt tttaatttag gagcgttaaa ttttggaaga 25980
tttcgggtta gttttggagg tgcgtggttt cgtaagtcgt taggttaagt ttgttttttt 26040
tgtttgtttt ttcggtaggt tgggcgcgtt atggtagtga gttttttcgcg taaacggaga 26100
gttggaatta aagttgatat ttaatagata tgttaattga gtatttattt tcgttttgag 26160
aataggaata aaaggtagtt tttttaaga gaggcggtgt aaaggtacgt tataggagtt 26220

```
tagaaaaggt tggcggcggg aaatttgtag tttgggggtt agttaatatt tttttttatt   26280
ttaagtattt attgatttgt tgttgttatt tttggcgacg tagaaggata tttgaaagaa   26340
tttttgatgg ggttttgatt tgagaaagga ggtgatttgt ttaggttttt attaaatttt   26400
taattattat attaattgtt tttttttatt ttttattcga tttttttttt tttgtttatt   26460
tttaattttt taattattta gaaatttttt tattttttag tggttttttt tttgtagtag   26520
ttttttattc gaattttttt ttcgtttttt cgtggtaggg tttgtatatt gatttttttg   26580
atttttggta tatttgggtt ttttgaaatt ttttaatttt tttagatttg aggatggtag   26640
gttttatttt ttttattgtg tgtatatatt tagagatatg aaaatttata tagattgttt   26700
ttaaatttag ggtatttaat agatgttttt tttttagttc gtttttttgat ttgaaatgtt   26760
tgtttgattt taatttggat attatttttt tttgtttttt tttttttaaa gtagtttgga   26820
tatgtgtgta agtgagttta gaatagtttt atttatattt tttattaaat tgtaaataaa   26880
agaagaatta atgaagtaga ttggtatata gattgtatta agagttcgaa tttttagttt   26940
ttggattttt tatttaattt tggttgttat ttatattgat agagttattt taagtagagg   27000
tttagagaaa tttgtattgt gggataatag gtaaagttat agtaaaaagt ggaataattt   27060
taaagttatt ttattagaat gtaaattgta tttttgggtt ttgttcgtaa ttatttagtt   27120
ttaatatata tagagttaga taggaaaaaa taggttaata tagttattgg tattagagaa   27180
gataaatttt atgggttttt tagtgaaaag aagattttta aagtttataa tttttgatta   27240
tttaatttta tttataattg tgggaatgaa taagatatta attgttttat gtattttatt   27300
tatattaatt aatttgtgtt tttattaaaa gtagttatat agaatttttt ttaatttttg   27360
gtagtaagtt tagaaaatga agtttatagt tattttgaat tggatatatt ttttgagttg   27420
attatttttg taagtgtagg aatataatat tgttttttta tggttttttt gtattttttt   27480
agggtttgta agtttttatt aggtttgata ttattgtttg ggtttatatt tattataagt   27540
aaatttgatt attatgttga ttttaaaata gtttatttgg ttagtataat tttagttttt   27600
aaattataaa aattttttaa tatacgaagt ttttagtttt tatttttttt agtttttttgt   27660
ttatttaaaa tttttatttt aattggtgta agtaataata atttgtatta ttatttgtat   27720
ttttttttatt tttttggaga ttgggttgga ttttagagag aatattagta ttattattat   27780
tataaataat aaaatttaaa agtaaagttt ttatttgtat gataattggt atttggaatg   27840
tttttgattt atttaatgtt attttataaa ggtattttgt aaattttttt ggaattttta   27900
gtaagagttt gtagtaattg gaataatttt ttgggaagat atttttttttg atgggttttt   27960
agtttttgga ggaatagatt gagagtaatt agggagggag gggatattgg aaattggtag   28020
ttacgttagt tgaaataagt ttgggtttag taaggtgatt gatgttgtgg ttgatttttt   28080
atttcgagtt tttttttaat tggggtattg attttttttta ttttgggatt ttaaggtatt   28140
cggtgtgtat gtagattttt tttttgtggt ttttattatg tggtttcgta gtaggttttt   28200
ggtttaatga tattttatag ttatagtttt tatatttatt attatgattt taatgtttag   28260
gtttttagtg tatttatatt aaatttgttt tattagtaag ttggagtata taggagagat   28320
gggggtaagt aaggatttag tagagtttaa atttagatat gtttaaatgg ttttgattgt   28380
gtaaagtgtg gtaatgtttt ttgttgtttt agttttttat tttaagtttt atatgttttt   28440
tggttaatga agtgtgatat aggttatatg ttaggaataa tagtatttgt tgagaataaa   28500
gtgaatttag gaaatttggt atatataaaa tgtatt                             28536
```

<210> 3
<211> 28536
<212> DNA
<213> Artificial Sequence

<220>
<223> chemically treated genomic DNA (Homo sapiens)

<400> 3

```
gatgtatttt gtgtatatta ggtttttga  gtttattttg tttttaataa atattattat    60
ttttagtatg tggtttatat tatattttat tagttagagg atatgtgaag tttagagtgg   120
aaagttaggg tagtaggaag tattgttata ttttatatag ttaaagttat ttaaatatgt   180
ttgaatttga gttttgttga gtttttattt gtttttattt tttttgtgtg ttttagttta   240
ttaataaagt aggtttaata taaatatatt aaaagtttaa atattgagat tatgataatg   300
aatatgaggg ttatgattat aaaatattat tgaattagag atttgttacg aaattatatg   360
gtgaagatta taagggagga atttgtatat atatcgagtg ttttgggatt ttaaagtggg   420
aagagttagt gttttaatta aagagaaatt cggggtaggg aattaattat aatattagtt   480
attttattga atttaggttt gttttagttg acgtagttgt taatttttaa tgtttttttt   540
tttttaatt  gtttttaatt tattttttta gaaattgaaa gtttattaaa gaggatattt   600
ttttagagag ttgttttagt tattataagt ttttgttaga aattttaaga aagtttataa   660
ggtattttta taaggtggta ttgagtaagt tagaagtatt ttaagtatta attattatgt   720
aaataagggt tttattttta gattttgttg tttgtggtgg tggtgatgtt ggtgtttttt   780
```

```
ttggaattta gtttaatttt taaaaaagta aaaggagtat aaatagtaat ataaattatt   840
attatttata ttaattaaaa tagaagtttt gaatgagtaa ggagttagag gaggtaaaaa   900
ttgggaattt cgtatattaa aaggttttta taatttgaaa attaagatta tgttggttaa   960
ataggttgtt ttaaaattag tatagtaatt aaatttgttt gtaatgaatg tagatttaaa  1020
tagtgatgtt agatttgata aggatttgta aattttaaaa gagtgtaaaa agattataga  1080
agaataatat tatattttttg tatttataga aatggttagt ttaagagatg tatttagttt  1140
agggtggttg taagttttat tttttgaatt tgttattaga agttaaaaga aattttgtat  1200
aattgttttt gatggaaata taaattggtt gatgtaaatg aaatatataa agtagttggt  1260
gttttattta tttttataat tataaatgaa attaaatgat taaaaattat agattttggg  1320
gatttttttt ttattgagga gtttatggaa tttgtttttt ttagtattaa taattgtgtt  1380
gatttatttt tttttgttta attttgtata tattaaaatt aggtggttac gaataaaatt  1440
tagaaatata atttatattt taataaaatg attttaaaat tattttattt tttattgtgg  1500
ttttatttgt tgttttataa tgtaggtttt tttgggtttt tgtttagaat gattttgtta  1560
atgtagatga tagttagagt tgaatgggga atttagaaat tggggattcg ggtttttgat  1620
gtaatttata tgttaattta ttttattagt ttttttttta tttatagttt ggtaaagaat  1680
atgggtggag ttgttttggg tttatttgta tatatgttta aattgttttg aaaaaggaag  1740
ggtaagaaag agtggtattt aagttggaat taggtaggta ttttagatta agagacgaat  1800
tggaaaggga atatttgtta gatattttgg gtttgaaggt agtttgtgta agtttttata  1860
tttttgagtg tgtgtatata gtggagaggg tggagtttgt tatttttaaa tttgaaaaga  1920
ttgagagatt ttagagggtt tagatgtgtt aaaggttaga gggattaata tataggtttt  1980
attacggaaa ggcggggaaa aggttcgaat agaaaattgt tgtagaaggg aagttattga  2040
gaggtaaggg agtttttgaa taattaaaaa gttaagaata agtaaaagga aggaggtcgg  2100
gtgggggata aaaaaaagta gttgatgtgg taattaagaa tttggtggga gtttgggtag  2160
gttatttttt tttttagatt agagttttat tagaaatttt tttaagtgtt tttttgcgtc  2220
gttaaagatg ataatagtaa attaataagt gtttgaaatg aaaggggatg ttgattagtt  2280
tttaggttat agatttttcg tcgttagttt tttttgaatt tttatagcgt gtttttgtat  2340
cgtttttttt aagaagagtt atttttttatt tttattttta ggacgaaggt aagtgtttag  2400
ttagtatatt tattaaatgt tagttttggt tttagttttt cgtttgcgcg gaaagtttat  2460
tgttatagcg cgtttagttt gtcggagggg tagatagaaa aagtaagttt ggtttggcga  2520
tttgcggggt tacgtatttt tagggttggt tcggagtttt ttagagttta acgtttttgg  2580
gttagaattg taaggtttcg gttcgagtaa agggtttgag ttatcgtagt cgtgggagcg  2640
tttttttttat ttgaatgtat ttatttataa ataagtataa aattttttta atagtagagg  2700
agaaagattt ttgttttaaa attaaagttg ggaattatcg gaaatttcgt tttgagtgcg  2760
agatattggt ttgttatttt tttaattttt atattttttt tgtaatatgt tttgatttaa  2820
taattttttt agcgtttagt attgttcgga cgttttaatt gggtaattta ttagtgagtt  2880
aatataggta tttatatatt tttttagttt aagtggttaa gtattaattt cgaaatgatt  2940
ataaaatatc ggaatcggta acgtatagta attttaattt atatgtaaat taattggttt  3000
attttaaatg ttttttttaa aaaaataatt attgtattgt agtatcggag gtatggatta  3060
aatttttaga atagataatt cggaaataag atttggatta ggaagataat ttagaatagt  3120
taataaatta ataatgtttg aggtagttaa atatcgttag ttattggtat ttatatattc  3180
gtttgttgtt agataaggag ttggggaaat tgtttgttag ggttgagatt ataatttaga  3240
gtgaagaaag taaatggtag tatatagttc gttatagcgg gttttgaaat aatattgtat  3300
tttttttaaa ttttgatttt tgggtgatag ggagttggcg gaggttattt tatatttgtt  3360
tacggttttg ttttaatttg attacgaaat tgttgttttt tttgagtttt ttaatttgat  3420
tattatttta acggttttgt tatttgtttt aatatattgg ggggaggagt gtaattgaga  3480
tttttattaa aaattatttg aatttattta gttagtattg ttttatttaa gtttagtttt  3540
atgggttgta tttaattttt tgtgtttttt atatattaaa attagattat taaaatgtcg  3600
gtaggaaagg gtgaaggaaa tggtttaatg ttttagttta ttggaagatt attattttta  3660
gatatagttt aaaatttgga ggaaataaaa aggatatacg ttttgggggg aaaatgtttt  3720
aatattttag aatgggggta ttattttttt tattttagag aattcgtatt ggagttgttt  3780
atgtaaaaat gtaatatttt tgaaatttat agatacgtaa ggttagtgtt ttttttttt  3840
taggttttta gtttaggcga tttagtttta aaggagttag tatttttgat gttataattt  3900
tgtttatatt tgtagggtag agaattgttt gttttgtttg gacgtttttt ttatttttttt  3960
ttaatttgaa gtaatcggaa tttaaatata gtcgttaagg ttcgtttttt ttttattgtt  4020
ttgataaggg aaaaatttga aatttacgtt ttaaattagt tcggtggttt gtagtttttt  4080
agtattttgt ttttacgatt gtatgtttaa tgtatttttt ggtgattttg ggtattaatt  4140
agttgtttaa taggagtatg attaaaaatg taaaagaagg attaggagcg tgaaacgtat  4200
gtttagtttt ttttatatat tcgaggaggg aatgagaatt attttgtatt ttttattttt  4260
ttaggagtta tttgtatttt ttattagttg tttattttag ttgtattggc gttgggtaag  4320
gcgaggattt aaaagtttag cgtagtgttt gcggcggtcg ggattggggt taattagttt  4380
ttggcgggcg agatttaga tagaaggggg gcgagaggaa cgtgagtttt tcgagttttt  4440
ttttttagt tttggtttgt aaatttttga aatttgaaag gggagggagt tgtacgcgcg  4500
tatttttgcg ttttttagc gtaatttttt tttttttttt tgtgtttttt cgcggatttt  4560
```

```
tgaatttttt tgtttttggt tttttatttt tttttaatt tttttatgag attgtttatt 4620
ttcgttatta gttgaaggta aggtcgtttt gttacgagcg ttttttaatt tttataaaat 4680
gaaaagaaaa aaagggagga ttattagttt attatttaga ggaatgggga ggttgtaaaa 4740
atcgtcgatg ggtagaggtg aagatgtttt tttcggattg tatttttcgg tgttttgtaa 4800
ttagagttta gttgtgggat ttgttgaaga aatttgattt ttttgtttcg gcgagatttt 4860
aaaaattaga aatagaaatt tttagagtta gagaggaaat ataattaaat agtacgtggg 4920
tattttttttt tttatttttt ttttttaaa taatattgtt ttgagttttt attgggtaaa 4980
gagagaaagt ttgagttttt acggatgtta cgtggaggtt agaaatggtt taaaatgtag 5040
atttttaatt agttttttc gtggttgaag aggttaattt tttttataaa atgagtttat 5100
ttgtcgattg ttagttattt taaagtgaag ggatttagta tttaaaataa attgagtaag 5160
tttgtttgtt tgtttttatt gttaatttaa atgaatttaa aatacggagt aatttaagaa 5220
aatatataat atgttttaga tagtttttaa aagtagggaa agtttagtat ttatatagtg 5280
attagggtta gttttaagcg ttaagttttt ttaaacgtat ttattttatg tatattttttt 5340
cgagttatta tatattttta aaattgcgag tattggtata ttgatttagg aagagtaata 5400
taatttttag agggaatttt atttttaatt agggattaaa gagatgtttt tttaatagcg 5460
ggtttgagtt ttgtttttaa gtaggaatta atattggtgg gaaaattcga atttaggagt 5520
aatggttgtg tttcggtatt ttttaaaaat atatattaat aggatgtttt tgagattgaa 5580
aaaatattgt tttatatgtt tggtagaagt ttttatattt ggttttttag gcgaattata 5640
tttatagttt ttttatttag aggtaggata gagttaaaat attttgttta ttattaaaat 5700
atatattttt gtttaagtta agaaattaga aaattagggt ttagaagtaa ggtatatttt 5760
tcgagtgaga atatgttttg taatttata tattttttgt tttgtaggag taaatgtgga 5820
tttgagggaa atttttttt ttattttat ttttatttcg tgtaatttaa tattattttc 5880
gttaggaatt ttaatttcgt tattttaaaa aatgagatat tcgtgattta gggtgaattt 5940
gttgaatgta ggtatagtag aggaaatttt agattttatg agcgtttgag ttttgtttag 6000
tgtaaatttt tcgtgaatat tgggttagtg cgtggtcgtg tttatttgtg cgtcgatatt 6060
tttagtatgt ttggtttatt cgttttgatt tcgggcgcgg tgttttagtt aagttgggtt 6120
tagcgtttcg gttttttttta gttgataagt ttagttcgtt cgttttcggt tgtggttttt 6180
ttattttttt ttattagttt attttatttt tttagatttt tttttattta ttttttttta 6240
tttttatcgc gtttattttt attttcgttt tttatcggtt ttttattttt tttttttcgt 6300
agtttttttt tgttgtgatt tttttttttta attttgtagg tttgaaagaa ggttatatac 6360
gtacgtttat atttatattt tatacgtttc gttttaaata attttatgaa tattgttttt 6420
tgtttcgttt tttgggttat tttttttgtc gttttttttt agttcgtttt gatttgtttt 6480
ttaaaagtac gtttttgttt ttttcgttgtt ttggcgtttt tttttgatt tattagggtt 6540
gtcgggttgg cgtagattgt tttttttttt tttttatttt atttttttttt ttggtttttt 6600
tttttatagt gggagttcgt gtttttgttt ttcggttggt ttttaagtgt ttcgttaggt 6660
tttttttttt ttcgtttttt cggtttcggt tttcgatttt tcggttcgtt ggtatttgtt 6720
tttttttttt gtttcgtttt tcgtcgtttt tgttcgtttt tttcggcgtt cgttcgggcg 6780
ttgtgttcgt ttttggatcg ttagtcgcgt agtcgggttc ggtcggtcgt tcgcgcgtta 6840
ttgtgtagtg gagtttggtg gaatttttgt tgacgttacg ttattttttta tacggagtag 6900
gagtagaggg aagagagagg gatgagaggg agggagagga gagagagtgc gagatcgagc 6960
gagaaagttg gagaggagta gaaagaaatt gttagtggcg gttagatttc ggaggtttta 7020
gtgtattcgt ggattttttc ggaatttggt atttttagga gtttgtagt tttttttaggt 7080
tcggttttcg ggcgtttgtc gtgtagtcgg aggttcggtt cgttggaaat cgtttcggga 7140
agtagtggga cgcggagata gtagtttttt ttcggtagtc ggtaagtgga ggttatttat 7200
ttcgtaggga tgtgagataa tgcgagtttg gaaatttgtt ttatttcgga gaatttttat 7260
cgtaggtgat ttgtggtttt tggggttaag tttcgtttaa ggtaacgtag tcggtaaata 7320
gattttgtaa agtttttgttt ttttcgtttt tcgttataga tattaataat ttatagggtg 7380
ttgaagtcga gagggaagtt agatcgtggt tggtatttaa aacgaggtat ttttttttaa 7440
atttcggtgt taatattgta ggaataaatt ttcgggttaa ggattagtat ttttaagata 7500
aagggttggg tataaagttt tagttattgg aagattagtt ttttttttat tgttatttat 7560
tgggaaaaaa aagaaaagaa aaagatttta ttttaattgg tagttagtga tttttttaggt 7620
ttaagcgaat tatttgggag ttaggtttgg atgttaagtt tttattattt ttttggattg 7680
taattttttt aaattgatta ttagttaatt ttaatttggt attttaggag atatatttta 7740
aatggatgta gagaattatt ttttagttgg agattaagaa aaaaatttc gattttaaat 7800
tttcgaaata tgttttttttt ttttagttta attattttat ttttttaagt aatttagaaa 7860
ttaaattatt ataaggtggt gtgatttttt tttatttttt tgtgtgagta ttgttttatt 7920
aaattaaacg gaaaaaattt ttattattat aaatgtaaat attagaattt atatatttta 7980
aaatattttt atgaaaaatt aatttgattt aaagaaattt ttttgtattt gttttagttt 8040
attaattaaa attaaagatg tttttattat ataaaatatt attttggtag aaatttattt 8100
aaaatttaaa tattaataat attaagaaaa taaagtatat aagtaaaata aattgaagat 8160
ttttgttgat gtaatatgag tatataatat tttaataatt aaattttttt taaaaaatta 8220
aatagttatt ttatttgtgg aatgttttat tttaatttag taaaattata tttaaattat 8280
ttaggtgttt tgttttttaa gttaagcgtg tttgttttta aatgttttta aagtatttat 8340
```

attaattggt tgtaaagaac gtatatatat ggtaaaatat agaattgaat tgagtagtat 8400
tttaattttt ttaaataatt atttattata aattaattta ttggttaatt ttataattta 8460
gtttatttaa aatatatgtt tttgtgttgt ttattttttaa atttttttatt aaagatttttg 8520
ttatggggta ataaagtgta tgaaaagggg ggaaatgtga aaggatttgg gattattcga 8580
attgtatttt ttttgtattt ttagttttgc ggtagttatt agaaattatt ttttagtaaa 8640
ttgttttatt ttttagggtt tgtttgtttg ttttgttatg gtttttcgtt tttcgttagt 8700
cgtgtagtgt tttttgtgtg tttataatat aaaatttaag ttggttaaaa taagagtttt 8760
tggtatatat attttaatta gaatatgaat tttgggggtg agaattattt tttattagga 8820
aaagtttttt attttaattt gtgagattag ttattgaagt tagtttcgaa gtttggtagt 8880
taaatttttt atagaagatt tgttttgata gggtaagttt aaggattagt aggcgggaat 8940
tggaggtttt tttttaaaaa attatttttt ttagttattt agatttagtt tttttagtag 9000
gtttggttat taaatgaagt ataaaaatgt aagttttaag gtttattttg attgtaaaat 9060
aaatttttaa gttataagga tatgtaggag tgagttaagg aatacgtttt gatttttttt 9120
ttagttttta gagtggagtt ttatgagttt ttgaagattt gttttgtatt gttttgtttg 9180
gtttttagta ttgaagtacg gggaagtggg gggaagaatg tgtaataatt gattgatttt 9240
atattaagta acgtaatttt tttttttttgt atattttattt ttttaaaaaa aataaataaa 9300
taaaaattat ttgtagttat tatttgtagt gtttcggtta ttagttaata atgtagttag 9360
tttagatata taaaaaaaaa agattatcga aatgatgatg atatgtaaat tttttttcgaa 9420
attattataa gtaaatattt gaagtttgga ttaataaaat tttatttgtg ttattttata 9480
tcgagttagt agaaagttgt gataatgaat tttgtaatat tttacgaata gatattttaa 9540
ttagggatta atttttgtgat tttatttgtag aattattaaa tttggagtcg ttaaattgtt 9600
atttttgggt ttacgggttt ataaggatcg aatcggtaga gttttcgttc gcgttttcgt 9660
tagcgggtgg gggaatcgtt tggtcgtttt tattttggat ttttacgtta tagcgtcggg 9720
tagttttttt tgtaggtagc gattttggtt agaggttttt tagggtttag ttttttttttag 9780
gagaggtcga gacgtaggga aacggtattt aggttagagg taggttcgta gtttttttgtt 9840
tcgtttttgt gttttcgtta attcgataac gtttgttttt atttcgattt tcgtattcgc 9900
gcgaagtggg tttttcggtc gtcggcgtat tttggttagc gtggagagag gtaggcgttg 9960
agatcgaagg ggtttaggga gttttggatt tttttttttt gtttttaaag taatcgcggt 10020
tttttttattt attcggtgga gtttttcgag atttattttt ttcggtttgt ttgtggtaga 10080
gaagggggag cgcgttaaat gtttggttcg ttgcgttgtg gttgaaaacg tgaaaaagat 10140
ttggttcgtt cgggagagaa aggggggagaa ttgggtagta gttatattag agttattttt 10200
tcgtttttgg cgggtagtaa atttttttaag aacgtttgtt ttgttttttttt tagtttcgtt 10260
tagtttattt agtgtttttt ttttgcgatt ttaaattata ttttagggta attatttgta 10320
gtaagtaaat aaatggtcgg gttagtattt ttaggagaaa gtgtggttaa atatggaaaa 10380
gtggtttttg atggatgaga ggttcgaatt tagttcgttt ttgaaatatt ttaggttaag 10440
agttcgttcg ttttagaatt atagaaaatc gagggaaatt gttgtttagg ataggggtac 10500
gttggcgttg atgttttata aatgtttatc gagttttaat taatggataa gtattgaagg 10560
gtggtttttg tatatagttt tttaaagaga aaagtttttt ttatttattt attttcgttg 10620

ttattgcgtt tagatgagtt tttaatttcg gtatcgagat ttttgaaagt aggtttatag 10680
tttttttagt atattgtggt tttatagttt ttttaatttt gggtattttt gcggtaattt 10740
tggagggaga tttttttttg ataaataaat gttttgggtt cgaggttagg ttggagatgt 10800
tgttgtatag ttagaggttg ttaggtcgga aaaatacgtt tgaagtttag tatatagtag 10860
gcgtttaata gttagtgtaa cgtagtttta tttgagtttt gtttattcga cggtcgtcgt 10920
tttttatagt tttttttttt ttttgttttg tagataacgg ggaatggaga ttaattgtcg 10980
taaattggtg tcggcgtgtg tgtaattagg taagaatttt tttttttttgt tcgggttatc 11040
ggacgggagg tcgcgttacg tgagggcggt aagagggtat tggtttgcg gcgaggtttt 11100
agcgaggggc gtttttcga ggggttagtt tgggtaggaa ggaaattaga attaaatcgt 11160
tagtggtttt tttttgtggc ggggcggtgg attaggaagt agcggcgcgt tgtgtatcga 11220
agtttttttag tttatttttt tcggttggaa tcgtcggtaa tcggggaggc gtagaaagag 11280
tacgttattt tgtttcgggt tgttagaggg ttcgggggac ggggatgtcg ttagtttttt 11340
tttttaattg ggtttttgtt ttttgttttt tttttttttt cggtttgttt cgtttttttt 11400
tttttttttcg ttttcggttt ttttcggtcg cggttcggga cgtttttttt cgtacgtggg 11460
gcgggcgcgc gcgtggttta ggcgtgtagt cggcggtcgt tgaatgtttt ttttttaaag 11520
atttcgaaat taaaaaggtc gagtttacgg atttttttga gagtcgaaaa gaggtagtta 11580
gtagtaagtt tttttcgcgg tagtattttg gcgttaatgg taaggtcggg agggaagcgt 11640
aggtcgcgcg ttgggtattc gttttcggga ttttgggttt cgggcgaagc gtaagaaggc 11700
gaggtcgtta gatttgacgc gtttgttgtt tgaatttaga tatttcgttt ttgggtggga 11760
cgggaagtag tcgttttagg gacgttaatt tttttttttta aattatattg tatttttgag 11820
atttaatatt tttttttttt tttcgttcgt tttttgtggt ttgatttttt gcgtacgttt 11880
tagtaaattt cggcgtttag gttggcgtgg aaaagtggtt taatagcgat ttttgtcgtt 11940
cgttatattt cgttgcgcgt agtattatta gggtttattt agttatttag gtttttagtt 12000
acgttttatt tagatttgtg ggtgcgcggt ttatcgcggg aggtaagtaa gggaaatttg 12060

agttggcgaa ggtttgtttt ggttggttgg gggaggggcg gggggtcgat aatatttttg 12120
aagagttgga gggtagttat tgtgtttagt agtttagggt agaatggagg tttgtttttg 12180
tcgacgcgaa tttgtttgaa gtattggttg ttaggttttg ggttttggcg atgtcgttgt 12240
ttgattggtt ggttttattt tggaggaatc gagggatatt gttagaggag gtttataggt 12300
ttatgtaaaa agttaaaaag tttttaattt acgttatagg tttttttga attgaaattt 12360
gttttatggg gcggaggggg gggtgtaagg gatggaggag ggaagatgtt tttttttta 12420
aatatatgga aaaaaatttt ttaaatttat tgttttttta ttttttggt ttcgtagtaa 12480
ataagtgttt agttttagga ggttattgat ttttgataat gtgagtagat aaagtttttt 12540
tttttttata gttttcggtt tttaattttt tttttcgga ttaaagtgta agaatataaa 12600
tgtaatatgg gatggagggg ggcgatttgg gatttcggtt aaaaaaataa atcgtattat 12660
taagaagaaa ataaaggttt tgtattggag ttttttcgtg aatttgagag aaaatgatta 12720
tttgttgaaa tgaagcgttt aaagcgattt agtgttttac gttcggatat tgtattatat 12780
cgttagtcgt tttgttgggt tagttaaacg tttatttgtt cgggattaat tttatggggt 12840
taaatggggg taatgtagag ataacgtcgc gtgatttttg ttatttagat tgtgttaaat 12900
tttttttttg tttgataatc ggtagtaaaa ataaattatt agatcgtagt atgtttggga 12960
tatggttaaa aattaagagt agcgatgatt tttggggaga atgttttgcg cgggtttagt 13020
tttggtttcg gttagattag aggagttttt taatttcgtt tcgcgcgggg cgggttcgta 13080
gtcgttaagt cgaggttgat atttttttatt gtgttgggag ttagagagac gtaaaatgtt 13140
tttttttttt agtttttatt ttaggttttt tagatatggg gaatgtattt tgaggatagg 13200
tggagaagtt tacggtagga tggggttttc gtaggtgagt aggaaacggt taagagtaga 13260
ggagttttgt ttgcgttagt tataagtcgc gtaggcgttt ttggtcgttc gtttttgata 13320
attagtatat aaagaattag aaataatgaa tgattgtttt tttaattatt atttttaggt 13380
tcgtattgtt ttagtgtacg tgaaaggttt tttttttata tttaatatgt ttttttttat 13440
tttttgatcg aaaagaaaaa ttgttgttta aatacgttta atgttattaa ttaagaaaag 13500
gtatgtaatg ggaagaaatg ttgaaaattt tgatttaatt ggtttttaag gaattagtag 13560
acgataaaaa aaaattatac gagtgggtaa agttatagta ttgttgaagg atagagtatt 13620
tatttttttt tgattttaag ttaatttatg gaatatttaa agttttggtt atagtttgtt 13680
tgtaaaataa aaggatttat tttttgtgtt tttttaaagt ttttttttgt ttttaaagag 13740
aaaaaaagtt tataatgata tatgattttt ttaaaaggtc gtgatagttt attacgttat 13800
ttttttcgtt tttgttttta acgtcgttta aaaatattat gttttcgtta aagattaaac 13860
gttttgtata ggtagagttt atttttaagt agtttaggtt ttgttttttt tttttagtga 13920
gttttatttt ttttggtatt tattgggcgg atgtttagtt tggatagaat ttcgaaacgg 13980
gggtagtacg agagcgattg gagattttta aaagttagag gtttgagaga gggtggacgt 14040
agttagtaga agatggtgta gaagttagtt gagaacgatt ttttagagta aagagatttt 14100
tttttggttt tttttgtttt gggggttttg aaaggaattt ataaaatggt ttttattttt 14160
aggaggagga cggattgatt tttttttgtt attggtttaa aaagttttag ggcggcggtt 14220
ttgggtttcg tgttgaaatc ggattgtatt gtagtttttt tggatttgac gtttggtttt 14280
gcgtttcgat aaggggcggg tattttttttc ggtttttttt aggaacgtat taattgttaa 14340
atagttttgg tttagtggat gggttgaaag tgttcgattt aagtcgttgg tgtgtataga 14400
tttttttttt ttgggaggtg ggttttatgg ttcgttgtgg tatttttagt cgcgatatat 14460
atttttatac gcggtagtag ttcggtttta atttttttcg aaggatttgg gttaattttg 14520
gcggttttgg cggtcgtaga ttttttttcg tcgtttcgtt ttcgcgtttt ttatttaatt 14580
agcgaatgtt tgcggagtat atattacgtg gatttttaat gtattttttg aaagtaaata 14640
atatagtttt tttcgtcgtt atgaagggat tttaatttta atatggatat tagcgagatt 14700
agtttagatc gtttttagta aaacgtaaaa tggtggtgcg tggggtggtg attaaggttt 14760
tgagttttgt tagaaagaag gggatgtgta gagaaaggtg gagaatttta gttgtggtta 14820
gcgcggaagg gataggtgtt tgtcgaaggg ggtatgaggt ttgaggaaaa agtaacgaaa 14880
taggggtaag gagagttttt tatttttttt ttttcgttcg attttcgtta ttttattttt 14940
tttttttttt ttttattttt cgcgttaatt aaatttgtag tttatttgaa aggtgtttcg 15000
tcgcgttgtg gtttttattt tttaggggaa attgtattag ttgttcgaaa gtagttagtt 15060
tttgcggatt tttgttcgta aaagtggttt ttataggtcg cgtttttcgt tgttgattcg 15120
gtatataaag tttttttaagg ttggttcggt tgttattttt tatcgttcgt tgttaatata 15180
tgtagtagtt gttagagcgg ttcgggggaa aaggaaatgt ataacgaaag tttattcgtg 15240
agtaggaata tattaatgga ataatttgat gttttttttaa ttttatgtaa aaagttttgt 15300
cgttttttta atattgattg aatgggtaat taatggtttt ttatttaggc gaatattttg 15360
taatttaaga taggtaaaag ataataagtt taaggtagaa gataaaaggt ttaattgtag 15420
cggcgtttgt tcgtttttta tttttaggg tttttgatta ggaaagtttt tttttagagg 15480
agaaaaaggt aggagtggga gaatatatat ttattatttc ggggttagat tttatcgtag 15540
tatttgatta tttagtttag tttttgtta ttttgttttt ttatttttag tttttttttt 15600
tgtatttttt tttttttttta attttttag gatgattttt tattattatc gttattatgg 15660
ttttaataat tttttttttt aaattttata ttttttattt tagtaattaa tgaggttgtt 15720
ttttgattta ggaggagatt tttttttttt agaatttaat gcgtagagtt tttgagaatt 15780
aaagtagttg gtaggggagg aagaaattaa tagaaaggga gagagtatat agaattgtgt 15840

```
gtgtatgtta aagagcgatt aggaatgata gagttaattt ttttgtgagg atttgacggg   15900
aagagtgttt aagattttat tagtatgttt ttaataggtt gatattttaa tttaaatttt   15960
tagaagtaat atattatttg ggttattata atgaggtggg tttttttttt tttgttagtt   16020
gatagttttt aaaatattat ttcgttaggg aaataaaagt tttattttag attataggtg   16080
ggtatttttg gatttaggtg atttatggtt attatgataa ttaatgttga atgttagtta   16140
ttagtatgtt tgggagagag aaaatagaaa gaagggagag taaaagaaat agaaaaggga   16200
gatggatata agttggagag ggaagaaaag agaaaaagag gaagatagat gagtgtttaa   16260
tttaattgtt gtttaaaaaa gtggcggggg ggtgggattt tatttagttt tttgttattt   16320
tttttttttt tgatttggat atttatgttt aattttatat tttattttt tttttttttt   16380
tttaaatata tgtgttatat tattttttt attttattta gttcggtaag tagttgtttt   16440
ttggagattt agtgatattt aggaaaattg tggtagtaat atgtaaatgt gaggaagtat   16500
taatagtatg ttcgttgagt gattttagta aatgtttttt ttttaattt tttttttttt   16560
ttttttttag gttattgtga ggtggtaatt tttattgtta tttgaatatt gtttttttag   16620
gtagttattt taaattttaa atggttgagt agttagagtt gtgggttgga aaaatgggta   16680
ttatttgtag ggatttagag agggtggttg ttgtttaata tatttataga tttttaattt   16740
agaaaataat tttttttttt tgataagtta gagtttttta aattttattt aggaaatggg   16800
gaaaaggata gttatagtga agtttttaat ttttgggtta tttggtttta tagttatgag   16860
gggtggtggg tagtggattg tttttagttt ggtttgtatg tagagaaaag ttagatattg   16920
gagggggtgg ggtatttttc gggtaggatg taaggttttt atttgatttt tgcgttttat   16980
taggagttta tatatttacg tttattacgt ggttttaagt tgagtttagg cgggtttcgt   17040
ttttgagtta gtttgggtag ggtaggattt ttattcgttt aaggtttaat agtttaggga   17100
gatgtttaat taagttattt tttgggtgaa ttttgaagat agattttttt taaaagttag   17160
agattatttg gttgagtttt aggttagatt gatacggaga gtttggcggt atagtttaat   17220
cgtttattgt tatggttaga gggattttgt ataattaata ttgaagagtg tgaaattaaa   17280
taagatttta agattggtaa tcggtggtaa atattagtat aaaatacggt tgattttatg   17340
ttatatattt tttttttta gggttttttt ttgaaagaat aagtaagaaa ttttaatcga   17400
gataattttt gatgtttttt agatttaaaa ttttacgtcg gtattgggtt tttttttttt   17460
gttttacgtg agttatgtag tatttttagt tttttttatta ggattttatt aatgtttttc   17520
gtattggaaa ttttgtgtt agaggttgaa tttatagtaa tttttaaaat taattaagaa   17580
gaatttagtt agaggttata gtaatgttgg aattataaaa tgtataagat ttattttttt   17640
ttggtttttt ttttatttat gttgttatg tttgtgtatt tataagtttt atgtatatta   17700
aatttttaaa attaattatt attatgttat agagttttta ttggatagtg tttttttagtt   17760
tttattatat attttttttt ttttatgtag atttattatg ttggtgtttt gttatatagg   17820
gggtttgaga agaatgttat ttaatcgtcg ttgttgtgag tgtgtaaagt gattaggaga   17880
ttaggagaat gttgaaattt ttgttggaaa aatgtaaaga aaatttttat tttgagttag   17940
ttgtttatag agttagtgtg tgtgtgcgtg tgtgtgtttg taatataaaa tggatgtgaa   18000
tatatatata taaatagata tggttttgtt tttattttaa tttgaattat ttagataatt   18060
gtttttattt attatttgat tttaatgggt ttatataaat taggatattt tattttttta   18120
ggtatttagg ttgttgttga tttttagtgt ttttaatatt tcgtatacgt tggtattatg   18180
aggagtagtt acgtgttttt gggtttttta attattttgg aggttgattg aggtttttta   18240
tatatgtata tttgtcgtga tgaaagtttt atcggtagag tggagttatt agagttttta   18300
ttaaaatttt gtgggtttat gagagatggg tttagaaatt tatatggttt tgtggggttt   18360
ttcggttttt taaaataagg tattaatatt taagttttta aaaatatttg tagttttggg   18420
gtttgaattt tgaaaaataa ggagtgaggg gttgtgtata ttaattatag tggagatttt   18480
ttttattttt taatgtgatg gagttttttt atgaaatgaa gtttaagggg gtatggtatt   18540
gtggggatta tagttatttt gaggtttaaa agaagaaatt ggaatatgat tagtaaatat   18600
atttagtaga aaagagttgg atttttattg atttagttat aggttatcgg ttggtagtgt   18660
aatgggagga aatatttatt ttatatatat attttatgat tttgggggaa ttagaggaaa   18720
tttaataaga aaacggttag aaatatttaa aatttttatt taaaagattt aagtaaatta   18780
gagttttatt agattaaaaa ttattataaa tgtaagagta ttgtttttag tgaaacgttg   18840
tggggtttga gaaggagatt tttcgttaaa ttttcgggat aaaatgcgtt atttaagtat   18900
tagataatga gtagaatgta aattaattta attttttttta ttaataggtt gttagtgtaa   18960
tgtgtataat ttagtgataa gattgtagga tttaatatag ttggatgtat gagttttagt   19020
taatgtagat ttgttatatg aggatgtgtt ttattttgag taggtgtttg tatgtgtgga   19080
atggggtaaa gtggaataaa aggttaaaag tagaaatgtt gatttaaagt ttattatgaa   19140
gaaatttttt ttttgtagtt aaattatttt taaagtggga tgatattggt gaagaaagat   19200
tgaaaaataa tttttatgtg cgtttttgga ttgtaagttt aaaatgggga ggagttgtag   19260
atagggtttg ggggtggtta gggtaaagga gagatatata agttgtaaat atatttgtag   19320
tttgttttat ttattttgtt ttatatcgaa taagtttttt aattttgtga ataaggataa   19380
ggagggagtg ttttaaagat attttatgtt ggtattgtaa attattgatt gtaatgttaa   19440
ataaatatat atttagagat gataatatta attttatagt aaaataatcg tttatgtaga   19500
aatttagagg agattagttt gtttttttta gttgatttat gttgggggat aaaaggattt   19560
ttaaaaatta ttttgaatat gtttggattt tttttttaa tttttttgga aattaaattt   19620
```

gtttggaaat agtgttataa agagttgatg tttttaaagg tgattttttt tgttttatat 19680
aaataaggtt ttgtttttgt tagttgagcg tagttttagg tttttcgttt ttagtttata 19740
tatatttttt ttgtttgttt ggattttaat ggtttaagat agttttgagt ttattgggaa 19800
aagaaaatga ttgttaaaaa ttatttttga aattggttat ttggtaatat ttttaattgt 19860
atggaaattt attaaggtat attttatata taattagttt aaggttgttg attttatagg 19920
ttttatggat ttaaatttga ttgataataa agtaaataag agagtcgaat ttaaagcgtg 19980
gtttttttcgg gttaggacga gtttaatata gtgtataagg aatttgaaag atttaggata 20040
tgtgttttaa ttaacgttaa gtagaatgga taagttttta gtattttgaa aacgttgggt 20100
tagggttttt tttttattgt gtgtttttttg tttggggatt aataagtatt atagagaacg 20160
tgatttgagg cgattttttta tttttgtata aatttagagt gaattattaa atagttgttc 20220
gtttaaagtt aaggtaattt ttttttgacg ggtttatttg tttttcgatt tttaatttat 20280
tagtttgttt ttttagggtt ttgttttttt tgtaattaaa gtttttttag attagcgtag 20340
tatttatttg ataggttgtt tggaaaattt aagatcggag aggtgatttg ttgttgtttt 20400
ttaaattttt tagttttaag taacgtgttt tttttttata tggggtgggg gattggaaat 20460
ggatgtagtg agatataaag agtgggtgtt ttgttgattt ttgtattttt tttttttttga 20520
ttattttatt tttttttttt aagtttttcga tttttagttt tatttttttta tttttgggtt 20580
cgtattaaaa gtcggatcgt tttgggttgg gtaggagttg aattttcggg agtttgtttg 20640
tgtagattta gtgcgtacgg cgaggtagta gttcggtttc gtattgttga taggtgtagg 20700
taggatagtt tttttatcgc ggttcggggc gttttgattg gtgcggagtt acgttagtcg 20760
tattcggaga agggtttggg aggaggcgga ggcggagagg gttggggagg gtcgcggcgg 20820
agtgacgttt cggtattagg aagttcgttt ttggttttaa gatgttaggt taatagggaa 20880
gcgcggagtc gtagatttgg ttcgtcgttc gtttgggtgt ttggagttga gttgcggtaa 20940
ggttcggttt ttgttcgatc gttcgagggg tgtgcgtgtg cgcgttgcgg agggtgcgtt 21000
tagagggtcg cgtcgtggtt gtagcggttg ttgtcgtcgt aggggattta atattattta 21060
tttgttttttg ttatttttga tattttttttg ttagggttgt cgcgtggggg gggggcgggt 21120
agagcgcggt cggcgttagt tttttttatt ggaggggttt ttgggggagg gagggagaga 21180
agaaggggggt ttttgtttat ttttgtttcg ttttggagtt tggaagtttg ttttttaaag 21240
acgttttgag tggtgttttt ttgtttatat tttatgtttt cgtttgttcg ttgatttttc 21300
gttttcggat tttttcgttt gagtttttcg gaggagacgg gggtagtttg gtttgagaat 21360
tcggcggggg ttgcgttttt tggttttttt cgtagcgggg aaatttcgcg tttagagcgc 21420
gattcggagc gggtagcggc ggttacgggg gttcggcggg gtagtagtta aggattagta 21480
gagcgtcgcg ttttttcgtt tatgaattgt atgaaaggtt cgttttattt ggagtatcga 21540
gtagcgggga ttaagttgtc ggtcgttttt ttattttttt gttattattt ttagtcgtta 21600
gttatggttt cggtttttggt tttcggttag tttcggtcgt tggatttttt taagtatagg 21660
ttggaggtgt atattatttt cgatattttt agttcggagg tcgtaggtaa ggcgtcgcgt 21720
cgttttgtag atattttcgt ttagttgttt tgcgttattc gttttttttc gttttaagga 21780
agttagtttt ttcgggggga ggcgtggtgg gagtggtcgt tcgtttggtt tttcgtagaa 21840
ttttcgggag tcggaatttt gattatttcg tattttttta gtttttttc gatcggttcg 21900
gtttttgggg cgttaagggc gcgagtaatt ttgtcgtttt ttttattcgt attttggttt 21960
ttttttttgtt ttttgggtta taaaaatttt agtattttga ttcgaggatt tttagaggtc 22020
gtcgattttt gtttttgttt tttttcggt ttttagtttt cgaggagttt tattcgttag 22080
gaaattgttt gaaattattt agaaatgttt ttcgcgaaga ggtatttttt tttttttttt 22140
gggaaagggt cggcgaattt cggtgtttaa tcgaattttt atattttttt ttagtttttt 22200
taaatcgtat ggaaatttga gtttttttgcg agggggaggg gggtttgtaa attacgcgcg 22260
tgtgcgcgtt ttaggagatt tggtgtgttt gcgtagaggt gtataaatat atttgaaagt 22320
ataggttata aaagtgaatg tgtcgttgta gtgagataaa tatgtaaata aaacgtgcgg 22380
cgttggggga ggggaggaaa tggggcgcgg atatttatat ttgcgtttgt atattttata 22440
ggcgtagcgt ttttcgcggt tcggagtcgt cgcgcgtatt tttttcggc gttaggtagt 22500
ttagtttttt tacggttttt gtcgtcggtt tagttggcgt tcgcgttgta ggtgggtatg 22560
ttgacgggaa agtgtgtgtg tttcgttttt agagaaagat aaaagttagt aggggaagaa 22620
tgaggacgtg ggcgtcgagg attcgtttaa gaagaagcgg taaaggcggt agcggattta 22680
ttttattagt tagtagtttt aggagttgga ggttattttt tagaggaatc gttattcgga 22740
tatgtttata cgcgaagaaa tcgttgtgtg gattaatttt acggaagttc gagttcgggt 22800
aggagttagt acggagtttg ggagggatgg ggggaggatg ttgtggaggt ataggttaag 22860
tagattagga gagaatgtgg aaggtagcgt cgtttgggag ggcgtcggtg gggcgtagtt 22920
ttgtaaaggt agaaggtttc gcggcggttt ggttgcgaga ttatagtttt tttttcgagg 22980
tcgataggat tgtcgttttg gtttaggttt ttagagcggt atcggtttat tgtttcgtta 23040
tttcgcgatt ttacgagttg ggttgtatgg gtaatttttt gtataggata ttgtgttttt 23100
ggtttgtagt tgttagagta gagttaataa aatttttatt aggttaagag tcgcgaatag 23160
gttttaattt gtgagttttt aataaggaaa attcgttaga gatacggaag agttggtttt 23220
ttttgggaaa tttttgtttc ggttttggtt tagttttttt ttttttgggt tcgcgttttt 23280
tatatttttt ttacggttgt ttcggttatt taggtttttt ttatatattt tatttttttag 23340
ttttgtgatt ttcgggagta aagttttaat atataattat tagttttttt agaaggagaa 23400

agaaaaaaag aagaaagatt tttttgtttg gtttatttat ttttttttag gagttgaatt 23460
ttggaaattg aaatttatat tttttttttt aaattataat tatagttttg taaaaagggt 23520
ttattttaat tttgtagtaa atttgtattt tatggattgg taaaaatgag tttaaataaa 23580
taatttaata gtaacgtttt ggtttatgtt ggtcggtgga agattttaaa tttgttagga 23640
ttttggaagt agaaaataga attaagtaaa ttaagcggta tttagaggtt ttgttgttaa 23700
aaaaaaaaaa ttaagtgttt tgggtagaaa aaataaagtt ttcggttaga gtagagtaaa 23760
taaaaagaag aaaataacga taaaaagaat aaagattaaa atgttttttt aaattagagg 23820
gaatgaagat attttttggg tggtatttgt gtaaggtatg aggttatgtt ggtggataaa 23880
aggtcgggaa gaagttgaaa atggttttag tttaattgtt tagagttaga gttgggtttt 23940
gggcggcgtg gttttgagta aggttagttt tttattagtt tttttgtata ttaagggaac 24000
gggtttttta cgtatttttt tcgtttgagt aaagtttaga tggtttaggg tagaaatggt 24060
aagtaattaa agatagagtt tatgggtttt ttgggatttt tcgaaaacgt ttttttattt 24120
cgttcgttat ttcgtagttt tattttagtg ttttgtagtc gcggcgttgg gttttttttg 24180
tagttgtttt tttttttagg gcggttgttt gtcgagttaa gtgggagtga ggcgtgtttt 24240
ttatagtagt cgggtgtaaa gaggaagggg gataaaaagg aaattaagaa tgaaaggaaa 24300
aagagaaaaa gcggattata cggttgggtt cggcggagat gtgtaatgtg aaatattatt 24360
ggtgttagtt cggatatttt aggttaggtt tttttttaat atataaaagt cgtcgtttgg 24420
ggcgataggg aggttcgatg tggattggga tcggggttgc ggttgggtta tcggatacgg 24480
gtggaagtcg gtcggtttgg gtggtcgttt gtaaagttaa acgattcggt tgggtttggc 24540
gcgcggatag gtttgtggtg ggtttagggt aaagaagagg tagagcgaaa gaagggggaa 24600
ttttttaaat tatttttttc gggttttcgg agtttaatat gttaagtttt tggagttaac 24660
gagttgacga agaggtggtt ttttgttttt tatttggttg ttttgttagg cgagaaagag 24720
tgttggcggt ttagtttttg ttaagggagt acgtattagg gggtggggga cgatagtgga 24780
ggttagggaa ggaagggagg aattgcgtgg gagaaagagc gattttttag tgttttttta 24840
gttttttttt tttattcgtg ggtttgtggt tttggaatgg aagtaagttt gtaaggtgtt 24900
tcgggaaggg ttggaaaagt ttgttgtttc gcgtttgttt tatattaagt gtttttggat 24960
ttggagaaac gtttggttga gtgattaaat cgttcgtagg tttttatgcg ttcggttgag 25020
gtttgtggcg tagtttcgag ttttagttcg taggttagag tagattaggt tttttgcgtt 25080
tggtggagat tcgggttagt aattgaaagt tggttttggt attttggtgt gtagggcggt 25140
gtagtgaagc gaggttaggg tgtgtgagtg cgttagcgtg tgtgtcgggg gaaggcgggg 25200
gttggttttc gatggaagtt ttagtaattt gtattgtggt atttgtttgt ttttttgttt 25260
taatcgtttt taggtttggt ttaagaatcg tcgggttaaa tggagaaaga gggagcgtaa 25320
ttagtaggtc gagttatgta agaatggttt cgggtcgtag tttaatgggt ttatgtagtt 25380
ttacgacgat atgtatttag gttattttta taataattgg gtcgttaagg gttttatatt 25440
cgttttttta tttattaaga gttttttttt ttttaatttt atgaacgtta attttttgtt 25500
attatagagt atgttttttt tatttaattt tatttcgttt atgagtatgt cgtttagtat 25560
ggtgttttta gtagtgatag gcgtttcggg ttttagtttt aatagtttga ataatttgaa 25620
taatttgagt agttcgtcgt tgaatttcgc ggtgtcgacg tttgtttgtt tttacgcgtc 25680
gtcgattttt tcgtatgttt atagggatac gtgtaattcg agtttggtta gtttgagatt 25740
gaaagtaaag tagtatttta gtttcggtta cgttagcgtg tagaattcgg tttttaattt 25800
gagtgtttgt tagtatgtag tggatcggtt cgtgtgagtc gtatttatag cgtcgggatt 25860
ttaggatttt gtcggatggg gtaatttcgt ttttgaaaga ttgggaatta tgttagaagg 25920
tcgtgggtat taaagaaagg gagagaaaga gaagttatat agagaaaagg aaattattga 25980

attaaagaga gagttttttt gattttaaag ggatgttttt agtgtttgat attttttatt 26040
ataagtattt ttaatagttg taaggatata tatataaata aatgtttgat tggatatgat 26100
attttaatat tattataagt ttgttatttt ttaagtttag tattgttaat atttaaatga 26160
ttgaaaggat gtatatatat cgaaatgtta aattaatttt ataaaagtag ttgttagtaa 26220
tattataata gtgtttttaa aggttaggtt ttaaaataaa gtatgttata tagaagcgat 26280
taggattttt cgtttgcgag taagggagtg tatatattaa atgttatatt gtatgttttt 26340
aatatattat tattattata aaaaatgtgt gaatattagt tttagaatag tttttttggt 26400
ggatgtaatg atgtttttga aattgttatg tataatttat tttgtgtata atatttcgta 26460
taatattatt gttttatttt ttagtaaata tgaaataaat gtgttttatt ttatgggagt 26520
aaaatatatt gtatataaat tggtttggat tttttttttt tttttttgtt attaatttgg 26580
ttaggatatt ttagttattg ttttttaaat aaattagttt tttttgtttg tttagttaaa 26640
tatataaggt agtagttttt atttaaattt ggtagaaata aatgatagtt atttattaga 26700
aattaaaaag aaaaaaaaag gtattttcgg gggggaaaag ggttataaaa tttaattttg 26760
tttttttaat ttttttttgg tttaaattta gaggatttta ttatggttag taaataatat 26820
gaaaaagaaa aaagaagaaa gaaatttagt aagtttatta gtttaaaatg atttttaagt 26880
ttattttttt acggggaaat ttatattttt agtaaattgt tttggagaaa tatttgtgta 26940
tgtatatatg tatagtttat atgtattttt tttaggagga atatatttat aataaattta 27000
tagggaaata tttttagttt aaaatattta ggtttttacg tttattttta ggtttaagta 27060
gagagatttt ttatgttata ttgtattatt atttttaaat tttttggaga tattaaaaga 27120

```
aataaagatg attttaata attatagttt tttagttttt taaagaattt aggggttgag   27180
aggttagagt ggagtttttt gagttttgtc gagtaatatg tagttgaggt aaaggttatg   27240
ttttcggtgt tttgttttaa ataatattga tttattaatt ttaaatttgt ttgtttttga   27300
aattatatag gattatagtt tgtaaattgt aggataatga agtaaattaa gatgaattat   27360
agttttggtt tttttgtta ttttttgata tttaaatagg gaatgagttc ggtgtgagtg   27420
tttaaatgaa ttttaagtat tcgatttttt tttattcgcg attttagtt ttaaaaaaat   27480
gtgaaatttg attttataat aaatagaaat aaatattatt tagttttaga gaatttattt   27540
ttatggcgtt aggagggtcg ttgtggaggt ggggggaggg atgtgttgag atttttgtt   27600
atgtttgtta atttttcgta taattaaagt gggcgagaat aaatattacg ttggggaatt   27660
tagagtaaaa agtaatcgtc gatttttgg agtcgataat attattgttt tttcgtttta   27720
gttgttttta tttgaatgaa attttattta gaagttttg gagttcgaat attgagtttt   27780
tgttttgtaa gaaattgttg ttgttattta aagagatcgt agataatgtt ttcgatttaa   27840
gattagtgtt taacgtatat tttttttttt tttaaagttt tgttttcgat ttgcggaggg   27900
attacgtagt agtgggggt agataaaagt tttttggacg agggttattt tttattatgt   27960
tgtttatttg agagtagtgg agaggaaaac ggtttttacg ggcggatttt ggtttttgga   28020
gtcgtagggt ttagcggttt cggtttttc gtttttagt ttggtagggg gcggggaggg   28080
ttaaagggcg gaggggaagg aaggagttag aagaggggat ttggggatgg gggttggaag   28140
cgttaacgag atttgttcgg aggatttagg tttttgtag gttggtgagt gatttgggag   28200
ttttgggtaa aagaggtgta tttcggttta gtttggttgt tgaattagaa taacgcgagg   28260
atattaatta atttgtagga aataaaaat ggaagtcgag gtttaggaag agttgttatt   28320
ttttcgattt gagtggtgta ggttgggggc ggagatttgg gatttaagag aggttatttt   28380
ttttatttta gttttttttt tttggatttt taaaaggaat aattttattt ttttttcgt   28440
tttttttata atttttattt tcgttagttc gtaggtcgtt tgttttttt cgtattttt   28500
ttttttattt agcgagagaa atttagtttt tagagt                          28536
```

<210> 4
<211> 28536
<212> DNA
<213> Artificial Sequence

<220>
<223> chemically treated genomic DNA (Homo sapiens)

<900> 4

```
gttttgggag ttaggttttt tttgttggat gggaaaagga ggtgtggaaa ggggtaggtg      60
gtttgtgggt tagtggggat gagagttgtg gggagaatgg aggagagagt aaaattattt     120
tttttggaag tttaagggaa gggagttgag gtgagagaaa tggttttttt taggttttaa     180
gtttttgttt ttagtttgta ttatttaagt tggggaagtg ataatttttt ttaaattttg     240
atttttattt tttgtttttt gtagattgat tgatgttttt gtgttgtttt agtttagtaa     300
ttaaattagg ttgaggtata ttttttttat ttagagtttt taaattattt attaatttgt     360
aggagattta aatttttttgg gtaggttttg ttagtgtttt tagtttttat ttttaaattt     420
tttttttag ttttttttt ttttttgtt ttttggtttt ttttgttttt tattaggttg     480
agaggtggag aggttgggat tgttgggttt tgtggtttta gaagttaaag tttgtttgtg     540
gggattgttt ttttttttat tgtttttaag tgaataatat ggtgggaggt gatttttgtt     600
taaagggttt ttatttgttt tttattgttg tgtgattttt ttgtaagttg gaggtagagt     660
tttaaaaaga aagaaagtgt gtgttgagta ttgattttaa attggggata ttatttgtga     720
tttttttaag tggtagtagt agtttttttgt agaataaaga tttagtgttt gagttttaaa     780
agtttttagg taaagtttta tttagatgaa agtaattaag gtgaaaaaat aatgatattg     840
ttggttttag aaaattggtg gttatttttt gttttaagtt ttttagtgtg gtgtttgttt     900
ttgtttattt tggttgtgtg gggggttgat aagtataata aaagatttta gtatattttt     960
tttttatttt ttataatgat ttttttagtg ttatgaggat gaatttttttg ggattaagtg    1020
gtatttgttt ttatttgtta tgaaattaaa ttttatattt ttttaaagtt gaaaattgtg    1080
gataaagaag gattgggtgt ttaaagttta tttaaatatt tatattgaat ttattttttg    1140
tttagatgtt agaggatggt agggagggtt agggttgtaa tttattttga tttgttttat    1200
tgttttgtag tttgtaaatt ataattttgt ataattttag gaataagtag gtttagaatt    1260
aatgggttaa tattatttaa aataaaatat tgggagtatg atttttgttt taattatata    1320
ttgtttgata agatttagga aattttattt taatttttta atttttgagt tttttgagaa    1380
attgaagggt tgtagttatt agaaattatt tttgtttttt ttaatgtttt taaaggattt    1440
ggaaatagta atgtaatata atatgaagga tttttttatt taagtttgaa gataaatgtg    1500
gaagtttaaa tattttgaat tggagatgtt tttttgtaaa tttattatag atgtattttt    1560
tttgagagaa atgtatataa attatatatg tatatatgta taaatatttt tttagaataa    1620
tttgttagag gtgtaggttt ttttgtaaag gagtaaattt gagagttatt ttaagttgat    1680
```

```
ggatttgtta aatttttttt tttttttttt ttttttatat tatttgttag ttataatgga 1740
attttttagg tttaagttaa agaaaaattg gagagataaa attagatttt gtagtttttt 1800
ttttttttgg gaatgttttt tttttttttt ttagtttttg atgaatggtt attatttatt 1860
tttattaaat ttaaataagg attgttgttt tgtatgttta attaggtagg tagagggaat 1920
tggtttgttt aggaagtagt gattgagatg ttttggttaa gttagtgata gaggagggga 1980
gaaagaattt agattaattt gtatgtagta tattttattt ttatgaaata aaatatattt 2040
gttttatatt tgttgaaaag taaaataata atattgtatg aaatgttata tatagggtag 2100
gttgtatata gtagttttag aaatattatt gtatttatta gagaaattat tttaaaattg 2160
atatttatat attttttata ataataataa tatgttagaa atatatagtg tggtatttag 2220
tatatatatt tttttgtttg taagtgaaaa attttaattg tttttgtata atatgtttta 2280
ttttaaagtt taatttttaa aaatattgtt gtgatattat taataattgt ttttataaaa 2340
tttaatttgat attttgatat atatatattt ttttagttat ttaaatgtta ataatgttaa 2400
atttaaaaaa taataagttt atagtaatgt taaaatgtta tatttagtta aatatttgtt 2460
tgtgtatgtg tttttgtaat tgttagaaat atttgtagtg aaagatgtta gatattgagg 2520
atattttttt gaaattaaag gagttttttt tttgatttag tggttttttt ttttttatat 2580
agtttttttt tttttttttt tttttagtgt ttatgatttt ttagtataat ttttagtttt 2640
ttaagggtgg agttgtttta tttggtaagg ttttaggatt ttggtgttgt gggtgtggtt 2700
tatatgggtt ggtttattgt atattggtaa gtatttaggt tggaggttgg gttttgtatg 2760
ttggtgtagt tgaagttgga gtgttgtttt gtttttagtt ttaggttggt taggtttgag 2820
ttatatgtgt ttttataaat atatggagga gttggtggtg tgtaaggata ggtaggtgtt 2880
ggtattgtgg aatttagtga tgggttattt aggttgttta agttatttag gttgttgaga 2940
ttggagtttg ggatgtttgt tattgttgag ggtattatgt tggatgatat gtttatggat 3000
gagatagagt tgggtgggga aaatatgttt tgtgatgata gggggttgat gtttatagag 3060
ttgaagaagg ggaagttttt ggtggatagg gaggtggatg taaggttttt ggtggtttag 3120
ttgttgtagg aatagtttgg gtatatgttg ttgtagggtt gtatgagttt attgaattgt 3180
ggtttgaagt tatttttgta tagtttggtt tgttggttgt gttttttttt tttttatttg 3240
gtttgatgat ttttgaatta aatttggggg tggttggggt aagggagtaa atagatgtta 3300
tagtgtagat tattaaaatt tttattggag gttaattttt gttttttttt gatatatatg 3360
ttagtgtatt tatatatttt ggttttgttt tattgtattg ttttgtatat taagatatta 3420
gggttagttt ttagttattg gtttgggttt ttattaagtg taggagattt ggtttgtttt 3480
ggtttgtgag ttgggatttg gagttatgtt ataaatttta gttgaatgta tggagatttg 3540
tggatggttt gattatttag ttaggtgttt ttttaggttt aaaaatattt aatgtaaaat 3600
aaatgtgggg tagtaggttt ttttaatttt ttttggggta ttttgtaaat ttgtttttat 3660
tttaaagtta tagatttatg gatgaggaga aggggttgga agggtattag aggattgttt 3720
ttttttttat gtaatttttt tttttttttt ttgattttta ttgttgtttt ttattttttg 3780
gtatgtgttt ttttaatagg gattaggttg ttaatatttt tttttgttta gtaaaataat 3840
taaataaaga gtaaaagatt atttttttgt tagtttgtta attttaggag tttggtatat 3900
taaattttgg gaatttggaa agggtagttt tggagatttt tttttttttt gttttgtttt 3960
ttttttattt taagtttatt ataggtttgt ttgtgtgtta ggtttagttg ggttgtttgg 4020
ttttgtaggt ggttatttag gttggttggt ttttatttgt gtttggtggt ttagttgtaa 4080
ttttgatttt aatttatatt gggttttttt gttgttttag atggtggttt ttgtgtattg 4140
gagagaggtt tggtttgaga tatttgagtt gatattagtg atgttttata ttatatattt 4200
ttgttgggtt tagttgtgta atttgttttt tttttttttt tttttatttt tgattttttt 4260
tttatttttt tttttttttg tatttgattg ttataaaaag tatgttttat ttttatttgg 4320
tttgataagt agttgttttg gaaggagagg tagttgtaag gagagtttag tgttgtggtt 4380
ataaagtatt agggtggagt tgtggaatag tgggtggggt gggagggtgt ttttgaagga 4440
ttttagaaaa tttatagatt ttgtttttaa ttatttgtta tttttatttt aggttattta 4500
aattttgttt aggtgagaag agtatgtgag aggtttgttt ttttgatgtg taagagagtt 4560
aatgaaagat tgattttgtt taaaattatg ttgtttagga tttagttttg gttttggata 4620
gttaaattaa aattattttt aatttttttt tggtttttta tttattagta tagttttatg 4680
ttttgtataa atgttattta gagagtgttt ttattttttt tgatttggga gagtattttg 4740
gtttttattt tttttattgt tgtttttttt tttttgtttg ttttgtttta attgggggtt 4800
ttattttttt tatttagagt atttaatttt ttttttttaa tagtaaagtt tttggatgtt 4860
gtttgatttg tttgattttg ttttttgttt ttagaatttt aataaatttg gaatttttta 4920
ttgattagta taaattagga tgttgttatt gggttattta tttgagttta tttttgttaa 4980
tttataaagt atagatttgt tataaagtta aggtaagttt tttttataaa attatgatta 5040
taatttagaa gagggggtgt gagttttaat ttttagagtt taatttttga gagaagataa 5100
ataaattaag tagaaaagtt tttttttttt tttttttttt ttttttaaga ggattagtag 5160
ttgtgtatta aaattttgtt tttggagatt ataaaattag gaaatagggt gtgtgggaga 5220
gatttgaatg gttgaaataa ttgtaaagaa ggtgtaagaa gtgtgagttt aggagggaaa 5280
aagttgggtt agggttggga taaaggtttt ttagggaggg ttaatttttt tgtgtttttg 5340
gtgggttttt tttgttaaag gtttataggt tggagtttgt ttgtggtttt tggtttggta 5400
gggattttat tagttttgtt ttggtaattg taagttagga atataatgtt ttgtgtaggg 5460
```

gattgtttat gtagtttagt ttgtgagatt gtgggatggt ggggtagtga gttggtgttg 5520
ttttgggagt ttgagttagg gtggtagttt tgttggtttt ggagagggaa ttgtaatttt 5580
gtaattaggt tgttgtgagg tttttgttt ttgtaaagtt gtgttttatt ggtgtttttt 5640
taggtggtgt tgtttttat attttttttt ggtttatttg gtttgtattt ttataatatt 5700
ttttttttat tttttttaga ttttgtgttg gtttttattt ggatttgggt ttttgtaagg 5760
ttggtttata tagtgatttt tttgtgtgtg gatatgtttg ggtagtggtt tttttggaaa 5820
gtggttttta gtttttggag ttgttggttg gtaaagtgag tttgttgttg tttttgttgt 5880
tttttttttag atgggttttt ggtgtttatg tttttatttt tttttgttg gtttttattt 5940
tttttgaaa atgaaatata tatattttt tgttagtatg tttatttgta atgtggatgt 6000
taattggatt ggtggtagaa gttgtggaag agttgggttg tttggtgttg gaggagggtg 6060
tgtgtggtgg ttttgggttg tgaggagtgt tgtgtttgtg gggtgtgtag gtgtaagtgt 6120
gggtgtttgt gttttatttt tttttttttt ttagtgttgt atgttttatt tatatgttta 6180
ttttattgta gtggtatatt tatttttata gtttgtgttt ttaagtatat ttatatattt 6240
ttgtgtagat atattaaatt ttttgggatg tgtatatgtg tgtggtttat agattttttt 6300
tttttttgta gaaagtttag atttttatgt ggtttgggaa ggttaggaaa agatgtgggg 6360
atttggttgg gtattgaagt ttgttggttt tttttaaaa aaaaaaaaa aatgtttttt 6420
tgtgaagggt atttttgagt ggttttaggt aattttttaa tgagtggagt tttttgggag 6480
ttgaaagttg agaggaaaat agggatagag gttggtggtt tttgaaggtt tttgaattaa 6540
gatgttggga tttttgtgat ttaggaaata gaagggaggt tagggtatga atagagaggg 6600
tggtagaatt gtttgtgttt ttagtgtttt aggagttggg ttggttgagg gagaattaaa 6660
gggatgtggg gtagttaaaa ttttggtttt tggaagtttt gtggggagtt aggtgaatga 6720
ttatttttat tatgtttttt tttggagggg ttgatttttt tggggtgaga gggagtgggt 6780
ggtgtagagt agttgagtgg gaatgtttgt agggtggtgt ggtgttttat ttgtggtttt 6840
tgggttggag gtgttggaga tggtgtgtat ttttagtttg tgtttggagg agtttagtga 6900
ttggggttga ttgggagtta gaattgaagt tatggttaat ggttggggat ggtgatagga 6960
agatgaggag atggttgata gtttggtttt tgttgtttgg tgttttaagt gaagtgggtt 7020
ttttatgtag tttatggatg agggagtgtg atgttttatt agtttttggt tattgttttg 7080
ttgagttttt gtagttgttg ttgtttgttt tgggttgtgt tttaggtgtg gagttttttt 7140
gttgtgggga gagttagggg atgtaatttt tgttgagttt ttaagttaag ttgtttttgt 7200
ttttttttgga aggtttaagt gaaaaagttt ggagatggaa agttagtggg taaatgaaga 7260
tatgggatgt gggtagaagg gtattattta gagtgttttt agggagtagg tttttaagtt 7320
ttaaagtgaa ataagagtgg gtaaagattt tttttttttt tttttttttt ttttaagaat 7380
ttttttaata aggaaagtta atgttgattg tgttttgttt gttttttttt tatgtggtag 7440
ttttgataga gaagtgttaa gagtgatagg gataggtagg tgatattaga tttttttgtgg 7500
tggtagtagt tgttgtagtt atgatgtggt tttttgagtg tatttttttgt aatgtgtata 7560
tgtatatttt ttgggtggtt gaataggagt tgggtttttgt tgtagtttag ttttaggtat 7620
ttaggtgagt gatggattag atttgtggtt ttgtgttttt ttgttggttt aatattttaa 7680
aattagaggt gggtttttttg gtgttgagat gttattttgt tgtggttttt tttagttttt 7740
tttgtttttg ttttttttta gatttttttt tgggtgtgat tgatgtggtt ttgtattaat 7800
taggatgttt tgagttgtgg tggagggatt gttttgtttg tatttattag tagtgtgggg 7860
ttgggttatt gttttgttgt gtgtattggg tttatatagg taagtttttg ggaatttagt 7920
ttttgtttag tttaaggtga tttggttttt agtatgaatt taaaggtgaa gagatgaggt 7980
taggagttga aggtttggga gaagagagtg gaatggttaa gaagagaaag gtataaggat 8040
taataagata tttatttttt gtgttttatt atatttattt ttaatttttt attttatata 8100
aaaaggagat atgttattta aaattagaaa atttgaaaaa tagtaataaa ttattttttt 8160
gattttaaat tttttaaata gtttgttaag tgaatgttgt gttaatttga agaagtttta 8220
attgtaaaga agatagagtt ttgaaaaggt aggttaataa attagaaatt gagaagtaaa 8280
tggatttgtt aaaagaaaat tattttgatt ttaaatgaat aattgtttgg tggtttattt 8340
tggatttata taagaataaa aagttgtttt agattatgtt ttttgtgatg tttattagtt 8400
tttagataga aaatatataa tagaagagaa attttaattt agtgtttttta aaatgttgaa 8460
agtttattta ttttatttaa tgttgattaa gatatatatt tttagatttt taaatttttt 8520
gtatattgta ttaagtttgt tttaatttga gagagttatg ttttaaattt gatttttttg 8580
tttattttat tattaattag atttaaattt ataaagtttg tagaattaat aattttgagt 8640
taattatata tgaaatatgt tttaatgaat ttttatataa ttaagaatgt tgttaaataa 8700
ttaattttaa ggataatttt taatagttat tttttttttt tagtgagttt aaggttgttt 8760
tgagttatta aagtttaagt aggtagaagg ggtgtgtgtg agttaagggt gaaaagttta 8820
gaattgtgtt taattagtaa aagtaaaatt ttatttatat aaaataaaaa aaattatttt 8880
tggagatatt aatttttat agtattgttt ttaagtaaat ttaattttta aagaaattaa 8940
agaaagaaat ttaaatatat ttaaaataat ttttgaaagt tttttttgttt tttagtatag 9000
gttagttgga gaggataaat taattttttt tgggtttttg tatgggtgat tgttttatta 9060
tggagttagt gttattattt ttgaatgtgt atttgtttga tattatagtt aatgatttgt 9120
aatgttagta tgaagtattt ttaaaatatt tttttttgt ttttgtttat aagattggga 9180
aatttatttg atgtggaata aagtggatga agtagattat aaatatattt gtaatttatg 9240

```
tgtttttttt ttgttttgat tatttttaaa ttttatttgt aatttttttt tattttaaat 9300
ttgtagttta aagatgtata tgagaattgt tttttagttt ttttttatta gtattatttt 9360
attttaagaa taatttagtt gtaagggagg aattttttta tagtaagttt taaattagta 9420
tttttgtttt taatttttta ttttatttta ttttatttta tatatataga tatttgttta 9480
gagtaaaata tatttttatg tgataggttt gtattagttg aggtttatat atttagttat 9540
attaggtttt gtaattttat tattaaatta tatatattat attagtagtt tgttggtaaa 9600
gaaggttaaa ttaatttata ttttgtttat tatttggtgt ttaaatgatg tattttattt 9660
tggagatttg gtggagaatt tttttttag atttatagt gttttattga agataatgtt 9720
tttatatttg tagtggtttt taatttgata agattttaat ttgtttaagt tttttaaata 9780
agggttttaa atgtttttag ttgttttttt attgaatttt ttttaatttt tttaagatta 9840
taaagtatat gtgtaaagta aatatttttt tttattgtat tgttagttga tgatttataa 9900
ttaagttaat aagaatttag ttttttttg ttgaatgtgt ttattaatta tattttagtt 9960
ttttttttta aattttagaa tagttgtggt ttttataata ttatgttttt taaagtttta 10020
ttttatgaag ggattttatt atattaaaga atgaaaaaaa ttttattgt agttagtata 10080
tatagttttt tattttttgt tttttaagat ttaaatttta gagttgtaaa tatttttgga 10140
agtttgggtg ttaatgtttt attttagaaa gttgagaagt tttatagagt tatatagatt 10200
tttaaattta ttttttataa atttatagaa ttttgataaa agttttggtg gttttatttt 10260
attgatggaa tttttattat gataaatata tatgtatgaa ggattttaat tagtttttaa 10320
agtggttgaa aaatttaagg gtatgtgatt gttttttata gtgttaatgt gtgtgagatg 10380
ttggaagtat tggggattag tagtagttta gatgtttaaa aagataaggt gttttaattt 10440
gtgtggattt attgaagtta agtggtgaat aaagataatt atttagataa tttagattaa 10500
agtaaaagta aaattatatt tatttgtata tatatattta tatttatttt atattataga 10560
tatatatatg tatatatata ttggttttgt aaataattga tttaaagtga ggattttttt 10620
tgtatttttt tagtaggagt tttaatattt ttttaatttt ttaattattt tatatattta 10680
tagtagtggt gattgggtga tatttttttt aggtttttg tgtggtagga tattaatatg 10740
ataagtttgt atggggaaaa ggaggtatgt ggtgggaatt aagaaatatt gtttagtgaa 10800
aattttgtgg tatggtggtg gttgattttg gagatttaat gtatataaga tttgtgggtg 10860
tataggtata ggtagtatgg atgagaaagg ggttagaaga aaataaattt tatgtatttt 10920
gtgattttag tattattgtg atttttggtt aagttttttt taattggttt tagaaattat 10980
tatgagttta gtttttaata tagaaatttt taatatggag aatattggtg ggattttggt 11040
agggaaatta gaggtgttgt atggtttatg tggggtaaag aaggaaagtt tagtgttggt 11100
gtgaggtttt gagtttggga gatattaggg gttgttttga ttgggtttt ttgtttattt 11160
ttttaaagaa agattttaga ggagggaaat gtgtgatatg gggttagttg tgttttgtgt 11220
tggtatttgt tattgattat tagttttaaa gttttattta attttatatt ttttagtgtt 11280
agttgtgtaa agttttttg gttatggtag tgagtggttg ggttgtgttg ttaaattttt 11340
tgtattaatt tggtttggga tttaattaag tgatttttga tttttggaaa gagtttgttt 11400
ttagagttta tttagaagat ggtttaatta gatattttt tgagttgtta ggttttagat 11460
gggtgggagt tttgttttgt ttaagttagt ttaaggatga ggtttgtttg gatttagttt 11520
ggagttatgt gatgggtgtg agtgtgtgag tttttggtaa ggtgtagagg ttagatggag 11580
atttgtattt ttgtttgaga agtgttttat ttttttttaat atttggtttt tttttgtata 11640
taaattaagt tgaaaatagt ttattattta ttattttta tagttatgga attaaataat 11700
ttagaaatta aaagttttat tgtagttgtt ttttttttta ttttttaaat ggaatttaaa 11760
aagttttggt ttgttaaaag gggaagatta tttttttgaat tggaagtttg tagatatatt 11820
gagtaatagt tatttttttt gggtttttgt aaatggtatt tatttttttta atttatagtt 11880
ttagttgttt aattatttga gatttggggt aattatttgg gggaatagtg tttagatggt 11940
agtgggagtt attattttat agtggtttgg ggaagagaag agaaagagat tagaggaggg 12000
ggtatttgtt aaaattattt aatgaatatg ttgttaatgt tttttttatat ttgtatgtta 12060
ttgttatagt tttttttaggt gttattgagt ttttagaaag taattatttg ttgaattaag 12120
taaaataagg agaatggtat agtatatgtg tttggagaag gggaaggaag ggtggaatat 12180
gaaattgagt atagatattt aggttaggaa agaaggaagt ggtaaggggt taaatgaagt 12240
tttatttttt tgttattttt ttaaataata gttggattaa atatttattt gttttttttt 12300
ttttttttt ttttttttt ttagtttatg tttatttttt ttttttattt tttttgtttt 12360
ttttttttt tgtttttttt tttttagata tgttggtagt taatatttag tattagttgt 12420
tatggtgatt ataaattatt taaatttaaa aatatttatt tataatttga gatgaagttt 12480
ttattttttt agtgaaataa tattttaaaa gttgttagtt gataaaaaaa aaggaattta 12540
ttttattgta gtaatttaag taatatatta tttttaaagg tttaaattaa aatgttagtt 12600
tgttaaaaat atgttggtag agttttggat attttttttg ttagattttt ataaagaagt 12660
tgattttgtt atttttggtt gttttttaat atatatatat aattttgtat gttttttttt 12720
tttttattaa ttttttttt ttttattaat tattttagtt tttaaagatt ttatgtattg 12780
ggttttaaaa gaaaagaatt tttttttgga ttagaaaata gttttattgg ttgttgaagt 12840
gaaagatgtg gggtttaggg ggaaaggtta ttaggattat aatggtggtg gtggtaggag 12900
gttattttag aggagttaag aagaaaaaaa aatgtaggga gaaggattgg aggtggaaag 12960
atagagtaat agaaaattga gttgggtggt taggtgttgt ggtgaagttt agttttgaaa 13020
```

tgataggtat atatttttt atttttgttt ttttttttt tgagagaaaa tttttttagt 13080
tagagatttt gggggggtagg aggtgggtaa atgttgttgt agttgggttt tttgtttttt 13140
attttgggtt tgttgttttt tgtttatttt ggattatagg gtatttgttt agatgaagag 13200
ttattaatta tttatttagt taatattagg aagatgataa agtttttat ataggattaa 13260
gaagatatta gattgtttta ttagtatatt tttgtttatg aataagtttt tgttatatat 13320
ttttttttt tttgagttgt tttaataatt gttgtatata ttagtagtgg gtggtgagga 13380
ataatagttg aattagtttt aagaaatttt gtgtattgag ttagtagtga ggaatgtgat 13440
ttgtgaagat tattttgtg ggtagggatt tgtagggatt gattattttt ggataattgg 13500
tataattttt tttggggtg aaaaattata atgtggtggg gtatttttta agtgagttgt 13560
agatttgatt ggtgtggggg gtgggggagg ggaggggaga atgggatggt ggaggttggg 13620
tggaggaaag aaaatggaaa attttttta tttttatttt gttgtttttt ttttaagttt 13680
tatgtttttt ttggtaagta tttgtttttt ttgtgttagt tatagttaga gtttttttatt 13740
tttttttata tattttttt tttttgataa ggtttaggat tttggttatt attttatgta 13800
ttattatttt gtgttttgtt agagatggtt tgggttgatt ttgttggtgt ttatgttagg 13860
attaaaattt ttttatgatg gtgaggaaaa ttgtattatt tgtttttagg gggtatatta 13920
ggagtttatg tagtatatgt tttgtaaata tttgttgatt gaatgagagg tgtgggggtg 13980
gggtggtgga gaggggtttg tggttgttaa ggttgttagg gttaatttag gttttttgaa 14040
gaaggttggg attgagttgt tgttgtgtgt gaaggtgtgt gttgtggttg ggggtgttat 14100

aatgggttat ggagtttatt ttttagaggg aggaagtttg tgtatattag tgatttgggt 14160
tgaatatttt tagtttattt attgggttaa agttatttaa taattaatgt gtttttgggg 14220
gaggttgggg gaagtatttg tttttttgttg ggatgtaaag ttaggtgtta ggtttaaagg 14280
ggttgtagtg tagtttgatt ttagtatgga atttagagtt gttgttttga aatttttttaa 14340
gttagtgata gaggagggtt agtttgtttt tttttttgagg gtgaagatta ttttatgagt 14400
tttttttagg atttttaaag taagaaaagt taaagaaagg tttttttgtt ttaggggggtt 14460
gtttttagtt ggtttttata ttattttttg ttagttgtgt ttattttttt ttaaattttt 14520
ggtttttagg ggtttttagt tgtttttgtg ttatttttgt tttggggttt tatttaggtt 14580
gggtatttgt ttaatggata ttaaggagaa tgggatttat tagggaagga aggtagagtt 14640
tggattgttt agaggtggat tttgtttata tagaatgttt agtttttaat gaggatatgg 14700
tatttttggg tggtgttggg ggtagaggtg gagagggtag tgtaatagat tattatggtt 14760
ttttgaagaa gttatatgtt attgtgaatt ttttttttt ttaaaagtaa agaaaaattt 14820
taaaaaaata taagaaataa attttttttgt tttataagta ggttgtggtt aggattttgg 14880
atatttata agttaattta aaattaggga aggataggtg ttttattttt tagtagtgtt 14940
atagttttgt ttatttgtgt gatttttttt tgttgtttat tagtttttta aaagttaatt 15000
aaattaagat ttttagtatt tttttttatt atatgttttt ttttaattaa tggtattaaa 15060
tgtgtttagg tagtaatttt ttttttggt taaaaagtag aaaaagatat attgagtgta 15120
ggggaagagt tttttatgtg tattaaaata atgtgggttt gaaagtaatg gttaagaaag 15180
taattattta ttattttttag tttttatgt gttagttatt aaaagtgaat gattaggggt 15240
gtttgtgtgg tttgtgattg gtgtaagtag aatttttttg tttttagttg tttttttgttt 15300
atttatgagg attttatttt attgtaggtt tttttatttg tttttagaat gtattttttta 15360
tgtttaggaa atttggggta gggattgggg gaaggagata ttttgtgttt ttttggtttt 15420
tagtataata agaaatgtta gttttggttt ggtgattgtg agtttgtttt gtgtgaagtg 15480
agattgggga gtttttttag tttggttgga gttagggttg agtttgtgta aagtattttt 15540
tttagaagtt attgttgttt ttgattttta attatattt aaatatatta tggtttaata 15600
atttattttt attattgatt attaaataga agaagaattt aatataattt aaatgataga 15660
aattatgtga tgttattttt gtattgtttt tatttaattt tatggggtta attttggata 15720
agtgagtgtt taattggttt agtagggtga ttggtggtgt agtgtagtgt ttgggtgtga 15780
agtattggat tgtttttagat gttttatttt aataaatgat tatttttttt tagatttatg 15840
gggaaatttt aatgtaagat ttttgttttt tttttagtaa tatggtttgt ttttttgatt 15900
ggggttttaa attgtttttt tttattttat attatattg tatttttata ttttaatttg 15960
gaaagagggg gttaggggtt gagggttgtg gggggggggg ggttttattt gtttatatta 16020
ttaaaggtta atagtttttt aaggttaggt atttatttat tatggagtta ggaaaataga 16080
ggaatagtaa atttgagggg tttttttta tgtatttgaa aagaaaggta tttttttttt 16140
tttattttt atatttttt ttttgtttta tagaataagt tttaatttag gaaaggtttg 16200
tggtgtaggt tggagatttt ttaattttt atataagttt gtagattttt tttggtaatg 16260
ttttttgatt tttttagagt gaaattagtt aattaagtaa tgatattgtt aaaatttaag 16320
gtttggtaat tagtatttta ggtaggtttg tgttgatagg ggtaaatttt tattttattt 16380
tgggttgtta agtatagtgg ttgtttttta gtttttagg gatgttgttg gttttttgtt 16440
tttttttaa ttagttaaag taaattttg ttaatttaag ttttttttgt ttgtttttg 16500
tgatgaattg tgtatttata agtttgggtg gggtgtggtt gagagtttga gtgattgagt 16560
gggttttggt ggtgttgtgt gtagtgggat ataatgagtg atagaggttg ttgttggatt 16620
attttttat gttagtttag atgttgaggt ttgttggagt gtgtgtaggg gattagatta 16680
tagggagtga gtgagaggga gagagaggtg ttgggtttta ggagtgtagt ataatttggg 16740

gaaaggaatt aatgtttttg ggatggttgt ttttgtttt atttagaggt ggagtgttta 16800
agtttaagta gtaggtgtgt taggtttggt ggtttttgttt ttttgtgttt tgtttgaggt 16860
ttagagtttt ggaggtgggt gtttagtgtg tggtttgtgt tttttttttg gttttattat 16920
tggtgttagg atgttgttgt gggaagaatt tgttgttggt tgtttttttt tggtttttag 16980
gagagtttgt gaatttgatt tttttgattt tggagttttt ggagaagaga tatttaatgg 17040
ttgttggttg tatgtttggg ttatgtgtgt gtttgtttta tgtgtggaga gaggtgtttt 17100
ggattgtggt tgaaaggagt tggggatggg aggaggggga ggggtgaggt aggttggagg 17160
agaaagaggg ataaagagta aagatttagt tagaggaaag agttgatggt atttttgttt 17220
tttggatttt ttggtaattt ggggtaggat ggtgtatttt ttttgtgttt ttttggttgt 17280
tggtggtttt agttgggagg agtaggttgg ggggttttgg tatatagtgt gttgttgttt 17340
tttagtttat tgttttgtta tagggagagg ttattggtga tttggttttg attttttttt 17400
tgtttaggtt ggtttttggg ggaagtgttt tttgttgggg ttttgttgta gggttagtgt 17460
tttttgttg tttttatgtg gtgtggtttt ttgtttgatg atttgggtag gagaaggggg 17520
tttttattta attgtatata tgttgatatt agtttgtggt agttggtttt tatttttttgt 17580
tatttgtaaa atagaagaga aggaaggttg taagaagtgg tggttgttga gtgagtaggg 17640
tttagatgag attatgttat attagttgtt aggtgtttat tgtgtgttag gttttaggtg 17700
tgtttttttg atttgatagt ttttggttgt gtagtagtat ttttagttta gttttgggtt 17760
taggatattt atttattaag aggggatttt tttttagagt tgttgtaaaa gtgtttagag 17820
gttagaggat tataaagtta tagtgtgttg gggaggttgt ggatttattt ttaagaattt 17880
tggtgttggg gttaagaatt tatttgaatg taatggtagt gggagtgggt gggtggagag 17940
gatttttttt tttgggaagt tgtatgtaaa gattattttt tagtgtttgt ttattagttg 18000
gagtttggta aatatttgta gaatattagt gttaatgtgt ttttgtttta gatagtagtt 18060
ttttttggtt ttttgtaatt ttgaaatgaa tgggtttttg gtttagggtg ttttaggagt 18120
gagttgagtt tgggtttttt atttattagg agttattttt ttatatttag ttatattttt 18180
ttttagagat attaatttgg ttatttattt atttattata aataattatt ttaaagtatg 18240
atttaagatt gtagaggaga gatattgggt ggattgagtg agattgagga gagtagggta 18300
aatgtttttg gagggtttat tgtttgttaa ggatggagaa atagttttgg tataattgtt 18360
atttagtttt tttttttttt tttttgggtg agttaaattt tttttatgtt tttaattata 18420
atgtagtgag ttaagtattt aatgtgtttt tttttttttg ttataggtaa gttgggagag 18480
gtgggttttg aggggtttta ttgggtgggt agaagagttg tggttgtttt aaagataaga 18540
aaagaaggtt tagggttttt taggtttttt tgattttagt gtttgttttt ttttatgtta 18600
attagggtat gttgatgatt ggagggttta ttttgtgtgg gtgtggggat tggggtggga 18660
gtaagtgttg ttgggttggt ggaggtatag aggtggggta gggagttgtg ggtttgtttt 18720
tggtttgagt attgtttttt tgtgttttgg tttttttttga agggagttgg gttttgggga 18780
gtttttggtt aaggttgttg tttataggag gggttgtttg gtgttgtggt gtggggattt 18840
agggtgggga tggttaggtg gtttttttat ttgttagtga gaatgtgggt ggggatttttg 18900
ttgatttgat ttttgtgggt ttgtgggttt agaagtagta gtttggtggt tttagattta 18960
gtgattttgt agtaaaatta taggattagt ttttgattga gatgtttgtt tgtgagatat 19020
tataaaattt attattatag ttttttatta atttgatatg aagtaatata gatgggattt 19080
tattagttta gattttaaat gtttatttat gataattttg gaggaaattt gtatgttatt 19140
attattttga taattttttt tttttatatg tttgaattgg ttgtattatt agttggtagt 19200
tggagtattg tagatggtaa ttgtaaatag tttttattta tttattttt ttaaagaatg 19260
aaatatataa aagaaaaaga ttgtgttgtt tggtgtaaag ttagttaatt attatatatt 19320
ttttttttta tttttttgtg ttttagtgtt gaagattaaa taaagtaata taaaataaat 19380
ttttaagaat ttatagagtt ttattttaag gattgaaaag aaggttaagg tgtgtttttt 19440
agtttatttt tatatgtttt tgtgatttgg agatttattt tgtagttaaa atgagttttg 19500
agatttgtat ttttatgttt tatttaatga ttaggtttat tagaagaatt gagtttaaat 19560
aattggggaa gataattttt taaaaagaga tttttaattt ttgtttgttg atttttaaat 19620
ttgttttatt aagataagtt ttttgtgaga aatttggttg ttagatttg gaattggttt 19680
taatggttaa ttttataaat tgagatggga gatttttttt gatgggaggt agtttttatt 19740
tttaaagttt atgttttagt tggaatgtat atgttaagga tttttgtttt ggttaatttg 19800
ggttttatat tgtgagtata taaaaagtat tatatggtta atggaggatg aggaattatg 19860
gtaaagtagg taggtaagtt ttaagaaata aaataatttg ttaaaaaata atttttgatg 19920
attattgtaa gattgaaagt gtaggaaaaa tatagtttga ataattttag atttttttat 19980
attttttttt tttttatata ttttgttatt ttataataaa atttttaatg gaaagtttaa 20040
aaataaatag tataggaata tgtgttttaa atgaattaaa ttgtgaaatt agttagtaaa 20100
ttaatttgta gtaagtaatt atttaaggaa attaaaatat tgtttagttt agttttgtat 20160
tttattatgt gtatgtgttt tttataatta attaatataa gtgttttagg aatatttgaa 20220
gataaatatg tttaatttaa ggaataaagt atttaaataa tttaagtgta attttgttga 20280
gttaaagtaa aatattttat aaatgaagtg gttatttaat tttttaggga aagtttggtt 20340
attgaaatgt tgtatgttta tgttatatta ataaaaattt ttaatttatt ttgtttatgt 20400
gttttgtttt tttgatatta ttggtatttg aattttagat ggatttttgt taaaatgata 20460
ttttgtgtga taaaagtatt tttagttttg attgatagat taaaataaat gtaaggaaat 20520

tttttaaat tagattaatt ttttataaaa atattttaga atgtatgaat tttgatattt 20580
atatttataa tggtaaaagt tttttttgtt tagtttagta agataatatt tatataaaag 20640
agtaaaaaaa aattatatta ttttatgata gtttgatttt taaattgttt aagaaagtaa 20700
agtggttaaa ttggaaaaga ggaatatatt ttggaggttt agaattgaaa attttttttt 20760
taatttttag ttggaaaata attttttgta tttatttaaa gtgtattttt tgaagtgtta 20820
gattggagtt gattggtgat taatttaaag gagttataat ttaaagaaat ggtgagagtt 20880
tggtatttag gtttggtttt taggtaattt gtttgggttt gagaggttat taattgttag 20940
ttaagatgga attttttttt tttttttttt tttttaatgg ataataatgg gaaggggggtt 21000
aatttttag tagttgaaat tttgtattta gtttttttatt ttgagaatgt taatttttgg 21060
tttgaggatt tgtttttgta gtgttggtat tgagatttaa gggaagatat tttgttttaa 21120
atgttagtta tggtttggtt ttttttttga ttttagtatt ttgtagattg ttagtgtttg 21180
tggtgggggga tgaaaggaat agggtttttgt aaggtttgtt tgttgattgt gttattttgg 21240
gtgaaattta gttttaaaag ttataaatta tttatggtga agatttttttg aagtggaata 21300
aatttttaga tttgtattat tttatatttt tgtgggatag atggtttttta tttattggtt 21360
attgggagag agttgttgtt tttgtgtttt attgtttttt ggggtgattt ttagtgagtt 21420
gagttttttgg ttgtatggta agtgtttgaa agttgggttt gagaggattg tagggttttt 21480
gagggtgtta agtttgaag gagtttatgg gtgtattggg gtttttgaaa tttagttgtt 21540
attggtagtt tttttttgtt tttttttagt tttttttgttt ggtttttgtat ttttttttttt 21600
ttttttttt tttattttt tttttttttt ttgtttttat tttgtgtggg gagtgatgtg 21660
atgttagtag agatttttatt aaattttatt gtatagtggt gtgtgggtgg ttggttgagt 21720
ttggttgtgt ggttggtgat ttaggagtga gtatagtgtt tgggtgagtg ttgggggggag 21780
tgagtagggg tgatgagaaa tgaggtaggg gagggaagta gatgttagtg ggttgaagag 21840
ttgggagttg gagttgggag agtgaaagga gaggggattt ggtggggtat ttaggagtta 21900
attgaggagt aggagtatgg atttttattg tggaaaggag gattagaagg gaggatggga 21960
tggaagagaa gaaaaagtaa tttgtgttaa tttggtagtt ttaataaatt aaaggggggag 22020
tgttagggta gtggggagat agaaatgtat ttttggggag taaattagga tgggttggga 22080
ggaagtgata gggaaagtgg tttaagagat ggaataaagg ataatgttta tggggttgtt 22140
tgggatgagg tgtgtggagt gtgggtgtga gtgtgtgtgt gtgatttttt tttaggtttg 22200
tagagttgag gaaagaggtt atagtaaaga gggattgtgg agggaggaaa gtgagagatt 22260
ggtagagggt gggagtggag gtgggtgtgg tggggatggg agaggatgag tgaagagaaa 22320
tttagaagaa tggagtgagt tagtgggaga gggtgggagg gttatagttg ggagtgaatg 22380
agttaggttt gttagttggg gaaggttggg atgttgggtt tagtttagtt gggatattgt 22440
gtttgaggtt aaggtgggtg gattaggtat gttgagagtg ttggtgtata ggtgggtatg 22500
gttatgtatt gatttagtgt ttatgaaggg tttgtattgg ataaggttta gatgtttata 22560
gagtttagaa tttttttttgt tgtatttata tttaataagt ttattttggg ttatggatat 22620
tttattttt aaaatgatga ggttaaggtt tttggtgagg atggtattaa attgtatggg 22680
atagaagtgg gggtggggga gagagttttt tttaagttta tatttgtttt tgtaaagtaa 22740
agagtatgtg aaattatagg gtatattttt atttgaaaag tgtgttttat ttttgaattt 22800
tgatttttttg atttttttgat ttgagtaaag atgtgtattt tggtagtgag tagaatattt 22860
tggtttttgtt ttgtttttga gtggaaggat tataaatata atttgtttgg aggattaggt 22920
gtgaaggttt ttgttaggta tatgggataa tgttttttta attttaaggg tattttgtta 22980
atgtatgttt ttggaaagtg ttggaatata gttattgttt ttggatttgg attttttttat 23040
taatattaat ttttgtttga gagtaaaatt taggtttgtt attaaaaaga tattttttttg 23100
gttttttaatt gagaataaag tttttttttaa aagttgtatt gtttttttta aattaatata 23160
ttaatatttg taattttaga aatatatagt gatttgggag aatgtgtata aaatagatat 23220
gtttaaaaaa gtttggtgtt taaaattaat tttagttatt atataggtgt tgggtttttt 23280
ttattttttgg gggttgtttg gaatatgtta tgtgtttttt tgaattattt tgtgttttga 23340
atttatttga gttagtagta aaaataggta aataaatttg tttaatttgt tttgagtgtt 23400
aaatttttt attttgaaat agttaatagt tgatagatgg atttatttta tggaaagggt 23460
tagttttttt agttatgaag aaaattgatt agagatttat attttaagtt atttttaatt 23520
tttatgtaat atttgtgaaa atttaaattt tttttttta tttagtggaa atttaaagta 23580
gtgttattta aggggagaga aatgaggggg aaaatgttta tgtgttgttt aattgtattt 23640
tttttttgat tttgagaatt tttattttttg gtttttgaaa ttttgttgag gtaagaaaat 23700
taaattttt taataagttt tataattgaa ttttagttat aggatattgg aaagtgtagt 23760
ttgagaaaga tatttttatt tttgtttatt gatgattttt gtagtttttt tattttttttg 23820
agtaatgggt taataatttt tttttttttt ttttttattt tgtagagatt aagaggtgtt 23880
tgtagtagaa tggtttttgtt tttagttggt ggtgaggata ggtaatttta tggaaaagtt 23940
ggaagagaat gagaaaatta aagatagaaa gatttagaga tttgtggaga gatataggga 24000
gagggaaggg agttgtgttg aaaagatgta aagatatgtg tgtgtaattt ttttttttt 24060
taggttttag aggtttgtaa attagggttg agaggaaggg gtttgggaag tttatgtttt 24120
ttttgttttt tttttgtttg gagttttgtt tgttagaggt tggttaattt tagttttggt 24180
tgttgtagat attgtgttga gtttttgggt ttttgttttg tttagtgtta gtgtagttga 24240
agtgagtagt tggtgggaaa tgtaaatggt ttttggagaa atagaagata tagaatgatt 24300

tttatttttt ttttgagtgt gtggaaggag ttggatatat gttttatgtt tttaattttt 24360
tttttatatt tttagttata tttttattaa ataattaatt aatgtttaga attattaggg 24420
aatatattag gtatgtaatt gtagaagtag ggtgttgggg ggttataaat tattgagttg 24480
atttaagatg tggattttag gttttttttt tgttaaagta gtaaaggaag agtgggtttt 24540
ggtgattgta tttagatttt gattatttta aattagaagg gggtggaggg agtgtttaag 24600
taaagtaagt aattttttgt tttgtagatg taaataagat tgtagtatta aaggtattag 24660
tttttttagg gttagattgt ttggattggg agtttgggga aggggagata ttaattttat 24720
gtatttgtga attttaagga tgttatattt ttatataaat aattttagtg tggatttttt 24780
ggaatggggg gagtaatatt tttattttag aatattaaaa tatttttttt ttaaagtgta 24840
tatttttttt atttttttaa aattttgaat tatgtttaaa gataatagtt ttttagtaaa 24900
ttggagtatt ggattatttt ttttattttt ttttattgat attttgatga tttgatttta 24960
atgtgtgggg ggtataggga attaaatata gtttataaaa ttaagtttag atgaaatagt 25020
gttggttaag tgggtttaga taatttttaa tgagaatttt aattatattt ttttttttaa 25080
tatgttgaga taagtgatag aattgttaga atggtaatta aattggaaag tttagggaga 25140
ataataattt tgtgattaaa ttggggtaaa attgtggata aatgtggggt gatttttgtt 25200
aattttttgt tatttaagag ttaggatttg ggaaaggtat agtattattt tagagtttgt 25260
tgtgatgggt tgtgtgttat tatttatttt ttttattttg gattatgatt ttaattttgg 25320
taagtaattt ttttagtttt ttatttgata ataagtgagt atgtaaatat taatggttag 25380
tgatgtttaa ttgttttaaa tattattgat ttgttggttg ttttaaattg tttttttagt 25440
ttaggttttg tttttgaatt gtttatttta gaggtttgat ttatgttttt gatgttataa 25500
tataataatt gtttttttaa aaaaggtatt taagatgaat taattgattt gtatataaat 25560
taaaattatt atgtgttgtt gattttggtg ttttataatt attttgaaat tagtatttaa 25620
ttatttgagt taaaagaata tataaatgtt tgtattgatt tattaatgaa ttatttaatt 25680
aaaatgtttg ggtaatgttg ggtgttggaa agattgttaa attaagatat attataggag 25740
ggatatgaag attagaaagg taatagatta atattttgta tttaaaatgg agtttttggt 25800
gatttttagt tttaattttg gagtaggggt tttttttttt tgttgttaaa aagattttgt 25860
gtttgtttgt gagtgagtgt atttaagtgg aaggaatgtt tttatggtta tggtggttta 25920
ggttttttgt ttggattggg attttatagt tttaatttag gagtgttaaa ttttggaaga 25980
ttttgggtta gttttggagg tgtgtggttt tgtaagttgt taggttaagt ttgttttttt 26040
tgtttgtttt tttggtaggt tgggtgtgtt atggtagtga gttttttgtg taaatggaga 26100
gttggaatta aagttgatat ttaatagata tgttaattga gtatttattt ttgttttgag 26160
aataggaata aaaggtagtt ttttttaaga gaggtggtgt aaaggtatgt tataggagtt 26220
tagaaaaggt tggtggtggg aaatttgtag tttgggggtt agttaatatt tttttttatt 26280
ttaagtattt attgatttgt tgttgttatt tttggtgatg tagaaggata tttgaaagaa 26340
tttttgatgg ggttttgatt tgagaaagga ggtgatttgt ttaggttttt attaaatttt 26400
taattattat attaattgtt tttttttatt ttttatttga tttttttttt tttgtttatt 26460
tttaattttt taattattta gaaatttttt tattttttag tggttttttt tttgtagtag 26520
ttttttattt gaattttttt tttgtttttt tgtggtaggg tttgtatatt gatttttttg 26580
atttttggta tatttgggtt ttttgaaatt ttttaatttt tttagatttg aggatggtag 26640
gttttatttt ttttattgtg tgtatatatt tagagatatg aaaatttata tagattgttt 26700
ttaaatttag ggtatttaat agatgttttt tttttagttt gttttttgat ttgaaatgtt 26760
tgtttgattt taatttggat attatttttt tttgtttttt tttttttaaa gtagtttgga 26820
tatgtgtgta agtgagttta gaatagtttt atttatattt tttattaaat tgtaaataaa 26880
agaagaatta atgaagtaga ttggtatata gattgtatta agagtttgaa tttttagttt 26940
ttggattttt tatttaattt tggttgttat ttatattgat agagttattt taagtagagg 27000
tttagagaaa tttgtattgt gggataatag gtaaagttat agtaaaaagt ggaataattt 27060
taaagttatt ttattagaat gtaaattgta tttttgggtt ttgtttgtaa ttatttagtt 27120
ttaatatata tagagttaga taggaaaaaa taggttaata tagttattgg tattagagaa 27180
gataaatttt atgggttttt tagtgaaaag aagattttta aagtttataa tttttgatta 27240
tttaatttta tttataattg tgggaatgaa taagatatta attgttttat gtattttatt 27300
tatattaatt aatttgtgtt tttattaaaa gtagttatat agaatttttt ttaatttttg 27360
gtagtaagtt tagaaaatga agtttatagt tattttgaat tggatatatt ttttgagttg 27420
attatttttg taagtgtagg aatataatat tgttttttta tggttttttt gtattttttt 27480
agggtttgta agtttttatt aggtttgata ttattgtttg ggtttatatt tattataagt 27540
aaatttgatt attatgttga ttttaaaata gtttatttgg ttagtataat tttagttttt 27600
aaattataaa aattttttaa tatatgaagt ttttagtttt tatttttttt agttttttgt 27660
ttatttaaaa tttttatttt aattggtgta agtaataata atttgtatta ttatttgtat 27720
tttttttatt tttttggaga ttgggttgga ttttagagag aatattagta ttattattat 27780
tataaataat aaaatttaaa agtaaagttt ttatttgtat gataattggt atttggaatg 27840
tttttgattt atttaatgtt attttataaa ggtattttgt aaattttttt ggaattttta 27900
gtaagagttt gtagtaattg gaataatttt ttgggaagat attttttttg atgggttttt 27960
agtttttgga ggaatagatt gagagtaatt agggagggag gggatattgg aaattggtag 28020
ttatgttagt tgaaataagt ttgggtttag taaggtgatt gatgttgtgg ttgatttttt 28080

```
atttgagtt ttttttaat tggggtattg atttttttta ttttgggatt ttaaggtatt   28140
tggtgtgtat gtagattttt tttttgtggt ttttattatg tggttttgta gtaggttttt   28200
ggtttaatga tattttatag ttatagtttt tatatttatt attatgattt taatgtttag   28260
gtttttagtg tatttatatt aaatttgttt tattagtaag ttggagtata taggagagat   28320
gggggtaagt aaggatttag tagagtttaa atttagatat gtttaaatgg ttttgattgt   28380
gtaaagtgtg gtaatgtttt ttgttgtttt agtttttat tttaagtttt atatgttttt   28440
tggttaatga agtgtgatat aggttatatg ttaggaataa tagtatttgt tgagaataaa   28500
gtgaatttag gaaatttggt atatataaaa tgtatt                           28536
```

<210> 5
<211> 28536
<212> DNA
<213> Artificial Sequence

<220>
<223> chemically treated genomic DNA (Homo sapiens)

<400> 5

```
gatgtatttt gtgtatatta ggttttttga gtttattttg ttttttaataa atattattat     60
ttttagtatg tggtttatat tatattttat tagttagagg atatgtgaag tttagagtgg    120
aaagttaggg tagtaggaag tattgttata ttttatatag ttaaagttat ttaaatatgt    180
ttgaatttga gttttgttga gtttttattt gtttttattt tttttgtgtg ttttagttta    240
ttaataaagt aggtttaata taaatatatt aaaagtttaa atattgagat tatgataatg    300
aatatgaggg ttatgattat aaaatattat tgaattagag atttgttatg aaattatatg    360
gtgaagatta taagggagga atttgtatat atattgagtg ttttgggatt ttaaagtggg    420
aagagttagt gttttaatta aagagaaatt tggggtaggg aattaattat aatattagtt    480
attttattga atttaggttt gttttagttg atgtagttgt taatttttaa tgttttttttt    540
ttttttaatt gtttttaatt tatttttttta gaaattgaaa gtttattaaa gaggatattt    600
ttttagagag ttgttttagt tattataagt ttttgttaga aattttaaga aagtttataa    660
ggtattttta taaggtggta ttgagtaagt tagaagtatt ttaagtatta attattatgt    720
aaataagggt tttattttta gattttgttg tttgtggtgg tggtgatgtt ggtgtttttt    780
ttggaattta gtttaatttt taaaaaagta aaaggagtat aaatagtaat ataaattatt    840
attatttata ttaattaaaa tagaagtttt gaatgagtaa ggagttagag gaggtaaaaa    900
ttgggaattt tgtatattaa aaggttttta taatttgaaa attaagatta tgttggttaa    960
ataggttgtt ttaaaattag tatagtaatt aaatttgttt gtaatgaatg tagatttaaa   1020
tagtgatgtt agatttgata aggatttgta aattttaaaa gagtgtaaaa agattataga   1080
agaataatat tatattttttg tatttataga aatggttagt ttaagagatg tatttagttt   1140
agggtggttg taagttttat tttttgaatt tgttattaga agttaaaaga aattttgtat   1200
aattgttttt gatggaaata taaattggtt gatgtaaatg aaatatataa agtagttggt   1260
gttttattta ttttttataat tataaatgaa attaaatgat taaaaattat agattttggg   1320
gatttttttt ttattgagga gtttatggaa tttgtttttt ttagtattaa taattgtgtt   1380
gatttatttt tttttgttta atttgtata tattaaaatt aggtggttat gaataaaatt   1440
tagaaatata atttatattt taataaaatg atttaaaat tatttttattt tttattgtgg   1500
ttttatttgt tgttttataa tgtaggtttt tttgggtttt tgtttagaat gattttgtta   1560
atgtagatga tagttagagt tgaatgggga atttagaaat tggggatttg ggtttttgat   1620
gtaatttata tgttaattta ttttattagt ttttttttta tttatagttt ggtaaagaat   1680
atgggtggag ttgttttggg tttatttgta tatatgttta aattgttttg aaaaaggaag   1740
ggtaagaaag agtggtattt aagttggaat taggtaggta ttttagatta agagatgaat   1800
tggaaaggga atatttgtta gatattttgg gtttgaaggt agtttgtgta agtttttata   1860
tttttgagtg tgtgtatata gtggagaggg tggagtttgt tatttttaaa tttgaaaaga   1920
ttgagagatt ttagagggtt tagatgtgtt aaaggttaga gggattaata tataggtttt   1980
attatggaaa ggtggggaaa aggtttgaat agaaaattgt tgtagaaggg aagttattga   2040
gaggtaaggg agtttttgaa taattaaaaa gttaagaata agtaaaagga aggaggttgg   2100
gtgggggata aaaaaaagta gttgatgtgg taattaagaa tttggtggga gtttgggtag   2160
gttatttttt tttttagatt agagttttat tagaaatttt tttaagtgtt tttttgtgtt   2220

gttaaagatg ataatagtaa attaataagt gtttgaaatg aaaggggatg ttgattagtt   2280
tttaggttat agatttttttg ttgttagttt tttttgaatt tttatagtgt gtttttgtat   2340
tgtttttttt aagaagagtt atttttttatt tttattttta ggatgaaggt aagtgtttag   2400
ttagtatatt tattaaatgt tagttttggt tttagttttt tgtttgtgtg gaaagtttat   2460
tgttatagtg tgtttagttt gttggagggg tagatagaaa aagtaagttt ggtttggtga   2520
tttgtggggt tatgtatttt tagggttggt ttggagtttt ttagagttta atgtttttgg   2580
```

gttagaattg taaggttttg gtttgagtaa agggtttgag ttattgtagt tgtgggagtg 2640
ttttttttat ttgaatgtat ttatttataa ataagtataa aattttttta atagtagagg 2700
agaaagattt ttgttttaaa attaaagttg ggaattattg gaaattttgt tttgagtgtg 2760
agatattggt ttgttatttt tttaattttt atattttttt tgtaatatgt tttgatttaa 2820
taattttttt agtgtttagt attgtttgga tgttttaatt gggtaattta ttagtgagtt 2880
aatataggta tttatatatt tttttagttt aagtggttaa gtattaattt tgaaatgatt 2940
ataaaatatt ggaattggta atgtatagta attttaattt atatgtaaat taattggttt 3000
attttaaatg ttttttttaa aaaaataatt attgtattgt agtattggag gtatggatta 3060
aattttttaga atagataatt tggaaataag atttggatta ggaagataat ttagaatagt 3120
taataaatta ataatgtttg aggtagttaa atattgttag ttattggtat ttatatattt 3180
gtttgttgtt agataaggag ttggggaaat tgtttgttag ggttgagatt ataatttaga 3240
gtgaagaaag taaatggtag tatatagttt gttatagtgg gttttgaaat aatattgtat 3300
tttttttaaa ttttgatttt tgggtgatag ggagttggtg gaggttattt tatatttgtt 3360
tatggttttg ttttaatttg attatgaaat tgttgttttt tttgagtttt ttaatttgat 3420
tattatttta atggttttgt tatttgtttt aatatattgg ggggaggagt gtaattgaga 3480
tttttattaa aaattatttg aatttattta gttagtattg ttttatttaa gtttagtttt 3540
atgggttgta tttaattttt tgtgtttttt atatattaaa attagattat taaaatgttg 3600
gtaggaaagg gtgaaggaaa tggtttaatg ttttagttta ttggaagatt attattttta 3660
gatatagttt aaaattttga ggaaataaaa aggatatatg ttttgggggg aaaatgtttt 3720
aatattttag aatgggggta ttattttttt tattttagag aatttgtatt ggagttgttt 3780
atgtaaaaat gtaatatttt tgaaatttat agatatgtaa ggttagtgtt tttttttttt 3840
taggttttta gtttaggtga tttagtttta aaggagttag tatttttgat gttataattt 3900
tgtttatatt tgtagggtag agaattgttt gttttgtttg gatgtttttt ttattttttt 3960
ttaatttgaa gtaattggaa tttaaatata gttgttaagg tttgtttttt ttttattgtt 4020
ttgataaggg aaaaatttga aatttatgtt ttaaattagt ttggtggttt gtagtttttt 4080
agtattttgt ttttatgatt gtatgtttaa tgtatttttt ggtgattttg ggtattaatt 4140
agttgtttaa taggagtatg attaaaaatg taaaagaagg attaggagtg tgaaatgtat 4200
gtttagtttt ttttatatat ttgaggaggg aatgagaatt attttgtatt ttttattttt 4260
ttaggagtta tttgtatttt ttattagttg tttattttag ttgtattggt gttgggtaag 4320
gtgaggattt aaaagtttag tgtagtgttt gtggtggttg ggattggggt taattagttt 4380
ttggtgggtg agattttaga tagaaggggg gtgagaggaa tgtgagtttt ttgagttttt 4440
ttttttagt tttggtttgt aaatttttga aatttgaaag gggagggagt tgtatgtgtg 4500
tatttttgtg ttttttagt gtaatttttt tttttttttt tgtgtttttt tgtggatttt 4560
tgaattttt tgtttttggt tttttattt tttttaatt tttttatgag attgtttatt 4620
tttgttatta gttgaaggta aggttgtttt gttatgagtg tttttaattt tttataaaat 4680
gaaaagaaaa aaagggagga ttattagttt attatttaga ggaatgggga ggttgtaaaa 4740
attgttgatg ggtagaggtg aagatgtttt ttttggattg tattttttgg tgttttgtaa 4800
ttagagttta gttgtgggat ttgttgaaga aatttgattt ttttgttttg gtgagatttt 4860
aaaaattaga aatagaaatt tttagagtta gagaggaaat ataattaaat agtatgtggg 4920
tatttttttt tttattttt ttttttaaa taatattgtt ttgagttttt attgggtaaa 4980
gagagaaagt ttgagttttt atggatgtta tgtggaggtt agaaatggtt taaaatgtag 5040
atttttaatt agtttttttt gtggttgaag aggttaattt tttttataaa atgagtttat 5100
ttgttgattg ttagttattt taaagtgaag ggatttagta tttaaaataa attgagtaag 5160
tttgtttgtt tgtttttattt gttaatttaa atgaatttaa aatatggagt aatttaagaa 5220
aatatataat atgttttaga tagtttttaa aagtagggaa agtttagtat ttatatagtg 5280
attagggtta gttttaagtg ttaagttttt ttaaatgtat ttattttatg tatatttttt 5340
tgagttatta tatattttta aaattgtgag tattggtata ttgatttagg aagagtaata 5400
taattttttag agggaatttt atttttaatt agggattaaa gagatgtttt tttaatagtg 5460
ggtttgagtt ttgtttttaa gtaggaatta atattggtgg gaaaatttga atttaggagt 5520
aatggttgtg ttttggtatt ttttaaaaat atatattaat aggatgtttt tgagattgaa 5580
aaaatattgt tttatatgtt tggtagaagt ttttatattt ggttttttag gtgaattata 5640
tttatagttt ttttatttag aggtaggata gagttaaaat attttgttta ttattaaaat 5700
atatattttt gtttaagtta agaaattaga aaattagggt ttagaagtaa ggtatatttt 5760
ttgagtgaga atatgttttg taattttata tattttttgt tttgtaggag taaatgtgga 5820
tttgagggaa attttttttt ttattttttat ttttattttg tgtaatttaa tattattttt 5880
gttaggaatt ttaattttgt tattttaaaa aatgagatat ttgtgattta gggtgaattt 5940
gttgaatgta ggtatagtag aggaaatttt agattttatg agtgtttgag ttttgtttag 6000
tgtaaatttt ttgtgaatat tgggttagtg tgtggttgtg tttatttgtg tgttgatatt 6060
tttagtatgt ttggtttatt tgttttgatt ttgggtgtgg tgttttagtt aagttgggtt 6120
tagtgttttg gtttttttta gttgataagt ttagtttgtt tgtttttggt tgtggttttt 6180
ttattttttt ttattagttt attttatttt tttagatttt tttttattta tttttttta 6240
tttttattgt gtttattttt atttttgttt tttattggtt ttttattttt ttttttttgt 6300
agtttttttt tgttgtgatt ttttttttta atttgtaggg tttgaaagaa ggttatatat 6360

```
gtatgtttat atttatattt tatatgtttt gttttaaata attttatgaa tattgttttt   6420
tgttttgttt tttgggttat tttttttgtt gttttttttt agtttgtttt gatttgtttt   6480
ttaaaagtat gtttttgttt ttttgttgtt ttggtgtttt ttttttgatt tattagggtt   6540
gttgggttgg tgtagattgt tttttttttt tttttatttt attttttttt ttggtttttt   6600
tttttatagt gggagtttgt gtttttgttt tttggttggt ttttaagtgt tttgttaggt   6660
tttttttttt tttgtttttt tggttttggt ttttgatttt ttggtttgtt ggtatttgtt   6720
tttttttttt gttttgtttt ttgttgtttt tgtttgtttt ttttggtgtt tgtttgggtg   6780
ttgtgtttgt ttttggattg ttagttgtgt agttgggttt ggttggttgt ttgtgtgtta   6840
ttgtgtagtg gagtttggtg gaatttttgt tgatgttatg ttattttta tatggagtag   6900
gagtagaggg aagagagagg gatgagaggg agggagagga gagagagtgt gagattgagt   6960
gagaaagttg gagaggagta gaaagaaatt gttagtggtg gttagatttt ggaggtttta   7020
gtgtatttgt ggattttttt ggaatttggt atttttagga gttttgtagt tttttttaggt   7080
ttggttttttg ggtgtttgtt gtgtagttgg aggtttggtt tgttggaaat tgttttggga   7140
agtagtggga tgtggagata gtagtttttt tttggtagtt ggtaagtgga ggttatttat   7200
tttgtaggga tgtgagataa tgtgagtttg gaaatttgtt ttattttgga gaatttttat   7260
tgtaggtgat ttgtggtttt tggggttaag ttttgtttaa ggtaatgtag ttggtaaata   7320
gattttgtaa agtttgtttt tttttgtttt ttgttataga tattaataat ttatagggtg   7380
ttgaagttga gagggaagtt agattgtggt tggtatttaa aatgaggtat tttttttttaa   7440
atttggtgt taatattgta ggaataaatt tttgggttaa ggattagtat ttttaagata   7500
aagggttggg tataaagttt tagttattgg aagattagtt ttttttttat tgttatttat   7560
tgggaaaaaa aagaaaagaa aaagatttta ttttaattgg tagttagtga tttttttaggt   7620
ttaagtgaat tatttgggag ttaggtttgg atgttaagtt tttattattt ttttggattg   7680
taatttttttt aaattgatta ttagttaatt ttaatttggt attttaggag atatatttta   7740
aatggatgta gagaattatt ttttagttgg agattaagaa aaaaattttt gattttaaat   7800
ttttgaaata tgtttttttt ttttagttta attattttat tttttttaagt aatttagaaa   7860
ttaaattatt ataaggtggt gtgatttttt tttattttt tgtgtgagta ttgttttatt   7920
aaattaaatg gaaaaaattt ttattattat aaatgtaaat attagaattt atatatttta   7980
aaatattttt atgaaaaatt aatttgattt aaagaaattt ttttgtattt gttttagttt   8040
attaattaaa attaaagatg tttttattat ataaaatatt attttggtag aaatttattt   8100
aaaatttaaa tattaataat attaagaaaa taaagtatat aagtaaaata aattgaagat   8160
ttttgttgat gtaatatgag tatataatat tttaataatt aaatttttttt taaaaaatta   8220
aatagttatt ttatttgtgg aatgttttat tttaatttag taaaattata tttaaattat   8280
ttaggtgttt tgttttttaa gttaagtgtg tttgttttta aatgttttta aagtatttat   8340
attaattggt tgtaaagaat gtatatatat ggtaaaatat agaattgaat tgagtagtat   8400
tttaattttt ttaaataatt atttattata aattaattta ttggttaatt ttataattta   8460
gtttatttaa aatatatgtt tttgtgttgt ttatttttaa atttttttatt aaagattttg   8520
ttatggggta ataaagtgta tgaaaagggg ggaaatgtga aaggatttgg gattatttga   8580
attgtatttt ttttgtattt ttagttttgt ggtagttatt agaaattatt ttttagtaaa   8640
ttgttttatt ttttagggtt tgtttgtttg ttttgttatg gtttttttgtt ttttgttagt   8700
tgtgtagtgt tttttgtgtg tttataatat aaaatttaag ttggttaaaa taagagtttt   8760
tggtatatat attttaatta gaatatgaat tttggggggtg agaattattt tttattagga   8820
aaagtttttt attttaattt gtgagattag ttattgaagt tagttttgaa gtttggtagt   8880
taaatttttt atagaagatt tgttttgata gggtaagttt aaggattagt aggtgggaat   8940
tggaggtttt tttttaaaaa attatttttt ttagttattt agatttagtt ttttttagtag   9000
gtttggttat taaatgaagt ataaaaatgt aagttttaag gtttattttg attgtaaaat   9060
aaatttttaa gttataagga tatgtaggag tgagttaagg aatatgtttt gatttttttttt   9120
ttagtttttta gagtggagtt ttatgagttt ttgaagattt gttttgtatt gttttgtttg   9180
gtttttagta ttgaagtatg gggaagtggg gggaagaatg tgtaataatt gattgatttt   9240
atattaagta atgtaatttt ttttttttgt atattttatt ttttaaaaaa aataaataaa   9300
taaaaattat ttgtagttat tatttgtagt gttttggtta ttagttaata atgtagttag   9360
tttagatata taaaaaaaaaa agattattga aatgatgatg atatgtaaat tttttttgaa   9420
attattataa gtaaatattt gaagtttgga ttaataaaat tttatttgtg ttattttata   9480
ttgagttagt agaaagttgt gataatgaat tttgtaatat tttatgaata gatattttaa   9540
ttagggatta attttgtgat tttattgtag aattattaaa tttggagttg ttaaattgtt   9600
atttttgggt ttatgggttt ataaggattg aattggtaga gttttttgttt gtgtttttgt   9660
tagtgggtgg gggaattgtt tggttgtttt tattttggat ttttatgtta tagtgttggg   9720
tagttttttt tgtaggtagt gattttggtt agaggttttt tagggtttag ttttttttag   9780
gagaggttga gatgtaggga aatggtattt aggttagagg taggtttgta gtttttttgtt   9840
ttgtttttgt gtttttgtta atttgataat gtttgttttt attttgattt ttgtatttgt   9900
gtgaagtggg tttttttggtt gttggtgtat tttggttagt gtggagagag gtaggtgttg   9960
agattgaagg ggtttaggga gttttggatt tttttttttt gtttttaaag taattgtggt   10020
tttttttattt atttggtgga gtttttttgag atttattttt tttggtttgt ttgtggtaga   10080
gaagggggag tgtgttaaat gtttggtttg ttgtgttgtg gttgaaaatg tgaaaaagat   10140
```

ttggtttgtt tgggagagaa agggggagaa ttgggtagta gttatattag agttattttt 10200
ttgtttttgg tgggtagtaa attttttaag aatgtttgtt ttgttttttt tagttttgtt 10260
tagtttattt agtgtttttt ttttgtgatt ttaaattata ttttagggta attatttgta 10320
gtaagtaaat aaatggttgg gttagtattt ttaggagaaa gtgtggttaa atatggaaaa 10380
gtggtttttg atggatgaga ggtttgaatt tagtttgttt ttgaaatatt ttaggttaag 10440
agtttgtttg ttttagaatt atagaaaatt gagggaaatt gttgtttagg atagggggtat 10500
gttggtgttg atgttttata aatgtttatt gagttttaat taatggataa gtattgaagg 10560
gtggttttttg tatatagttt tttaaagaga aaagtttttt ttatttattt atttttgttg 10620
ttattgtgtt tagatgagtt tttaattttg gtattgagat ttttgaaagt aggtttatag 10680
tttttttagt atattgtggt tttatagttt tttaattttt gggtattttt gtggtaattt 10740
tggagggaga tttttttttg ataaataaat gttttgggtt tgaggttagg ttggagatgt 10800
tgttgtatag ttagaggttg ttaggttgga aaaatatgtt tgaagtttag tatatagtag 10860
gtgtttaata gttagtgtaa tgtagtttta tttgagtttt gtttatttga tggttgttgt 10920
tttttatagt tttttttttt ttttgttttg tagataatgg ggaatggaga ttaattgttg 10980
taaattggtg ttggtgtgtg tgtaattagg taagaatttt tttttttgt ttgggttatt 11040
ggatgggagg ttgtgttatg tgagggtggt aagagggtat tggtttttgtg gtgaggtttt 11100
agtgaggggt gttttttttga ggggttagtt tgggtaggaa ggaaattaga attaaattgt 11160
tagtggtttt tttttgtggt ggggtggtgg attaggaagt agtggtgtgt tgtgtattga 11220
agtttttttag tttatttttt ttggttggaa ttgttggtaa ttggggaggt gtagaaagag 11280
tatgttattt tgttttgggt tgttagaggg tttgggggat ggggatgttg ttagtttttt 11340
tttttaattg ggtttttgtt ttttgttttt tttttttttt tggtttgttt tgtttttttt 11400
ttttttttttg tttttggttt tttttggttg tggtttggga tgtttttttt tgtatgtggg 11460
gtgggtgtgt gtgtggttta ggtgtgtagt tggtggttgt tgaatgtttt ttttttaaag 11520
attttgaaat taaaaaggtt gagtttatgg atttttttga gagttgaaaa gaggtagtta 11580
gtagtaagtt tttttttgtgg tagtattttg gtgttaatgg taaggttggg agggaagtgt 11640
aggttgtgtg ttgggtattt gtttttggga ttttgggttt tgggtgaagt gtaagaaggt 11700
gaggttgtta gatttgatgt gtttgttgtt tgaatttaga tattttgttt ttgggtggga 11760
tgggaagtag ttgttttagg gatgttaatt ttttttttta aattatattg tatttttgag 11820
atttaatatt ttttttttttt ttttgtttgt tttttgtggt ttgatttttt gtgtatgttt 11880
tagtaaattt tggtgtttag gttggtgtgg aaaagtggtt taatagtgat ttttgttgtt 11940
tgttatattt tgttgtgtgt agtattatta gggtttattt agttatttag gtttttagtt 12000
atgttttatt tagatttgtg ggtgtgtggt ttattgtggg aggtaagtaa gggaaatttg 12060
agttggtgaa ggtttgtttt ggttggttgg gggaggggtg gggggttgat aatattttttg 12120
aagagttgga gggtagttat tgtgtttagt agtttagggt agaatggagg tttgtttttg 12180
ttgatgtgaa tttgtttgaa gtattggttg ttaggttttg ggttttggtg atgttgttgt 12240
ttgattggtt ggttttattt tggaggaatt gagggatatt gttagaggag gtttataggt 12300
ttatgtaaaa agttaaaaag tttttaattt atgttatagg tttttttttga attgaaattt 12360
gtttttatggg gtggaggggg gggtgtaagg gatggaggag ggaagatgtt ttttttttta 12420
aatatatgga aaaaaatttt ttaaatttat tgttttttta ttttttttggt tttgtagtaa 12480
ataagtgttt agtttagga ggttattgat ttttgataat gtgagtagat aaagtttttt 12540
tttttttata gtttttggtt tttaattttt ttttttttgga ttaaagtgta agaatataaa 12600
tgtaatatgg gatggagggg ggtgatttgg gattttggtt aaaaaaataa attgtattat 12660
taagaagaaa ataaaggttt tgtattggag ttttttttgtg aatttgagag aaaatgatta 12720
tttgttgaaa tgaagtgttt aaagtgattt agtgttttat gtttggatat tgtattatat 12780
tgttagttgt tttgttgggt tagttaaatg tttatttgtt tgggattaat tttatggggt 12840
taaatggggg taatgtagag ataatgttgt gtgattttttg ttatttagat tgtgttaaat 12900
ttttttttttg tttgataatt ggtagtaaaa ataaattatt agattgtagt atgtttggga 12960
tatggttaaa aattaagagt agtgatgatt tttggggaga atgtttgtg tgggtttagt 13020
tttggttttg gttagattag aggagttttt taattttgtt ttgtgtgggg tgggtttgta 13080
gttgttaagt tgaggttgat atttttttatt gtgttgggag ttagagagat gtaaaatgtt 13140
tttttttttt agttttttatt ttaggttttt tagatatggg gaatgtattt tgaggatagg 13200
tggagaagtt tatggtagga tggggttttt gtaggtgagt aggaaatggt taagagtaga 13260
ggagttttgt ttgtgttagt tataagttgt gtaggtgttt ttggttgttt gtttttgata 13320
attagtatat aaagaattag aaataatgaa tgattgtttt tttaattatt atttttaggt 13380
ttgtattgtt ttagtgtatg tgaaaggttt ttttttttata tttaatatgt ttttttttat 13440
tttttgattg aaaagaaaaa ttgttgttta aatatgttta atgttattaa ttaagaaaag 13500
gtatgtaatg ggaagaaatg ttgaaaattt tgatttaatt ggtttttaag gaattagtag 13560
atgataaaaa aaaattatat gagtgggtaa agttatagta ttgttgaagg atagagtatt 13620
tatttttttt tgattttaag ttaatttatg gaatatttaa agttttggtt atagtttgtt 13680
tgtaaaataa aaggatttat tttttgtgtt tttttaaagt ttttttttgt ttttaaagag 13740
aaaaaaagtt tataatgata tatgattttt ttaaaaggtt gtgatagttt attatgttat 13800
tttttttgtt tttgttttta atgttgttta aaaatattat gtttttgtta aagattaaat 13860
gttttgtata ggtagagttt atttttaagt agtttaggtt ttgttttttt tttttagtga 13920

gttttatttt ttttggtatt tattgggtgg atgtttagtt tggatagaat tttgaaatgg 13980
gggtagtatg agagtgattg gagattttta aaagttagag gtttgagaga gggtggatgt 14040
agttagtaga agatggtgta gaagttagtt gagaatgatt ttttagagta aagagatttt 14100
tttttggttt tttttgtttt gggggttttg aaaggaattt ataaaatggt ttttattttt 14160
aggaggagga tggattgatt tttttttgtt attggtttaa aaagttttag ggtggtggtt 14220
ttgggttttg tgttgaaatt ggattgtatt gtagtttttt tggatttgat gtttggtttt 14280
gtgttttgat aaggggtggg tatttttttt ggtttttttt aggaatgtat taattgttaa 14340
atagttttgg tttagtggat gggttgaaag tgtttgattt aagttgttgg tgtgtataga 14400
tttttttttt ttgggaggtg ggttttatgg tttgttgtgg tatttttagt tgtgatatat 14460
atttttatat gtggtagtag tttggtttta atttttttttg aaggatttgg gttaattttg 14520
gtggttttgg tggttgtaga tttttttttg ttgttttgtt tttgtgtttt ttatttaatt 14580
agtgaatgtt tgtggagtat atattatgtg gatttttaat gtatttttttg aaagtaaata 14640
atatagtttt ttttgttgtt atgaagggat tttaatttta atatggatat tagtgagatt 14700
agtttagatt gtttttagta aaatgtaaaa tggtggtgtg tggggtggtg attaaggttt 14760
tgagttttgt tagaaagaag gggatgtgta gagaaaggtg gagaatttta gttgtggtta 14820
gtgtggaagg gataggtgtt tgttgaaggg ggtatgaggt ttgaggaaaa agtaatgaaa 14880
taggggtaag gagagttttt tatttttttt tttttgtttg atttttgtta ttttattttt 14940
tttttttttt ttttattttt tgtgttaatt aaatttgtag tttatttgaa aggtgttttg 15000
ttgtgttgtg gtttttttatt tttaggggaa attgtattag ttgtttgaaa gtagttagtt 15060
tttgtggatt tttgtttgta aaagtggttt ttataggttg tgttttttgt tgttgatttg 15120
gtatataaag tttttttaagg ttggtttggt tgttattttt tattgtttgt tgttaatata 15180
tgtagtagtt gttagagtgg tttgggggaa aaggaaatgt ataatgaaag tttatttgtg 15240
agtaggaata tattaatgga ataatttgat gtttttttaa ttttatgtaa aaagttttgt 15300
tgttttttta atattgattg aatgggtaat taatggtttt ttatttaggt gaatattttg 15360
taatttaaga taggtaaaag ataataagtt taaggtagaa gataaaaggt ttaattgtag 15420
tggtgtttgt ttgtttttta tttttagggg tttttgatta ggaaagtttt tttttagagg 15480
agaaaaaggt aggagtggga gaatatatat ttattatttt ggggttagat tttattgtag 15540
tatttgatta tttagtttag tttttttgtta ttttgttttt ttatttttag tttttttttt 15600
tgtatttttt tttttttttta atttttttag gatgattttt tattattatt gttattatgg 15660
ttttaataat tttttttttt aaatttttata tttttttattt tagtaattaa tgaggttgtt 15720
ttttgattta ggaggagatt tttttttttt agaatttaat gtgtagagtt tttgagaatt 15780
aaagtagttg gtaggggagg aagaaattaa tagaaaggga gagagtatat agaattgtgt 15840
gtgtatgtta aagagtgatt aggaatgata gagttaattt ttttgtgagg atttgatggg 15900
aagagtgttt aagattttat tagtatgttt ttaataggtt gatattttaa tttaaatttt 15960
tagaagtaat atattatttg ggttattata atgaggtggg tttttttttt tttgttagtt 16020
gatagttttt aaaatattat tttgttaggg aaataaaagt tttattttag attataggtg 16080
ggtatttttg gatttaggtg atttatggtt attatgataa ttaatgttga atgttagtta 16140
ttagtatgtt tgggagagag aaaatagaaa gaagggagag taaaagaaat agaaaaggga 16200
gatggatata agttggagag ggaagaaaag agaaaaagag gaagatagat gagtgtttaa 16260
tttaattgtt gtttaaaaaa gtggtggggg ggtgggattt tatttagttt tttgttattt 16320
tttttttttt tgatttggat atttatgttt aattttatat tttatttttt tttttttttt 16380
tttaaatata tgtgttatat tattttttttt attttattta gtttggtaag tagttgtttt 16440
ttggagattt agtgatattt aggaaaattg tggtagtaat atgtaaatgt gaggaagtat 16500
taatagtatg tttgttgagt gattttagta aatgtttttt tttttaattt tttttttttt 16560
tttttttttag gttattgtga ggtggtaatt tttattgtta tttgaatatt gtttttttag 16620
gtagttattt taaattttaa atggttgagt agttagagtt gtgggttgga aaaatgggta 16680
ttatttgtag ggatttagag agggtggttg ttgtttaata tatttataga tttttaattt 16740
agaaaataat tttttttttt tgataagtta gagtttttta aattttattt aggaaatggg 16800
gaaaaggata gttatagtga agtttttaat ttttgggtta tttggtttta tagttatgag 16860
gggtggtggg tagtggattg tttttagttt ggtttgtatg tagagaaaag ttagatattg 16920
gagggggtgg ggtatttttt gggtaggatg taaggttttt atttgatttt tgtgttttat 16980
taggagttta tatatttatg tttattatgt ggttttaagt tgagtttagg tgggttttgt 17040
ttttgagtta gtttgggtag ggtaggattt ttatttgttt aaggtttaat agtttaggga 17100
gatgtttaat taagttattt tttgggtgaa ttttgaagat agattttttt taaaagttag 17160
agattatttg gttgagtttt aggttagatt gatatggaga gtttggtggt atagtttaat 17220
tgtttattgt tatggttaga gggatttttgt ataattaata ttgaagagtg tgaaattaaa 17280
taagatttta agattggtaa ttggtggtaa atattagtat aaaatatggt tgattttatg 17340
ttatatattt tttttttttta gggttttttt ttgaaagaat aagtaagaaa ttttaattga 17400
gataattttt gatgtttttt agatttaaaa ttttatgttg gtattgggtt tttttttttt 17460
gttttatgtg agttatgtag tatttttagt tttttttatta ggattttatt aatgtttttt 17520
gtattggaaa tttttgtgtt agaggttgaa tttatagtaa tttttaaaat taattaagaa 17580

gaatttagtt agaggttata gtaatgttgg aattataaaa tgtataagat ttattttttt 17640

ttggtttttt ttttatttat gttgtttatg tttgtgtatt tataagtttt atgtatatta 17700
aatttttaaa attaattatt attatgttat agagttttta ttggatagtg ttttttagtt 17760
tttattatat attttttttt ttttatgtag atttattatg ttggtgtttt gttatatagg 17820
gggtttgaga agaatgttat ttaattgttg ttgttgtgag tgtgtaaagt gattaggaga 17880
ttaggagaat gttgaaattt ttgttggaaa aatgtaaaga aaatttttat tttgagttag 17940
ttgtttatag agttagtgtg tgtgtgtgtg tgtgtgtttg taatataaaa tggatgtgaa 18000
tatatatata taaatagata tggttttgtt tttattttaa tttgaattat ttagataatt 18060
gttttttattt attatttgat tttaatgggt ttatataaat taggatattt tatttttta 18120
ggtatttagg ttgttgttga tttttagtgt ttttaatatt ttgtatatgt tggtattatg 18180
aggagtagtt atgtgttttt gggtttttta attattttgg aggttgattg aggtttttta 18240
tatatgtata tttgttgtga tgaaagtttt attggtagag tggagttatt agagttttta 18300
ttaaaatttt gtgggtttat gagagatggg tttagaaatt tatatggttt tgtggggttt 18360
tttggttttt taaaataagg tattaatatt taagttttta aaaatatttg tagttttggg 18420
gtttgaattt tgaaaaataa ggagtgaggg gttgtgtata ttaattatag tggagatttt 18480
ttttattttt taatgtgatg gagttttttt atgaaatgaa gttttaaggg gtatggtatt 18540
gtggggatta tagttatttt gaggtttaaa agaagaaatt ggaatatgat tagtaaatat 18600
atttagtaga aaagagttgg atttttattg atttagttat aggttattgg ttggtagtgt 18660
aatgggagga aatatttatt ttatatatat attttatgat tttgggggaa ttagaggaaa 18720
tttaataaga aaatggttag aaatatttaa aatttttatt taaaagattt aagtaaatta 18780
gagttttatt agattaaaaa ttattataaa tgtaagagta ttgtttttag tgaaatgttg 18840
tggggtttga gaaggagatt ttttgttaaa tttttgggat aaaatgtgtt atttaagtat 18900
tagataatga gtagaatgta aattaattta atttttttta ttaataggtt gttagtgtaa 18960
tgtgtataat ttagtgataa gattgtagga tttaatatag ttggatgtat gagttttagt 19020
taatgtagat ttgttatatg aggatgtgtt ttattttgag taggtgtttg tatgtgtgga 19080
atggggtaaa gtggaataaa aggttaaaag tagaaatgtt gatttaaagt ttattatgaa 19140
gaaatttttt ttttgtagtt aaattatttt taaagtggga tgatattggt gaagaaagat 19200
tgaaaaataa tttttatgtg tgtttttgga ttgtaagttt aaaatgggga ggagttgtag 19260
atagggtttg ggggtggtta gggtaaagga gagatatata agttgtaaat atatttgtag 19320
tttgttttat ttattttgtt ttatattgaa taagtttttt aattttgtga ataaggataa 19380
ggagggagtg ttttaaagat attttatgtt ggtattgtaa attattgatt gtaatgttaa 19440
ataaatatat atttagagat gataatatta attttatagt aaaataattg tttatgtaga 19500
aatttagagg agattagttt gtttttttta gttgatttat gttgggggat aaaaggattt 19560
ttaaaaatta ttttgaatat gtttggattt tttttttaa ttttttgga aattaaattt 19620
gtttggaaat agtgttataa agagttgatg tttttaaagg tgattttttt tgttttatat 19680
aaataaggtt ttgtttttgt tagttgagtg tagttttagg tttttgtttt ttagtttata 19740
tatatttttt ttgtttgttt ggattttaat ggtttaagat agttttgagt ttattgggaa 19800
aagaaaatga ttgttaaaaa ttatttttga aattggttat ttggtaatat ttttaattgt 19860
atggaaattt attaaggtat attttatata taattagttt aaggttgttg attttatagg 19920
ttttatggat ttaaatttga ttgataataa agtaaataag agagttgaat ttaaagtgtg 19980
gttttttttgg gttaggatga gtttaatata gtgtataagg aatttgaaag atttaggata 20040
tgtgttttaa ttaatgttaa gtagaatgga taagttttta gtattttgaa aatgttgggt 20100
tagggttttt tttttattgt gtgtttttttg tttggggatt aataagtatt atagagaatg 20160
tgatttgagg tgattttttta tttttgtata aatttagagt gaattattaa atagttgttt 20220
gtttaaagtt aaggtaattt ttttttgatg ggtttatttg ttttttgatt tttaatttat 20280
tagtttgttt ttttagggtt ttgttttttt tgtaattaaa gttttttttag attagtgtag 20340
tatttatttg ataggttgtt tggaaaattt aagattggag aggtgatttg ttgttgtttt 20400
ttaaattttt tagttttaag taatgtgttt tttttttata tggggtgggg gattggaaat 20460
ggatgtagtg agatataaag agtgggtgtt ttgttgattt ttgtattttt tttttttttga 20520
ttattttatt tttttttttt aagtttttga tttttagttt tattttttta tttttgggtt 20580
tgtattaaaa gttggattgt tttgggttgg gtaggagttg aatttttggg agtttgtttg 20640
tgtagattta gtgtgtatgg tgaggtagta gtttggtttt gtattgttga taggtgtagg 20700
taggatagtt tttttattgt ggtttggggt gttttgattg gtgtggagtt atgttagttg 20760
tatttggaga agggtttggg aggaggtgga ggtggagagg gttggggagg gttgtggtgg 20820
agtgatgttt tggtattagg aagtttgttt ttggttttaa gatgttaggt taatagggaa 20880
gtgtggagtt gtagatttgg tttgttgttt gtttgggtgt ttggagttga gttgtggtaa 20940
ggtttggttt ttgtttgatt gtttgagggg tgtgtgtgtg tgtgttgtgg agggtgtgtt 21000
tagagggttg tgttgtggtt gtagtggttg ttgttgttgt aggggattta atattattta 21060
tttgttttttg ttatttttga tattttttttg ttagggttgt tgtgtggggg gggggtgggt 21120
agagtgtggt tggtgttagt tttttttatt ggagggggttt ttgggggagg gagggagaga 21180
agaagggggt ttttgtttat ttttgttttg ttttggagtt tggaagtttg tttttttaaag 21240
atgttttgag tggtgttttt ttgtttatat tttatgtttt tgtttgtttg ttgatttttt 21300
gtttttggat tttttttgttt gagtttttttg gaggagatgg gggtagtttg gtttgagaat 21360
ttggtggggg ttgtgttttt tggttttttt tgtagtgggg aaattttgtg tttagagtgt 21420

```
gatttggagt gggtagtggt ggttatgggg gtttggtggg gtagtagtta aggattagta 21480
gagtgttgtg tttttttgtt tatgaattgt atgaaaggtt tgttttattt ggagtattga 21540
gtagtgggga ttaagttgtt ggttgttttt ttattttttt gttattattt ttagttgtta 21600
gttatggttt tggttttggt ttttggttag ttttggttgt tggatttttt taagtatagg 21660
ttggaggtgt atattatttt tgatattttt agtttggagg ttgtaggtaa ggtgttgtgt 21720
tgttttgtag atatttttgt ttagttgttt tgtgttattt gttttttttt gttttaagga 21780
agttagtttt tttgggggga ggtgtggtgg gagtggttgt ttgtttggtt ttttgtagaa 21840
tttttgggag ttggaatttt gattattttg tatttttttta gttttttttt gattggtttg 21900
gtttttgggg tgttaagggt gtgagtaatt ttgttgtttt ttttatttgt atttttggttt 21960
tttttttgtt ttttgggtta taaaaatttt agtattttga tttgaggatt tttagaggtt 22020
gttgattttt gtttttgttt tttttttggt ttttagtttt tgaggagttt tatttgttag 22080
gaaattgttt gaaattattt agaaatgttt tttgtgaaga ggtatttttt ttttttttt 22140
gggaaagggt tggtgaattt tggtgtttaa ttgaattttt atattttttt ttagtttttt 22200
taaattgtat ggaaatttga gtttttttgtg agggggaggg gggtttgtaa attatgtgtg 22260
tgtgtgtgtt ttaggagatt tggtgtgttt gtgtagaggt gtataaatat atttgaaagt 22320
ataggttata aaagtgaatg tgttgttgta gtgagataaa tatgtaaata aaatgtgtgg 22380
tgttgggggga ggggaggaaa tggggtgtgg atatttatat ttgtgtttgt atattttata 22440
ggtgtagtgt tttttgtggt ttggagttgt tgtgtgtatt tttttttggt gttaggtagt 22500
ttagtttttt tatggttttt gttgttggtt tagttggtgt ttgtgttgta ggtgggtatg 22560
ttgatgggaa agtgtgtgtg ttttgttttt agagaaagat aaaagttagt aggggaagaa 22620
tgaggatgtg ggtgttgagg atttgtttaa gaagaagtgg taaaggtggt agtggattta 22680
ttttattagt tagtagtttt aggagttgga ggttattttt tagaggaatt gttatttgga 22740
tatgtttata tgtgaagaaa ttgttgtgtg gattaatttt atggaagttt gagtttgggt 22800
aggagttagt atggagtttg ggagggatgg ggggaggatg ttgtggaggt ataggttaag 22860
tagattagga gagaatgtgg aaggtagtgt tgtttgggag ggtgttggtg gggtgtagtt 22920
ttgtaaaggt agaaggtttt gtggtggttt ggttgtgaga ttatagtttt tttttttgagg 22980
ttgataggat tgttgttttg gtttaggttt ttagagtggt attggtttat tgttttgtta 23040
ttttgtgatt ttatgagttg ggttgtatgg gtaatttttt gtataggata ttgtgttttt 23100
ggtttgtagt tgttagagta gagttaataa aatttttatt aggttaagag ttgtgaatag 23160
gttttaattt gtgagttttt aataaggaaa atttgttaga gatatggaag agttggtttt 23220
ttttgggaaa ttttttgtttt ggttttggtt tagttttttt ttttttgggt ttgtgttttt 23280
tatatttttt ttatggttgt tttggttatt taggtttttt ttatatattt tatttttttag 23340
ttttgtgatt tttgggagta aagttttaat atataattat tagttttttt agaaggagaa 23400
agaaaaaaag aagaaagatt tttttgtttg gtttatttat tttttttttag gagttgaatt 23460
ttggaaattg aaatttatat tttttttttt aaattataat tatagttttg taaaaagggt 23520
ttattttaat tttgtagtaa atttgtattt tatggattgg taaaaatgag tttaaataaa 23580
taatttaata gtaatgtttt ggtttatgtt ggttggtgga agattttaaa tttgttagga 23640
ttttggaagt agaaaataga attaagtaaa ttaagtggta tttagaggtt ttgttgttaa 23700
aaaaaaaaaa ttaagtgttt tgggtagaaa aaataaagtt tttggttaga gtagagtaaa 23760
taaaaagaag aaaataatga taaaaagaat aaagattaaa atgttttttt aaattagagg 23820
gaatgaagat atttttttggg tggtatttgt gtaaggtatg aggttatgtt ggtggataaa 23880
aggttgggaa gaagttgaaa atggtttttag tttaattgtt tagagttaga gttgggtttt 23940
gggtggtgtg gtttttgagta aggttagttt tttattagtt tttttgtata ttaagggaat 24000
gggtttttta tgtatttttt ttgtttgagt aaagtttaga tggtttaggg tagaaatggt 24060
aagtaattaa agatagagtt tatgggtttt ttgggatttt ttgaaaatgt tttttttattt 24120
tgtttgttat tttgtagttt tattttagtg ttttgtagtt gtggtgttgg gtttttttttg 24180
tagttgtttt ttttttttagg gtggttgttt gttgagttaa gtgggagtga ggtgtgtttt 24240
ttatagtagt tgggtgtaaa gaggaagggg gataaaaagg aaattaagaa tgaaaggaaa 24300
aagagaaaaa gtggattata tggttgggtt tggtggagat gtgtaatgtg aaatattatt 24360
ggtgttagtt tggatatttt aggttaggtt tttttttaat atataaaagt tgttgtttgg 24420
ggtgataggg aggtttgatg tggattggga ttggggttgt ggttgggtta ttggatatgg 24480
gtggaagttg gttggtttgg gtggttgttt gtaaagttaa atgatttggt tgggtttggt 24540
gtgtggatag gtttgtggtg ggtttagggt aaagaagagg tagagtgaaa gaagggggaa 24600
tttttaaaat tattttttttt gggtttttgg agtttaatat gttaagtttt tggagttaat 24660
gagttgatga agaggtggtt ttttgttttt tatttggttg ttttgttagg tgagaaagag 24720
tgttggtggt ttagtttttg ttaagggagt atgtattagg gggtggggga tgatagtgga 24780
ggttagggaa ggaagggagg aattgtgtgg gagaaagagt gattttttag tgtttttttta 24840
gttttttttt tttatttgtg ggtttgtggt tttggaatgg aagtaagttt gtaaggtgtt 24900
ttgggaaggg ttggaaaagt ttgttgtttt gtgtttgttt tatattaagt gtttttggat 24960
ttggagaaat gtttggttga gtgattaaat tgtttgtagg tttttatgtg tttggttgag 25020
gtttgtggtg tagttttgag ttttagtttg taggttagag tagattaggt tttttgtgtt 25080
tggtggagat ttgggttagt aattgaaagt tggttttggt attttggtgt gtagggtggt 25140
gtagtgaagt gaggttaggg tgtgtgagtg tgttagtgtg tgtgttgggg gaaggtgggg 25200
```

```
gttggttttt gatggaagtt ttagtaattt gtattgtggt atttgtttgt tttttgttt   25260
taattgtttt taggtttggt ttaagaattg ttgggttaaa tggagaaaga gggagtgtaa   25320
ttagtaggtt gagttatgta agaatggttt tgggttgtag tttaatgggt ttatgtagtt   25380
ttatgatgat atgtatttag gttattttta taataattgg gttgttaagg gttttatatt   25440
tgtttttttta tttattaaga gtttttttt ttttaatttt atgaatgtta atttttttgtt   25500
attatagagt atgttttttt tatttaattt tattttgttt atgagtatgt tgtttagtat   25560
ggtgttttta gtagtgatag gtgttttggg ttttagtttt aatagtttga ataatttgaa   25620
taatttgagt agtttgttgt tgaattttgt ggtgttgatg tttgtttgtt tttatgtgtt   25680
gttgattttt ttgtatgttt atagggatat gtgtaatttg agtttggtta gtttgagatt   25740
gaaagtaaag tagtatttta gttttggtta tgttagtgtg tagaatttgg tttttaattt   25800
gagtgtttgt tagtatgtag tggattggtt tgtgtgagtt gtatttatag tgttgggatt   25860
ttaggatttt gttggatggg gtaattttgt ttttgaaaga ttgggaatta tgttagaagg   25920
ttgtgggtat taaagaaagg gagagaaaga gaagttatat agagaaaagg aaattattga   25980
attaaagaga gagttttttt gattttaaag ggatgttttt agtgtttgat attttttatt   26040
ataagtattt ttaatagttg taaggatata tatataaata aatgtttgat tggatatgat   26100
attttaatat tattataagt ttgttatttt ttaagtttag tattgttaat atttaaatga   26160
ttgaaaggat gtatatatat tgaaatgtta aattaatttt ataaaagtag ttgttagtaa   26220
tattataata gtgtttttaa aggttaggtt ttaaaataaa gtatgttata tagaagtgat   26280
taggattttt tgtttgtgag taagggagtg tatatattaa atgttatatt gtatgttttt   26340
aatatattat tattattata aaaaatgtgt gaatattagt tttagaatag ttttttttggt   26400
ggatgtaatg atgtttttga aattgttatg tataatttat tttgtgtata atattttgta   26460
taatattatt gttttatttt ttagtaaata tgaaataaat gtgttttatt ttatgggagt   26520
aaaatatatt gtatataaat tggtttggat ttttttttt tttttttgtt attaatttgg   26580
ttaggatatt ttagttattg tttttaaat aaattagttt tttttgtttg tttagttaaa   26640
tatataaggt agtagttttt atttaaattt ggtagaaata aatgatagtt atttattaga   26700
aattaaaaag aaaaaaaaag gtatttttgg gggggaaaag ggttataaaa tttaattttg   26760
tttttttaat ttttttttgg tttaaattta gaggatttta ttatggttag taaataatat   26820
gaaaaagaaa aaagaagaaa gaaatttagt aagtttatta gtttaaaatg atttttaagt   26880
ttatttttttt atggggaaat ttatattttt agtaaattgt tttggagaaa tatttgtgta   26940
tgtatatatg tatagtttat atgtattttt tttaggagga atatatttat aataaattta   27000
tagggaaata tttttagttt aaaatattta ggtttttatg tttattttta ggtttaagta   27060
gagagatttt ttatgttata ttgtattatt atttttaaat tttttggaga tattaaaaga   27120
aataaagatg atttttaata attatagttt tttagttttt taaagaattt aggggttgag   27180
aggttagagt ggagtttttt gagttttgtt gagtaatatg tagttgaggt aaaggttatg   27240
tttttggtgt tttgttttaa ataatattga tttattaatt ttaaatttgt ttgtttttga   27300
aattatatag gattatagtt tgtaaattgt aggataatga agtaaattaa gatgaattat   27360
agttttggtt ttttttgtta ttttttgata tttaaatagg gaatgagttt ggtgtgagtg   27420
tttaaatgaa ttttaagtat ttgatttttt tttatttgtg atttttagtt ttaaaaaaat   27480
gtgaaatttg attttataat aaatagaaat aaatattatt tagttttaga gaatttattt   27540
ttatggtgtt aggagggttg ttgtggaggt gggggggaggg atgtgttgag atttttttgtt   27600
atgtttgtta atttttttgta taattaaagt gggtgagaat aaatattatg ttggggaatt   27660
tagagtaaaa agtaattgtt gatttttttgg agttgataat attattgttt ttttgtttta   27720
gttgttttta tttgaatgaa attttattta gaagtttttg gagtttgaat attgagtttt   27780
tgttttgtaa gaaattgttg ttgttattta aagagattgt agataatgtt tttgatttaa   27840
gattagtgtt taatgtatat ttttttttt tttaaagttt tgtttttgat ttgtggaggg   27900
attatgtagt agtgggggt agataaaagt tttttggatg agggttattt tttattatgt   27960
tgtttatttg agagtagtgg agaggaaaat ggtttttatg ggtggatttt ggtttttgga   28020
gttgtagggt ttagtggttt tggtttttt gtttttagt ttggtagggg gtggggaggg   28080
ttaaagggtg gaggggaagg aaggagttag aagaggggat ttggggatgg gggttggaag   28140
tgttaatgag atttgtttgg aggatttagg tttttgtag gttggtgagt gatttgggag   28200
ttttgggtaa aagaggtgta ttttggttta gtttggttgt tgaattagaa taatgtgagg   28260
atattaatta atttgtagga aataaaaaat ggaagttgag gtttaggaag agttgttatt   28320
tttttgattt gagtggtgta ggttgggggt ggagatttgg gatttaagag aggttatttt   28380
ttttatttta gttttttttt tttggatttt taaaaggaat aattttattt ttttttttgt   28440
ttttttata atttttattt ttgttagttt gtaggttgtt tgtttttttt tgtattttttt   28500
ttttttattt agtgagagaa atttagtttt tagagt                             28536
```